# EUROPÄISCHE PATENTSCHRIFT

(11) **EP 2 081 954 B1**
(45) Veröffentlichungstag und Bekanntmachung des Hinweises auf die Patenterteilung: **13.06.2012**
(21) Anmeldenummer: 07821799.9
(22) Anmeldetag: 24.10.2007
(51) Int. Cl.: C12N 15/82

(54) **VERFAHREN ZUR ERHÖHUNG DER RESISTENZ GEGEN BIOTROPHE PILZE IN PFLANZEN**
METHODS FOR INCREASING THE RESISTANCE OF PLANTS TO FUNGI
PROCEDE D'AUGMENTATION DE LA RESISTANCE AUX CHAMPIGNONS DANS LES PLANTES

(30) Priorität: 24.10.2006 EP 06122870
(43) Veröffentlichungstag der Anmeldung: 29.07.2009
(73) Patentinhaber: BASF Plant Science GmbH, 67056 Ludwigshafen (DE)
(72) Erfinder: SCHULTHEISS, Holger, 67435 Neustadt (DE); FRANK, Markus, 67434 Neustadt (DE); HÖFLE, Caroline, 85395 Wolfersdorf (DE)
(74) Vertreter: Popp, Andreas
(86) Internationale Anmeldenummer: PCT/EP2007/061436
(87) Internationale Veröffentlichungsnummer: WO 2008/049865

(56) Entgegenhaltungen:
- WO-A-02/101079
- WO-A-2004/081217
- WO-A-2007/080143
- IMANI JAFARGHOLI ET AL: "Expression of barley BAX Inhibitor-1 in carrots confers resistance to Botrytis cinerea" MOLECULAR PLANT PATHOLOGY, Bd. 7, Nr. 4, Juli 2006 (2006-07), Seiten 279-284, XP002475154 ISSN: 1464-6722
- EICHMANN R ET AL: "THE BARLEY APOPTOSIS SUPPRESSOR HOMOLOGUE BAX INHIBITOR-1 COMPROMISES NONHOST PENETRATION RESISTANCE OF BARLEY TO THE INAPPROPRIATE PATHOGEN BLUMERIA GRAMINIS F. SP. TRITICI" MOLECULAR PLANT-MICROBE INTERACTIONS, APS PRESS, ST. PAUL, MN, US, Bd. 17, Nr. 5, Mai 2004 (2004-05), Seiten 484-490, XP009033393 ISSN: 0894-0282
- HUECKELHOVEN R ET AL: "Overexpression of barley BAX inhibitor 1 induces breakdown of mlo-mediated penetration resistance to Blumeria graminis" PROCEEDINGS OF THE NATIONAL ACADEMY OF SCIENCES OF USA, NATIONAL ACADEMY OF SCIENCE, WASHINGTON, DC, US, Bd. 100, Nr. 9, 29. April 2003 (2003-04-29), Seiten 5555-5560, XP002286823 ISSN: 0027-8424
- HUECKELHOVEN RALPH ET AL: "Differential expression of putative cell death regulator genes in near-isogenic, resistant and susceptible barley lines during interaction with the powdery mildew fungus" PLANT MOLECULAR BIOLOGY, Bd. 47, Nr. 6, Dezember 2001 (2001-12), Seiten 739-748, XP002475155 ISSN: 0167-4412
- SANCHEZ P ET AL: "AtBI-1, a plant homologue of bax inhibitor-1 suppresses bax-induced, cell death in yeast and is rapidly upregulated during wounding and pathogen challenge" PLANT JOURNAL, BLACKWELL SCIENTIFIC PUBLICATIONS, OXFORD, GB, Bd. 21, Nr. 4, 2000, Seiten 393-399, XP002968581 ISSN: 0960-7412
- ADENDORFF ET AL: "Scanning electron microscopy of direct host leaf penetration by urediospore-derived infection structures of Phakopsora apoda" MYCOLOGICAL RESEARCH, ELSEVIER,, GB, Bd. 104, Nr. 3, März 2000 (2000-03), Seiten 317-324, XP022454098 ISSN: 0953-7562
- CHAE H-J ET AL: "Evolutionarily conserved cytoprotection provided by Bax Inhibitor-1 homologs from animals, plants, and yeast" GENE: AN INTERNATIONAL JOURNAL ON GENES AND GENOMES, ELSEVIER, AMSTERDAM, NL, Bd. 323, 24. Dezember 2003 (2003-12-24), Seiten 101-113, XP004477034 ISSN: 0378-1119

### SEQUENZPROTOKOLL

<110> BASF Plant Science GmbH
<120> verfahren zur Erhöhung der Resistenz gegen Pilze in Pflanzen
<130> PF 58494
<150> EP 06122870.6
   <141> 2006-10-24
<160> 48
<170> PatentIn Ver. 2.1
<210> 1
   <211> 744
   <212> DNA
   <213> Hordeum vulgare
<220>
   <221> CDS
   <222> (1)..(741)
   <223> coding for BI1-protein
<400> 1
   atg gac gcc ttc tac tcg acc tcg tcg gcg gcg gcg agc ggc tgg ggc      48
   Met Asp Ala Phe Tyr Ser Thr Ser Ser Ala Ala Ala Ser Gly Trp Gly
    1               5                  10                  15
   cac gac tcc ctc aag aac ttc cgc cag atc tcc ccc gcc gtg cag tcc      96
   His Asp Ser Leu Lys Asn Phe Arg Gln Ile Ser Pro Ala Val Gln Ser
               20                  25                  30
   cac ctc aag ctc gtt tac ctg act cta tgc ttt gca ctg gcc tca tct     144
   His Leu Lys Leu Val Tyr Leu Thr Leu Cys Phe Ala Leu Ala Ser Ser
           35                  40                  45
   gcc gtg ggt gct tac cta cac att gcc ctg aac atc ggc ggg atg ctg     192
   Ala Val Gly Ala Tyr Leu His Ile Ala Leu Asn Ile Gly Gly Met Leu
       50                  55                  60
   aca atg ctc gct tgt gtc gga act atc gcc tgg atg ttc tcg gtg cca     240
   Thr Met Leu Ala Cys Val Gly Thr Ile Ala Trp Met Phe Ser Val Pro
   65                  70                  75                  80
   gtc tat gag gag agg aag agg ttt ggg ctg ctg atg ggt gca gcc ctc     288
   Val Tyr Glu Glu Arg Lys Arg Phe Gly Leu Leu Met Gly Ala Ala Leu
                   85                  90                  95
   ctg gaa ggg gct tcg gtt gga cct ctg att gag ctt gcc ata gac ttt     336
   Leu Glu Gly Ala Ser Val Gly Pro Leu Ile Glu Leu Ala Ile Asp Phe
               100                 105                 110
   gac cca agc atc ctc gtg aca ggg ttt gtc gga acc gcc atc gcc ttt     384
   Asp Pro Ser Ile Leu Val Thr Gly Phe Val Gly Thr Ala Ile Ala Phe
           115                 120                 125
   ggg tgc ttc tct ggc gcc gcc atc atc gcc aag cgc agg gag tac ctg     432
   Gly Cys Phe Ser Gly Ala Ala Ile Ile Ala Lys Arg Arg Glu Tyr Leu
       130                 135                 140
   tac ctc ggt ggc ctg ctc tcg tct ggc ctg tcg atc ctg ctc tgg ctg     480
   Tyr Leu Gly Gly Leu Leu Ser Ser Gly Leu Ser Ile Leu Leu Trp Leu
   145                 150                 155                 160
   cag ttt gtc acg tcc atc ttt ggc cac tcc tct ggc agc ttc atg ttt     528
   Gln Phe Val Thr Ser Ile Phe Gly His Ser Ser Gly Ser Phe Met Phe
                   165                 170                 175
   gag gtt tac ttt ggc ctg ttg atc ttc ctg ggg tac atg gtg tac gac     576
   Glu Val Tyr Phe Gly Leu Leu Ile Phe Leu Gly Tyr Met Val Tyr Asp
               180                 185                 190
   acg cag gag atc atc gag agg gcg cac cat ggc gac atg gac tac atc     624
   Thr Gln Glu Ile Ile Glu Arg Ala His His Gly Asp Met Asp Tyr Ile
           195                 200                 205
   aag cac gcc ctc acc ctc ttc acc gac ttt gtt gcc gtc ctc gtc cga     672
   Lys His Ala Leu Thr Leu Phe Thr Asp Phe Val Ala Val Leu Val Arg
       210                 215                 220
   gtc ctc atc atc atg ctc aag aac gca ggc gac aag tcg gag gac aag     720
   Val Leu Ile Ile Met Leu Lys Asn Ala Gly Asp Lys Ser Glu Asp Lys
   225                 230                 235                 240
   aag aag agg aag agg ggg tcc tga                                     744
   Lys Lys Arg Lys Arg Gly Ser
                   245
<210> 2
   <211> 247
   <212> PRT
   <213> Hordeum vulgare
<400> 2
   Met Asp Ala Phe Tyr Ser Thr Ser Ser Ala Ala Ala Ser Gly Trp Gly
   1               5                   10                  15
   His Asp Ser Leu Lys Asn Phe Arg Gln Ile Ser Pro Ala Val Gln Ser
               20                  25                  30
   His Leu Lys Leu Val Tyr Leu Thr Leu Cys Phe Ala Leu Ala Ser Ser
           35                  40                  45
   Ala Val Gly Ala Tyr Leu His Ile Ala Leu Asn Ile Gly Gly Met Leu
       50                  55                  60
   Thr Met Leu Ala Cys Val Gly Thr Ile Ala Trp Met Phe Ser Val Pro
   65                  70                  75                  80
   Val Tyr Glu Glu Arg Lys Arg Phe Gly Leu Leu Met Gly Ala Ala Leu
                   85                  90                  95
   Leu Glu Gly Ala Ser Val Gly Pro Leu Ile Glu Leu Ala Ile Asp Phe
               100                 105                 110
   Asp Pro Ser Ile Leu Val Thr Gly Phe Val Gly Thr Ala Ile Ala Phe
           115                 120                 125
   Gly Cys Phe Ser Gly Ala Ala Ile Ile Ala Lys Arg Arg Glu Tyr Leu
       130                 135                 140
   Tyr Leu Gly Gly Leu Leu Ser Ser Gly Leu Ser Ile Leu Leu Trp Leu
   145                 150                 155                 160
   Gln Phe Val Thr Ser Ile Phe Gly His Ser Ser Gly Ser Phe Met Phe
                   165                 170                 175
   Glu Val Tyr Phe Gly Leu Leu Ile Phe Leu Gly Tyr Met Val Tyr Asp
               180                 185                 190
   Thr Gln Glu Ile Ile Glu Arg Ala His His Gly Asp Met Asp Tyr Ile
           195                 200                 205
   Lys His Ala Leu Thr Leu Phe Thr Asp Phe Val Ala Val Leu Val Arg
       210                 215                 220
   Val Leu Ile Ile Met Leu Lys Asn Ala Gly Asp Lys Ser Glu Asp Lys
   225                 230                 235                 240
   Lys Lys Arg Lys Arg Gly Ser
                   245
<210> 3
   <211> 1067
   <212> DNA
   <213> Arabidopsis thaliana
<220>
   <221> CDS
   <222> (1)..(741)
   <223> coding for BI1-protein
<400> 3
   atg gat gcg ttc tct tcc ttc ttc gat tct caa cct ggt agc aga agc      48
   Met Asp Ala Phe Ser Ser Phe Phe Asp Ser Gln Pro Gly Ser Arg Ser
    1               5                  10                  15
   tgg agc tat gat tct ctt aaa aac ttc cgt cag att tct cca gcc gtt      96
   Trp Ser Tyr Asp Ser Leu Lys Asn Phe Arg Gln Ile Ser Pro Ala Val
               20                  25                  30
   cag aat cat ctt aaa cgg gtt tat ttg acc tta tgt tgt gct ctt gtg     144
   Gln Asn His Leu Lys Arg Val Tyr Leu Thr Leu Cys Cys Ala Leu Val
           35                  40                  45
   gcg tct gcc ttt gga gct tac ctc cat gtg ctc tgg aat atc ggc ggt     192
   Ala Ser Ala Phe Gly Ala Tyr Leu His Val Leu Trp Asn Ile Gly Gly
       50                  55                  60
   att ctt aca acg att gga tgt att gga act atg att tgg ctc ctt tca     240
   Ile Leu Thr Thr Ile Gly Cys Ile Gly Thr Met Ile Trp Leu Leu Ser
   65                  70                  75                  80
   tgt cct cct tat gaa cac caa aaa agg ctt tct ctt ctg ttt gtg tct     288
   Cys Pro Pro Tyr Glu His Gln Lys Arg Leu Ser Leu Leu Phe Val Ser
                   85                  90                  95
   gct gtt ctt gaa ggt gct tct gtt ggc ccc ttg atc aaa gtg gca att     336
   Ala Val Leu Glu Gly Ala Ser Val Gly Pro Leu Ile Lys Val Ala Ile
               100                 105                 110
   gat gtt gac cca agc atc ctt atc act gca ttt gtt gga act gcg ata     384
   Asp Val Asp Pro Ser Ile Leu Ile Thr Ala Phe Val Gly Thr Ala Ile
           115                 120                 125
   gcg ttt gtc tgt ttc tca gca gca gca atg tta gca aga cgc agg gag     432
   Ala Phe Val Cys Phe Ser Ala Ala Ala Met Leu Ala Arg Arg Arg Glu
       130                 135                 140
   tat ctc tac ctt gga gga ctg ctt tca tct ggc ttg tct atg cta atg     480
   Tyr Leu Tyr Leu Gly Gly Leu Leu Ser Ser Gly Leu Ser Met Leu Met
   145                 150                 155                 160
   tgg ctc cag ttt gcc tct tca atc ttt ggt ggc tct gca tct atc ttt     528
   Trp Leu Gln Phe Ala Ser Ser Ile Phe Gly Gly Ser Ala Ser Ile Phe
                   165                 170                 175
   aag ttt gag ttg tac ttt gga ctt ttg atc ttt gtg gga tac atg gtg     576
   Lys Phe Glu Leu Tyr Phe Gly Leu Leu Ile Phe Val Gly Tyr Met Val
               180                 185                 190
   gtg gac aca caa gag att ata gaa aag gca cac ctc ggt gac atg gac     624
   Val Asp Thr Gln Glu Ile Ile Glu Lys Ala His Leu Gly Asp Met Asp
           195                 200                 205
   tat gta aaa cat tcg ttg acc ctt ttc act gac ttt gta gct gtg ttt     672
   Tyr Val Lys His Ser Leu Thr Leu Phe Thr Asp Phe Val Ala Val Phe
       210                 215                 220
   gtt cgg att ctc atc ata atg ttg aag aac tca gca gat aaa gaa gag     720
   Val Arg Ile Leu Ile Ile Met Leu Lys Asn Ser Ala Asp Lys Glu Glu
   225                 230                 235                 240
   aag aag aag aaa agg aga aac tgaggggatg taaagtaaat ttaactttat        771
   Lys Lys Lys Lys Arg Arg Asn
                   245
   ggttgttatc gtgtgtggcc actttgaaga tattacttgt tagcactctc tattggtgac 831
   cagacatgtt tccactaaaa aggatctgct tgtttcactt ctgcacaagt accatcttca 891
   gattgtaaat gactcgagtg ttgttcttct tttcataaac ttttgttctt taagagtttg 951
   gttctactga ttgcatctta ccaagctaag aataatgtag gaaaatgata atcctgttta 1011
   aattttctaa aatgtgtgca tttcagaaaa aaaaaaaaaa aaaaaaaaaa aaaaaa      1067
<210> 4
   <211> 247
   <212> PRT
   <213> Arabidopsis thaliana
<400> 4
   Met Asp Ala Phe Ser Ser Phe Phe Asp Ser Gln Pro Gly Ser Arg Ser
   1               5                   10                  15
   Trp Ser Tyr Asp Ser Leu Lys Asn Phe Arg Gln Ile Ser Pro Ala Val
               20                  25                  30
   Gln Asn His Leu Lys Arg Val Tyr Leu Thr Leu Cys Cys Ala Leu Val
           35                  40                  45
   Ala Ser Ala Phe Gly Ala Tyr Leu His Val Leu Trp Asn Ile Gly Gly
       50                  55                  60
   Ile Leu Thr Thr Ile Gly Cys Ile Gly Thr Met Ile Trp Leu Leu Ser
   65                  70                  75                  80
   Cys Pro Pro Tyr Glu His Gln Lys Arg Leu Ser Leu Leu Phe Val Ser
                   85                  90                  95
   Ala Val Leu Glu Gly Ala Ser Val Gly Pro Leu Ile Lys Val Ala Ile
               100                 105                 110
   Asp Val Asp Pro Ser Ile Leu Ile Thr Ala Phe Val Gly Thr Ala Ile
           115                 120                 125
   Ala Phe Val Cys Phe Ser Ala Ala Ala Met Leu Ala Arg Arg Arg Glu
       130                 135                 140
   Tyr Leu Tyr Leu Gly Gly Leu Leu Ser Ser Gly Leu Ser Met Leu Met
   145                 150                 155                 160
   Trp Leu Gln Phe Ala Ser Ser Ile Phe Gly Gly Ser Ala Ser Ile Phe
                   165                 170                 175
   Lys Phe Glu Leu Tyr Phe Gly Leu Leu Ile Phe Val Gly Tyr Met Val
               180                 185                 190
   Val Asp Thr Gln Glu Ile Ile Glu Lys Ala His Leu Gly Asp Met Asp
           195                 200                 205
   Tyr Val Lys His Ser Leu Thr Leu Phe Thr Asp Phe Val Ala Val Phe
       210                 215                 220
   Val Arg Ile Leu Ile Ile Met Leu Lys Asn Ser Ala Asp Lys Glu Glu
   225                 230                 235                 240
   Lys Lys Lys Lys Arg Arg Asn
                   245
<210> 5
   <211> 1160
   <212> DNA
   <213> Nicotiana tabacum
<220>
   <221> CDS
   <222> (1)..(747)
   <223> coding for BI1-protein
<400> 5
   atg gag tct tgc aca tcg ttc ttc aat tca cag tcg gcg tcg tct cgc     48
   Met Glu Ser Cys Thr Ser Phe Phe Asn Ser Gln Ser Ala Ser Ser Arg
   1               5                   10                  15
   aat cgc tgg agt tac gat tct ctt aag aac ttc cgc cag atc tct ccc     96
   Asn Arg Trp Ser Tyr Asp Ser Leu Lys Asn Phe Arg Gln Ile Ser Pro
               20                  25                  30
   ttt gtt caa act cat ctc aaa aag gtc tac ctt tca tta tgt tgt gct     144
   Phe Val Gln Thr His Leu Lys Lys Val Tyr Leu Ser Leu Cys Cys Ala
           35                  40                  45
   tta gtt gct tcg gct gct gga gct tac ctt cac att ctt tgg aac att     192
   Leu Val Ala Ser Ala Ala Gly Ala Tyr Leu His Ile Leu Trp Asn Ile
       50                  55                  60
   ggt ggc tta ctt acg aca ttg gga tgt gtg gga agc ata gtg tgg ctg     240
   Gly Gly Leu Leu Thr Thr Leu Gly Cys Val Gly Ser Ile Val Trp Leu
   65                  70                  75                  80
   atg gcg aca cct ctg tat gaa gag caa aag agg ata gca ctt ctg atg     288
   Met Ala Thr Pro Leu Tyr Glu Glu Gln Lys Arg Ile Ala Leu Leu Met
                   85                  90                  95
   gca gct gca ctg ttt aaa gga gca tct att ggt cca ctg att gaa ttg     336
   Ala Ala Ala Leu Phe Lys Gly Ala Ser Ile Gly Pro Leu Ile Glu Leu
               100                 105                 110
   gct att gac ttt gac cca agc att gtg atc ggt gct ttt gtt ggt tgt     384
   Ala Ile Asp Phe Asp Pro Ser Ile Val Ile Gly Ala Phe Val Gly Cys
           115                 120                 125
   gct gtg gct ttt ggt tgc ttc tca gct gct gcc atg gtg gca agg cgc     432
   Ala Val Ala Phe Gly Cys Phe Ser Ala Ala Ala Met Val Ala Arg Arg
       130                 135                 140
   aga gag tac ttg tat ctt gga ggt ctt ctt tca tct ggt ctc tct atc     480
   Arg Glu Tyr Leu Tyr Leu Gly Gly Leu Leu Ser Ser Gly Leu Ser Ile
   145                 150                 155                 160
   ctt ttc tgg ttg cac ttc gcg tcc tcc att ttt ggt ggt tct atg gcc     528
   Leu Phe Trp Leu His Phe Ala Ser Ser Ile Phe Gly Gly Ser Met Ala
                   165                 170                 175
   ttg ttc aag ttc gag gtt tat ttt ggg ctc ttg gtg ttt gtg ggc tat     576
   Leu Phe Lys Phe Glu Val Tyr Phe Gly Leu Leu Val Phe Val Gly Tyr
               180                 185                 190
   atc att ttt gac acc caa gat ata att gag aag gca cac ctt ggg gat     624
   Ile Ile Phe Asp Thr Gln Asp Ile Ile Glu Lys Ala His Leu Gly Asp
           195                 200                 205
   ttg gac tac gtg aag cat gct ctg acc ctc ttt aca gat ttt gtt gct     672
   Leu Asp Tyr Val Lys His Ala Leu Thr Leu Phe Thr Asp Phe Val Ala
       210                 215                 220
   gtt ttt gtg cga ata tta atc ata atg ctg aag aat gca tcc gac aag     720
   Val Phe Val Arg Ile Leu Ile Ile Met Leu Lys Asn Ala Ser Asp Lys
   225                 230                 235                 240
   gaa gag aag aag aag aag agg aga aac taatgcataa gcggttattc          767
   Glu Glu Lys Lys Lys Lys Arg Arg Asn
                   245
   aaagactctg taactctaga atctggcatt ttcttgttca taaacttctg tagaccttcg 827
   acaagtatgt tgttaatagt ttggtaacgc ctcagattaa gctgcgaggc tctgttatgc 887
   cgcatgccaa tgtggttatg gtggtacata gatggttttg tttccgaagc ataccatcaa 947
   ataacatgca tgtttacact atatcgataa cctacgagtg tactacttat ttctgctccc 1007
   ttttgctgtg ttaggttgtt catgattgta tagttgattt tccgttatgt tagaccatct 1067
   tctttcttga cgtttaattt ctcatattga tgggagaaat gaaaattcac accgtcgccc 1127
   caacttgttt aagactgagg cgcaattgta gtt                              1160
<210> 6
   <211> 249
   <212> PRT
   <213> Nicotiana tabacum
<400> 6
   Met Glu Ser Cys Thr Ser Phe Phe Asn Ser Gln Ser Ala Ser Ser Arg
   1               5                   10                  15
   Asn Arg Trp Ser Tyr Asp Ser Leu Lys Asn Phe Arg Gln Ile Ser Pro
               20                  25                  30
   Phe Val Gln Thr His Leu Lys Lys Val Tyr Leu Ser Leu Cys Cys Ala
           35                  40                  45
   Leu Val Ala Ser Ala Ala Gly Ala Tyr Leu His Ile Leu Trp Asn Ile
       50                  55                  60
   Gly Gly Leu Leu Thr Thr Leu Gly Cys Val Gly Ser Ile Val Trp Leu
   65                  70                  75                  80
   Met Ala Thr Pro Leu Tyr Glu Glu Gln Lys Arg Ile Ala Leu Leu Met
                   85                  90                  95
   Ala Ala Ala Leu Phe Lys Gly Ala Ser Ile Gly Pro Leu Ile Glu Leu
               100                 105                 110
   Ala Ile Asp Phe Asp Pro Ser Ile Val Ile Gly Ala Phe Val Gly Cys
           115                 120                 125
   Ala Val Ala Phe Gly Cys Phe Ser Ala Ala Ala Met Val Ala Arg Arg
       130                 135                 140
   Arg Glu Tyr Leu Tyr Leu Gly Gly Leu Leu Ser Ser Gly Leu Ser Ile
   145                 150                 155                 160
   Leu Phe Trp Leu His Phe Ala Ser Ser Ile Phe Gly Gly Ser Met Ala
                   165                 170                 175
   Leu Phe Lys Phe Glu Val Tyr Phe Gly Leu Leu Val Phe Val Gly Tyr
               180                 185                 190
   Ile Ile Phe Asp Thr Gln Asp Ile Ile Glu Lys Ala His Leu Gly Asp
           195                 200                 205
   Leu Asp Tyr Val Lys His Ala Leu Thr Leu Phe Thr Asp Phe Val Ala
       210                 215                 220
   Val Phe Val Arg Ile Leu Ile Ile Met Leu Lys Asn Ala Ser Asp Lys
   225                 230                 235                 240
   Glu Glu Lys Lys Lys Lys Arg Arg Asn
                   245
<210> 7
   <211> 1056
   <212> DNA
   <213> Oryza sativa
<220>
   <221> CDS
   <222> (1)..(747)
   <223> coding for BI1-protein
<400> 7
   atg gac gcc ttc tac tcg acc tcg tcg gcg tac gga gcg gcg gcg agc      48
   Met Asp Ala Phe Tyr Ser Thr Ser Ser Ala Tyr Gly Ala Ala Ala Ser
    1               5                  10                  15
   ggc tgg ggc tac gac tcg ctg aag aac ttc cgc cag atc tcc ccc gcc      96
   Gly Trp Gly Tyr Asp Ser Leu Lys Asn Phe Arg Gln Ile Ser Pro Ala
               20                  25                  30
   gtc cag tcc cac ctc aag ctc gtt tac ctg aca cta tgc gtc gcc ctg     144
   Val Gln Ser His Leu Lys Leu Val Tyr Leu Thr Leu Cys Val Ala Leu
           35                  40                  45
   gct gcg tcg gcg gtg ggc gca tac ctg cac gtc gcc ttg aac atc ggc     192
   Ala Ala Ser Ala Val Gly Ala Tyr Leu His Val Ala Leu Asn Ile Gly
       50                  55                  60
   ggg atg ttg act atg ctc ggg tgc gtg ggg agc atc gcc tgg ttg ttc     240
   Gly Met Leu Thr Met Leu Gly Cys Val Gly Ser Ile Ala Trp Leu Phe
   65                  70                  75                  80
   tcg gtg cct gtc ttt gag gag agg aag agg ttt ggg att ctc ttg gcc     288
   Ser Val Pro Val Phe Glu Glu Arg Lys Arg Phe Gly Ile Leu Leu Ala
                   85                  90                  95
   gct gcc ctg ctg gaa ggg gct tca gtt ggg cct ctg atc aag ctt gct     336
   Ala Ala Leu Leu Glu Gly Ala Ser Val Gly Pro Leu Ile Lys Leu Ala
               100                 105                 110
   gta gac ttt gac tca agc att ctc gta aca gca ttt gtt gga act gcc     384
   Val Asp Phe Asp Ser Ser Ile Leu Val Thr Ala Phe Val Gly Thr Ala
           115                 120                 125
   att gca ttt ggg tgc ttc act tgc gct gcc atc gtt gcc aag cgt agg     432
   Ile Ala Phe Gly Cys Phe Thr Cys Ala Ala Ile Val Ala Lys Arg Arg
       130                 135                 140
   gag tac ctc tac ctt ggt ggt ttg ctc tct tct ggc ctc tcc atc ctg     480
   Glu Tyr Leu Tyr Leu Gly Gly Leu Leu Ser Ser Gly Leu Ser Ile Leu
   145                 150                 155                 160
   ctc tgg ctg cag ttt gcc gca tcc atc ttt ggc cac tcc acc ggc agc     528
   Leu Trp Leu Gln Phe Ala Ala Ser Ile Phe Gly His Ser Thr Gly Ser
                   165                 170                 175
   ttc atg ttt gag gtt tac ttt ggc ctg ttg atc ttc ctg ggg tac atg     576
   Phe Met Phe Glu Val Tyr Phe Gly Leu Leu Ile Phe Leu Gly Tyr Met
               180                 185                 190
   gtg tat gac acg cag gag atc atc gag agg gct cac cac ggt gac atg     624
   Val Tyr Asp Thr Gln Glu Ile Ile Glu Arg Ala His His Gly Asp Met
           195                 200                 205
   gac tac atc aag cac gca ctc acc ctc ttc act gac ttc gtg gcc gtc     672
   Asp Tyr Ile Lys His Ala Leu Thr Leu Phe Thr Asp Phe Val Ala Val
       210                 215                 220
   ctt gtc cgg atc ctc gtc atc atg ctc aag aac gcg tct gac aag tcg     720
   Leu Val Arg Ile Leu Val Ile Met Leu Lys Asn Ala Ser Asp Lys Ser
   225                 230                 235                 240
   gag gag aag aag agg aag aag agg tct tgagagcttc tcttcccgct           767
   Glu Glu Lys Lys Arg Lys Lys Arg Ser
                   245
   ttgcacataa gaaaaaacca ccgcggctat tgcctctacg tattatgaca gagccgcact 827
   tcaactgggt tttatggtga atacaagttc ttttgcattt tgttgatacg gtgtgaatct 887
   tctcaggttt gtcgtcgtag tagctttgca aatactagca tgctacatga cacggatctt 947
   tctgtaatgg tggtcgcgtt gatcgaaacg tgaaaacaca tcttcatttg cgactaattt 1007
   gtttgccttt tggtgattga tgatgatcct ttccccaaaa aaaaaaaaa            1056
<210> 8
   <211> 249
   <212> PRT
   <213> Oryza sativa
<400> 8
   Met Asp Ala Phe Tyr Ser Thr Ser Ser Ala Tyr Gly Ala Ala Ala Ser
   1               5                   10                  15
   Gly Trp Gly Tyr Asp Ser Leu Lys Asn Phe Arg Gln Ile Ser Pro Ala
               20                  25                  30
   Val Gln Ser His Leu Lys Leu Val Tyr Leu Thr Leu Cys Val Ala Leu
           35                  40                  45
   Ala Ala Ser Ala Val Gly Ala Tyr Leu His Val Ala Leu Asn Ile Gly
       50                  55                  60
   Gly Met Leu Thr Met Leu Gly Cys Val Gly Ser Ile Ala Trp Leu Phe
   65                  70                  75                  80
   Ser Val Pro Val Phe Glu Glu Arg Lys Arg Phe Gly Ile Leu Leu Ala
                   85                  90                  95
   Ala Ala Leu Leu Glu Gly Ala Ser Val Gly Pro Leu Ile Lys Leu Ala
               100                 105                 110
   Val Asp Phe Asp Ser Ser Ile Leu Val Thr Ala Phe Val Gly Thr Ala
           115                 120                 125
   Ile Ala Phe Gly Cys Phe Thr Cys Ala Ala Ile Val Ala Lys Arg Arg
       130                 135                 140
   Glu Tyr Leu Tyr Leu Gly Gly Leu Leu Ser Ser Gly Leu Ser Ile Leu
   145                 150                 155                 160
   Leu Trp Leu Gln Phe Ala Ala Ser Ile Phe Gly His Ser Thr Gly Ser
                   165                 170                 175
   Phe Met Phe Glu Val Tyr Phe Gly Leu Leu Ile Phe Leu Gly Tyr Met
               180                 185                 190
   Val Tyr Asp Thr Gln Glu Ile Ile Glu Arg Ala His His Gly Asp Met
           195                 200                 205
   Asp Tyr Ile Lys His Ala Leu Thr Leu Phe Thr Asp Phe Val Ala Val
       210                 215                 220
   Leu Val Arg Ile Leu Val Ile Met Leu Lys Asn Ala Ser Asp Lys Ser
   225                 230                 235                 240
   Glu Glu Lys Lys Arg Lys Lys Arg Ser
                   245
<210> 9
   <211> 973
   <212> DNA
   <213> Brassica napus
<220>
   <221> CDS
   <222> (1)..(741)
   <223> coding for BI1-protein
<400> 9
   atg gat tca ttc tcg tcc ttc ttc gat tct caa cct ggt agc aga agc      48
   Met Asp Ser Phe Ser Ser Phe Phe Asp Ser Gln Pro Gly Ser Arg Ser
   1               5                   10                  15
   tgg agc tat gat tct ctc aaa aac ctc cgt cag att tct ccc tcc gtc      96
   Trp Ser Tyr Asp Ser Leu Lys Asn Leu Arg Gln Ile Ser Pro Ser Val
               20                  25                  30
   cag aat cat ctc aag agg gtt tat ctc act ctg tgt tgt gct ctc gtt      144
   Gln Asn His Leu Lys Arg Val Tyr Leu Thr Leu Cys Cys Ala Leu Val
           35                  40                  45
   gcg tct gcg ttt gga gct tac ctc cac gtg ctc tgg aac ata ggt ggt      192
   Ala Ser Ala Phe Gly Ala Tyr Leu His Val Leu Trp Asn Ile Gly Gly
       50                  55                  60
   att ctc act acc att gga tgc ttt gga agc atg att tgg ctg ctc tcc      240
   Ile Leu Thr Thr Ile Gly Cys Phe Gly Ser Met Ile Trp Leu Leu Ser
   65                  70                  75                  80
   tgt cct cct tat gaa caa caa aag agg ctt tcc ctt ctg ttt ctg tct      288
   Cys Pro Pro Tyr Glu Gln Gln Lys Arg Leu Ser Leu Leu Phe Leu Ser
                   85                  90                  95
   gct gtt ctc gaa ggt gct tca gtt ggt ccc ttg atc aaa gtg gca gtt      336
   Ala Val Leu Glu Gly Ala Ser Val Gly Pro Leu Ile Lys Val Ala Val
               100                 105                 110
   gat ttt gac cca agc atc ctc atc act gcg ttt gtc gga act gcg ata      384
   Asp Phe Asp Pro Ser Ile Leu Ile Thr Ala Phe Val Gly Thr Ala Ile
           115                 120                 125
   gcc ttt atc tgt ttc tca ggg gca gcg atg ttg gca aga cgc aga gag      432
   Ala Phe Ile Cys Phe Ser Gly Ala Ala Met Leu Ala Arg Arg Arg Glu
       130                 135                 140
   tac ctc tac ctc gga gga ctg ctt tca tct ggc ttg tcc atg ctt atg      480
   Tyr Leu Tyr Leu Gly Gly Leu Leu Ser Ser Gly Leu Ser Met Leu Met
   145                 150                 155                 160
   tgg ctt cag ttt gcc tct tcc atc ttt ggt ggc tct gca tcc atc ttt      528
   Trp Leu Gln Phe Ala Ser Ser Ile Phe Gly Gly Ser Ala Ser Ile Phe
                   165                 170                 175
   aag ttt gag ctc tac ttt gga ctc ttg atc ttt gtg gga tac atg gtg      576
   Lys Phe Glu Leu Tyr Phe Gly Leu Leu Ile Phe Val Gly Tyr Met Val
               180                 185                 190
   gtg gac act caa gat att ata gag aag gcc cac ctc ggt gac atg gat      624
   Val Asp Thr Gln Asp Ile Ile Glu Lys Ala His Leu Gly Asp Met Asp
           195                 200                 205
   tac gtg aaa cat tcg ttg acc ctt ttc acc gat ttt gta gct gtg ttt      672
   Tyr Val Lys His Ser Leu Thr Leu Phe Thr Asp Phe Val Ala Val Phe
       210                 215                 220
   gtt cgt gtt ctc atc att atg ctg aag aac tcg gca gat aaa gaa gat      720
   Val Arg Val Leu Ile Ile Met Leu Lys Asn Ser Ala Asp Lys Glu Asp
   225                 230                 235                 240
   aaa aag aag agg agg agg aac tgagactaaa aagtgagaaa gaaagctaaa         771
   Lys Lys Lys Arg Arg Arg Asn
                   245
   tagagtgggt gttatgtgtg tttcaaaaaa taaaaaagag tgggtgttat aagtacagac 831
   atgatagcgt tggtgttttt tacttgtttg gaacagtttt ggtaacaaca cacgttacgt 891
   atttgtgtat tcctcttagt gactccagat tgtgaatgga tcagtatctt gaaactgtgt 951
   tgaaaattat cagttgggag ct                                             973
<210> 10
   <211> 247
   <212> PRT
   <213> Brassica napus
<400> 10
   Met Asp Ser Phe Ser Ser Phe Phe Asp Ser Gln Pro Gly Ser Arg Ser
   1               5                   10                  15
   Trp Ser Tyr Asp Ser Leu Lys Asn Leu Arg Gln Ile Ser Pro Ser Val
               20                  25                  30
   Gln Asn His Leu Lys Arg Val Tyr Leu Thr Leu Cys Cys Ala Leu Val
           35                  40                  45
   Ala Ser Ala Phe Gly Ala Tyr Leu His Val Leu Trp Asn Ile Gly Gly
       50                  55                  60
   Ile Leu Thr Thr Ile Gly Cys Phe Gly Ser Met Ile Trp Leu Leu Ser
   65                  70                  75                  80
   Cys Pro Pro Tyr Glu Gln Gln Lys Arg Leu Ser Leu Leu Phe Leu Ser
                   85                  90                  95
   Ala Val Leu Glu Gly Ala Ser Val Gly Pro Leu Ile Lys Val Ala Val
               100                 105                 110
   Asp Phe Asp Pro Ser Ile Leu Ile Thr Ala Phe Val Gly Thr Ala Ile
           115                 120                 125
   Ala Phe Ile Cys Phe Ser Gly Ala Ala Met Leu Ala Arg Arg Arg Glu
       130                 135                 140
   Tyr Leu Tyr Leu Gly Gly Leu Leu Ser Ser Gly Leu Ser Met Leu Met
   145                 150                 155                 160
   Trp Leu Gln Phe Ala Ser Ser Ile Phe Gly Gly Ser Ala Ser Ile Phe
                   165                 170                 175
   Lys Phe Glu Leu Tyr Phe Gly Leu Leu Ile Phe Val Gly Tyr Met Val
               180                 185                 190
   Val Asp Thr Gln Asp Ile Ile Glu Lys Ala His Leu Gly Asp Met Asp
           195                 200                 205
   Tyr Val Lys His Ser Leu Thr Leu Phe Thr Asp Phe Val Ala Val Phe
       210                 215                 220
   Val Arg Val Leu Ile Ile Met Leu Lys Asn Ser Ala Asp Lys Glu Asp
   225                 230                 235                 240
   Lys Lys Lys Arg Arg Arg Asn
                   245
<210> 11
   <211> 747
   <212> DNA
   <213> Glycine max
<220>
   <221> CDS
   <222> (1)..(744)
   <223> coding for BI1-protein
<400> 11
   cga ttg caa gca atg gac gcc ttc aat tcc ttc ttc gat tca aga aac      48
   Arg Leu Gln Ala Met Asp Ala Phe Asn Ser Phe Phe Asp Ser Arg Asn
   1               5                   10                  15
   cga tgg aat tac gat act ctc aaa aac ttc cgt cag att tct ccg gtc      96
   Arg Trp Asn Tyr Asp Thr Leu Lys Asn Phe Arg Gln Ile Ser Pro Val
               20                  25                  30
   gtg cag aat cac ctg aag cag gtt tat ttt act ctg tgt ttt gcc gtg      144
   Val Gln Asn His Leu Lys Gln Val Tyr Phe Thr Leu Cys Phe Ala Val
           35                  40                  45
   gtt gct gcg gct gtc ggg gct tac ctt cat gtc ctc ttg aac att ggg      192
   Val Ala Ala Ala Val Gly Ala Tyr Leu His Val Leu Leu Asn Ile Gly
       50                  55                  60
   ggt ttt ctt act aca gtg gca tgc atg gga agc agc ttt tgg tta ctc      240
   Gly Phe Leu Thr Thr Val Ala Cys Met Gly Ser Ser Phe Trp Leu Leu
   65                  70                  75                  80
   tcc aca cct cct ttt gaa gag agg aag agg gtg act ttg ttg atg gcc      288
   Ser Thr Pro Pro Phe Glu Glu Arg Lys Arg Val Thr Leu Leu Met Ala
                   85                  90                  95
   gca tca ctg ttt cag ggt tcc tct att gga ccc ttg att gat ttg gct      336
   Ala Ser Leu Phe Gln Gly Ser Ser Ile Gly Pro Leu Ile Asp Leu Ala
               100                 105                 110
   att cat atc gat cca agc ctt atc ttt agt gca ttt gtg gga aca gcc      384
   Ile His Ile Asp Pro Ser Leu Ile Phe Ser Ala Phe Val Gly Thr Ala
           115                 120                 125
   ttg gcc ttt gca tgc ttc tca gga gca gct ttg gtt gct agg cgt agg      432
   Leu Ala Phe Ala Cys Phe Ser Gly Ala Ala Leu Val Ala Arg Arg Arg
       130                 135                 140
   gag tac ctg tac ctt ggt ggc ttg gtt tct tct gga ttg tcc atc ctt      480
   Glu Tyr Leu Tyr Leu Gly Gly Leu Val Ser Ser Gly Leu Ser Ile Leu
   145                 150                 155                 160
   ctc tgg ttg cac ttt gct tct tcc atc ttt gga ggc tca aca gct ctc      528
   Leu Trp Leu His Phe Ala Ser Ser Ile Phe Gly Gly Ser Thr Ala Leu
                   165                 170                 175
   ttt aag ttt gag ttg tac ttt ggg ctt ttg gtg ttt gta ggt tac att      576
   Phe Lys Phe Glu Leu Tyr Phe Gly Leu Leu Val Phe Val Gly Tyr Ile
               180                 185                 190
   gta gta gac acc caa gaa ata gtt gag agg gca cac ttg ggc gat ctg      624
   Val Val Asp Thr Gln Glu Ile Val Glu Arg Ala His Leu Gly Asp Leu
           195                 200                 205
   gac tat gta aag cat gcc ttg acc ttg ttt acc gat ttg gtc gca gtt      672
   Asp Tyr Val Lys His Ala Leu Thr Leu Phe Thr Asp Leu Val Ala Val
       210                 215                 220
   ttt gtc cgg att ctt gtt att atg ttg aag aat tcg act gag agg aat      720
   Phe Val Arg Ile Leu Val Ile Met Leu Lys Asn Ser Thr Glu Arg Asn
   225                 230                 235                 240
   gag aag aaa aag aag aga aga gat tga                                  747
   Glu Lys Lys Lys Lys Arg Arg Asp
                   245
<210> 12
   <211> 248
   <212> PRT
   <213> Glycine max
<400> 12
   Arg Leu Gln Ala Met Asp Ala Phe Asn Ser Phe Phe Asp Ser Arg Asn
   1               5                   10                  15
   Arg Trp Asn Tyr Asp Thr Leu Lys Asn Phe Arg Gln Ile Ser Pro Val
               20                  25                  30
   Val Gln Asn His Leu Lys Gln Val Tyr Phe Thr Leu Cys Phe Ala Val
           35                  40                  45
   Val Ala Ala Ala Val Gly Ala Tyr Leu His Val Leu Leu Asn Ile Gly
       50                  55                  60
   Gly Phe Leu Thr Thr Val Ala Cys Met Gly Ser Ser Phe Trp Leu Leu
   65                  70                  75                  80
   Ser Thr Pro Pro Phe Glu Glu Arg Lys Arg Val Thr Leu Leu Met Ala
                   85                  90                  95
   Ala Ser Leu Phe Gln Gly Ser Ser Ile Gly Pro Leu Ile Asp Leu Ala
               100                 105                 110
   Ile His Ile Asp Pro Ser Leu Ile Phe Ser Ala Phe Val Gly Thr Ala
           115                 120                 125
   Leu Ala Phe Ala Cys Phe Ser Gly Ala Ala Leu Val Ala Arg Arg Arg
       130                 135                 140
   Glu Tyr Leu Tyr Leu Gly Gly Leu Val Ser Ser Gly Leu Ser Ile Leu
   145                 150                 155                 160
   Leu Trp Leu His Phe Ala Ser Ser Ile Phe Gly Gly Ser Thr Ala Leu
                   165                 170                 175
   Phe Lys Phe Glu Leu Tyr Phe Gly Leu Leu Val Phe Val Gly Tyr Ile
               180                 185                 190
   Val Val Asp Thr Gln Glu Ile Val Glu Arg Ala His Leu Gly Asp Leu
           195                 200                 205
   Asp Tyr Val Lys His Ala Leu Thr Leu Phe Thr Asp Leu Val Ala Val
       210                 215                 220
   Phe Val Arg Ile Leu Val Ile Met Leu Lys Asn Ser Thr Glu Arg Asn
   225                 230                 235                 240
   Glu Lys Lys Lys Lys Arg Arg Asp
                   245
<210> 13
   <211> 1510
   <212> DNA
   <213> Glycine max
<220>
   <221> CDS
   <222> (1)..(777)
   <223> coding for BI-1 protein
<400> 13
   atc acg aaa act ata cga ttc gat tcc ttg ttt tcg atg gac act ttc    48
   Ile Thr Lys Thr Ile Arg Phe Asp Ser Leu Phe Ser Met Asp Thr Phe
    1               5                  10                  15
   ttc aag tcc cca tct tct tct tct tcg aga agc cgc tgg agt tac gat    96
   Phe Lys Ser Pro Ser Ser Ser Ser Ser Arg Ser Arg Trp Ser Tyr Asp
               20                  25                  30
   act ctc aag aat ttc cgc gag atc tct ccg ctc gtt cag aat cac atc   144
   Thr Leu Lys Asn Phe Arg Glu Ile Ser Pro Leu Val Gln Asn His Ile
           35                  40                  45
   aaa ctg gtt tat ttt acg tta tgt tgc gct gtg gtg gct gct gct gtt   192
   Lys Leu Val Tyr Phe Thr Leu Cys Cys Ala Val Val Ala Ala Ala Val
       50                  55                  60
   gga gct ttc ctt cat gtt ctg tgg aac att ggc ggt ttt ctc acc acg   240
   Gly Ala Phe Leu His Val Leu Trp Asn Ile Gly Gly Phe Leu Thr Thr
   65                  70                  75                  80
   ttg gct tcc att ggg agc atg ttt tgg ttg cta tct aca ccc cct ttt   288
   Leu Ala Ser Ile Gly Ser Met Phe Trp Leu Leu Ser Thr Pro Pro Phe
                   85                  90                  95
   gaa gag caa aag agg ttg tct ctg ttg atg gct tcg gcc ctg ttt cag   336
   Glu Glu Gln Lys Arg Leu Ser Leu Leu Met Ala Ser Ala Leu Phe Gln
               100                 105                 110
   ggt gct tcc att gga cct ctg att gat ttg gct ttt gcc att gat cct   384
   Gly Ala Ser Ile Gly Pro Leu Ile Asp Leu Ala Phe Ala Ile Asp Pro
           115                 120                 125
   ggc ctt atc att ggc gca ttt gtg gca act tct ttg gct ttt gct tgc   432
   Gly Leu Ile Ile Gly Ala Phe Val Ala Thr Ser Leu Ala Phe Ala Cys
       130                 135                 140
   ttt tct gca gta gcc tta gtt gca agg cga agg gag tac ctc tac ctt      480
   Phe Ser Ala Val Ala Leu Val Ala Arg Arg Arg Glu Tyr Leu Tyr Leu
   145                 150                 155                 160
   ggt ggt ttg ctt tct tct tgg ctt tcc att ctt atg tgg ttg cac tct      528
   Gly Gly Leu Leu Ser Ser Trp Leu Ser Ile Leu Met Trp Leu His Ser
                   165                 170                 175
   gat tcc tct ctc ttt ggg ggc tca att gca ctc ttc aag ttt gag ctg      576
   Asp Ser Ser Leu Phe Gly Gly Ser Ile Ala Leu Phe Lys Phe Glu Leu
               180                 185                 190
   tac ttt ggg ctt ttg gtg ttt gtg ggc tac gtt ata gta gac act caa      624
   Tyr Phe Gly Leu Leu Val Phe Val Gly Tyr Val Ile Val Asp Thr Gln
           195                 200                 205
   gaa att att gaa agg gct cac ttt ggt gac ctg gat tat gtg aag cat      672
   Glu Ile Ile Glu Arg Ala His Phe Gly Asp Leu Asp Tyr Val Lys His
       210                 215                 220
   gca ttg aca ttg ttc act gat ttg gct gca atc ttt gtg cga att ctt      720
   Ala Leu Thr Leu Phe Thr Asp Leu Ala Ala Ile Phe Val Arg Ile Leu
   225                 230                 235                 240
   att ata atg ttg aag aat tca tct gag aga aat gag aag aag aag aaa      768
   Ile Ile Met Leu Lys Asn Ser Ser Glu Arg Asn Glu Lys Lys Lys Lys
                   245                 250                 255
   agg aga gat tagtaggctg accgaccgac tcgagctcag gcttctctac              817
   Arg Arg Asp
   agtaatttag tttgtggaga atacataatt agctgtttag atgatgttgg tcccttgtgt 877
   agttagttag ctatgtgttt gctgtaatgg taaatgtcag gatttctttt aaacatcttc 937
   atatgtattt gccaatatca taatgtgtcg tataacatca taccttggtt taaaaaaaaa 997
   aaaaaaaaaa aaaaaaaaaa aaaaaaaaaa aaaaaaaaaa aaaaaaaann nnnnnnnnnn 1057
   nnnnnnnnnn nnnnnnnnnn nnnnnnnnnn nnnnnnnngg tgtttgtggg ctacgttata 1117
   gtagacactc aagtaatcat tgagagggct cactttggtg acctggatta tgttaagcat 1177
   gcattgacac tgttcactga tttggctgca atctttgtgc gaattcttaa tataatgttg 1237
   aataattcat ctaagagaaa tgagaagaag aggaggagag attaataggt tgaccgattg 1297
   ctatgtgtag agtaatttgg tttgtagaga atacataatt agctgtttag aagttgttgg 1357
   tccccttgtg tagttagtag ttagctatgt gtttgctgta atggtaaatg tcaggatttc 1417
   ttttaaacat tttcatatgt atttgctaat aatcataata tatagtataa acatcattcc 1477
   ttggtttaaa aaaagaaaaa aaaaaaaaaa aaa                               1510
<210> 14
   <211> 259
   <212> PRT
   <213> Glycine max
<400> 14
   Ile Thr Lys Thr Ile Arg Phe Asp Ser Leu Phe Ser Met Asp Thr Phe
   1               5                   10                  15
   Phe Lys Ser Pro Ser Ser Ser Ser Ser Arg Ser Arg Trp Ser Tyr Asp
               20                  25                  30
   Thr Leu Lys Asn Phe Arg Glu Ile Ser Pro Leu Val Gln Asn His Ile
           35                  40                  45
   Lys Leu Val Tyr Phe Thr Leu Cys Cys Ala Val Val Ala Ala Ala Val
       50                  55                  60
   Gly Ala Phe Leu His Val Leu Trp Asn Ile Gly Gly Phe Leu Thr Thr
   65                  70                  75                  80
   Leu Ala Ser Ile Gly Ser Met Phe Trp Leu Leu Ser Thr Pro Pro Phe
                   85                  90                  95
   Glu Glu Gln Lys Arg Leu Ser Leu Leu Met Ala Ser Ala Leu Phe Gln
               100                 105                 110
   Gly Ala Ser Ile Gly Pro Leu Ile Asp Leu Ala Phe Ala Ile Asp Pro
           115                 120                 125
   Gly Leu Ile Ile Gly Ala Phe Val Ala Thr Ser Leu Ala Phe Ala Cys
       130                 135                 140
   Phe Ser Ala Val Ala Leu Val Ala Arg Arg Arg Glu Tyr Leu Tyr Leu
   145                 150                 155                 160
   Gly Gly Leu Leu Ser Ser Trp Leu Ser Ile Leu Met Trp Leu His Ser
                   165                 170                 175
   Asp Ser Ser Leu Phe Gly Gly Ser Ile Ala Leu Phe Lys Phe Glu Leu
               180                 185                 190
   Tyr Phe Gly Leu Leu Val Phe Val Gly Tyr Val Ile Val Asp Thr Gln
           195                 200                 205
   Glu Ile Ile Glu Arg Ala His Phe Gly Asp Leu Asp Tyr Val Lys His
       210                 215                 220
   Ala Leu Thr Leu Phe Thr Asp Leu Ala Ala Ile Phe Val Arg Ile Leu
   225                 230                 235                 240
   Ile Ile Met Leu Lys Asn Ser Ser Glu Arg Asn Glu Lys Lys Lys Lys
                   245                 250                 255
   Arg Arg Asp
<210> 15
   <211> 651
   <212> DNA
   <213> Triticum aestivum
<220>
   <221> CDS
   <222> (1)..(651)
   <223> coding for BI-1 protein
<400> 15
   gtc gca atg ccg ggt cga cga ttt cgt ctg acc tat gct ttg cct ggc      48
   Val Ala Met Pro Gly Arg Arg Phe Arg Leu Thr Tyr Ala Leu Pro Gly
    1               5                  10                  15
   ctc atc tgc cgt ggg tgc tta cct gca cat tgc cct gaa cat tgg cgg      96
   Leu Ile Cys Arg Gly Cys Leu Pro Ala His Cys Pro Glu His Trp Arg
               20                  25                  30
   gat gct gac aat gct cgc gtg tat cgg aac cat cgc ctg gat gtt ctc     144
   Asp Ala Asp Asn Ala Arg Val Tyr Arg Asn His Arg Leu Asp Val Leu
           35                  40                  45
   ggt gcc agt cta cga gga gag gaa gag gtt tgg gct gct gat ggg tgc     192
   Gly Ala Ser Leu Arg Gly Glu Glu Glu Val Trp Ala Ala Asp Gly Cys
       50                  55                  60
   agc ctc ctg gaa ggg gct tca gtt gga cct ctg att gag ctt gcc ata     240
   Ser Leu Leu Glu Gly Ala Ser Val Gly Pro Leu Ile Glu Leu Ala Ile
   65                  70                  75                  80
   gac ttt gac cca agt atc ctc gtg aca ggg ttt gtc gga acc gcc atc     288
   Asp Phe Asp Pro Ser Ile Leu Val Thr Gly Phe Val Gly Thr Ala Ile
                   85                  90                  95
   gcc ttc ggg tgc ttc tct ggc gcc gcc atc atc gcc aag cgc agg gag     336
   Ala Phe Gly Cys Phe Ser Gly Ala Ala Ile Ile Ala Lys Arg Arg Glu
               100                 105                 110
   tac ctg tac ctc ggt ggt ctg ctc tcc tcc ggc ctg tcg atc ctg ctc     384
   Tyr Leu Tyr Leu Gly Gly Leu Leu Ser Ser Gly Leu Ser Ile Leu Leu
           115                 120                 125
   tgg ctg cag ttt gcc acg tcc atc ttt ggc cac tcc tct ggc agc ttc     432
   Trp Leu Gln Phe Ala Thr Ser Ile Phe Gly His Ser Ser Gly Ser Phe
       130                 135                 140
   atg ttt gag gtt tac ttt ggc ctg ttg atc ttc ctg gga tac atg gtg     480
   Met Phe Glu Val Tyr Phe Gly Leu Leu Ile Phe Leu Gly Tyr Met Val
   145                 150                 155                 160
   tac gac acg cag gag atc atc gag agg gcg cac cac ggc gac atg gat     528
   Tyr Asp Thr Gln Glu Ile Ile Glu Arg Ala His His Gly Asp Met Asp
                   165                 170                 175
   tac atc aag cac gcg ctc acc ctc ttc acc gac ttc gtc gcc gtt ctc     576
   Tyr Ile Lys His Ala Leu Thr Leu Phe Thr Asp Phe Val Ala Val Leu
               180                 185                 190
   gtc cgc gtc ctc atc atc ttg ctc aag aac gca gcg gac aag gtc gga     624
   Val Arg Val Leu Ile Ile Leu Leu Lys Asn Ala Ala Asp Lys Val Gly
           195                 200                 205
   ggc caa gaa gag gag gaa gag aag tcc                                 651
   Gly Gln Glu Glu Glu Glu Glu Lys Ser
       210                 215
<210> 16
   <211> 217
   <212> PRT
   <213> Triticum aestivum
<400> 16
   Val Ala Met Pro Gly Arg Arg Phe Arg Leu Thr Tyr Ala Leu Pro Gly
   1               5                   10                  15
   Leu Ile Cys Arg Gly Cys Leu Pro Ala His Cys Pro Glu His Trp Arg
               20                  25                  30
   Asp Ala Asp Asn Ala Arg Val Tyr Arg Asn His Arg Leu Asp Val Leu
           35                  40                  45
   Gly Ala Ser Leu Arg Gly Glu Glu Glu Val Trp Ala Ala Asp Gly Cys
       50                  55                  60
   Ser Leu Leu Glu Gly Ala Ser Val Gly Pro Leu Ile Glu Leu Ala Ile
   65                  70                  75                  80
   Asp Phe Asp Pro Ser Ile Leu Val Thr Gly Phe Val Gly Thr Ala Ile
                   85                  90                  95
   Ala Phe Gly Cys Phe Ser Gly Ala Ala Ile Ile Ala Lys Arg Arg Glu
               100                 105                 110
   Tyr Leu Tyr Leu Gly Gly Leu Leu Ser Ser Gly Leu Ser Ile Leu Leu
           115                 120                 125
   Trp Leu Gln Phe Ala Thr Ser Ile Phe Gly His Ser Ser Gly Ser Phe
       130                 135                 140
   Met Phe Glu Val Tyr Phe Gly Leu Leu Ile Phe Leu Gly Tyr Met Val
   145                 150                 155                 160
   Tyr Asp Thr Gln Glu Ile Ile Glu Arg Ala His His Gly Asp Met Asp
                   165                 170                 175
   Tyr Ile Lys His Ala Leu Thr Leu Phe Thr Asp Phe Val Ala Val Leu
               180                 185                 190
   Val Arg Val Leu Ile Ile Leu Leu Lys Asn Ala Ala Asp Lys Val Gly
           195                 200                 205
   Gly Gln Glu Glu Glu Glu Glu Lys Ser
       210                 215
<210> 17
   <211> 412
   <212> DNA
   <213> Zea mays
<220>
   <221> CDS
   <222> (3)..(410)
   <223> coding for BI1-protein
<400> 17
   tt gtt att gac ttg gat tcg agg att ctc gtc act gcg ttc gtc ggg       47
      Val Ile Asp Leu Asp Ser Arg Ile Leu Val Thr Ala Phe Val Gly
       1               5                  10                  15
   acc gca gtt gct ttt gca tgc ttc tct ggc gct gcc atc atc gcc aag      95
   Thr Ala Val Ala Phe Ala Cys Phe Ser Gly Ala Ala Ile Ile Ala Lys
                   20                  25                  30
   cgc agg gaa tac ctg tac ctc ggc ggt ctg ctt tca tct ggc ctc tcc     143
   Arg Arg Glu Tyr Leu Tyr Leu Gly Gly Leu Leu Ser Ser Gly Leu Ser
               35                  40                  45
   att ctt ctc tgg ctg cag ttt gct act tca atc ttt ggc cac acc agc     191
   Ile Leu Leu Trp Leu Gln Phe Ala Thr Ser Ile Phe Gly His Thr Ser
           50                  55                  60
   gcg acc ttc atg ttt gag ctc tac ttt ggc ctc ctg gtt ttc ctg gga     239
   Ala Thr Phe Met Phe Glu Leu Tyr Phe Gly Leu Leu Val Phe Leu Gly
       65                  70                  75
   tat atg gtg ttt gac acc cag gag atc atc gag agg gcg cac cgt ggg     287
   Tyr Met Val Phe Asp Thr Gln Glu Ile Ile Glu Arg Ala His Arg Gly
   80                  85                  90                  95
   gac atg gac tac atc aag cac gcg ctg act ctc ttc acc gac ttt gtt     335
   Asp Met Asp Tyr Ile Lys His Ala Leu Thr Leu Phe Thr Asp Phe Val
                   100                 105                 110
   gcg gtt ctt gtt cga atc ctt gtc atc atg atg aag aat gca cag gag     383
   Ala Val Leu Val Arg Ile Leu Val Ile Met Met Lys Asn Ala Gln Glu
               115                 120                 125
   aaa tcc caa gac gag aag aag agg aag aa                              412
   Lys Ser Gln Asp Glu Lys Lys Arg Lys
           130                 135
<210> 18
   <211> 136
   <212> PRT
   <213> Zea mays
<400> 18
   Val Ile Asp Leu Asp Ser Arg Ile Leu Val Thr Ala Phe Val Gly Thr
   1               5                   10                  15
   Ala Val Ala Phe Ala Cys Phe Ser Gly Ala Ala Ile Ile Ala Lys Arg
               20                  25                  30
   Arg Glu Tyr Leu Tyr Leu Gly Gly Leu Leu Ser Ser Gly Leu Ser Ile
           35                  40                  45
   Leu Leu Trp Leu Gln Phe Ala Thr Ser Ile Phe Gly His Thr Ser Ala
       50                  55                  60
   Thr Phe Met Phe Glu Leu Tyr Phe Gly Leu Leu Val Phe Leu Gly Tyr
   65                  70                  75                  80
   Met Val Phe Asp Thr Gln Glu Ile Ile Glu Arg Ala His Arg Gly Asp
                   85                  90                  95
   Met Asp Tyr Ile Lys His Ala Leu Thr Leu Phe Thr Asp Phe Val Ala
               100                 105                 110
   Val Leu Val Arg Ile Leu Val Ile Met Met Lys Asn Ala Gln Glu Lys
           115                 120                 125
   Ser Gln Asp Glu Lys Lys Arg Lys
       130                 135
<210> 19
   <211> 345
   <212> DNA
   <213> Triticum aestivum
<220>
   <221> CDS
   <222> (1)..(342)
<400> 19
   gcc gcc atc atc gcc aag cgc agg gag tac ctg tac ctc ggt ggc ctg      48
   Ala Ala Ile Ile Ala Lys Arg Arg Glu Tyr Leu Tyr Leu Gly Gly Leu
   1               5                   10                  15
   ctc tcc tcc ggc ctg tcg atc ctg ctc tgg ctg cag ttt gcc acg tcc      96
   Leu Ser Ser Gly Leu Ser Ile Leu Leu Trp Leu Gln Phe Ala Thr Ser
               20                  25                  30
   atc ttt ggc cac tcc tct ggc agc ttc atg ttt gag gtt tac ttt ggc      144
   Ile Phe Gly His Ser Ser Gly Ser Phe Met Phe Glu Val Tyr Phe Gly
           35                  40                  45
   ctg ttg atc ttt ctg gga tac atg gtg tac gac acg cag gag atc atc      192
   Leu Leu Ile Phe Leu Gly Tyr Met Val Tyr Asp Thr Gln Glu Ile Ile
       50                  55                  60
   gag agg gcg cac cac ggc gac atg gac tac atc aag cac gcg ctc acc      240
   Glu Arg Ala His His Gly Asp Met Asp Tyr Ile Lys His Ala Leu Thr
   65                  70                  75                  80
   ctc ttc acc gac ttt gtc gcc gtc ctc gtc cgg atc ctc atc atc atg      288
   Leu Phe Thr Asp Phe Val Ala Val Leu Val Arg Ile Leu Ile Ile Met
                   85                  90                  95
   ctc aag aac gca ggc gac aag tcg gag gac aag aag aag agg aag agg      336
   Leu Lys Asn Ala Gly Asp Lys Ser Glu Asp Lys Lys Lys Arg Lys Arg
               100                 105                 110
   agg tcc tga                                                          345
   Arg Ser
<210> 20
   <211> 114
   <212> PRT
   <213> Triticum aestivum
<400> 20
   Ala Ala Ile Ile Ala Lys Arg Arg Glu Tyr Leu Tyr Leu Gly Gly Leu
   1               5                   10                  15
   Leu Ser Ser Gly Leu Ser Ile Leu Leu Trp Leu Gln Phe Ala Thr Ser
               20                  25                  30
   Ile Phe Gly His Ser Ser Gly Ser Phe Met Phe Glu Val Tyr Phe Gly
           35                  40                  45
   Leu Leu Ile Phe Leu Gly Tyr Met Val Tyr Asp Thr Gln Glu Ile Ile
       50                  55                  60
   Glu Arg Ala His His Gly Asp Met Asp Tyr Ile Lys His Ala Leu Thr
   65                  70                  75                  80
   Leu Phe Thr Asp Phe Val Ala Val Leu Val Arg Ile Leu Ile Ile Met
                   85                  90                  95
   Leu Lys Asn Ala Gly Asp Lys Ser Glu Asp Lys Lys Lys Arg Lys Arg
               100                 105                 110
   Arg Ser
<210> 21
   <211> 403
   <212> DNA
   <213> Zea mays
<220>
   <221> CDS
   <222> (1)..(402)
   <223> coding for BI1-protein
<400> 21
   ggc agc atc gcc tgg ctc ttc tcg gtg ccc gtc tac gag gag agg aag      48
   Gly Ser Ile Ala Trp Leu Phe Ser Val Pro Val Tyr Glu Glu Arg Lys
   1               5                   10                  15
   agg tac tgg ctg ctg atg gcg gct gcc ctc ctg gaa ggg gcg tcg gtt      96
   Arg Tyr Trp Leu Leu Met Ala Ala Ala Leu Leu Glu Gly Ala Ser Val
               20                  25                  30
   gga ccc ctc atc aag ctc gcc gtg gaa ttt gac cca agc atc ctg gtg      144
   Gly Pro Leu Ile Lys Leu Ala Val Glu Phe Asp Pro Ser Ile Leu Val
           35                  40                  45
   aca gcg ttc gtg ggg act gcc att gcg ttc gcg tgc ttc tct tgc gcg      192
   Thr Ala Phe Val Gly Thr Ala Ile Ala Phe Ala Cys Phe Ser Cys Ala
       50                  55                  60
   gcc atg gtg gcc aag cgc agg gag tac ctc tac ctg ggc ggg ctg ctc      240
   Ala Met Val Ala Lys Arg Arg Glu Tyr Leu Tyr Leu Gly Gly Leu Leu
   65                  70                  75                  80
   tct tct ggc ctc tcc atc ctg ctc tgg ctg cag ttc gcc gcc tcc atc      288
   Ser Ser Gly Leu Ser Ile Leu Leu Trp Leu Gln Phe Ala Ala Ser Ile
                   85                  90                  95
   ttc ggc cac caa tcc act agc agc ttc atg ttt gag gtc tac ttt ggg      336
   Phe Gly His Gln Ser Thr Ser Ser Phe Met Phe Glu Val Tyr Phe Gly
               100                 105                 110
   ctg ctc atc ttc ctg ggc tac atg gtg tac gac acg cag gag gtc atc      384
   Leu Leu Ile Phe Leu Gly Tyr Met Val Tyr Asp Thr Gln Glu Val Ile
           115                 120                 125
   gag agg gcg cac cac ggc g                                            403
   Glu Arg Ala His His Gly
       130
<210> 22
   <211> 134
   <212> PRT
   <213> Zea mays
<400> 22
   Gly Ser Ile Ala Trp Leu Phe Ser Val Pro Val Tyr Glu Glu Arg Lys
   1               5                   10                  15
   Arg Tyr Trp Leu Leu Met Ala Ala Ala Leu Leu Glu Gly Ala Ser Val
               20                  25                  30
   Gly Pro Leu Ile Lys Leu Ala Val Glu Phe Asp Pro Ser Ile Leu Val
           35                  40                  45
   Thr Ala Phe Val Gly Thr Ala Ile Ala Phe Ala Cys Phe Ser Cys Ala
       50                  55                  60
   Ala Met Val Ala Lys Arg Arg Glu Tyr Leu Tyr Leu Gly Gly Leu Leu
   65                  70                  75                  80
   Ser Ser Gly Leu Ser Ile Leu Leu Trp Leu Gln Phe Ala Ala Ser Ile
                   85                  90                  95
   Phe Gly His Gln Ser Thr Ser Ser Phe Met Phe Glu Val Tyr Phe Gly
               100                 105                 110
   Leu Leu Ile Phe Leu Gly Tyr Met Val Tyr Asp Thr Gln Glu Val Ile
           115                 120                 125
   Glu Arg Ala His His Gly
       130
<210> 23
   <211> 410
   <212> DNA
   <213> Zea mays
<220>
   <221> CDS
   <222> (3)..(410)
   <223> coding for BI1-protein
<400> 23
   gc tgg aac atc ggc gtg agg ctg aca atg ctc ggt tgc atc ggc agc       47
      Trp Asn Ile Gly Val Arg Leu Thr Met Leu Gly Cys Ile Gly Ser
       1               5                   10                  15
   atc gac tgg ctc ttc tcg gtg ccc gtc tac gag gag agg aag agg tat      95
   Ile Asp Trp Leu Phe Ser Val Pro Val Tyr Glu Glu Arg Lys Arg Tyr
                   20                  25                  30
   ggg ctg ctg atg gcg gct gcc ctc ctg gaa ggc gct tcg gtc gga ccc     143
   Gly Leu Leu Met Ala Ala Ala Leu Leu Glu Gly Ala Ser Val Gly Pro
               35                  40                  45
   ctc gtc aag ctc gcc gtg gaa ttt gac cca agc atc ctg gtg acg gcg     191
   Leu Val Lys Leu Ala Val Glu Phe Asp Pro Ser Ile Leu Val Thr Ala
           50                  55                  60
   ttc gtg ggg act gcc atc gcg ttc gcg tgc ttc tcc ggc gcg gcc atg     239
   Phe Val Gly Thr Ala Ile Ala Phe Ala Cys Phe Ser Gly Ala Ala Met
       65                  70                  75
   gtg gcc agg cgc agg gag tac ctc tac ctg ggc ggg ctg ctc tcg tcg     287
   Val Ala Arg Arg Arg Glu Tyr Leu Tyr Leu Gly Gly Leu Leu Ser Ser
   80                  85                  90                  95
   ggg ctc tcc atc ctg ctc tgg ctg cag ctc gcc gcc tcc atc ttc ggc     335
   Gly Leu Ser Ile Leu Leu Trp Leu Gln Leu Ala Ala Ser Ile Phe Gly
                   100                 105                 110
   cac tcc gca acc agc ttc atg ttc gag gtc tac ttc ggg ctg ctc atc     383
   His Ser Ala Thr Ser Phe Met Phe Glu Val Tyr Phe Gly Leu Leu Ile
               115                 120                 125
   ttc ctg ggc tac gtg gtg tac gac acg                                 410
   Phe Leu Gly Tyr Val Val Tyr Asp Thr
           130                 135
<210> 24
   <211> 136
   <212> PRT
   <213> **Zea** mays
<400> 24
   Trp Asn Ile Gly Val Arg Leu Thr Met Leu Gly Cys Ile Gly Ser Ile
   1               5                   10                  15
   Asp Trp Leu Phe Ser Val Pro Val Tyr Glu Glu Arg Lys Arg Tyr Gly
               20                  25                  30
   Leu Leu Met Ala Ala Ala Leu Leu Glu Gly Ala Ser Val Gly Pro Leu
           35                  40                  45
   Val Lys Leu Ala Val Glu Phe Asp Pro Ser Ile Leu Val Thr Ala Phe
       50                  55                  60
   Val Gly Thr Ala Ile Ala Phe Ala Cys Phe Ser Gly Ala Ala Met Val
   65                  70                  75                  80
   Ala Arg Arg Arg Glu Tyr Leu Tyr Leu Gly Gly Leu Leu Ser Ser Gly
                   85                  90                  95
   Leu Ser Ile Leu Leu Trp Leu Gln Leu Ala Ala Ser Ile Phe Gly His
               100                 105                 110
   Ser Ala Thr Ser Phe Met Phe Glu Val Tyr Phe Gly Leu Leu Ile Phe
           115                 120                 125
   Leu Gly Tyr Val Val Tyr Asp Thr
   130 135
<210> 25
   <211> 463
   <212> DNA
   <213> Triticum aestivum
<220>
   <221> CDS
   <222> (1)..(462)
   <223> coding for BI1-protein
<400> 25
   ttc tca ggt acg ttc cgc aat tcc cgg agc gac gat ttc gtg ctc tgc      48
   Phe Ser Gly Thr Phe Arg Asn Ser Arg Ser Asp Asp Phe Val Leu Cys
   1               5                   10                  15
   gaa ctt cag cga gag ctc ccc cga tgc cgg gac gca acc ttg acg gtc      96
   Glu Leu Gln Arg Glu Leu Pro Arg Cys Arg Asp Ala Thr Leu Thr Val
               20                  25                  30
   gta tac gtg atc cca ata gtg ggc cga ata aaa tct gcc gcg ggt gct      144
   Val Tyr Val Ile Pro Ile Val Gly Arg Ile Lys Ser Ala Ala Gly Ala
           35                  40                  45
   tac ctg cac att gcc ctg aac atc ggt ggg atg ctg aca atg ctt gcg      192
   Tyr Leu His Ile Ala Leu Asn Ile Gly Gly Met Leu Thr Met Leu Ala
       50                  55                  60
   tgt atc gga acc att gcc tgg atg ttc tct gtg cca gtc tat gag gag      240
   Cys Ile Gly Thr Ile Ala Trp Met Phe Ser Val Pro Val Tyr Glu Glu
   65                  70                  75                  80
   agg aag agg ttt ggg ctg ctg atg ggt gca gcc ctc ctg gaa ggg gct      288
   Arg Lys Arg Phe Gly Leu Leu Met Gly Ala Ala Leu Leu Glu Gly Ala
                   85                  90                  95
   tcg gtt gga cct ctg att gag ctt gcc ata gac ttt gac cca agc atc      336
   Ser Val Gly Pro Leu Ile Glu Leu Ala Ile Asp Phe Asp Pro Ser Ile
               100                 105                 110
   ctc gtg aca ggg ttt gtt gga acc gcc atc gcc ttt ggg tgc ttc tct      384
   Leu Val Thr Gly Phe Val Gly Thr Ala Ile Ala Phe Gly Cys Phe Ser
           115                 120                 125
   ggc gcc gcc atc atc gcc aag cgc agg gag tac ctg tac ctc gga ggc      432
   Gly Ala Ala Ile Ile Ala Lys Arg Arg Glu Tyr Leu Tyr Leu Gly Gly
       130                 135                 140
   ctg ctc tcc tcc ggc ctg acg atc ctg ctc t                            463
   Leu Leu Ser Ser Gly Leu Thr Ile Leu Leu
   145                 150
<210> 26
   <211> 154
   <212> PRT
   <213> Triticum aestivum
<400> 26
   Phe Ser Gly Thr Phe Arg Asn Ser Arg Ser Asp Asp Phe Val Leu Cys
   1               5                   10                  15
   Glu Leu Gln Arg Glu Leu Pro Arg Cys Arg Asp Ala Thr Leu Thr Val
               20                  25                  30
   Val Tyr Val Ile Pro Ile Val Gly Arg Ile Lys Ser Ala Ala Gly Ala
           35                  40                  45
   Tyr Leu His Ile Ala Leu Asn Ile Gly Gly Met Leu Thr Met Leu Ala
       50                  55                  60
   Cys Ile Gly Thr Ile Ala Trp Met Phe Ser Val Pro Val Tyr Glu Glu
   65                  70                  75                  80
   Arg Lys Arg Phe Gly Leu Leu Met Gly Ala Ala Leu Leu Glu Gly Ala
                   85                  90                  95
   Ser Val Gly Pro Leu Ile Glu Leu Ala Ile Asp Phe Asp Pro Ser Ile
               100                 105                 110
   Leu Val Thr Gly Phe Val Gly Thr Ala Ile Ala Phe Gly Cys Phe Ser
           115                 120                 125
   Gly Ala Ala Ile Ile Ala Lys Arg Arg Glu Tyr Leu Tyr Leu Gly Gly
       130                 135                 140
   Leu Leu Ser Ser Gly Leu Thr Ile Leu Leu
   145                 150
<210> 27
   <211> 388
   <212> DNA
   <213> Zea mays
<220>
   <221> CDS
   <222> (3)..(386)
   <223> coding for BI1-protein
<400> 27
   tc tgg aac atc ggc ggg acg ctg aca atg ctc ggt tgc gtc ggc agc      47
      Trp Asn Ile Gly Gly Thr Leu Thr Met Leu Gly Cys Val Gly Ser
      1               5                   10                  15
   atc gcc tgg ctc ttc tcg gtg ccc gtc tac gag gag agg aag agg tat     95
   Ile Ala Trp Leu Phe Ser Val Pro Val Tyr Glu Glu Arg Lys Arg Tyr
                   20                  25                  30
   ggg ctg ctg atg gcg gct gcc ctc ctg gaa ggc gct tcg gtc gga ccc    143
   Gly Leu Leu Met Ala Ala Ala Leu Leu Glu Gly Ala Ser Val Gly Pro
               35                  40                  45
   ctc gtc aag ctc gcc gtg gaa ttt gac cca agc atc ctg gtg acg gcg    191
   Leu Val Lys Leu Ala Val Glu Phe Asp Pro Ser Ile Leu Val Thr Ala
           50                  55                  60
   ttc gtg ggg act gcc atc gcg ttc gcg tgc ttc tcc ggc gcg cca tgg    239
   Phe Val Gly Thr Ala Ile Ala Phe Ala Cys Phe Ser Gly Ala Pro Trp
       65                  70                  75
   tgg cag gcc agg gag tac ctc tac ctg ggc ggc tgc tct cgt cga ggc    287
   Trp Gln Ala Arg Glu Tyr Leu Tyr Leu Gly Gly Cys Ser Arg Arg Gly
   80                  85                  90                  95
   tct cca tcc tgc tct ggc tgc agc tcg ccg cct cca tct tcg gca ctc    335
   Ser Pro Ser Cys Ser Gly Cys Ser Ser Pro Pro Pro Ser Ser Ala Leu
                   100                 105                 110
   cgc aac agc ttc atg ttc gag gtc tac ttc ggg ctg ctc att ctt ctg    383
   Arg Asn Ser Phe Met Phe Glu Val Tyr Phe Gly Leu Leu Ile Leu Leu
               115                 120                 125
   ggc ta                                                             388
                                       Gly
<210> 28
   <211> 128
   <212> PRT
   <213> **Zea** mays
<400> 28
   Trp Asn Ile Gly Gly Thr Leu Thr Met Leu Gly Cys Val Gly Ser Ile
   1               5                   10                  15
   Ala Trp Leu Phe Ser Val Pro Val Tyr Glu Glu Arg Lys Arg Tyr Gly
               20                  25                  30
   Leu Leu Met Ala Ala Ala Leu Leu Glu Gly Ala Ser Val Gly Pro Leu
           35                  40                  45
   Val Lys Leu Ala Val Glu Phe Asp Pro Ser Ile Leu Val Thr Ala Phe
       50                  55                  60
   Val Gly Thr Ala Ile Ala Phe Ala Cys Phe Ser Gly Ala Pro Trp Trp
   65                  70                  75                  80
   Gln Ala Arg Glu Tyr Leu Tyr Leu Gly Gly Cys Ser Arg Arg Gly Ser
                   85                  90                  95
   Pro Ser Cys Ser Gly Cys Ser Ser Pro Pro Pro Ser Ser Ala Leu Arg
               100                 105                 110
   Asn Ser Phe Met Phe Glu Val Tyr Phe Gly Leu Leu Ile Leu Leu Gly
           115                 120                 125
<210> 29
   <211> 1737
   <212> DNA
   <213> Solanum tuberosum
<220>
   <221> promoter
   <222> (1)..(1737)
   <223> patatin promotor
<400> 29
   aagcttatgt tgccatatag agtagtttgt gatggtatac ttcataaact ttaacttatg 60
   ttaaatttgt aatgataaaa tttttattgt aaattaaaaa ttacttataa aattgggcat 120
   tataacatat gaaagacaaa ttgtgttaca tattttactt ttgactttaa tatgaatatt 180
   tcaatttaaa tcattgtttt attttctctt tctttttaca ggtataaaag gtgaaaattg 240
   aagcaagatt gattgcaagc tatgtgtcac cacgttattg atactttgga agaaattttt 300
   acttatatgt ctttgtttag gagtaatatt tgatatgttt tagttagatt ttcttgtcat 360
   ttatgcttta gtataatttt agttattttt attatatgat catgggtgaa ttttgataca 420
   aatatttttg tcattaaata aattaattta tcacaacttg attactttca gtgacaaaaa 480
   atgtattgtc gtagtaccct tttttgttga atatgaataa ttttttttat tttgtgacaa 540
   ttgtaattgt cactacttat gataatattt agtgacatat atgtcgtcgg taaaagcaaa 600
   cactttcagt gacaaaataa tagatttaat cacaaaatta ttaacctttt ttataataat 660
   aaatttatcc ctaatttata catttaagga caaagtattt tttttatata taaaaaatag 720
   tctttagtga cgatcgtagt gttgagtcta gaaatcataa tgttgaatct agaaaaatct 780
   catgcagtgt aaaataaacc tcaaaaagga cgttcagtcc atagaggggg tgtatgtgac 840
   accccaacct cagcaaaaga aaacctccct tcaacaagga catttgcggt gctaaacaat 900
   ttcaagtctc atcacacata tatttattat ataatactaa taaagaatag aaaaggaaag 960
   gtaaacatca ttaaatcgtc tttgtatatt tttagtgaca actgattgac gaaatctttt 1020
   tcgtcacaca aaatttttag tgacgaaaca tgatttatag atgatgaaat tatttgtccc 1080
   tcataatcta atttgttgta gtgatcatta ctcctttgtt tgttttattt gtcatgttag 1140
   tccattaaaa aaaaatatct ctcttcttat gtacgtgaat ggttggaacg gatctattat 1200
   ataatactaa taaagaatag aaaaaggaaa gtgagtgagg ttcgagggag agaatctgtt 1260
   taatatcaga gtcgatcatg tgtcaatttt atcgatatga ccctaacttc aactgagttt 1320
   aaccaattcc gataaggcga gaaatatcat agtattgagt ctagaaaaat ctcatgtagt 1380
   gtggggtaaa cctcagcaag gacgttgagt ccatagaggg gggtgtatgt gacaccccaa 1440
   cctcagcaaa agaaaacctc ccctcaagaa ggacatttgc ggtgctaaac aatttcaagt 1500
   ctcatcacac atatatatat attatataat actaataaat aatagaaaaa ggaaaggtaa 1560
   acatcactaa cgacagttgc ggtgcaaact gagtgaggta ataaacatca ctaactttta 1620
   ttggttatgt caaactcaaa gtaaaatttc tcaacttgtt tacgtgccta tatataccat 1680
   gcttgttata tgctcaaagc accaacaaaa tttaaaaaca ctttgaacat ttgcaaa    1737
<210> 30
   <211> 1317
   <212> DNA
   <213> Triticum aestivum
<220>
   <221> promoter
   <222> (1)..(1317)
   <223> germin 9f-3.8 gene promotor
<400> 30
   gaattcaagc tatcactctc gaaccaagca cattgatgta aggtatcatt ggattccaga 60
   tgtcgtgagt tccaagttgc tgaaacttga gaagatccat accgacgaca atggttcaga 120
   tatgatgacc aagatattgc gaaataagaa gctacaagca tgttgcaagg tagcgggcat 180
   ggcggtgccc ccatcatgag tcggaggggg agatttgttg ggatatcctc ctcatgtggg 240
   ttctgaggag atgaccattt gaggcctttt agccagccca aagaggtgca gaagcccact 300
   acccattagg gttatgacct agggtcattt tggactttgc acatgagtgg atggggatgc 360
   tttaccctcc atccagcagc caccaccaag ggtgacgaaa atcagttcat cctccaagag 420
   agaagaagag agaaaaccaa gagagcaagg gaagaagagg aagattgaag gaagaagaaa 480
   agggagctcc tccccaaggt tgtgatggtc catatccact atcttgtctc cttcaaactt 540
   cggttccacc atctttggta agattgttct aatccctagt tcttgagccc caaatcttgt 600
   tgtgttcatc caagattcag aaatcttgat gtatgagatc ctctagtgct gtctagagaa 660
   gaatttgttg tatcccacat ttgataatag tggaagagga tttgggtggc ttcggcccat 720
   ggtttttccc ctcaagttga ggggttttcc acgtaaaatc tggtgtctct ttgttgatgc 780
   ttggtgttgt ccagaaactt actcctacca caagacacta ggggccagtt cttttgggaa 840
   attctcccag aattgaccct ctccccagct tctcccagaa ttgtcactcc atttttcttt 900
   acaattccta gctagttaag gtctaattag ttaggaattg taaaaaaata tcaagtggca 960
   attctgggag aagctgggga gggggtcaat tctggaagaa ttgcccaaaa gaactggccc 1020
   taggctgagg agtgtcttgc ctgctgctta acattttctg cctccatata tgttgttgca 1080
   tatgtttcct tccgtgctaa gcaacgatcc ttgagttagt acatgatgtg gtgctgagat 1140
   tactttgttt tcgctgcagt tatcagttaa ccacaagtgc atttgcgtgc taattcccaa 1200
   caatatgcca cccgcaactc atccaccata gctcagcagc aaccaccaat gccatagaca 1260
   ctctcggtaa acaacctgta gcttatcagt ctagctaagc gtgctgcata gcaagca 1317
<210> 31
   <211> 959
   <212> DNA
   <213> Arabidopsis thaliana
<220>
   <221> promoter
   <222> (1)..(959)
   <223> CAB-2 promotor
<400> 31
   gaattcatgt gtgagggcaa ttagtgattg taaaaataaa attgtgtttt gtaaaaaact 60
   tttactgtcg aaattattta gggtgatgaa aaaatcagta aactacgaat gatagcttaa 120
   agagtttcta tcaaagtgat tgaggaatag tttgttgcaa attaaacctc taacaaaatg 180
   ttttctgttg tggtttttca tctctacaaa ttttgaattt tatgatgaat tagaaagata 240
   gaatgagtta ctttagattt taaaaggttg ttcaagttta caaaacagat tactagaatc 300
   atgattaaaa atttacaagc tacatattgt ctaaaccaat gatgttgaac ataccagatg 360
   atagtttttc agtgtttgaa caatcaattg gatagttttt atgtttctgc aaaatatgca 420
   aataatcagt gtttttgagt ctttgcattt tgatttaaaa gcaaaaacaa ctgagtttca 480
   aggttaaatt aattacatta ttcatgagat ttatcaggtt agtggataaa ctgacaatgg 540
   aatcaatgtt attgtaaatt ggtagtgatg ttggacttct aatgttactc tctatgatgt 600
   ttcggtcatc ggtatcacac tatctttact tttatttaaa ggaaagatca cacaaataag 660
   ttatctctat tcagaactat taagctgctt ccaaaagact tgcaacatgt ggtctcgaaa 720
   tgctttggct gcaatgaaaa aatcatagca aaagctagtg gactagagac tgccacataa 780
   gaatagtaaa cgttaaaacc aaaatctcaa aaatccaatg agtaaagaga tatagattac 840
   ttcatagata acaaacgtta ctcgcaattt tcctatataa tccaacccta cctaaccatt 900
   ttcaatcact ctcactcaca agttagtcac caaaaaaaaa aaaaacacaa aaagtttca 959
<210> 32
   <211> 445
   <212> DNA
   <213> Zea mays
<220>
   <221> promoter
   <222> (1)..(445)
   <223> PPCZm1 promoter
<400> 32
   gaattccaaa aatagacacg gcaattttct tattcacaga aaaaatataa ctacaactaa 60
   tccccaagtc cacagggatt agggatcaat ctgcaaaact aaaagtactt ttacagttgt 120
   acttggcatg agtcatgtga ccatgagaga ggcgcacggt tcagcaaagc aacataaaat 180
   tctccaaacg ggccccgcca cacacgatca ccatcacccc cgggctcccg acccagtaca 240
   aatagacacg cacactccca actccccacc catctccgcc gcgcacaccg cccaatcagc 300
   caatctcctc ctcctcctcc gctctcagac gagcagcggt tgccatcact ctccacttcc 360
   cacgcccgct gcgggctcgc aggcggcaga gaattgtctg tgccgccggg tgggaatttg 420
   attcggtcgg attccgtgcg ccgcg                                       445
<210> 33
   <211> 5455
   <212> DNA
   <213> Künstliche Sequenz
<220>
   <223> Beschreibung der künstlichen Sequenz: recombinant expression vector pUbiBI-1
<400> 33
   ggggatcctc tagagtcgac ctgcaggcgg ccgcactagt gattaggatt ccaacgcgag 60
   ccaggacaag cgaggaacct tgcgtgcgag gcgaggccgc cccgctccga ttcgattcga 120
   cgcgcaggcg caggcgcagg gatggacgcc ttctactcga cctcgtcggc ggcggcgagc 180
   ggctggggcc acgactccct caagaacttc cgccagatct cccccgccgt gcagtcccac 240
   ctcaagctcg tttacctgac tctatgcttt gcactggcct catctgccgt gggtgcttac 300
   ctacacattg ccctgaacat cggcgggatg ctgacaatgc tcgcttgtgt cggaactatc 360
   gcctggatgt tctcggtgcc agtctatgag gagaggaaga ggtttgggct gctgatgggt 420
   gcagccctcc tggaaggggc ttcggttgga cctctgattg agcttgccat agactttgac 480
   ccaagcatcc tcgtgacagg gtttgtcgga accgccatcg cctttgggtg cttctctggc 540
   gccgccatca tcgccaagcg cagggagtac ctgtacctcg gtggcctgct ctcgtctggc 600
   ctgtcgatcc tgctctggct gcagtttgtc acgtccatct ttggccactc ctctggcagc 660
   ttcatgtttg aggtttactt tggcctgttg atcttcctgg ggtacatggt gtacgacacg 720
   caggagatca tcgagagggc gcaccatggc gacatggact acatcaagca cgccctcacc 780
   ctcttcaccg actttgttgc cgtcctcgtc cgagtcctca tcatcatgct caagaacgca 840
   ggcgacaagt cggaggacaa gaagaagagg aagagggggt cctgaacgtw tctcccgcac 900
   atgtagatac cgtcaccgcg tcgacctgca ggcatgcccg ctgaaatcac cagtctctct 960
   ctacaaatct atctctctca taataatgtg tgagtagttc ccagataagg gaattagggt 1020
   tcttataggg tttcgctcat gtgttgagca tataagaaac ccttagtatg tatttgtatt 1080
   tgtaaaatac ttctatcaat aaaatttcta attcctaaaa ccaaaatcca gtgggtaccg 1140
   agctcgaatt caagcttggc actggccgtc gttttacaac gtcgtgactg ggaaaaccct 1200
   ggcgttaccc aacttaatcg ccttgcagca catccccctt tcgccagctg gcgtaatagc 1260
   gaagaggccc gcaccgatcg cccttcccaa cagttgcgca gcctgaatgg cgaatggcgc 1320
   ctgatgcggt attttctcct tacgcatctg tgcggtattt cacaccgcat atggtgcact 1380
   ctcagtacaa tctgctctga tgccgcatag ttaagccagc cccgacaccc gccaacaccc 1440
   gctgacgcgc cctgacgggc ttgtctgctc ccggcatccg cttacagaca agctgtgacc 1500
   gtctccggga gctgcatgtg tcagaggttt tcaccgtcat caccgaaacg cgcgagacga 1560
   aagggcctcg tgatacgcct atttttatag gttaatgtca tgataataat ggtttcttag 1620
   acgtcaggtg gcacttttcg gggaaatgtg cgcggaaccc ctatttgttt atttttctaa 1680
   atacattcaa atatgtatcc gctcatgaga caataaccct gataaatgct tcaataatat 1740
   tgaaaaagga agagtatgag tattcaacat ttccgtgtcg cccttattcc cttttttgcg 1800
   gcattttgcc ttcctgtttt tgctcaccca gaaacgctgg tgaaagtaaa agatgctgaa 1860
   gatcagttgg gtgcacgagt gggttacatc gaactggatc tcaacagcgg taagatcctt 1920
   gagagttttc gccccgaaga acgttttcca atgatgagca cttttaaagt tctgctatgt 1980
   ggcgcggtat tatcccgtat tgacgccggg caagagcaac tcggtcgccg catacactat 2040
   tctcagaatg acttggttga gtactcacca gtcacagaaa agcatcttac ggatggcatg 2100
   acagtaagag aattatgcag tgctgccata accatgagtg ataacactgc ggccaactta 2160
   cttctgacaa cgatcggagg accgaaggag ctaaccgctt ttttgcacaa catgggggat 2220
   catgtaactc gccttgatcg ttgggaaccg gagctgaatg aagccatacc aaacgacgag 2280
   cgtgacacca cgatgcctgt agcaatggca acaacgttgc gcaaactatt aactggcgaa 2340
   ctacttactc tagcttcccg gcaacaatta atagactgga tggaggcgga taaagttgca 2400
   ggaccacttc tgcgctcggc ccttccggct ggctggttta ttgctgataa atctggagcc 2460
   ggtgagcgtg ggtctcgcgg tatcattgca gcactggggc cagatggtaa gccctcccgt 2520
   atcgtagtta tctacacgac ggggagtcag gcaactatgg atgaacgaaa tagacagatc 2580
   gctgagatag gtgcctcact gattaagcat tggtaactgt cagaccaagt ttactcatat 2640
   atactttaga ttgatttaaa acttcatttt taatttaaaa ggatctaggt gaagatcctt 2700
   tttgataatc tcatgaccaa aatcccttaa cgtgagtttt cgttccactg agcgtcagac 2760
   cccgtagaaa agatcaaagg atcttcttga gatccttttt ttctgcgcgt aatctgctgc 2820
   ttgcaaacaa aaaaaccacc gctaccagcg gtggtttgtt tgccggatca agagctacca 2880
   actctttttc cgaaggtaac tggcttcagc agagcgcaga taccaaatac tgttcttcta 2940
   gtgtagccgt agttaggcca ccacttcaag aactctgtag caccgcctac atacctcgct 3000
   ctgctaatcc tgttaccagt ggctgctgcc agtggcgata agtcgtgtct taccgggttg 3060
   gactcaagac gatagttacc ggataaggcg cagcggtcgg gctgaacggg gggttcgtgc 3120
   acacagccca gcttggagcg aacgacctac accgaactga gatacctaca gcgtgagctt 3180
   tgagaaagcg ccacgcttcc cgaagggaga aaggcggaca ggtatccggt aagcggcagg 3240
   gtcggaacag gagagcgcac gagggagctt ccagggggaa acgcctggta tctttatagt 3300
   cctgtcgggt ttcgccacct ctgacttgag cgtcgatttt tgtgatgctc gtcagggggg 3360
   cggagcctat ggaaaaacgc cagcaacgcg gcctttttac ggttcctggc cttttgctgg 3420
   ccttttgctc acatgttctt tcctgcgtta tcccctgatt ctgtggataa ccgtattacc 3480
   gcctttgagt gagctgatac cgctcgccgc agccgaacga ccgagcgcag cgagtcagtg 3540
   agcgaggaag cggaagagcg cccaatacgc aaaccgcctc tccccgcgcg ttggccgatt 3600
   cattaatgca gctggcacga caggtttccc gactggaaag cgggcagtga gcgcaacgca 3660
   attaatgtga gttagctcac tcattaggca ccccaggctt tacactttat gcttccggct 3720
   cgtatgttgt gtggaattgt gagcggataa caatttcaca caggaaacag ctatgaccat 3780
   gattacgaat tcccatgcct cgaggatcta acatgcttag atacatgaag taacatgctg 3840
   ctacggttta ataattcttg agttgatttt tactggtact tagatagatg tatatacatg 3900
   cttagataca tgaagtaaca tgctcctaca gttcctttaa tcattattga gtacctatat 3960
   attctaataa atcagtatgt tttaaattat tttgatttta ctggtactta gatagatgta 4020
   tatatacatg ctcaaacatg cttagataca tgaagtaaca tgctgctacg gtttagtcat 4080
   tattgagtgc ctataatttc taataaatca gtatgtttta aattattttg attttactgg 4140
   tacttagata gatgtatata tacatgctca aacatgctta gatacatgaa gtaatatgct 4200
   actacggttt aattgttctt gagtacctat atattctaat aaatcagtat gttttaaatt 4260
   atttcgattt tactggtact tagatagatg tatatataca tgcttagata catgaagtaa 4320
   catgctacta cggtttaatt gttcttgaat acctatatat tctaataaat cagtatgttt 4380
   taaattattt cgattttact ggtacttaga tagatgtata tatacatgct cgaacatgct 4440
   tagatacatg aagtaacatg ctacatatat attataataa atcagtatgt cttaaattat 4500
   tttgatttta ctggtactta gatagatgta tatacatgct caaacatgct tagatacatg 4560
   aagtaacatg ctactacggt ttaatcatta ttgagtacct atatattcta ataaatcagt 4620
   atgttttcaa ttgttttgat tttactggta cttagatata tgtatatata catgctcgaa 4680
   catgcttaga tacgtgaagt aacatgctac tatggttaat tgttcttgag tacctatata 4740
   ttctaataaa tcagtatgtt ttaaattatt tcgattttac tggtacttag atagatgtat 4800
   atatacatgc tcgaacatgc ttagatacat gaagtaacat gctactacgg tttaatcgtt 4860
   cttgagtacc tatatattct aataaatcag tatgtcttaa attatcttga ttttactggt 4920
   acttagatag atgtatatac atgcttagat acatgaagta acatgctact atgatttaat 4980
   cgttcttgag tacctatata ttctaataaa tcagtatgtt tttaattatt ttgattttac 5040
   tggtacttag atagatgtat atatacatgc tcgaacatgc ttagatacat gaagtaacat 5100
   gctactacgg tttaatcatt cttgagtacc tatatattct aataaatcag tatgttttta 5160
   attattttga tattactggt acttaacatg tttagataca tcatatagca tgcacatgct 5220
   gctactgttt aatcattcgt gaatacctat atattctaat atatcagtat gtcttctaat 5280
   tattatgatt ttgatgtact tgtatggtgg catatgctgc agctatgtgt agattttgaa 5340
   tacccagtgt gatgagcatg catggcgcct tcatagttca tatgctgttt atttcctttg 5400
   agactgttct tttttgttga tagtcaccct gttgtttggt gattcttatg caccc      5455
<210> 34
   <211> 12633
   <212> DNA
   <213> Künstliche Sequenz
<220>
   <223> Beschreibung der künstlichen Sequenz: recombinant expression vector pLo114ubiBI-1
<400> 34
   aattcactgg ccgtcgtttt acaacgactc agagcttgac aggaggcccg atctagtaac 60
   atagatgaca ccgcgcgcga taatttatcc tagtttgcgc gctatatttt gttttctatc 120
   gcgtattaaa tgtataattg cgggactcta atcataaaaa cccatctcat aaataacgtc 180
   atgcattaca tgttaattat tacatgctta acgtaattca acagaaatta tatgataatc 240
   atcgcaagac cggcaacagg attcaatctt aagaaacttt attgccaaat gtttgaacga 300
   tcggggatca tccgggtctg tggcgggaac tccacgaaaa tatccgaacg cagcaagatc 360
   tagagcttgg gtcccgctca gaagaactcg tcaagaaggc gatagaaggc gatgcgctgc 420
   gaatcgggag cggcgatacc gtaaagcacg aggaagcggt cagcccattc gccgccaagc 480
   tcttcagcaa tatcacgggt agccaacgct atgtcctgat agcggtccgc cacacccagc 540
   cggccacagt cgatgaatcc agaaaagcgg ccattttcca ccatgatatt cggcaagcag 600
   gcatcgccat gggtcacgac gagatcctcg ccgtcgggca tgcgcgcctt gagcctggcg 660
   aacagttcgg ctggcgcgag cccctgatgc tcttcgtcca gatcatcctg atcgacaaga 720
   ccggcttcca tccgagtacg tgctcgctcg atgcgatgtt tcgcttggtg gtcgaatggg 780
   caggtagccg gatcaagcgt atgcagccgc cgcattgcat cagccatgat ggatactttc 840
   tcggcaggag caaggtgaga tgacaggaga tcctgccccg gcacttcgcc caatagcagc 900
   cagtcccttc ccgcttcagt gacaacgtcg agcacagctg cgcaaggaac gcccgtcgtg 960
   gccagccacg atagccgcgc tgcctcgtcc tgcagttcat tcagggcacc ggacaggtcg 1020
   gtcttgacaa aaagaaccgg gcgcccctgc gctgacagcc ggaacacggc ggcatcagag 1080
   cagccgattg tctgttgtgc ccagtcatag ccgaatagcc tctccaccca agcggccgga 1140
   gaacctgcgt gcaatccatc ttgttcaatc atgcgaaacg atccagatcc ggtgcagatt 1200
   atttggattg agagtgaata tgagactcta attggatacc gaggggaatt tatggaacgt 1260
   cagtggagca tttttgacaa gaaatatttg ctagctgata gtgaccttag gcgacttttg 1320
   aacgcgcaat aatggtttct gacgtatgtg cttagctcat taaactccag aaacccgcgg 1380
   ctgagtggct ccttcaacgt tgcggttctg tcagttccaa acgtaaaacg gcttgtcccg 1440
   cgtcatcggc gggggtcata acgtgactcc cttaattctc cgctcatgat cagattgtcg 1500
   tttcccgcct tcagtttaaa ctatcagtgt ttgacaggat cctgcttggt aataattgtc 1560
   attagattgt ttttatgcat agatgcactc gaaatcagcc aattttagac aagtatcaaa 1620
   cggatgttaa ttcagtacat taaagacgtc cgcaatgtgt tattaagttg tctaagcgtc 1680
   aatttgttta caccacaata tatcctgcca ccagccagcc aacagctccc cgaccggcag 1740
   ctcggcacaa aatcaccacg cgttaccacc acgccggccg gccgcatggt gttgaccgtg 1800
   ttcgccggca ttgccgagtt cgagcgttcc ctaatcatcg accgcacccg gagcgggcgc 1860
   gaggccgcca aggcccgagg cgtgaagttt ggcccccgcc ctaccctcac cccggcacag 1920
   atcgcgcacg cccgcgagct gatcgaccag gaaggccgca ccgtgaaaga ggcggctgca 1980
   ctgcttggcg tgcatcgctc gaccctgtac cgcgcacttg agcgcagcga ggaagtgacg 2040
   cccaccgagg ccaggcggcg cggtgccttc cgtgaggacg cattgaccga ggccgacgcc 2100
   ctggcggccg ccgagaatga acgccaagag gaacaagcat gaaaccgcac caggacggcc 2160
   aggacgaacc gtttttcatt accgaagaga tcgaggcgga gatgatcgcg gccgggtacg 2220
   tgttcgagcc gcccgcgcac gtctcaaccg tgcggctgca tgaaatcctg gccggtttgt 2280
   ctgatgccaa gctggcggcc tggccggcca gcttggccgc tgaagaaacc gagcgccgcc 2340
   gtctaaaaag gtgatgtgta tttgagtaaa acagcttgcg tcatgcggtc gctgcgtata 2400
   tgatgcgatg agtaaataaa caaatacgca aggggaacgc atgaaggtta tcgctgtact 2460
   taaccagaaa ggcgggtcag gcaagacgac catcgcaacc catctagccc gcgccctgca 2520
   actcgccggg gccgatgttc tgttagtcga ttccgatccc cagggcagtg cccgcgattg 2580
   ggcggccgtg cgggaagatc aaccgctaac cgttgtcggc atcgaccgcc cgacgattga 2640
   ccgcgacgtg aaggccatcg gccggcgcga cttcgtagtg atcgacggag cgccccaggc 2700
   ggcggacttg gctgtgtccg cgatcaaggc agccgacttc gtgctgattc cggtgcagcc 2760
   aagcccttac gacatatggg ccaccgccga cctggtggag ctggttaagc agcgcattga 2820
   ggtcacggat ggaaggctac aagcggcctt tgtcgtgtcg cgggcgatca aaggcacgcg 2880
   catcggcggt gaggttgccg aggcgctggc cgggtacgag ctgcccattc ttgagtcccg 2940
   tatcacgcag cgcgtgagct acccaggcac tgccgccgcc ggcacaaccg ttcttgaatc 3000
   agaacccgag ggcgacgctg cccgcgaggt ccaggcgctg gccgctgaaa ttaaatcaaa 3060
   actcatttga gttaatgagg taaagagaaa atgagcaaaa gcacaaacac gctaagtgcc 3120
   ggccgtccga gcgcacgcag cagcaaggct gcaacgttgg ccagcctggc agacacgcca 3180
   gccatgaagc gggtcaactt tcagttgccg gcggaggatc acaccaagct gaagatgtac 3240
   gcggtacgcc aaggcaagac cattaccgag ctgctatctg aatacatcgc gcagctacca 3300
   gagtaaatga gcaaatgaat aaatgagtag atgaatttta gcggctaaag gaggcggcat 3360
   ggaaaatcaa gaacaaccag gcaccgacgc cgtggaatgc cccatgtgtg gaggaacggg 3420
   cggttggcca ggcgtaagcg gctgggttgt ctgccggccc tgcaatggca ctggaacccc 3480
   caagcccgag gaatcggcgt gagcggtcgc aaaccatccg gcccggtaca aatcggcgcg 3540
   gcgctgggtg atgacctggt ggagaagttg aaggccgcgc aggccgccca gcggcaacgc 3600
   atcgaggcag aagcacgccc cggtgaatcg tggcaagcgg ccgctgatcg aatccgcaaa 3660
   gaatcccggc aaccgccggc agccggtgcg ccgtcgatta ggaagccgcc caagggcgac 3720
   gagcaaccag attttttcgt tccgatgctc tatgacgtgg gcacccgcga tagtcgcagc 3780
   atcatggacg tggccgtttt ccgtctgtcg aagcgtgacc gacgagctgg cgaggtgatc 3840
   cgctacgagc ttccagacgg gcacgtagag gtttccgcag ggccggccgg catggccagt 3900
   gtgtgggatt acgacctggt actgatggcg gtttcccatc taaccgaatc catgaaccga 3960
   taccgggaag ggaagggaga caagcccggc cgcgtgttcc gtccacacgt tgcggacgta 4020
   ctcaagttct gccggcgagc cgatggcgga aagcagaaag acgacctggt agaaacctgc 4080
   attcggttaa acaccacgca cgttgccatg cagcgtacga agaaggccaa gaacggccgc 4140
   ctggtgacgg tatccgaggg tgaagccttg attagccgct acaagatcgt aaagagcgaa 4200
   accgggcggc cggagtacat cgagatcgag ctagctgatt ggatgtaccg cgagatcaca 4260
   gaaggcaaga acccggacgt gctgacggtt caccccgatt actttttgat cgatcccggc 4320
   atcggccgtt ttctctaccg cctggcacgc cgcgccgcag gcaaggcaga agccagatgg 4380
   ttgttcaaga cgatctacga acgcagtggc agcgccggag agttcaagaa gttctgtttc 4440
   accgtgcgca agctgatcgg gtcaaatgac ctgccggagt acgatttgaa ggaggaggcg 4500
   gggcaggctg gcccgatcct agtcatgcgc taccgcaacc tgatcgaggg cgaagcatcc 4560
   gccggttcct aatgtacgga gcagatgcta gggcaaattg ccctagcagg ggaaaaaggt 4620
   cgaaaaggtc tctttcctgt ggatagcacg tacattggga acccaaagcc gtacattggg 4680
   aaccggaacc cgtacattgg gaacccaaag ccgtacattg ggaaccggtc acacatgtaa 4740
   gtgactgata taaaagagaa aaaaggcgat ttttccgcct aaaactcttt aaaacttatt 4800
   aaaactctta aaacccgcct ggcctgtgca taactgtctg gccagcgcac agccgaagag 4860
   ctgcaaaaag cgcctaccct tcggtcgctg cgctccctac gccccgccgc ttcgcgtcgg 4920
   cctatcgcgg ccgctggccg ctcaaaaatg gctggcctac ggccaggcaa tctaccaggg 4980
   cgcggacaag ccgcgccgtc gccactcgac cgccggcgcc cacatcaagg caccctgcct 5040
   cgcgcgtttc ggtgatgacg gtgaaaacct ctgacacatg cagctcccgg agacggtcac 5100
   agcttgtctg taagcggatg ccgggagcag acaagcccgt cagggcgcgt cagcgggtgt 5160
   tggcgggtgt cggggcgcag ccatgaccca gtcacgtagc gatagcggag tgtatactgg 5220
   cttaactatg cggcatcaga gcagattgta ctgagagtgc accatatgcg gtgtgaaata 5280
   ccgcacagat gcgtaaggag aaaataccgc atcaggcgct cttccgcttc ctcgctcact 5340
   gactcgctgc gctcggtcgt tcggctgcgg cgagcggtat cagctcactc aaaggcggta 5400
   atacggttat ccacagaatc aggggataac gcaggaaaga acatgtgagc aaaaggccag 5460
   caaaaggcca ggaaccgtaa aaaggccgcg ttgctggcgt ttttccatag gctccgcccc 5520
   cctgacgagc atcacaaaaa tcgacgctca agtcagaggt ggcgaaaccc gacaggacta 5580
   taaagatacc aggcgtttcc ccctggaagc tccctcgtgc gctctcctgt tccgaccctg 5640
   ccgcttaccg gatacctgtc cgcctttctc ccttcgggaa gcgtggcgct ttctcatagc 5700
   tcacgctgta ggtatctcag ttcggtgtag gtcgttcgct ccaagctggg ctgtgtgcac 5760
   gaaccccccg ttcagcccga ccgctgcgcc ttatccggta actatcgtct tgagtccaac 5820
   ccggtaagac acgacttatc gccactggca gcagccactg gtaacaggat tagcagagcg 5880
   aggtatgtag gcggtgctac agagttcttg aagtggtggc ctaactacgg ctacactaga 5940
   aggacagtat ttggtatctg cgctctgctg aagccagtta ccttcggaaa aagagttggt 6000
   agctcttgat ccggcaaaca aaccaccgct ggtagcggtg gtttttttgt ttgcaagcag 6060
   cagattacgc gcagaaaaaa aggatctcaa gaagatcctt tgatcttttc tacggggtct 6120
   gacgctcagt ggaacgaaaa ctcacgttaa gggattttgg tcatgcatga tatatctccc 6180
   aatttgtgta gggcttatta tgcacgctta aaaataataa aagcagactt gacctgatag 6240
   tttggctgtg agcaattatg tgcttagtgc atctaacgct tgagttaagc cgcgccgcga 6300
   agcggcgtcg gcttgaacga atttctagct agacattatt tgccgactac cttggtgatc 6360
   tcgcctttca cgtagtggac aaattcttcc aactgatctg cgcgcgaggc caagcgatct 6420
   tcttcttgtc caagataagc ctgtctagct tcaagtatga cgggctgata ctgggccggc 6480
   aggcgctcca ttgcccagtc ggcagcgaca tccttcggcg cgattttgcc ggttactgcg 6540
   ctgtaccaaa tgcgggacaa cgtaagcact acatttcgct catcgccagc ccagtcgggc 6600
   ggcgagttcc atagcgttaa ggtttcattt agcgcctcaa atagatcctg ttcaggaacc 6660
   ggatcaaaga gttcctccgc cgctggacct accaaggcaa cgctatgttc tcttgctttt 6720
   gtcagcaaga tagccagatc aatgtcgatc gtggctggct cgaagatacc tgcaagaatg 6780
   tcattgcgct gccattctcc aaattgcagt tcgcgcttag ctggataacg ccacggaatg 6840
   atgtcgtcgt gcacaacaat ggtgacttct acagcgcgga gaatctcgct ctctccaggg 6900
   gaagccgaag tttccaaaag gtcgttgatc aaagctcgcc gcgttgtttc atcaagcctt 6960
   acggtcaccg taaccagcaa atcaatatca ctgtgtggct tcaggccgcc atccactgcg 7020
   gagccgtaca aatgtacggc cagcaacgtc ggttcgagat ggcgctcgat gacgccaact 7080
   acctctgata gttgagtcga tacttcggcg atcaccgctt cccccatgat gtttaacttt 7140
   gttttagggc gactgccctg ctgcgtaaca tcgttgctgc tccataacat caaacatcga 7200
   cccacggcgt aacgcgcttg ctgcttggat gcccgaggca tagactgtac cccaaaaaaa 7260
   cagtcataac aagccatgaa aaccgccact gcgggggttc catggacata caaatggacg 7320
   aacggataaa ccttttcacg cccttttaaa tatccgatta ttctaataaa cgctcttttc 7380
   tcttaggttt acccgccaat atatcctgtc aaacactgat agtttaaact gaaggcggga 7440
   aacgacaatc agatctagta ggaaacagct atgaccatga ttacgccaag cttgcatgcc 7500
   tgcaggtcga ctctagagga tcgatccccg ggtaggtcag tcccttatgt tacgtcctgt 7560
   agaaacccca acccgtgaaa tcaaaaaact cgacggcctg tgggcattca gtctggatcg 7620
   cgaaaactgt ggaattggtc agcgttggtg ggaaagcgcg ttacaagaaa gccgggcaat 7680
   tgctgtgcca ggcagtttta acgatcagtt cgccgatgca gatattcgta attatgcggg 7740
   caacgtctgg tatcagcgcg aagtctttat accgaaaggt tgggcaggcc agcgtatcgt 7800
   gctgcgtttc gatgcggtca ctcattacgg caaagtgtgg gtcaataatc aggaagtgat 7860
   ggagcatcag ggcggctata cgccatttga agccgatgtc acgccgtatg ttattgccgg 7920
   gaaaagtgta cgtaagtttc tgcttctacc tttgatatat atataataat tatcattaat 7980
   tagtagtaat ataatatttc aaatattttt ttcaaaataa aagaatgtag tatatagcaa 8040
   ttgcttttct gtagtttata agtgtgtata ttttaattta taacttttct aatatatgac 8100
   caaaatttgt tgatgtgcag gtatcaccgt ttgtgtgaac aacgaactga actggcagac 8160
   tatcccgccg ggaatggtga ttaccgacga aaacggcaag aaaaagcagt cttacttcca 8220
   tgatttcttt aactatgccg gaatccatcg cagcgtaatg ctctacacca cgccgaacac 8280
   ctgggtggac gatatcaccg tggtgacgca tgtcgcgcaa gactgtaacc acgcgtctgt 8340
   tgactggcag gtggtggcca atggtgatgt cagcgttgaa ctgcgtgatg cggatcaaca 8400
   ggtggttgca actggacaag gcactagcgg gactttgcaa gtggtgaatc cgcacctctg 8460
   gcaaccgggt gaaggttatc tctatgaact gtgcgtcaca gccaaaagcc agacagagtg 8520
   tgatatctac ccgcttcgcg tcggcatccg gtcagtggca gtgaagggcg aacagttcct 8580
   gattaaccac aaaccgttct actttactgg ctttggtcgt catgaagatg cggacttgcg 8640
   tggcaaagga ttcgataacg tgctgatggt gcacgaccac gcattaatgg actggattgg 8700
   ggccaactcc taccgtacct cgcattaccc ttacgctgaa gagatgctcg actgggcaga 8760
   tgaacatggc atcgtggtga ttgatgaaac tgctgctgtc ggctttaacc tctctttagg 8820
   cattggtttc gaagcgggca acaagccgaa agaactgtac agcgaagagg cagtcaacgg 8880
   ggaaactcag caagcgcact tacaggcgat taaagagctg atagcgcgtg acaaaaacca 8940
   cccaagcgtg gtgatgtgga gtattgccaa cgaaccggat acccgtccgc aaggtgcacg 9000
   ggaatatttc gcgccactgg cggaagcaac gcgtaaactc gacccgacgc gtccgatcac 9060
   ctgcgtcaat gtaatgttct gcgacgctca caccgatacc atcagcgatc tctttgatgt 9120
   gctgtgcctg aaccgttatt acggatggta tgtccaaagc ggcgatttgg aaacggcaga 9180
   gaaggtactg gaaaaagaac ttctggcctg gcaggagaaa ctgcatcagc cgattatcat 9240
   caccgaatac ggcgtggata cgttagccgg gctgcactca atgtacaccg acatgtggag 9300
   tgaagagtat cagtgtgcat ggctggatat gtatcaccgc gtctttgatc gcgtcagcgc 9360
   cgtcgtcggt gaacaggtat ggaatttcgc cgattttgcg acctcgcaag gcatattgcg 9420
   cgttggcggt aacaagaaag ggatcttcac tcgcgaccgc aaaccgaagt cggcggcttt 9480
   tctgctgcaa aaacgctgga ctggcatgaa cttcggtgaa aaaccgcagc agggaggcaa 9540
   acaatgagag ctcgaatttc cccgatcggt caaacatttg gcaataaagn ttcttaagat 9600
   tgaatcctgt tgccggtctt gcgatgatta tcatataatt tctgttgaat tacgttaagc 9660
   atgtaataat taacatgtaa tgcatgacgt tatttatgag atgggttttt atgattagag 9720
   tcccgcaatt atacatttaa tacgcgatag aaaacaaaat atancgcgca aactaggata 9780
   aattatcgcg cgcggtgtca tctatgttac tagatcggga attcccatgc ctcgaggatc 9840
   taacatgctt agatacatga agtaacatgc tgctacggtt taataattct tgagttgatt 9900
   tttactggta cttagataga tgtatataca tgcttagata catgaagtaa catgctccta 9960
   cagttccttt aatcattatt gagtacctat atattctaat aaatcagtat gttttaaatt 10020
   attttgattt tactggtact tagatagatg tatatataca tgctcaaaca tgcttagata 10080
   catgaagtaa catgctgcta cggtttagtc attattgagt gcctataatt tctaataaat 10140
   cagtatgttt taaattattt tgattttact ggtacttaga tagatgtata tatacatgct 10200
   caaacatgct tagatacatg aagtaatatg ctactacggt ttaattgttc ttgagtacct 10260
   atatattcta ataaatcagt atgttttaaa ttatttcgat tttactggta cttagataga 10320
   tgtatatata catgcttaga tacatgaagt aacatgctac tacggtttaa ttgttcttga 10380
   atacctatat attctaataa atcagtatgt tttaaattat ttcgatttta ctggtactta 10440
   gatagatgta tatatacatg ctcgaacatg cttagataca tgaagtaaca tgctacatat 10500
   atattataat aaatcagtat gtcttaaatt attttgattt tactggtact tagatagatg 10560
   tatatacatg ctcaaacatg cttagataca tgaagtaaca tgctactacg gtttaatcat 10620
   tattgagtac ctatatattc taataaatca gtatgttttc aattgttttg attttactgg 10680
   tacttagata tatgtatata tacatgctcg aacatgctta gatacgtgaa gtaacatgct 10740
   actatggtta attgttcttg agtacctata tattctaata aatcagtatg ttttaaatta 10800
   tttcgatttt actggtactt agatagatgt atatatacat gctcgaacat gcttagatac 10860
   atgaagtaac atgctactac ggtttaatcg ttcttgagta cctatatatt ctaataaatc 10920
   agtatgtctt aaattatctt gattttactg gtacttagat agatgtatat acatgcttag 10980
   atacatgaag taacatgcta ctatgattta atcgttcttg agtacctata tattctaata 11040
   aatcagtatg tttttaatta ttttgatttt actggtactt agatagatgt atatatacat 11100
   gctcgaacat gcttagatac atgaagtaac atgctactac ggtttaatca ttcttgagta 11160
   cctatatatt ctaataaatc agtatgtttt taattatttt gatattactg gtacttaaca 11220
   tgtttagata catcatatag catgcacatg ctgctactgt ttaatcattc gtgaatacct 11280
   atatattcta atatatcagt atgtcttcta attattatga ttttgatgta cttgtatggt 11340
   ggcatatgct gcagctatgt gtagattttg aatacccagt gtgatgagca tgcatggcgc 11400
   cttcatagtt catatgctgt ttatttcctt tgagactgtt cttttttgtt gatagtcacc 11460
   ctgttgtttg gtgattctta tgcacccggg gatcctctag agtcgacctg caggcggccg 11520
   cactagtgat taggattcca acgcgagcca ggacaagcga ggaaccttgc gtgcgaggcg 11580
   aggccgcccc gctccgattc gattcgacgc gcaggcgcag gcgcagggat ggacgccttc 11640
   tactcgacct cgtcggcggc ggcgagcggc tggggccacg actccctcaa gaacttccgc 11700
   cagatctccc ccgccgtgca gtcccacctc aagctcgttt acctgactct atgctttgca 11760
   ctggcctcat ctgccgtggg tgcttaccta cacattgccc tgaacatcgg cgggatgctg 11820
   acaatgctcg cttgtgtcgg aactatcgcc tggatgttct cggtgccagt ctatgaggag 11880
   aggaagaggt ttgggctgct gatgggtgca gccctcctgg aaggggcttc ggttggacct 11940
   ctgattgagc ttgccataga ctttgaccca agcatcctcg tgacagggtt tgtcggaacc 12000
   gccatcgcct ttgggtgctt ctctggcgcc gccatcatcg ccaagcgcag ggagtacctg 12060
   tacctcggtg gcctgctctc gtctggcctg tcgatcctgc tctggctgca gtttgtcacg 12120
   tccatctttg gccactcctc tggcagcttc atgtttgagg tttactttgg cctgttgatc 12180
   ttcctggggt acatggtgta cgacacgcag gagatcatcg agagggcgca ccatggcgac 12240
   atggactaca tcaagcacgc cctcaccctc ttcaccgact ttgttgccgt cctcgtccga 12300
   gtcctcatca tcatgctcaa gaacgcaggc gacaagtcgg aggacaagaa gaagaggaag 12360
   agggggtcct gaacgtwtct cccgcacatg tagataccgt caccgcgtcg acctgcaggc 12420
   atgcccgctg aaatcaccag tctctctcta caaatctatc tctctcataa taatgtgtga 12480
   gtagttccca gataagggaa ttagggttct tatagggttt cgctcatgtg ttgagcatat 12540
   aagaaaccct tagtatgtat ttgtatttgt aaaatacttc tatcaataaa atttctaatt 12600
   cctaaaacca aaatccagtg ggtaccgagc tcg                              12633
<210> 35
   <211> 5598
   <212> DNA
   <213> Künstliche Sequenz
<220>
   <223> Beschreibung der künstlichen Sequenz: recombinant expression vector pOXoBI-1
<400> 35
   ggggatcctc tagagtcgac ctgcaggcgg ccgcactagt gattaggatt ccaacgcgag 60
   ccaggacaag cgaggaacct tgcgtgcgag gcgaggccgc cccgctccga ttcgattcga 120
   cgcgcaggcg caggcgcagg gatggacgcc ttctactcga cctcgtcggc ggcggcgagc 180
   ggctggggcc acgactccct caagaacttc cgccagatct cccccgccgt gcagtcccac 240
   ctcaagctcg tttacctgac tctatgcttt gcactggcct catctgccgt gggtgcttac 300
   ctacacattg ccctgaacat cggcgggatg ctgacaatgc tcgcttgtgt cggaactatc 360
   gcctggatgt tctcggtgcc agtctatgag gagaggaaga ggtttgggct gctgatgggt 420
   gcagccctcc tggaaggggc ttcggttgga cctctgattg agcttgccat agactttgac 480
   ccaagcatcc tcgtgacagg gtttgtcgga accgccatcg cctttgggtg cttctctggc 540
   gccgccatca tcgccaagcg cagggagtac ctgtacctcg gtggcctgct ctcgtctggc 600
   ctgtcgatcc tgctctggct gcagtttgtc acgtccatct ttggccactc ctctggcagc 660
   ttcatgtttg aggtttactt tggcctgttg atcttcctgg ggtacatggt gtacgacacg 720
   caggagatca tcgagagggc gcaccatggc gacatggact acatcaagca cgccctcacc 780
   ctcttcaccg actttgttgc cgtcctcgtc cgagtcctca tcatcatgct caagaacgca 840
   ggcgacaagt cggaggacaa gaagaagagg aagagggggt cctgaacgtw tctcccgcac 900
   atgtagatac cgtcaccgcg tcgacctgca ggcatgcccg ctgaaatcac cagtctctct 960
   ctacaaatct atctctctca taataatgtg tgagtagttc ccagataagg gaattagggt 1020
   tcttataggg tttcgctcat gtgttgagca tataagaaac ccttagtatg tatttgtatt 1080
   tgtaaaatac ttctatcaat aaaatttcta attcctaaaa ccaaaatcca gtgggtaccg 1140
   agctcgaatt caagcttggc actggccgtc gttttacaac gtcgtgactg ggaaaaccct 1200
   ggcgttaccc aacttaatcg ccttgcagca catccccctt tcgccagctg gcgtaatagc 1260
   gaagaggccc gcaccgatcg cccttcccaa cagttgcgca gcctgaatgg cgaatggcgc 1320
   ctgatgcggt attttctcct tacgcatctg tgcggtattt cacaccgcat atggtgcact 1380
   ctcagtacaa tctgctctga tgccgcatag ttaagccagc cccgacaccc gccaacaccc 1440
   gctgacgcgc cctgacgggc ttgtctgctc ccggcatccg cttacagaca agctgtgacc 1500
   gtctccggga gctgcatgtg tcagaggttt tcaccgtcat caccgaaacg cgcgagacga 1560
   aagggcctcg tgatacgcct atttttatag gttaatgtca tgataataat ggtttcttag 1620
   acgtcaggtg gcacttttcg gggaaatgtg cgcggaaccc ctatttgttt atttttctaa 1680
   atacattcaa atatgtatcc gctcatgaga caataaccct gataaatgct tcaataatat 1740
   tgaaaaagga agagtatgag tattcaacat ttccgtgtcg cccttattcc cttttttgcg 1800
   gcattttgcc ttcctgtttt tgctcaccca gaaacgctgg tgaaagtaaa agatgctgaa 1860
   gatcagttgg gtgcacgagt gggttacatc gaactggatc tcaacagcgg taagatcctt 1920
   gagagttttc gccccgaaga acgttttcca atgatgagca cttttaaagt tctgctatgt 1980
   ggcgcggtat tatcccgtat tgacgccggg caagagcaac tcggtcgccg catacactat 2040
   tctcagaatg acttggttga gtactcacca gtcacagaaa agcatcttac ggatggcatg 2100
   acagtaagag aattatgcag tgctgccata accatgagtg ataacactgc ggccaactta 2160
   cttctgacaa cgatcggagg accgaaggag ctaaccgctt ttttgcacaa catgggggat 2220
   catgtaactc gccttgatcg ttgggaaccg gagctgaatg aagccatacc aaacgacgag 2280
   cgtgacacca cgatgcctgt agcaatggca acaacgttgc gcaaactatt aactggcgaa 2340
   ctacttactc tagcttcccg gcaacaatta atagactgga tggaggcgga taaagttgca 2400
   ggaccacttc tgcgctcggc ccttccggct ggctggttta ttgctgataa atctggagcc 2460
   ggtgagcgtg ggtctcgcgg tatcattgca gcactggggc cagatggtaa gccctcccgt 2520
   atcgtagtta tctacacgac ggggagtcag gcaactatgg atgaacgaaa tagacagatc 2580
   gctgagatag gtgcctcact gattaagcat tggtaactgt cagaccaagt ttactcatat 2640
   atactttaga ttgatttaaa acttcatttt taatttaaaa ggatctaggt gaagatcctt 2700
   tttgataatc tcatgaccaa aatcccttaa cgtgagtttt cgttccactg agcgtcagac 2760
   cccgtagaaa agatcaaagg atcttcttga gatccttttt ttctgcgcgt aatctgctgc 2820
   ttgcaaacaa aaaaaccacc gctaccagcg gtggtttgtt tgccggatca agagctacca 2880
   actctttttc cgaaggtaac tggcttcagc agagcgcaga taccaaatac tgttcttcta 2940
   gtgtagccgt agttaggcca ccacttcaag aactctgtag caccgcctac atacctcgct 3000
   ctgctaatcc tgttaccagt ggctgctgcc agtggcgata agtcgtgtct taccgggttg 3060
   gactcaagac gatagttacc ggataaggcg cagcggtcgg gctgaacggg gggttcgtgc 3120
   acacagccca gcttggagcg aacgacctac accgaactga gatacctaca gcgtgagctt 3180
   tgagaaagcg ccacgcttcc cgaagggaga aaggcggaca ggtatccggt aagcggcagg 3240
   gtcggaacag gagagcgcac gagggagctt ccagggggaa acgcctggta tctttatagt 3300
   cctgtcgggt ttcgccacct ctgacttgag cgtcgatttt tgtgatgctc gtcagggggg 3360
   cggagcctat ggaaaaacgc cagcaacgcg gcctttttac ggttcctggc cttttgctgg 3420
   ccttttgctc acatgttctt tcctgcgtta tcccctgatt ctgtggataa ccgtattacc 3480
   gcctttgagt gagctgatac cgctcgccgc agccgaacga ccgagcgcag cgagtcagtg 3540
   agcgaggaag cggaagagcg cccaatacgc aaaccgcctc tccccgcgcg ttggccgatt 3600
   cattaatgca gctggcacga caggtttccc gactggaaag cgggcagtga gcgcaacgca 3660
   attaatgtga gttagctcac tcattaggca ccccaggctt tacactttat gcttccggct 3720
   cgtatgttgt gtggaattgt gagcggataa caatttcaca caggaaacag ctatgaccat 3780
   gattacgaat tcccatgcct cgagcagaaa gatataatat gtaaaaaaat gggtctatat 3840
   atatggaagg tttcaggaag acaaaggttc tagaaacttc caaaaaaaat ccagaatata 3900
   ttttggaaga aataccctct tgggttggcc ccggcgcagc ccctagtggg ccaaaaagcc 3960
   acgatctaat cccggtctaa ttggtctaat agtttagact tctaattaga cgggctctta 4020
   tgccggtcta attggtctaa ttagattaaa atcctaatta aatatgaacg caactaggct 4080
   tcccctctct ctagttttct cggagctctt tttcatggac cttgaagtat tgccggatca 4140
   ctacttcgga actcgtggat acttcagagt gcacatctac tttgaatctt gattggtaga 4200
   tcatctcgga gaaattctca cagttgggag gtataaccag ttgccgaaat tgccatgctt 4260
   cactcacagc caggatcagc ccatgtccca aggcaaccct tgtagctaca tgccgaggcc 4320
   tgactacttg gggcctcgcg ccctgcattt ttgcatgttc atgtgacacg ttaaatgttg 4380
   agagaaatag attactaaat atcacccatt tcgttattct agatgagtat cctacaatat 4440
   gtataccgaa aaatgtattt taaactgtgg taggtgagaa agatctatta aaaagaactc 4500
   tacgtatact cccccctccc aatccccatc caggtttgta agacactttc gtcttttttt 4560
   gccgaatttt aaccgtaaat ttgactagta aaaataagtt atactgaatg taataaatat 4620
   cgtacattcg gatgttggag acagggagag gctggctggt gcgctggatg gatcacggtc 4680
   agaaagtctg acttgcaacg ccacaggccc gttgattgcc actgacaacc aagttttcgt 4740
   tgtttcgctg gtgccatatt ttccgcgatc gaatatttaa actgcgagga gaaaggcaag 4800
   cagggcgcca tatcagcact tgatcactca ctgatcgatc agtagtagcc accttctctg 4860
   cgccgacgtg ttatatatta ttggcaacaa gtcatcgatt gagaacagaa acaaaacaag 4920
   aagagaacta tttgagagag agtagttacg ccgcagcgag tagcctccca tttctgacga 4980
   tcatgccata cgataaaccg gccggcggcg agaccagtta gcaaggttga aatgccaaca 5040
   catgtcgcgc tcatttctcg gctttttcat tttgcatgtc gtcatgcagg ccctggacac 5100
   tgacatttct ctcttttgct gttgaatgaa gaccctaacc tttcaccatc agcacgcccc 5160
   tcaacttgat aagcctagac gaaacccata tgcatgattg atgagtaatg gtgtgcacga 5220
   atattatgaa cccgtttcca agagcaatac tccattgaga tacacctcct ccttgtatct 5280
   gttcgttggt cccatttcca tagcagccgg cagtggcctt gactctgact gccacgcaag 5340
   taatatatct ttaataaact cgctgccttg cttcgtgtgt ccatttgcaa atgcatgcag 5400
   tgacgacatg cacatgcata gcttaattag ctccatgcat ccactgcttc cattaatccc 5460
   ctatataaag gactccatat gcctcaccat tcactcatcc accacagctt agcagcagca 5520
   acaaccagtg ccatagacac tctccatcaa caaactctag ctgatcaatc ctagctaagc 5580
   ttattacata gcaagccc                                                5598
<210> 36
   <211> 12776
   <212> DNA
   <213> Künstliche Sequenz
<220>
   <223> Beschreibung der künstlichen Sequenz : recombinant expression vector pLo114OxoBI-1
<400> 36
   aattcactgg ccgtcgtttt acaacgactc agagcttgac aggaggcccg atctagtaac 60
   atagatgaca ccgcgcgcga taatttatcc tagtttgcgc gctatatttt gttttctatc 120
   gcgtattaaa tgtataattg cgggactcta atcataaaaa cccatctcat aaataacgtc 180
   atgcattaca tgttaattat tacatgctta acgtaattca acagaaatta tatgataatc 240
   atcgcaagac cggcaacagg attcaatctt aagaaacttt attgccaaat gtttgaacga 300
   tcggggatca tccgggtctg tggcgggaac tccacgaaaa tatccgaacg cagcaagatc 360
   tagagcttgg gtcccgctca gaagaactcg tcaagaaggc gatagaaggc gatgcgctgc 420
   gaatcgggag cggcgatacc gtaaagcacg aggaagcggt cagcccattc gccgccaagc 480
   tcttcagcaa tatcacgggt agccaacgct atgtcctgat agcggtccgc cacacccagc 540
   cggccacagt cgatgaatcc agaaaagcgg ccattttcca ccatgatatt cggcaagcag 600
   gcatcgccat gggtcacgac gagatcctcg ccgtcgggca tgcgcgcctt gagcctggcg 660
   aacagttcgg ctggcgcgag cccctgatgc tcttcgtcca gatcatcctg atcgacaaga 720
   ccggcttcca tccgagtacg tgctcgctcg atgcgatgtt tcgcttggtg gtcgaatggg 780
   caggtagccg gatcaagcgt atgcagccgc cgcattgcat cagccatgat ggatactttc 840
   tcggcaggag caaggtgaga tgacaggaga tcctgccccg gcacttcgcc caatagcagc 900
   cagtcccttc ccgcttcagt gacaacgtcg agcacagctg cgcaaggaac gcccgtcgtg 960
   gccagccacg atagccgcgc tgcctcgtcc tgcagttcat tcagggcacc ggacaggtcg 1020
   gtcttgacaa aaagaaccgg gcgcccctgc gctgacagcc ggaacacggc ggcatcagag 1080
   cagccgattg tctgttgtgc ccagtcatag ccgaatagcc tctccaccca agcggccgga 1140
   gaacctgcgt gcaatccatc ttgttcaatc atgcgaaacg atccagatcc ggtgcagatt 1200
   atttggattg agagtgaata tgagactcta attggatacc gaggggaatt tatggaacgt 1260
   cagtggagca tttttgacaa gaaatatttg ctagctgata gtgaccttag gcgacttttg 1320
   aacgcgcaat aatggtttct gacgtatgtg cttagctcat taaactccag aaacccgcgg 1380
   ctgagtggct ccttcaacgt tgcggttctg tcagttccaa acgtaaaacg gcttgtcccg 1440
   cgtcatcggc gggggtcata acgtgactcc cttaattctc cgctcatgat cagattgtcg 1500
   tttcccgcct tcagtttaaa ctatcagtgt ttgacaggat cctgcttggt aataattgtc 1560
   attagattgt ttttatgcat agatgcactc gaaatcagcc aattttagac aagtatcaaa 1620
   cggatgttaa ttcagtacat taaagacgtc cgcaatgtgt tattaagttg tctaagcgtc 1680
   aatttgttta caccacaata tatcctgcca ccagccagcc aacagctccc cgaccggcag 1740
   ctcggcacaa aatcaccacg cgttaccacc acgccggccg gccgcatggt gttgaccgtg 1800
   ttcgccggca ttgccgagtt cgagcgttcc ctaatcatcg accgcacccg gagcgggcgc 1860
   gaggccgcca aggcccgagg cgtgaagttt ggcccccgcc ctaccctcac cccggcacag 1920
   atcgcgcacg cccgcgagct gatcgaccag gaaggccgca ccgtgaaaga ggcggctgca 1980
   ctgcttggcg tgcatcgctc gaccctgtac cgcgcacttg agcgcagcga ggaagtgacg 2040
   cccaccgagg ccaggcggcg cggtgccttc cgtgaggacg cattgaccga ggccgacgcc 2100
   ctggcggccg ccgagaatga acgccaagag gaacaagcat gaaaccgcac caggacggcc 2160
   aggacgaacc gtttttcatt accgaagaga tcgaggcgga gatgatcgcg gccgggtacg 2220
   tgttcgagcc gcccgcgcac gtctcaaccg tgcggctgca tgaaatcctg gccggtttgt 2280
   ctgatgccaa gctggcggcc tggccggcca gcttggccgc tgaagaaacc gagcgccgcc 2340
   gtctaaaaag gtgatgtgta tttgagtaaa acagcttgcg tcatgcggtc gctgcgtata 2400
   tgatgcgatg agtaaataaa caaatacgca aggggaacgc atgaaggtta tcgctgtact 2460
   taaccagaaa ggcgggtcag gcaagacgac catcgcaacc catctagccc gcgccctgca 2520
   actcgccggg gccgatgttc tgttagtcga ttccgatccc cagggcagtg cccgcgattg 2580
   ggcggccgtg cgggaagatc aaccgctaac cgttgtcggc atcgaccgcc cgacgattga 2640
   ccgcgacgtg aaggccatcg gccggcgcga cttcgtagtg atcgacggag cgccccaggc 2700
   ggcggacttg gctgtgtccg cgatcaaggc agccgacttc gtgctgattc cggtgcagcc 2760
   aagcccttac gacatatggg ccaccgccga cctggtggag ctggttaagc agcgcattga 2820
   ggtcacggat ggaaggctac aagcggcctt tgtcgtgtcg cgggcgatca aaggcacgcg 2880
   catcggcggt gaggttgccg aggcgctggc cgggtacgag ctgcccattc ttgagtcccg 2940
   tatcacgcag cgcgtgagct acccaggcac tgccgccgcc ggcacaaccg ttcttgaatc 3000
   agaacccgag ggcgacgctg cccgcgaggt ccaggcgctg gccgctgaaa ttaaatcaaa 3060
   actcatttga gttaatgagg taaagagaaa atgagcaaaa gcacaaacac gctaagtgcc 3120
   ggccgtccga gcgcacgcag cagcaaggct gcaacgttgg ccagcctggc agacacgcca 3180
   gccatgaagc gggtcaactt tcagttgccg gcggaggatc acaccaagct gaagatgtac 3240
   gcggtacgcc aaggcaagac cattaccgag ctgctatctg aatacatcgc gcagctacca 3300
   gagtaaatga gcaaatgaat aaatgagtag atgaatttta gcggctaaag gaggcggcat 3360
   ggaaaatcaa gaacaaccag gcaccgacgc cgtggaatgc cccatgtgtg gaggaacggg 3420
   cggttggcca ggcgtaagcg gctgggttgt ctgccggccc tgcaatggca ctggaacccc 3480
   caagcccgag gaatcggcgt gagcggtcgc aaaccatccg gcccggtaca aatcggcgcg 3540
   gcgctgggtg atgacctggt ggagaagttg aaggccgcgc aggccgccca gcggcaacgc 3600
   atcgaggcag aagcacgccc cggtgaatcg tggcaagcgg ccgctgatcg aatccgcaaa 3660
   gaatcccggc aaccgccggc agccggtgcg ccgtcgatta ggaagccgcc caagggcgac 3720
   gagcaaccag attttttcgt tccgatgctc tatgacgtgg gcacccgcga tagtcgcagc 3780
   atcatggacg tggccgtttt ccgtctgtcg aagcgtgacc gacgagctgg cgaggtgatc 3840
   cgctacgagc ttccagacgg gcacgtagag gtttccgcag ggccggccgg catggccagt 3900
   gtgtgggatt acgacctggt actgatggcg gtttcccatc taaccgaatc catgaaccga 3960
   taccgggaag ggaagggaga caagcccggc cgcgtgttcc gtccacacgt tgcggacgta 4020
   ctcaagttct gccggcgagc cgatggcgga aagcagaaag acgacctggt agaaacctgc 4080
   attcggttaa acaccacgca cgttgccatg cagcgtacga agaaggccaa gaacggccgc 4140
   ctggtgacgg tatccgaggg tgaagccttg attagccgct acaagatcgt aaagagcgaa 4200
   accgggcggc cggagtacat cgagatcgag ctagctgatt ggatgtaccg cgagatcaca 4260
   gaaggcaaga acccggacgt gctgacggtt caccccgatt actttttgat cgatcccggc 4320
   atcggccgtt ttctctaccg cctggcacgc cgcgccgcag gcaaggcaga agccagatgg 4380
   ttgttcaaga cgatctacga acgcagtggc agcgccggag agttcaagaa gttctgtttc 4440
   accgtgcgca agctgatcgg gtcaaatgac ctgccggagt acgatttgaa ggaggaggcg 4500
   gggcaggctg gcccgatcct agtcatgcgc taccgcaacc tgatcgaggg cgaagcatcc 4560
   gccggttcct aatgtacgga gcagatgcta gggcaaattg ccctagcagg ggaaaaaggt 4620
   cgaaaaggtc tctttcctgt ggatagcacg tacattggga acccaaagcc gtacattggg 4680
   aaccggaacc cgtacattgg gaacccaaag ccgtacattg ggaaccggtc acacatgtaa 4740
   gtgactgata taaaagagaa aaaaggcgat ttttccgcct aaaactcttt aaaacttatt 4800
   aaaactctta aaacccgcct ggcctgtgca taactgtctg gccagcgcac agccgaagag 4860
   ctgcaaaaag cgcctaccct tcggtcgctg cgctccctac gccccgccgc ttcgcgtcgg 4920
   cctatcgcgg ccgctggccg ctcaaaaatg gctggcctac ggccaggcaa tctaccaggg 4980
   cgcggacaag ccgcgccgtc gccactcgac cgccggcgcc cacatcaagg caccctgcct 5040
   cgcgcgtttc ggtgatgacg gtgaaaacct ctgacacatg cagctcccgg agacggtcac 5100
   agcttgtctg taagcggatg ccgggagcag acaagcccgt cagggcgcgt cagcgggtgt 5160
   tggcgggtgt cggggcgcag ccatgaccca gtcacgtagc gatagcggag tgtatactgg 5220
   cttaactatg cggcatcaga gcagattgta ctgagagtgc accatatgcg gtgtgaaata 5280
   ccgcacagat gcgtaaggag aaaataccgc atcaggcgct cttccgcttc ctcgctcact 5340
   gactcgctgc gctcggtcgt tcggctgcgg cgagcggtat cagctcactc aaaggcggta 5400
   atacggttat ccacagaatc aggggataac gcaggaaaga acatgtgagc aaaaggccag 5460
   caaaaggcca ggaaccgtaa aaaggccgcg ttgctggcgt ttttccatag gctccgcccc 5520
   cctgacgagc atcacaaaaa tcgacgctca agtcagaggt ggcgaaaccc gacaggacta 5580
   taaagatacc aggcgtttcc ccctggaagc tccctcgtgc gctctcctgt tccgaccctg 5640
   ccgcttaccg gatacctgtc cgcctttctc ccttcgggaa gcgtggcgct ttctcatagc 5700
   tcacgctgta ggtatctcag ttcggtgtag gtcgttcgct ccaagctggg ctgtgtgcac 5760
   gaaccccccg ttcagcccga ccgctgcgcc ttatccggta actatcgtct tgagtccaac 5820
   ccggtaagac acgacttatc gccactggca gcagccactg gtaacaggat tagcagagcg 5880
   aggtatgtag gcggtgctac agagttcttg aagtggtggc ctaactacgg ctacactaga 5940
   aggacagtat ttggtatctg cgctctgctg aagccagtta ccttcggaaa aagagttggt 6000
   agctcttgat ccggcaaaca aaccaccgct ggtagcggtg gtttttttgt ttgcaagcag 6060
   cagattacgc gcagaaaaaa aggatctcaa gaagatcctt tgatcttttc tacggggtct 6120
   gacgctcagt ggaacgaaaa ctcacgttaa gggattttgg tcatgcatga tatatctccc 6180
   aatttgtgta gggcttatta tgcacgctta aaaataataa aagcagactt gacctgatag 6240
   tttggctgtg agcaattatg tgcttagtgc atctaacgct tgagttaagc cgcgccgcga 6300
   agcggcgtcg gcttgaacga atttctagct agacattatt tgccgactac cttggtgatc 6360
   tcgcctttca cgtagtggac aaattcttcc aactgatctg cgcgcgaggc caagcgatct 6420
   tcttcttgtc caagataagc ctgtctagct tcaagtatga cgggctgata ctgggccggc 6480
   aggcgctcca ttgcccagtc ggcagcgaca tccttcggcg cgattttgcc ggttactgcg 6540
   ctgtaccaaa tgcgggacaa cgtaagcact acatttcgct catcgccagc ccagtcgggc 6600
   ggcgagttcc atagcgttaa ggtttcattt agcgcctcaa atagatcctg ttcaggaacc 6660
   ggatcaaaga gttcctccgc cgctggacct accaaggcaa cgctatgttc tcttgctttt 6720
   gtcagcaaga tagccagatc aatgtcgatc gtggctggct cgaagatacc tgcaagaatg 6780
   tcattgcgct gccattctcc aaattgcagt tcgcgcttag ctggataacg ccacggaatg 6840
   atgtcgtcgt gcacaacaat ggtgacttct acagcgcgga gaatctcgct ctctccaggg 6900
   gaagccgaag tttccaaaag gtcgttgatc aaagctcgcc gcgttgtttc atcaagcctt 6960
   acggtcaccg taaccagcaa atcaatatca ctgtgtggct tcaggccgcc atccactgcg 7020
   gagccgtaca aatgtacggc cagcaacgtc ggttcgagat ggcgctcgat gacgccaact 7080
   acctctgata gttgagtcga tacttcggcg atcaccgctt cccccatgat gtttaacttt 7140
   gttttagggc gactgccctg ctgcgtaaca tcgttgctgc tccataacat caaacatcga 7200
   cccacggcgt aacgcgcttg ctgcttggat gcccgaggca tagactgtac cccaaaaaaa 7260
   cagtcataac aagccatgaa aaccgccact gcgggggttc catggacata caaatggacg 7320
   aacggataaa ccttttcacg cccttttaaa tatccgatta ttctaataaa cgctcttttc 7380
   tcttaggttt acccgccaat atatcctgtc aaacactgat agtttaaact gaaggcggga 7440
   aacgacaatc agatctagta ggaaacagct atgaccatga ttacgccaag cttgcatgcc 7500
   tgcaggtcga ctctagagga tcgatccccg ggtaggtcag tcccttatgt tacgtcctgt 7560
   agaaacccca acccgtgaaa tcaaaaaact cgacggcctg tgggcattca gtctggatcg 7620
   cgaaaactgt ggaattggtc agcgttggtg ggaaagcgcg ttacaagaaa gccgggcaat 7680
   tgctgtgcca ggcagtttta acgatcagtt cgccgatgca gatattcgta attatgcggg 7740
   caacgtctgg tatcagcgcg aagtctttat accgaaaggt tgggcaggcc agcgtatcgt 7800
   gctgcgtttc gatgcggtca ctcattacgg caaagtgtgg gtcaataatc aggaagtgat 7860
   ggagcatcag ggcggctata cgccatttga agccgatgtc acgccgtatg ttattgccgg 7920
   gaaaagtgta cgtaagtttc tgcttctacc tttgatatat atataataat tatcattaat 7980
   tagtagtaat ataatatttc aaatattttt ttcaaaataa aagaatgtag tatatagcaa 8040
   ttgcttttct gtagtttata agtgtgtata ttttaattta taacttttct aatatatgac 8100
   caaaatttgt tgatgtgcag gtatcaccgt ttgtgtgaac aacgaactga actggcagac 8160
   tatcccgccg ggaatggtga ttaccgacga aaacggcaag aaaaagcagt cttacttcca 8220
   tgatttcttt aactatgccg gaatccatcg cagcgtaatg ctctacacca cgccgaacac 8280
   ctgggtggac gatatcaccg tggtgacgca tgtcgcgcaa gactgtaacc acgcgtctgt 8340
   tgactggcag gtggtggcca atggtgatgt cagcgttgaa ctgcgtgatg cggatcaaca 8400
   ggtggttgca actggacaag gcactagcgg gactttgcaa gtggtgaatc cgcacctctg 8460
   gcaaccgggt gaaggttatc tctatgaact gtgcgtcaca gccaaaagcc agacagagtg 8520
   tgatatctac ccgcttcgcg tcggcatccg gtcagtggca gtgaagggcg aacagttcct 8580
   gattaaccac aaaccgttct actttactgg ctttggtcgt catgaagatg cggacttgcg 8640
   tggcaaagga ttcgataacg tgctgatggt gcacgaccac gcattaatgg actggattgg 8700
   ggccaactcc taccgtacct cgcattaccc ttacgctgaa gagatgctcg actgggcaga 8760
   tgaacatggc atcgtggtga ttgatgaaac tgctgctgtc ggctttaacc tctctttagg 8820
   cattggtttc gaagcgggca acaagccgaa agaactgtac agcgaagagg cagtcaacgg 8880
   ggaaactcag caagcgcact tacaggcgat taaagagctg atagcgcgtg acaaaaacca 8940
   cccaagcgtg gtgatgtgga gtattgccaa cgaaccggat acccgtccgc aaggtgcacg 9000
   ggaatatttc gcgccactgg cggaagcaac gcgtaaactc gacccgacgc gtccgatcac 9060
   ctgcgtcaat gtaatgttct gcgacgctca caccgatacc atcagcgatc tctttgatgt 9120
   gctgtgcctg aaccgttatt acggatggta tgtccaaagc ggcgatttgg aaacggcaga 9180
   gaaggtactg gaaaaagaac ttctggcctg gcaggagaaa ctgcatcagc cgattatcat 9240
   caccgaatac ggcgtggata cgttagccgg gctgcactca atgtacaccg acatgtggag 9300
   tgaagagtat cagtgtgcat ggctggatat gtatcaccgc gtctttgatc gcgtcagcgc 9360
   cgtcgtcggt gaacaggtat ggaatttcgc cgattttgcg acctcgcaag gcatattgcg 9420
   cgttggcggt aacaagaaag ggatcttcac tcgcgaccgc aaaccgaagt cggcggcttt 9480
   tctgctgcaa aaacgctgga ctggcatgaa cttcggtgaa aaaccgcagc agggaggcaa 9540
   acaatgagag ctcgaatttc cccgatcggt caaacatttg gcaataaagn ttcttaagat 9600
   tgaatcctgt tgccggtctt gcgatgatta tcatataatt tctgttgaat tacgttaagc 9660
   atgtaataat taacatgtaa tgcatgacgt tatttatgag atgggttttt atgattagag 9720
   tcccgcaatt atacatttaa tacgcgatag aaaacaaaat atancgcgca aactaggata 9780
   aattatcgcg cgcggtgtca tctatgttac tagatcggga attcccatgc ctcgagcaga 9840
   aagatataat atgtaaaaaa atgggtctat atatatggaa ggtttcagga agacaaaggt 9900
   tctagaaact tccaaaaaaa atccagaata tattttggaa gaaataccct cttgggttgg 9960
   ccccggcgca gcccctagtg ggccaaaaag ccacgatcta atcccggtct aattggtcta 10020
   atagtttaga cttctaatta gacgggctct tatgccggtc taattggtct aattagatta 10080
   aaatcctaat taaatatgaa cgcaactagg cttcccctct ctctagtttt ctcggagctc 10140
   tttttcatgg accttgaagt attgccggat cactacttcg gaactcgtgg atacttcaga 10200
   gtgcacatct actttgaatc ttgattggta gatcatctcg gagaaattct cacagttggg 10260
   aggtataacc agttgccgaa attgccatgc ttcactcaca gccaggatca gcccatgtcc 10320
   caaggcaacc cttgtagcta catgccgagg cctgactact tggggcctcg cgccctgcat 10380
   ttttgcatgt tcatgtgaca cgttaaatgt tgagagaaat agattactaa atatcaccca 10440
   tttcgttatt ctagatgagt atcctacaat atgtataccg aaaaatgtat tttaaactgt 10500
   ggtaggtgag aaagatctat taaaaagaac tctacgtata ctcccccctc ccaatcccca 10560
   tccaggtttg taagacactt tcgtcttttt ttgccgaatt ttaaccgtaa atttgactag 10620
   taaaaataag ttatactgaa tgtaataaat atcgtacatt cggatgttgg agacagggag 10680
   aggctggctg gtgcgctgga tggatcacgg tcagaaagtc tgacttgcaa cgccacaggc 10740
   ccgttgattg ccactgacaa ccaagttttc gttgtttcgc tggtgccata ttttccgcga 10800
   tcgaatattt aaactgcgag gagaaaggca agcagggcgc catatcagca cttgatcact 10860
   cactgatcga tcagtagtag ccaccttctc tgcgccgacg tgttatatat tattggcaac 10920
   aagtcatcga ttgagaacag aaacaaaaca agaagagaac tatttgagag agagtagtta 10980
   cgccgcagcg agtagcctcc catttctgac gatcatgcca tacgataaac cggccggcgg 11040
   cgagaccagt tagcaaggtt gaaatgccaa cacatgtcgc gctcatttct cggctttttc 11100
   attttgcatg tcgtcatgca ggccctggac actgacattt ctctcttttg ctgttgaatg 11160
   aagaccctaa cctttcacca tcagcacgcc cctcaacttg ataagcctag acgaaaccca 11220
   tatgcatgat tgatgagtaa tggtgtgcac gaatattatg aacccgtttc caagagcaat 11280
   actccattga gatacacctc ctccttgtat ctgttcgttg gtcccatttc catagcagcc 11340
   ggcagtggcc ttgactctga ctgccacgca agtaatatat ctttaataaa ctcgctgcct 11400
   tgcttcgtgt gtccatttgc aaatgcatgc agtgacgaca tgcacatgca tagcttaatt 11460
   agctccatgc atccactgct tccattaatc ccctatataa aggactccat atgcctcacc 11520
   attcactcat ccaccacagc ttagcagcag caacaaccag tgccatagac actctccatc 11580
   aacaaactct agctgatcaa tcctagctaa gcttattaca tagcaagccc ggggatcctc 11640
   tagagtcgac ctgcaggcgg ccgcactagt gattaggatt ccaacgcgag ccaggacaag 11700
   cgaggaacct tgcgtgcgag gcgaggccgc cccgctccga ttcgattcga cgcgcaggcg 11760
   caggcgcagg gatggacgcc ttctactcga cctcgtcggc ggcggcgagc ggctggggcc 11820
   acgactccct caagaacttc cgccagatct cccccgccgt gcagtcccac ctcaagctcg 11880
   tttacctgac tctatgcttt gcactggcct catctgccgt gggtgcttac ctacacattg 11940
   ccctgaacat cggcgggatg ctgacaatgc tcgcttgtgt cggaactatc gcctggatgt 12000
   tctcggtgcc agtctatgag gagaggaaga ggtttgggct gctgatgggt gcagccctcc 12060
   tggaaggggc ttcggttgga cctctgattg agcttgccat agactttgac ccaagcatcc 12120
   tcgtgacagg gtttgtcgga accgccatcg cctttgggtg cttctctggc gccgccatca 12180
   tcgccaagcg cagggagtac ctgtacctcg gtggcctgct ctcgtctggc ctgtcgatcc 12240
   tgctctggct gcagtttgtc acgtccatct ttggccactc ctctggcagc ttcatgtttg 12300
   aggtttactt tggcctgttg atcttcctgg ggtacatggt gtacgacacg caggagatca 12360
   tcgagagggc gcaccatggc gacatggact acatcaagca cgccctcacc ctcttcaccg 12420
   actttgttgc cgtcctcgtc cgagtcctca tcatcatgct caagaacgca ggcgacaagt 12480
   cggaggacaa gaagaagagg aagagggggt cctgaacgtw tctcccgcac atgtagatac 12540
   cgtcaccgcg tcgacctgca ggcatgcccg ctgaaatcac cagtctctct ctacaaatct 12600
   atctctctca taataatgtg tgagtagttc ccagataagg gaattagggt tcttataggg 12660
   tttcgctcat gtgttgagca tataagaaac ccttagtatg tatttgtatt tgtaaaatac 12720
   ttctatcaat aaaatttcta attcctaaaa ccaaaatcca gtgggtaccg agctcg     12776
<210> 37
   <211> 744
   <212> DNA
   <213> Triticum aestivum
<220>
   <221> CDS
   <222> (1)..(741)
   <223> coding for TaBI-1
<400> 37
   atg gac gcc ttc tac tcg acc tcg tcg gcg gcg gcg agc ggc tgg ggc      48
   Met Asp Ala Phe Tyr Ser Thr Ser Ser Ala Ala Ala Ser Gly Trp Gly
   1               5                   10                  15
   tac gac tcc ctc aag aac ttc cgc gag atc tcc ccc gcc gtg cag tcc      96
   Tyr Asp Ser Leu Lys Asn Phe Arg Glu Ile Ser Pro Ala Val Gln Ser
               20                  25                  30
   cac ctc aag ctc gtt tac ctg acc cta tgc ttt gcc ctg gcc tca tct      144
   His Leu Lys Leu Val Tyr Leu Thr Leu Cys Phe Ala Leu Ala Ser Ser
           35                  40                  45
   gcc gtg ggt gct tac ctg cac att gcc ctg aac atc ggt ggg atg ctg      192
   Ala Val Gly Ala Tyr Leu His Ile Ala Leu Asn Ile Gly Gly Met Leu
       50                  55                  60
   aca atg ctc gcg tgt gtt gga acc atc gcc tgg atg ttc tct gtg cca      240
   Thr Met Leu Ala Cys Val Gly Thr Ile Ala Trp Met Phe Ser Val Pro
   65                  70                  75                  80
   gtc tat gag gag agg aag agg ttt ggg ctg ctg atg ggt gca gcc ctc      288
   Val Tyr Glu Glu Arg Lys Arg Phe Gly Leu Leu Met Gly Ala Ala Leu
                   85                  90                  95
   ctg gaa ggg gct tcg gtt gga cct ctg att gag ctt gcc ata gac ttt      336
   Leu Glu Gly Ala Ser Val Gly Pro Leu Ile Glu Leu Ala Ile Asp Phe
               100                 105                 110
   gac cca agt atc ctc gtg aca ggg ttt gtc gga acc gcc atc gcc ttc      384
   Asp Pro Ser Ile Leu Val Thr Gly Phe Val Gly Thr Ala Ile Ala Phe
           115                 120                 125
   ggg tgc ttc tct ggc gcc gcc atc atc gcc aag cgc agg gag tac ctg      432
   Gly Cys Phe Ser Gly Ala Ala Ile Ile Ala Lys Arg Arg Glu Tyr Leu
       130                 135                 140
   tac ctc ggt ggt ctg ctc tcc tcc ggc ctg tcg atc ctg ctc tgg ctg      480
   Tyr Leu Gly Gly Leu Leu Ser Ser Gly Leu Ser Ile Leu Leu Trp Leu
   145                 150                 155                 160
   cag ttt gcc acg tcc atc ttt ggc cac tcc tct ggc agc ttc atg ttt      528
   Gln Phe Ala Thr Ser Ile Phe Gly His Ser Ser Gly Ser Phe Met Phe
                   165                 170                 175
   gag gtt tac ttt ggc ctg ttg atc ttc ctg gga tac atg gtg tac gac      576
   Glu Val Tyr Phe Gly Leu Leu Ile Phe Leu Gly Tyr Met Val Tyr Asp
               180                 185                 190
   acg cag gag atc atc gag agg gcg cac cac ggc gac atg gat tac atc      624
   Thr Gln Glu Ile Ile Glu Arg Ala His His Gly Asp Met Asp Tyr Ile
           195                 200                 205
   aag cac gcg ctc acc ctc ttc acc gac ttc gtc gcc gtt ctc gtc cgc      672
   Lys His Ala Leu Thr Leu Phe Thr Asp Phe Val Ala Val Leu Val Arg
       210                 215                 220
   gtc ctc atc atc atg ctc aag aac gca ggc gac aag tcg gag gac aag      720
   Val Leu Ile Ile Met Leu Lys Asn Ala Gly Asp Lys Ser Glu Asp Lys
   225                 230                 235                 240
   aag aag agg aag agg ggg tcc tga                                      744
   Lys Lys Arg Lys Arg Gly Ser
                   245
<210> 38
   <211> 247
   <212> PRT
   <213> Triticum aestivum
<400> 38
   Met Asp Ala Phe Tyr Ser Thr Ser Ser Ala Ala Ala Ser Gly Trp Gly
   1               5                   10                  15
   Tyr Asp Ser Leu Lys Asn Phe Arg Glu Ile Ser Pro Ala Val Gln Ser
               20                  25                  30
   His Leu Lys Leu Val Tyr Leu Thr Leu Cys Phe Ala Leu Ala Ser Ser
           35                  40                  45
   Ala Val Gly Ala Tyr Leu His Ile Ala Leu Asn Ile Gly Gly Met Leu
       50                  55                  60
   Thr Met Leu Ala Cys Val Gly Thr Ile Ala Trp Met Phe Ser Val Pro
   65                  70                  75                  80
   Val Tyr Glu Glu Arg Lys Arg Phe Gly Leu Leu Met Gly Ala Ala Leu
                   85                  90                  95
   Leu Glu Gly Ala Ser Val Gly Pro Leu Ile Glu Leu Ala Ile Asp Phe
               100                 105                 110
   Asp Pro Ser Ile Leu Val Thr Gly Phe Val Gly Thr Ala Ile Ala Phe
           115                 120                 125
   Gly Cys Phe Ser Gly Ala Ala Ile Ile Ala Lys Arg Arg Glu Tyr Leu
       130                 135                 140
   Tyr Leu Gly Gly Leu Leu Ser Ser Gly Leu Ser Ile Leu Leu Trp Leu
   145                 150                 155                 160
   Gln Phe Ala Thr Ser Ile Phe Gly His Ser Ser Gly Ser Phe Met Phe
                   165                 170                 175
   Glu Val Tyr Phe Gly Leu Leu Ile Phe Leu Gly Tyr Met Val Tyr Asp
               180                 185                 190
   Thr Gln Glu Ile Ile Glu Arg Ala His His Gly Asp Met Asp Tyr Ile
           195                 200                 205
   Lys His Ala Leu Thr Leu Phe Thr Asp Phe Val Ala Val Leu Val Arg
       210                 215                 220
   Val Leu Ile Ile Met Leu Lys Asn Ala Gly Asp Lys Ser Glu Asp Lys
   225                 230                 235                 240
   Lys Lys Arg Lys Arg Gly Ser
                   245
<210> 39
   <211> 1293
   <212> DNA
   <213> Hordeum vulgare
<220>
   <221> CDS
   <222> (173)..(1126)
   <223> coding for Hordeum vulgare subsp. vulgare syntaxin (Ror2)
<400> 39
   gtaactaacc ccttcttcct cccttgtcca ctccgcttct ccccatccaa gaaacagcgc 60
   caacagctcc acccatcgag gagaatcaag aaaccgcgcc ggcgtggtga tcaaggacat 120
   ccatcgatcg atcgaccgac cctgccttgc ctgagtcaac ccggcggcag cc atg aac 178
                                                             Met Asn
                                                              1
   aac ctc ttc tcg agc tcg tgg aag cgg gcg ggc gcg ggg ggc gac ggg   226
   Asn Leu Phe Ser Ser Ser Trp Lys Arg Ala Gly Ala Gly Gly Asp Gly
           5                   10                  15
   gac ctg gag tcg ggc ggc ggc ggc gtg gag atg acg gcg ccg ccg ggc   274
   Asp Leu Glu Ser Gly Gly Gly Gly Val Glu Met Thr Ala Pro Pro Gly
       20                  25                  30
   gcc gcg gcg ggg gcg agc ctg gac cgc ttc ttc gag gac gtg gag tcg   322
   Ala Ala Ala Gly Ala Ser Leu Asp Arg Phe Phe Glu Asp Val Glu Ser
   35                  40                  45                  50
   atc aag gac gac ctg cgg gag ctg gag cgg atc cag cgc tcc ctc cac   370
   Ile Lys Asp Asp Leu Arg Glu Leu Glu Arg Ile Gln Arg Ser Leu His
                   55                  60                  65
   gac ggc aac gag tcg ggc aag tcg ctc cac gac gcg tcg gcg gtg cgc   418
   Asp Gly Asn Glu Ser Gly Lys Ser Leu His Asp Ala Ser Ala Val Arg
               70                  75                  80
   gcg ctc cgc tcc cgc atg gac gcc gac gtg gcc gcc gcc atc aag aag   466
   Ala Leu Arg Ser Arg Met Asp Ala Asp Val Ala Ala Ala Ile Lys Lys
           85                  90                  95
   gcc aag gtg gtg aag ttg cgg ctc gag tcg ctc gac cgc gcc aac gcc   514
   Ala Lys Val Val Lys Leu Arg Leu Glu Ser Leu Asp Arg Ala Asn Ala
       100                 105                 110
   gcc aac cgg tcc gtg gcc ggg tgc ggg ccg ggg tcg tcc acg gac cgc   562
   Ala Asn Arg Ser Val Ala Gly Cys Gly Pro Gly Ser Ser Thr Asp Arg
   115                 120                 125                 130
   acc cgc acc tcc gtc gtg gcc ggg ctg cgc aag aag ctg cgg gat gcc   610
   Thr Arg Thr Ser Val Val Ala Gly Leu Arg Lys Lys Leu Arg Asp Ala
                   135                 140                 145
   atg gag tcc ttc tcc tcc ctc cgc tcc cgc atc acc tcc gag tac cgg   658
   Met Glu Ser Phe Ser Ser Leu Arg Ser Arg Ile Thr Ser Glu Tyr Arg
               150                 155                 160
   gaa acc gtg gcc cgc cgc tac ttc acg gtg acg ggg tcc cag ccc gac   706
   Glu Thr Val Ala Arg Arg Tyr Phe Thr Val Thr Gly Ser Gln Pro Asp
           165                 170                 175
   gag gcc acg ctg gac acg ctg gcg gag acg ggg gag ggg gag cgg ctc   754
   Glu Ala Thr Leu Asp Thr Leu Ala Glu Thr Gly Glu Gly Glu Arg Leu
       180                 185                 190
   ctg cag cgc gcc atc gcg gag cag cag ggg aga ggg gag gtg ctg ggc   802
   Leu Gln Arg Ala Ile Ala Glu Gln Gln Gly Arg Gly Glu Val Leu Gly
   195                 200                 205                 210
   gtg gtg gcg gag atc cag gag cgg cac ggc gcc gtg gcg gac ctg gag   850
   Val Val Ala Glu Ile Gln Glu Arg His Gly Ala Val Ala Asp Leu Glu
                   215                 220                 225
   cgg tcc ctg ctg gag ctg cag cag gtg ttc aac gac atg gcc gtg ctg   898
   Arg Ser Leu Leu Glu Leu Gln Gln Val Phe Asn Asp Met Ala Val Leu
               230                 235                 240
   gtg gcg gcg cag ggg gag cag ctg gac gac atc gag ggc cac gtc ggg   946
   Val Ala Ala Gln Gly Glu Gln Leu Asp Asp Ile Glu Gly His Val Gly
           245                 250                 255
   cgg gcg agg tcg ttc gtc gac cgc ggg cgc gag cag ctg cag gtg gca      994
   Arg Ala Arg Ser Phe Val Asp Arg Gly Arg Glu Gln Leu Gln Val Ala
       260                 265                 270
   cgc aag cac cag aag agc tcc cgc aag tgg acc ttc atc ggc atc ggc     1042
   Arg Lys His Gln Lys Ser Ser Arg Lys Trp Thr Phe Ile Gly Ile Gly
   275                 280                 285                 290
   atc ctg ctc gtc gtc atc ctc atc atc gtc atc ccc atc gtg ctc aag     1090
   Ile Leu Leu Val Val Ile Leu Ile Ile Val Ile Pro Ile Val Leu Lys
                   295                 300                 305
   aac acc aac aag agc aac aac aac aac agc cag cag tagtggtagg          1136
   Asn Thr Asn Lys Ser Asn Asn Asn Asn Ser Gln Gln
               310                 315
   aacagcctgt ggatctgttg tctgtctctg atgatcctgg tcctggattg cttcctggtt 1196
   gttgttgttg attgtctttt gtggaatttt ttgcgattgt aattactcca tccatgtggt 1256
   tcgttgagcc actcgattat tatttcatga ctatata                            1293
<210> 40
   <211> 318
   <212> PRT
   <213> Hordeum vulgare
<400> 40
   Met Asn Asn Leu Phe Ser Ser Ser Trp Lys Arg Ala Gly Ala Gly Gly
   1               5                   10                  15
   Asp Gly Asp Leu Glu Ser Gly Gly Gly Gly Val Glu Met Thr Ala Pro
               20                  25                  30
   Pro Gly Ala Ala Ala Gly Ala Ser Leu Asp Arg Phe Phe Glu Asp Val
           35                  40                  45
   Glu Ser Ile Lys Asp Asp Leu Arg Glu Leu Glu Arg Ile Gln Arg Ser
       50                  55                  60
   Leu His Asp Gly Asn Glu Ser Gly Lys Ser Leu His Asp Ala Ser Ala
   65                  70                  75                  80
   Val Arg Ala Leu Arg Ser Arg Met Asp Ala Asp Val Ala Ala Ala Ile
                   85                  90                  95
   Lys Lys Ala Lys Val Val Lys Leu Arg Leu Glu Ser Leu Asp Arg Ala
               100                 105                 110
   Asn Ala Ala Asn Arg Ser Val Ala Gly Cys Gly Pro Gly Ser Ser Thr
           115                 120                 125
   Asp Arg Thr Arg Thr Ser Val Val Ala Gly Leu Arg Lys Lys Leu Arg
       130                 135                 140
   Asp Ala Met Glu Ser Phe Ser Ser Leu Arg Ser Arg Ile Thr Ser Glu
   145                 150                 155                 160
   Tyr Arg Glu Thr Val Ala Arg Arg Tyr Phe Thr Val Thr Gly Ser Gln
                   165                 170                 175
   Pro Asp Glu Ala Thr Leu Asp Thr Leu Ala Glu Thr Gly Glu Gly Glu
               180                 185                 190
   Arg Leu Leu Gln Arg Ala Ile Ala Glu Gln Gln Gly Arg Gly Glu Val
           195                 200                 205
   Leu Gly Val Val Ala Glu Ile Gln Glu Arg His Gly Ala Val Ala Asp
       210                 215                 220
   Leu Glu Arg Ser Leu Leu Glu Leu Gln Gln Val Phe Asn Asp Met Ala
   225                 230                 235                 240
   Val Leu Val Ala Ala Gln Gly Glu Gln Leu Asp Asp Ile Glu Gly His
                   245                 250                 255
   Val Gly Arg Ala Arg Ser Phe Val Asp Arg Gly Arg Glu Gln Leu Gln
               260                 265                 270
   Val Ala Arg Lys His Gln Lys Ser Ser Arg Lys Trp Thr Phe Ile Gly
           275                 280                 285
   Ile Gly Ile Leu Leu Val Val Ile Leu Ile Ile Val Ile Pro Ile Val
       290                 295                 300
   Leu Lys Asn Thr Asn Lys Ser Asn Asn Asn Asn Ser Gln Gln
   305                 310                 315
<210> 41
   <211> 948
   <212> DNA
   <213> Arabidopsis thaliana
<220>
   <221> CDS
   <222> (1)..(945)
   <223> coding for Arabidopsis thaliana syntaxin 121 (SYP121) / syntaxin-related protein (SYR1) (At3g11820)
<400> 41
   atg gcg aat ccc gcg gga tca acc ggt ggt gtg aac ctc gac aag ttc      48
   Met Ala Asn Pro Ala Gly Ser Thr Gly Gly Val Asn Leu Asp Lys Phe
   1               5                   10                  15
   ttc gaa gat gtt gaa tct gtg aaa gaa gag cta aag gag cta gat cgg      96
   Phe Glu Asp Val Glu Ser Val Lys Glu Glu Leu Lys Glu Leu Asp Arg
               20                  25                  30
   ctc aac gaa aca ctc tct tca tgt cac gag cag agc aag acg ctt cac     144
   Leu Asn Glu Thr Leu Ser Ser Cys His Glu Gln Ser Lys Thr Leu His
           35                  40                  45
   aat gct aaa gcc gtt aaa gat ctc cgg tct aaa atg gac ggt gac gtt     192
   Asn Ala Lys Ala Val Lys Asp Leu Arg Ser Lys Met Asp Gly Asp Val
       50                  55                  60
   gga gtc gcg ttg aag aag gcg aag atg att aaa gtt aaa ctc gag gcg     240
   Gly Val Ala Leu Lys Lys Ala Lys Met Ile Lys Val Lys Leu Glu Ala
   65                  70                  75                  80
   cta gat cgt gcc aat gct gct aat cgg agt ctc cct ggc tgt gga cct     288
   Leu Asp Arg Ala Asn Ala Ala Asn Arg Ser Leu Pro Gly Cys Gly Pro
                   85                  90                  95
   ggt tct tcc tcc gat cga acc agg acc tct gtc ctc aat ggt ctc agg     336
   Gly Ser Ser Ser Asp Arg Thr Arg Thr Ser Val Leu Asn Gly Leu Arg
               100                 105                 110
   aag aaa ttg atg gac tct atg gat agt ttc aac cga ttg agg gag ctt     384
   Lys Lys Leu Met Asp Ser Met Asp Ser Phe Asn Arg Leu Arg Glu Leu
           115                 120                 125
   atc tcg tcc gag tat aga gaa act gta cag agg agg tac ttc acc gtc     432
   Ile Ser Ser Glu Tyr Arg Glu Thr Val Gln Arg Arg Tyr Phe Thr Val
               130                 135                 140
   acc ggc gag aat ccg gat gaa cga acc cta gat cga ctg att tcc act      480
   Thr Gly Glu Asn Pro Asp Glu Arg Thr Leu Asp Arg Leu Ile Ser Thr
   145                 150                 155                 160
   gga gag agt gag aga ttc ttg cag aaa gca ata caa gaa caa gga aga      528
   Gly Glu Ser Glu Arg Phe Leu Gln Lys Ala Ile Gln Glu Gln Gly Arg
                   165                 170                 175
   gga agg gtg tta gac acc att aac gag att caa gaa agg cat gat gcg      576
   Gly Arg Val Leu Asp Thr Ile Asn Glu Ile Gln Glu Arg His Asp Ala
               180                 185                 190
   gtt aaa gac att gag aag aat ctc agg gag ctt cac cag gtg ttt cta      624
   Val Lys Asp Ile Glu Lys Asn Leu Arg Glu Leu His Gln Val Phe Leu
           195                 200                 205
   gac atg gcc gtg ctg gta gag cac cag gga gct cag ctt gat gac atc      672
   Asp Met Ala Val Leu Val Glu His Gln Gly Ala Gln Leu Asp Asp Ile
       210                 215                 220
   gag agt cat gtg ggt cga gct agc tcc ttt atc aga ggc gga act gac      720
   Glu Ser His Val Gly Arg Ala Ser Ser Phe Ile Arg Gly Gly Thr Asp
   225                 230                 235                 240
   cag cta caa acc gct cgg gtt tac cag aag aac acg cga aaa tgg aca      768
   Gln Leu Gln Thr Ala Arg Val Tyr Gln Lys Asn Thr Arg Lys Trp Thr
                   245                 250                 255
   tgt att gcc att att att ctc atc atc atc ata act gtt gtg gtt ctt      816
   Cys Ile Ala Ile Ile Ile Leu Ile Ile Ile Ile Thr Val Val Val Leu
               260                 265                 270
   gct gtt tta aaa ccg tgg aac aac agc agt ggc ggc ggc ggc ggt ggt      864
   Ala Val Leu Lys Pro Trp Asn Asn Ser Ser Gly Gly Gly Gly Gly Gly
           275                 280                 285
   ggt ggt ggg ggt acc act gga gga agt caa cca aat tca ggg aca cca      912
   Gly Gly Gly Gly Thr Thr Gly Gly Ser Gln Pro Asn Ser Gly Thr Pro
       290                 295                 300
   cca aat cct cct cag gca agg cgt cta ttg cgt tga                      948
   Pro Asn Pro Pro Gln Ala Arg Arg Leu Leu Arg
   305                 310                 315
<210> 42
   <211> 315
   <212> PRT
   <213> Arabidopsis thaliana
<400> 42
   Met Ala Asn Pro Ala Gly Ser Thr Gly Gly Val Asn Leu Asp Lys Phe
   1               5                   10                  15
   Phe Glu Asp Val Glu Ser Val Lys Glu Glu Leu Lys Glu Leu Asp Arg
               20                  25                  30
   Leu Asn Glu Thr Leu Ser Ser Cys His Glu Gln Ser Lys Thr Leu His
           35                  40                  45
   Asn Ala Lys Ala Val Lys Asp Leu Arg Ser Lys Met Asp Gly Asp Val
       50                  55                  60
   Gly Val Ala Leu Lys Lys Ala Lys Met Ile Lys Val Lys Leu Glu Ala
   65                  70                  75                  80
   Leu Asp Arg Ala Asn Ala Ala Asn Arg Ser Leu Pro Gly Cys Gly Pro
                   85                  90                  95
   Gly Ser Ser Ser Asp Arg Thr Arg Thr Ser Val Leu Asn Gly Leu Arg
               100                 105                 110
   Lys Lys Leu Met Asp Ser Met Asp Ser Phe Asn Arg Leu Arg Glu Leu
           115                 120                 125
   Ile Ser Ser Glu Tyr Arg Glu Thr Val Gln Arg Arg Tyr Phe Thr Val
       130                 135                 140
   Thr Gly Glu Asn Pro Asp Glu Arg Thr Leu Asp Arg Leu Ile Ser Thr
   145                 150                 155                 160
   Gly Glu Ser Glu Arg Phe Leu Gln Lys Ala Ile Gln Glu Gln Gly Arg
                   165                 170                 175
   Gly Arg Val Leu Asp Thr Ile Asn Glu Ile Gln Glu Arg His Asp Ala
               180                 185                 190
   Val Lys Asp Ile Glu Lys Asn Leu Arg Glu Leu His Gln Val Phe Leu
           195                 200                 205
   Asp Met Ala Val Leu Val Glu His Gln Gly Ala Gln Leu Asp Asp Ile
       210                 215                 220
   Glu Ser His Val Gly Arg Ala Ser Ser Phe Ile Arg Gly Gly Thr Asp
   225                 230                 235                 240
   Gln Leu Gln Thr Ala Arg Val Tyr Gln Lys Asn Thr Arg Lys Trp Thr
                   245                 250                 255
   Cys Ile Ala Ile Ile Ile Leu Ile Ile Ile Ile Thr Val Val Val Leu
               260                 265                 270
   Ala Val Leu Lys Pro Trp Asn Asn Ser Ser Gly Gly Gly Gly Gly Gly
           275                 280                 285
   Gly Gly Gly Gly Thr Thr Gly Gly Ser Gln Pro Asn Ser Gly Thr Pro
       290                 295                 300
   Pro Asn Pro Pro Gln Ala Arg Arg Leu Leu Arg
   305                 310                 315
<210> 43
   <211> 1275
   <212> DNA
   <213> Hordeum vulgare
<220>
   <221> CDS
   <222> (80)..(1006)
   <223> coding for Hordeum vulgare subsp. vulgare SNAP-34
<400> 43
   ggcccctcca ccccacccca cccagtcgct gcggatactt gattctgcta ctcggccagc 60
   gatcgatctc gcctccgcc atg agc gcc acc agg ccc tcc ttc ttc ccc tcc  112
                        Met Ser Ala Thr Arg Pro Ser Phe Phe Pro Ser
                        1               5                   10
   aac aac aac agg aac aag ccc gcc acc cgg aac ccc ttc gac tcc gac   160
   Asn Asn Asn Arg Asn Lys Pro Ala Thr Arg Asn Pro Phe Asp Ser Asp
               15                  20                  25
   tcg gac gac gac ggc ggc atg gcc cgg cgc ggc ccg gcg cgg gcc tcg   208
   Ser Asp Asp Asp Gly Gly Met Ala Arg Arg Gly Pro Ala Arg Ala Ser
               30                  35                  40
   tcc gtc ccg acc ccc gcc gcg ggg ccg gcc agg gcc tcc tcg gcc ccg     256
   Ser Val Pro Thr Pro Ala Ala Gly Pro Ala Arg Ala Ser Ser Ala Pro
        45                  50                  55
   atc ccc gcc gac gag gcg gac cag cgg ggc gcc ctg ttc ggc gcg ggc     304
   Ile Pro Ala Asp Glu Ala Asp Gln Arg Gly Ala Leu Phe Gly Ala Gly
    60                  65                  70                  75
   ccc gcg ccg tcc ggc ttc gcg tcc tcc tcc tcc gcg gcc gcc agg ggc     352
   Pro Ala Pro Ser Gly Phe Ala Ser Ser Ser Ser Ala Ala Ala Arg Gly
                    80                  85                  90
   cgg tac agg aac gac ttc cgc gac tcg ggc ggc gtg gag gcg cag tcc     400
   Arg Tyr Arg Asn Asp Phe Arg Asp Ser Gly Gly Val Glu Ala Gln Ser
                95                 100                 105
   gtg cag gag ctc gag ggc tac gcg gcc tac aag gcc gag gag acc acg     448
   Val Gln Glu Leu Glu Gly Tyr Ala Ala Tyr Lys Ala Glu Glu Thr Thr
           110                 115                 120
   cgc cgg gtc gac ggc tgc ctc cgg gtc gcc gag gag atg cgg gac acc     496
   Arg Arg Val Asp Gly Cys Leu Arg Val Ala Glu Glu Met Arg Asp Thr
       125                 130                 135
   gcg tca aag acc ctg ctc cag gtg cac cag cag ggc cag cag atc agg     544
   Ala Ser Lys Thr Leu Leu Gln Val His Gln Gln Gly Gln Gln Ile Arg
   140                 145                 150                 155
   cgc acc cac gcc atg gcc gtc gac atc gac cag gat ctc tcc agg ggg     592
   Arg Thr His Ala Met Ala Val Asp Ile Asp Gln Asp Leu Ser Arg Gly
                   160                 165                 170
   gaa aag cta cta ggt gat ctt ggt ggt ttg ttt tcc aag aag tgg aag     640
   Glu Lys Leu Leu Gly Asp Leu Gly Gly Leu Phe Ser Lys Lys Trp Lys
               175                 180                 185
   cca aag aag aac ggc gca atc agg ggc cct atg ctg acc aga gac gat     688
   Pro Lys Lys Asn Gly Ala Ile Arg Gly Pro Met Leu Thr Arg Asp Asp
           190                 195                 200
   tcc ttc ata cgc aag ggc agc cat atg gag cag agg cat aaa ctg ggg     736
   Ser Phe Ile Arg Lys Gly Ser His Met Glu Gln Arg His Lys Leu Gly
       205                 210                 215
   ctg tca gat cgt ccg cat cga tcc aat gca cgc cag ttc cta tct gaa     784
   Leu Ser Asp Arg Pro His Arg Ser Asn Ala Arg Gln Phe Leu Ser Glu
   220                 225                 230                 235
   ccc aca tca ggc ctt gag aaa gtc gag gtg gag aag gca aag cag gat     832
   Pro Thr Ser Gly Leu Glu Lys Val Glu Val Glu Lys Ala Lys Gln Asp
                   240                 245                 250
   gat ggc ctg tct gac ctt agc gac ata ctg aca gag ttg aaa gga atg     880
   Asp Gly Leu Ser Asp Leu Ser Asp Ile Leu Thr Glu Leu Lys Gly Met
               255                 260                 265
   gcc att gac atg gga act gag att gag ggg caa aca aag gat ctt ggt     928
   Ala Ile Asp Met Gly Thr Glu Ile Glu Gly Gln Thr Lys Asp Leu Gly
           270                 275                 280
   cat gcg gag aag gac ttt gac gaa ctt aac tac agg gtc aag ggg gca     976
   His Ala Glu Lys Asp Phe Asp Glu Leu Asn Tyr Arg Val Lys Gly Ala
       285                 290                 295
   aac gct cga aca cgt cgc ctg ctt ggc aga taggcaagaa gcatatgttg      1026
   Asn Ala Arg Thr Arg Arg Leu Leu Gly Arg
   300                 305
   ttcaccagag gattctgtga cactccttat cttctgcatt tgctttcgtg ggctgttaat 1086
   tcagatcatt ttgtgcataa aactctggtt aggaaggtct gttggggagt tgtatcaggg 1146
   tttattgtgt atatacgcta gacgggcggt tcgttttcta tgttgcagtt gtactacatt 1206
   tgctatggac agtagatacg tttgtattcg gttttcttgt tttgcaatcg ctatgctgca 1266
   ggaaagcac 1275
<210> 44
   <211> 309
   <212> PRT
   <213> Hordeum vulgare
<400> 44
   Met Ser Ala Thr Arg Pro Ser Phe Phe Pro Ser Asn Asn Asn Arg Asn
   1               5                   10                  15
   Lys Pro Ala Thr Arg Asn Pro Phe Asp Ser Asp Ser Asp Asp Asp Gly
               20                  25                  30
   Gly Met Ala Arg Arg Gly Pro Ala Arg Ala Ser Ser Val Pro Thr Pro
           35                  40                  45
   Ala Ala Gly Pro Ala Arg Ala Ser Ser Ala Pro Ile Pro Ala Asp Glu
       50                  55                  60
   Ala Asp Gln Arg Gly Ala Leu Phe Gly Ala Gly Pro Ala Pro Ser Gly
   65                  70                  75                  80
   Phe Ala Ser Ser Ser Ser Ala Ala Ala Arg Gly Arg Tyr Arg Asn Asp
                   85                  90                  95
   Phe Arg Asp Ser Gly Gly Val Glu Ala Gln Ser Val Gln Glu Leu Glu
               100                 105                 110
   Gly Tyr Ala Ala Tyr Lys Ala Glu Glu Thr Thr Arg Arg Val Asp Gly
           115                 120                 125
   Cys Leu Arg Val Ala Glu Glu Met Arg Asp Thr Ala Ser Lys Thr Leu
       130                 135                 140
   Leu Gln Val His Gln Gln Gly Gln Gln Ile Arg Arg Thr His Ala Met
   145                 150                 155                 160
   Ala Val Asp Ile Asp Gln Asp Leu Ser Arg Gly Glu Lys Leu Leu Gly
                   165                 170                 175
   Asp Leu Gly Gly Leu Phe Ser Lys Lys Trp Lys Pro Lys Lys Asn Gly
               180                 185                 190
   Ala Ile Arg Gly Pro Met Leu Thr Arg Asp Asp Ser Phe Ile Arg Lys
           195                 200                 205
   Gly Ser His Met Glu Gln Arg His Lys Leu Gly Leu Ser Asp Arg Pro
       210                 215                 220
   His Arg Ser Asn Ala Arg Gln Phe Leu Ser Glu Pro Thr Ser Gly Leu
   225                 230                 235                 240
   Glu Lys Val Glu Val Glu Lys Ala Lys Gln Asp Asp Gly Leu Ser Asp
                   245                 250                 255
   Leu Ser Asp Ile Leu Thr Glu Leu Lys Gly Met Ala Ile Asp Met Gly
               260                 265                 270
   Thr Glu Ile Glu Gly Gln Thr Lys Asp Leu Gly His Ala Glu Lys Asp
           275                 280                 285
   Phe Asp Glu Leu Asn Tyr Arg Val Lys Gly Ala Asn Ala Arg Thr Arg
       290                 295                 300
   Arg Leu Leu Gly Arg
   305
<210> 45
   <211> 1398
   <212> DNA
   <213> Glycine max
<220>
   <221> CDS
   <222> (212)..(946)
<220>
   <221> misc_feature
   <222> <1367>..<1367>
   <223> n is a, c, g, or t
<220>
   <221> misc_feature
   <222> <1368>..<1368>
   <223> n is a, c, g, or t
<220>
   <221> misc_feature
   <222> <1369>..<1369>
   <223> n is a, c, g, or t
<220>
   <221> misc_feature
   <222> <1370>..<1370>
   <223> n is a, c, g, or t
<220>
   <221> misc_feature
   <222> <1371>..<1371>
   <223> n is a, c, g, or t
<220>
   <221> misc_feature
   <222> <1372>..<1372>
   <223> n is a, c, g, or t
<220>
   <221> misc_feature
   <222> <1373>..<1373>
   <223> n is a, c, g, or t
<220>
   <221> misc_feature
   <222> <1374>..<1374>
   <223> n is a, c, g, or t
<220>
   <221> misc_feature
   <222> <1375>..<1375>
   <223> n is a, c, g, or t
<220>
   <221> misc_feature
   <222> <1376>..<1376>
   <223> n is a, c, g, or t
<220>
   <221> misc_feature
   <222> <1377>..<1377>
   <223> n is a, c, g, or t
<220>
   <221> misc_feature
   <222> <1378>..<1378>
   <223> n is a, c, g, or t
<220>
   <221> misc_feature
   <222> <1379>..<1379>
   <223> n is a, c, g, or t
<220>
   <221> misc_feature
   <222> <1380>..<1380>
   <223> n is a, c, g, or t
<220>
   <221> misc_feature
   <222> <1381>..<1381>
   <223> n is a, c, g, or t
<220>
   <221> misc_feature
   <222> <1382>..<1382>
   <223> n is a, c, g, or t
<220>
   <221> misc_feature
   <222> <1383>..<1383>
   <223> n is a, c, g, or t
<220>
   <221> misc_feature
   <222> <1384>..<1384>
   <223> n is a, c, g, or t
<400> 45
   ggaatttccg tggcgaccca tcggacgctc gtccgacgca tcaacactac ggccgactgg      60
   aggcgacttg aatgaaaggg aggtaatctt aagtggaacc gtttgcaaaa ttacccgttc     120
   ctcgtacctc gaatgcagtc taaacttggg gtcgctgaag tcacagccat atacgacact     180
   agacatattt gtgtgtctcc gattgcaagc a atg gac tcc ttc aat tcc ttc        232
                                      Met Asp Ser Phe Asn Ser Phe
                                      1               5
   ttc gat tca aca aac cga tgg aat tac gat act ctc aaa aac ttc cgt       280
   Phe Asp Ser Thr Asn Arg Trp Asn Tyr Asp Thr Leu Lys Asn Phe Arg
           10                  15                  20
   caa att tct ccg gtc gtt cag aat cac ctc aag cag gtt tat ttt act       328
   Gln Ile Ser Pro Val Val Gln Asn His Leu Lys Gln Val Tyr Phe Thr
       25                  30                  35
   ctg tgt ttc gcc gtg gtt gct gcg gct gtt ggg gct tac ctt cat gtc       376
   Leu Cys Phe Ala Val Val Ala Ala Ala Val Gly Ala Tyr Leu His Val
   40                  45                  50                  55
   ctc ttg aac att ggg ggt ttt ctt act aca gtg gca tgc gtg gga agc       424
   Leu Leu Asn Ile Gly Gly Phe Leu Thr Thr Val Ala Cys Val Gly Ser
                   60                  65                  70
   agt gtt tgg tta ctc tcg aca cct cct ttt gaa gag agg aaa aga gtg       472
   Ser Val Trp Leu Leu Ser Thr Pro Pro Phe Glu Glu Arg Lys Arg Val
               75                  80                  85
   act ttg ttg atg gcc gca tca ctg ttt cag ggt gcc tct att gga ccc       520
   Thr Leu Leu Met Ala Ala Ser Leu Phe Gln Gly Ala Ser Ile Gly Pro
           90                  95                  100
   ttg ata gat ttg gct att caa atc gat cca agc ctt atc ttt agt gca      568
   Leu Ile Asp Leu Ala Ile Gln Ile Asp Pro Ser Leu Ile Phe Ser Ala
       105                 110                 115
   ttt gtg gga aca tcc ttg gcc ttt gca tgc ttc tca gga gca gct ttg      616
   Phe Val Gly Thr Ser Leu Ala Phe Ala Cys Phe Ser Gly Ala Ala Leu
   120                 125                 130                 135
   gtt gct agg cgt agg gag tac ctg tac ctt ggt ggc ttg gtt tct tct      664
   Val Ala Arg Arg Arg Glu Tyr Leu Tyr Leu Gly Gly Leu Val Ser Ser
                   140                 145                 150
   gga ttg tcc atc ctt ctc tgg ttg cac ttt gct tct tcc atc ttt gga      712
   Gly Leu Ser Ile Leu Leu Trp Leu His Phe Ala Ser Ser Ile Phe Gly
               155                 160                 165
   ggc tca aca gct ctc ttt aag ttt gag ttg tac ttt ggg cta ttg gtg      760
   Gly Ser Thr Ala Leu Phe Lys Phe Glu Leu Tyr Phe Gly Leu Leu Val
           170                 175                 180
   ttt gta ggt tac att gta gta gac acc caa gaa ata gtt gag agg gca      808
   Phe Val Gly Tyr Ile Val Val Asp Thr Gln Glu Ile Val Glu Arg Ala
       185                 190                 195
   cac ttg ggc gat ctg gac tat gta aag cat gcc ttg acc ttg ttt acc      856
   His Leu Gly Asp Leu Asp Tyr Val Lys His Ala Leu Thr Leu Phe Thr
   200                 205                 210                 215
   gat ttg gtc gca gtt ttt gtc cgg att ctt gtt att atg ttg aag aat      904
   Asp Leu Val Ala Val Phe Val Arg Ile Leu Val Ile Met Leu Lys Asn
                   220                 225                 230
   tcg act gag agg aat gag aag aaa aag aag aga aga gat tga ttttcttacc   956
   Ser Thr Glu Arg Asn Glu Lys Lys Lys Lys Arg Arg Asp
               235                 240
   aatctgctcg attaacactt cacctcgctt ggtctgtaat acataaaaac agccgtctat    1016
   acagtgtttt ccacttttta agcttgctcc tcctactgtg cagttaagtc gtttgtttac     1076
   caatagtaca tgttgacatg ttttgagctc tctaataaga gaaatgttta cattacattt    1136
   gttttaaaga tgaaaagggg agtggggaag aagtcgatgg tgaaggtccc taataaattc    1196
   ttactcccaa gactcagaat ttttcttggg gaggaagtgg aattcaggga aacacttttt    1256
   tttcacacat tattgagtat attttatact agactcgcca aatcacgaat ttcattatct    1316
   atttagcttc ttttttttcc ccctgtaaaa aaaaaaaaaa aaaaaaaaaa nnnnnnnnnn    1376
   nnnnnnnnaa aaaaaaaaaa aa                                             1398
<210> 46
   <211> 244
   <212> PRT
   <213> Glycine max
<400> 46
   Met Asp Ser Phe Asn Ser Phe Phe Asp Ser Thr Asn Arg Trp Asn Tyr
   1               5                   10                  15
   Asp Thr Leu Lys Asn Phe Arg Gln Ile Ser Pro Val Val Gln Asn His
               20                  25                  30
   Leu Lys Gln Val Tyr Phe Thr Leu Cys Phe Ala Val Val Ala Ala Ala
           35                  40                  45
   Val Gly Ala Tyr Leu His Val Leu Leu Asn Ile Gly Gly Phe Leu Thr
       50                  55                  60
   Thr Val Ala Cys Val Gly Ser Ser Val Trp Leu Leu Ser Thr Pro Pro
   65                  70                  75                  80
   Phe Glu Glu Arg Lys Arg Val Thr Leu Leu Met Ala Ala Ser Leu Phe
                   85                  90                  95
   Gln Gly Ala Ser Ile Gly Pro Leu Ile Asp Leu Ala Ile Gln Ile Asp
               100                 105                 110
   Pro Ser Leu Ile Phe Ser Ala Phe Val Gly Thr Ser Leu Ala Phe Ala
           115                 120                 125
   Cys Phe Ser Gly Ala Ala Leu Val Ala Arg Arg Arg Glu Tyr Leu Tyr
       130                 135                 140
   Leu Gly Gly Leu Val Ser Ser Gly Leu Ser Ile Leu Leu Trp Leu His
   145                 150                 155                 160
   Phe Ala Ser Ser Ile Phe Gly Gly Ser Thr Ala Leu Phe Lys Phe Glu
                   165                 170                 175
   Leu Tyr Phe Gly Leu Leu Val Phe Val Gly Tyr Ile Val Val Asp Thr
               180                 185                 190
   Gln Glu Ile Val Glu Arg Ala His Leu Gly Asp Leu Asp Tyr Val Lys
           195                 200                 205
   His Ala Leu Thr Leu Phe Thr Asp Leu Val Ala Val Phe Val Arg Ile
       210                 215                 220
   Leu Val Ile Met Leu Lys Asn Ser Thr Glu Arg Asn Glu Lys Lys Lys
   225                 230                 235                 240
   Lys Arg Arg Asp
<210> 47
   <211> 20
   <212> DNA
   <213> Artificial sequence
<220>
   <223> GFP-Primer 1
<400> 47
   atggtgagca agggcgagga 20
<210> 48
   <211> 43
   <212> DNA
   <213> Artificial sequence
<220>
   <223> GFP-Primer 2
<400> 48
   ttgaacaacg atgtgcaaga ctccttgtac agctcgtcca tgc 43
   PF 58494
   92

## Beschreibung

Die vorliegende Erfindung betrifft Verfahren zur Erzeugung oder Erhöhung von Resistenz gegen mindestens ein biotrophes Pathogen aus dem Genus Phakopsora in einer Sojapflanze oder einem Teil einer Sojapflanze durch Erhöhung der Proteinmenge oder Funktion mindestens eines Bax Inhibitor-1 (BI-1) Proteins in mindestens einem Teil der Sojapflanze.

Der Anbau landwirtschaftlicher Kulturpflanzen dient in erster Linie der Nahrungsmittelproduktion für den Menschen und der Produktion von Futtermitteln für Tiere. In den letzten 25 Jahren sind im Pflanzenbau deutliche Steigerungen der Erträge erreicht worden. Dies gelang durch ein gutes Zusammenspiel von veränderter Anbautechnik, neuen Züchtungen, Düngung und nicht zuletzt verbessertem Pflanzenschutz. Angesichts einer ständig wachsenden Weltbevölkerung gewinnt die Nahrungssicherung zunehmend an Bedeutung. Es wird geschätzt, dass im Jahr 2010 7 Mrd. Menschen auf der Erde leben werden. Um diese zu ernähren, ohne dass der Anteil unterernährter Menschen zunimmt, müsste die Nahrungsmittelproduktion um 60 % gesteigert werden (Entrup N.L. et al., Lehrbuch des Pflanzenbaues, Thomas Mann Verlag, Gelsenkirchen, 2000). Hierfür ist effektiver Pflanzenschutz ein entscheidender Faktor. Besonders in den heute üblichen Monokulturen kommt es leicht zu einer epidemieartigen Ausbreitung von Krankheiten. Die Folge sind deutlich reduzierte Erträge. Bisher wurden die pathogenen Organismen vor allem durch den Einsatz von Pestiziden bekämpft. Heute dagegen steht dem Menschen die Möglichkeit offen die genetische Disposition einer Pflanze gegenüber einem Pathogen direkt zu verändern.

Pilze sind weltweit verbreitet und bilden eine heterogene Gruppe mit großer Artenvielfalt. Sie sind Eukaryonten, besitzen kein Chlorophyll und sind daher heterotroph. Damit sind sie auf externe Kohlenstoffquellen angewiesen, die sie sich als Parasiten, Saprophyten oder Symbionten erschließen. Saprophyten leben ausschließlich auf abgestorbenem Pflanzenmaterial. Parasitierende Pilze ernähren sich von lebendem Gewebe und müssen ihre Entwicklung bis zum Absterben der Pflanze vollendet haben. Fakultative Parasiten können sich sowohl von lebenden als auch von totem Gewebe ernähren. Symbionten, wie die Mykorrhiza, leben in enger Gesellschaft mit den Pflanzen. Pilze besitzen einen oder mehrere Zellkerne pro Zelle und sind homo- oder heterokaryotisch. Mindestens in einem Lebensstadium haben Pilze eine feste Zellwand. Diese besteht meist aus Chitin oder in einigen Fällen wie den Oomycota aus Cellulose. Der vegetative Pilzkörper (Thallus) ist meist haploid, seltener diploid. Der Thallus niederer Pilze (u.a. Myxomycota) besteht aus amöboiden Zellen oder Plasmodien (nacktem Protoplasma mit vielen Kernen). Eumycota haben Sprosszellen, wie die Hefen (z.B. Saccharomyces cerevisiae), oder bilden ein Myzel das aus fadenförmigen Hyphen besteht. Durch die Aggregation von Hyphen können spezielle Organe für die Vermehrung (Fruchtkörper) oder zum Überdauern ungünstiger Umweltbedingungen (Sklerotien) gebildet werden. Die Fortpflanzung und Vermehrung erfolgt meist durch Sporen; asexuell mit Konidien, Uredosporen, Sporangiosporen, Chlamydosporen und Zoosporen, sexuell mit Oosporen, Ascosporen, Zygosporen und Basidiosporen.

Bis heute sind ca. 100 000 verschiedene Pilzarten bekannt. Von diesen treten allerdings nur 5 % als Pflanzenpathogene auf. Die Basidiomycota sind eine Abteilung der echten Pilze, welche durch die Bildung einer besonderen Struktur, der Basidie, an der die Basidiosporen reifen, charakterisiert sind. Zu den Basidiomycota gehören auch die allgemein bekannten Hutpilze. Die Basidiomycota sind überwiegend heterothallisch und selbsteril. Die Kopulation erfolgt durch Somatogamie. Dabei verschmelzen zwei kompatible, haploide, einkernige Myzelien oder Sporidien miteinander. Das daraus hervorgehende, dikaryotische Myzel stellt über einen langen Zeitraum die Hauptlebensphase dar. Die beiden einzigen phytopathogenen Gattungen der Basidiomycota sind die Brandpilze (Ustilages) und die Rostpilze (Uredinales). Brandpilze befallen nur Angiospermen und besaßen früher eine große ökonomische Bedeutung. Heute werden sie durch entsprechende Wirkstoffe und strenge Kontrollen des Saatgutes erfolgreich bekämpft. Eine größere Bedeutung haben heute noch die Rostpilze. Sie sind biotroph und können einen komplizierten Entwicklungszyklus mit bis zu fünf verschiedenen Sporenstadien (Spermatium, Äzidiospore, Uredospore, Teleutospore und Basidiospore) haben. Rostpilze die alle Sporenstadien ausbilden, werden als makrozyklische Roste bezeichnet. Fehlen einige Stadien spricht man von mikrozyklischen Rosten. Bei den ,imperfekten Rosten' fehlen die Basidiosporen. Einige Roste wechseln während ihrer Entwicklung den Wirt. Diese werden als heterözisch bezeichnet. Der Wirtswechsel kann mit einem Kernphasenwechsel verbunden sein. Autözische Roste durchlaufen dagegen ihre gesamte Entwicklung an einem Wirt. Ein klassisches Beispiel für einen makrozyklischen, heterözischen Rost ist der Getreideschwarzrost Puccinia graminis. P. graminis befällt in seinem dikaryotischen Stadium vor allem Weizen. Der Haplont ist auf der Berberitze pathogen (Börner H., Pflanzenkrankheiten und Pflanzenschutz, Ulmer Verlag Stuttgart, 1997; Sitte P. et al., Strasburger - Lehrbuch der Botanik, Gustav Fischer Verlag, Stuttgart, 1998; Entrup N.L. et al., Lehrbuch des Pflanzenbaues, Thomas Mann Verlag, Gelsenkirchen, 2000).

Bei der Infektion von Pflanzen mit pathogenen Pilzen beobachtet man im Allgemeinen unterschiedliche Phasen. Die ersten Phasen der Interaktion zwischen phytopathogenen Pilzen und ihren potentiellen Wirtspflanzen sind für die Besiedlung der Pflanze durch den Pilz entscheidend. Im ersten Schritt der Infektion heften sich die Sporen an die Pflanzenoberfläche an, keimen aus und der Pilz dringt in die Pflanze ein. Für das Anheften der Sporen ist entweder ein aktiver Metabolismus nötig, dies ist bei Colletotrichum graminicola der Fall, oder sie erfolgt passiv, wie bei Magnaporthe grisea. Bei letzterem führt Feuchtigkeit zur Ausscheidung eines ,Klebeschleimes' mit dem sich die Spore anheftet (Howard R.J. et al., Annu. Rev. Microbiol. 50, 491 (1996)). Die Keimung der Spore wird durch unspezifische Induktoren wie Wasser, Nährstoffe, Ethylen oder seltener wie bei Phyllosticta ampelicida, durch hydrophobe Oberflächen induziert (Kuo K. et al., Fungal Genet. Biol. 20, 18 (1996)). Einige Pilze bilden zwei Keimschläuche aus, wie der Getreidemehltau Blumeria graminis, andere Pilze hingegen nur einen (Green J.R. et al., The powdery mildrews, a comprehensive treatise; The formation and function of infection and feeding structures, APS Press, 2002). Pilze können durch schon vorhandene Öffnungen wie Stomata, Lentizellen, Hydatoden und Wunden in die Pflanze eindringen, oder sie penetrieren die Epidermis der Pflanze direkt durch mechanische Kraft und mit der Hilfe von Zellwand auflösenden Enzymen. Zum Eindringen in die Pflanze werden spezielle Infektionsstrukturen gebildet. Diese Druckorgane werden als Appressorien bezeichnet und erlauben dem Pilz über einem diskreten Punkt einen hohen Druck aufzubauen. Es wird geschätzt das M. grisea mit seinem Appressorium Drücke von 80 bar erreicht (Howard R.J. et al., Annu. Rev. Microbiol. 50, 491 (1996)). Die meisten Rostpilze dringen dagegen über die Spaltöffnungen in die Pflanze ein. Der Sojarost Phakopsora pachyrhizi penetriert die Epidermis der Pflanze direkt und ist damit in seinem Penetrationsverhalten dem Getreidemehltau B. graminis ähnlich (Koch E. et al., Phytopath. Z. 106, 302 (1983); Tucker S.L. et al., Annu. Rev. Phytopathol. 39, 385 (2001); Green J.R. et al., The powdery mildrews, a comprehensive treatise; The formation and function of infection and feeding structures, APS Press, 2002).

Phytopathogene Pilze besiedeln durchaus nicht immer die vollständige Pflanze, sondern nur bestimmte Bereiche oder Gewebe. Nach der erfolgreichen Invasion der Pflanze verfolgen phytopathogene Pilze verschiedene Ernährungsstrategien. Pertotrophe bzw. nekrotrophe Pathogene töten die Wirtszellen durch extrazelluläre Enzyme oder Toxine und ernähren sich durch Abbau der toten Zellen. Einige Gattungen mit pertotropher Ernährung sind die Fusarien sp., Alternaria sp. und Cochliobolus. Die Mehrzahl der Pilze verfolgt diese Ernährungsstrategie. Die biotrophen phytopathogenen Pilze, wie der Mehltau und viele Rostpilze, sind für ihre Ernährung auf den Stoffwechsel lebender Zellen angewiesen. Eine Zwischenstellung haben die hemibiotrophen pathogenen Pilze zu denen die Gattungen Phytophtora und Peronospora zählen. Diese sind meist zu Beginn ihrer Entwicklung biotroph und gehen erst zu einer pertotrophen Lebensweise über, wenn ihre Entwicklung weit fortgeschritten ist (Prell H.H., Interaktionen von Pflanzen und phytopathogenen Pilzen, Gustav Fischer Verlag, Jena, 1996). Um der Infektion zu entgehen, haben die Pflanzen komplexe Abwehrmechanismen entwickelt. Eine andere Zwischenstellung nimmt beispielsweise der Sojarost ein, der die Epidermis direkt penetriert, worauf die penetrierte Zelle nekrotisch wird; nach der Penetration geht er zu einer obligat-biotrophen Lebensweise über. Die Untergruppe der biotrophen Pilzpathogene, die im wesentlichen eine solche Infektionsstrategie zur Anwendung bringt, wird für die Zwecke der vorliegenden Beschreibung als "heminekrotroph" bezeichnet.

Es ist wichtig, hervorzuheben, dass Pflanzen während ihrer Entwicklung dem ständigen Angriff zahlreicher phytopathogener Organismen ausgesetzt sind. Trotzdem ist die Besiedlung der Pflanze durch phytopathogene Organismen eher die Ausnahme als die Regel. Bevor ein Pathogen die Pflanze befallen kann, muss es eine Reihe von Barrieren überwinden. Häufig hat das Pathogen spezielle, an die Pflanze angepasste Pathogenitätsfaktoren, sogenannte Virulenzfaktoren, entwickelt, um diese Barrieren zu überwinden. In diesem Fall wird die Pflanze eine Wirtspflanze des Pathogens, d.h. es ist virulent auf der Pflanze. Es besteht eine Basiskompatibilität zwischen der Pflanze und dem Pathogen. Dies bedeutet, dass die zur Besiedlung erforderlichen physiologischen und biochemischen Vorraussetzungen bereits bestehen oder hergestellt werden (Prell H.H., Interaktionen von Pflanzen und phytopathogenen Pilzen, Gustav Fischer Verlag, Jena, 1996). Das Pathogen entwickelt sich bei einer kompatiblen Interaktion auf Kosten der Pflanze und bewirkt so die Ausbildung von Krankheitssymptomen wie Welken, Nekrosen und Chlorosen. Besteht eine Basiskompatibilität kann das Pathogen aber trotzdem noch von der Pflanze abgewehrt werden, wenn in dieser eine Resistenzmutation stattgefunden hat. Die dadurch erworbene Resistenz der Pflanze wird als Wirtsresistenz bezeichnet. Sie ist nur gegen ein bestimmtes einzelnes Pathogen gerichtet und kann von diesem leicht überwunden werden. Meist, aber nicht immer, handelt es sich dabei um biotrophe Pathogene. Die Wirts-resistenz kann weiter in eine nichtrassenspezifische horizontale Resistenz, an der meist mehrere Gene beteiligt sind, und in eine rassenspezifische vertikale Resistenz unterteilt werden. Letztere ist nur gegen bestimmte einzelne Rassen eines Pathogens wirksam. Ein Großteil der Pathogene wird von der Pflanze von vornherein abgewehrt. Dieses Phänomen wird als Basisinkompatibilität oder Nicht-Wirtsresistenz ("non-host resistence") bezeichnet. Im Gegensatz zu der Wirtsresistenz beruht die Nicht-Wirtsresistenz auf einer Reihe von Ursachen und nicht auf einzelnen Genen. Zum einen können dem Pathogen die notwendigen Pathogenitätsfaktoren fehlen oder die Pflanze ist in der Lage das Pathogen zu erkennen und erfolgreich abzuwehren. Ein weiterer Begriff, der vor allem eine Bedeutung für die Landwirtschaft hat, ist die Toleranz. Eine Pflanze ist gegenüber einem Pathogen tolerant, wenn sie zwar befallen werden kann, der Befall allerdings nicht zur Ausbildung von Krankheitssymptomen und zu keiner Ertragsminderung führt (Prell H.H., Interaktionen von Pflanzen und phytopathogenen Pilzen, Gustav Fischer Verlag, Jena, 1996). Ein Erzeugen bzw. ein Erhöhen einer Resistenz soll für die Zwecke der Beschreibung der vorliegenden Erfindung sowohl eine Erhöhung oder Erzeugung von jeglicher Art Resistenz als auch Toleranz umfassen, d.h. insbesondere alle Fälle der erhöhten Toleranz oder Resistenz, die zu einer Verminderung der Ertragseinbuße unter Einwirken des Pathogens führen.

Bei Resistenzreaktionen von Pflanzen werden mit dem Begriff Resistenzfaktoren Strukturen, Substanzen und Prozesse zusammengefasst, die den Befall der Pflanze durch potentielle Pathogene verhindern oder hemmen. Sind die Resistenzfaktoren bereits konstitutiv in der Pflanze vorhanden, werden sie als präformierte Resistenzfaktoren bezeichnet. Induzierte Resistenzfaktoren werden erst gebildet, wenn es zu einer Erkennungsreaktion zwischen der Pflanze und dem potentiellen Pathogen gekommen ist. Die Erkennung kann als eine Signal-Sensor-Reaktion beschrieben werden (Elicitor-Rezeptor-Modell, Keen N.T. et al., Phytopathology 62, 768 (1972)). Das Signal sind pflanzliche oder vom Pathogen stammende Stoffe, sogenannte Elicitoren, die an einen für den jeweiligen Elicitor spezifischen Sensor oder Rezeptor binden. Diese Bindung löst einen oder mehrere Effektoren aus, die z.B. Signaltransduktionsketten darstellen können und Resistenzreaktion induzieren. Eine ganze Reihe von Stoffen wirken als Elicitoren. Zu diesen gehören Proteine, Glykoproteine, Glykane und Lipide (Garcia Brugger et al., MPMI 19, 711 (2006)). Auch Abbauprodukte der Pflanzenzellwand, die durch Enzyme des Pathogens oder aber auch durch Verwundung der Pflanze freigesetzt werden, können eine Resistenzreaktion induzieren. In diesem Zusammenhang wird häufig von einem Avirulenzfaktor des Pathogens und dem zugehörigen Resistenzgen der Pflanze gesprochen ("Gen-für-Gen-Hypothese", Flor, J. Agric. Res. 74, 241 (1947)). Das Pathogen kann durch strukturelle Veränderungen des Elicitors, Maskierung der Erkennungssequenz oder durch Kompetition mit einem anderen Stoff um die Bindungsstellen am Elicitor oder Rezeptor die Erkennung durch die Pflanze verhindern.

Präformierte Resistenzfaktoren bilden die erste Abwehr gegen die Besiedlung durch pathogene Organismen. Diese Faktoren können morphologischer Natur sein oder Substanzen des sekundären pflanzlichen Stoffwechsels (Phytoanticipine) darstellen. Morphologische Faktoren, die eine Besiedlung verhindern sind die Blattbehaarung, die Dichte und Form der Stomata sowie die Beschaffenheit der Kutikula und der Zellwand.

Das Erkennen des Pathogens durch die Pflanze kann auch zur Induktion von Resistenzfaktoren, d.h. morphologischer sowie physiologischer Resistenzreaktionen führen. Viele dieser Reaktionen sind die Folge einer Signalkaskade. Signalmoleküle, wie Ca²⁺, NO, Reaktive Sauerstoffverbindungen und Phytohormone, wie Ethylen und Jasmonat, sind an den Kaskaden beteiligt und tragen zu der Vernetzung der Signalwege bei. Als Resistenzreaktionen bei denen morphologische Strukturen in der Pflanzenzelle verändert werden, sind die Bildung von Zellwandappositionen (Papillen), Kork- und Abscissionsschichten, Thyllen und die Imprägnierung der Zellwand zu nennen. Der Beginn der Penetration durch einen pathogenen Pilz kann die Bildung von Papillen auslösen. Gegenüber der potentiellen Penetrationstelle werden an der inneren Zellwand Lignin, Kallose, Suberin und hydroxyprolinreiche Proteine abgelagert und miteinander vernetzt. Die Kallose kann durch Einlagerung von Anillinblau angefärbt werden. Zusätzlich akkumulieren in der gebildeten Papille Phenole, reaktive Sauerstoffverbindungen und Hydrolasen (Hückelhoven R. et al., Plant Physiol. 119, 1251 (1999); Assaad F.F. et al., Mol. Biol. of the Cell, 15, 5118 (2004)). Die Papillenbildung führt zu einer erheblich verdickten Zellwand und kann das Eindringen des pathogenen Pilzes verhindern. Physiologische Prozesse die zu der induzierten Resistenz beitragen sind die Depolarisierung der Zellmembran, der ,Oxidative burst' (oxidative Stressantwort), die ,Hypersensitive Reaktion', die Bildung von Phytoalexinen und die Expression von ,Pathogenesis related proteins' (PR-Proteine). Eine der ersten Reaktionen auf den Kontakt mit einem Elicitor ist die Depolarisation der Zellmembran. Dabei kommt es zu einem starken Efflux von CI-- und K⁺- Ionen verbunden mit einem deutlichen Wasserverlust. Es wird angenommen, dass die Depolarisation eine Erhöhung der Ca²⁺-Konzentration, eines wichtigen Signalmoleküls, auslöst (Ward J.M. et al., Plant Cell 7, 833 (1995)) und eine Rolle bei der ,Hypersensitiven Reaktion' (HR) spielt (Wendehenne D. et al., Plant Cell 14, 1937 (2002)). Die HR in Pflanzen ist eine Form des programmierten Zelltods. Sie ermöglicht der Pflanze den Pilz auch noch nach dem Eindringen zu stoppen, indem sie ihm die Nahrungsgrundlage entzieht. Der Verlauf der HR scheint dabei von der Kombination von Pflanze und Pathogen abhängig zu sein. Für die Induktion der HR scheint aber die Proteinbiosynthese, ein intaktes Cytoskelett und Salicylsäure nötig zu sein (Heath M., Plant Mol. Biol. 44, 321 (2000)).

Eine sehr schnelle Reaktion auf das Pathogen ist der ,Oxidative burst', die Bildung reaktiver Sauerstoffverbindungen, wie das Superoxidanion O₂⁻, das Hydroxylradikal OH. und Wasserstoffperoxid. Diese Verbindungen werden durch verschiedene Oxidasen gebildet. Das Hydroxylradikal wirkt lokal, während H₂O₂ über die Membranen diffundieren kann. Beide oxidieren polyungesättigte Fettsäuren und können so Membranen zerstören (Grant J.J. et al., Plant Physiol. 124, 21 (2000)). Für H₂O₂ wird auch eine Funktion in der Genregulation vermutet. Außerdem unterstützen die Verbindungen die Abwehrreaktionen durch Vernetzung der Zellwandkomponenten, verstärkte Lignifizierung und haben eine toxische Wirkung auf Pathogene (Garcia-Brugger A. et al., MPMI 19, 711 (2006)). Nicht zuletzt führt der Angriff des Pathogens zur Expression von Genen die für PR-Proteine und Phytoalexine codieren. PR-Proteine sind eine heterogene Gruppe von Proteinen, die toxisch auf eindringende Pilze wirken. Als Phytoalexine werden niedermolekulare antimikrobiell wirksame Substanzen bezeichnet, deren Synthese durch biotischen oder abiotischen Stress ausgelöst wird (Prell H.H., Interaktionen von Pflanzen und phytopathogenen Pilzen, Gustav Fischer Verlag, Jena, 1996; van Loon L.C. et al., Physiol. Mol. Plant Physiol. 55, 85 (1999)). Die beschriebenen Reaktionen laufen zum Teil sowohl bei der Interaktion des Pathogens mit einer Wirts- als auch einer Nicht-Wirtspflanze ab. Entscheidend für die Abwehr des Pathogens ist die Qualität der Erkennung und die Quantität und Geschwindigkeit der Resistenzreaktion (Thordal-Christensen H., Current Opinion in Plant Biology 6, 351 (2003)).

Eine Pflanzenkrankheit, die in der letzten Zeit an Bedeutung gewonnen hat, ist der Sojabohnenrost. Die Krankheit wird durch die pathogenen Rostpilze Phakopsora pachyrhizi (Sydow) und Phakopsora meibomiae (Arthur) verursacht. Sie gehören zu der Klasse der Basidiomycota, Ordnung Uredinales und zu der Familie der Phakopsoraceae. Die beiden Spezies sind sehr eng miteinander verwandt. Die intergenen Sequenzen ihrer rRNA-Gene zeigen eine Ähnlichkeit von 80 % (Frederick R.D. et al., Phytopathology 92, 217 (2002)). Die Arten werden anhand morphologischer Charakteristika der Teliosporen unterschieden (Ono Y. et al., Mycol. Res. 96, 825 (1992)). Beide Rostpilze infizieren ein weites Spektrum von Wirtspflanzen. P. pachyrhizi, der auch als asiatischer Sojarost bezeichnet wird, ist auf Sojabohnen (Glycine max) das aggressivere Pathogen und besitzt daher, zumindest derzeit, eine größere Bedeutung für die Landwirtschaft. P. pachyrhizi kann unter natürlichen Bedingungen 31 Arten aus 17 Familien der Leguminosae infizieren und ist in der Lage unter kontrollierten Bedingungen auf weiteren 60 Arten zu wachsen (Sinclair et al.(eds.), Proceedings of the soybean rust workshop (1995), National Soybean Research Laboratory, Publication No. 1 (1996); Rytter J.L. et al., Plant Dis. 87, 818 (1984)). P. meiboniae wurde im Karibischen Bassin und Puerto Rico gefunden, bisher ohne größere Schäden zu verursachen.

Ursprünglich wurde P. pachyrhizi 1902 in Japan entdeckt. Von dort breitete sich P. pachyrhizi über weite Teile Asiens sowie über Indien und Australien aus, um schließlich 1996 Afrika zu erreichen. Der Pilz gelangte 2001 nach Süd- Amerika und trat erstmals 2004 in Amerika auf (Sconyers E.L. et al., www.ers.usda.gov/Features/SoyBeanRust/ (2005)). Besonders in Süd-Amerika verursacht P. pachyrhizi große Ertragseinbußen von bis zu 80 %. Es wird geschätzt das allein 1,5 Mio. t der brasilanischen Sojabohnenernte 2005/2006 der Infektion mit Sojabohnenrost zum Opfer gefallen sind. P. pachyrhizi ist ein hemicyclischer Rost, der drei Arten von Sporen bildet. Eine Bildung von Teliosporen wurde in Asien gegen Ende der Vegetationsperiode beobachtet (Yeh C.C. et al., Phytopathology 71, 1111 (1981)). Die Bildung von Basidiosporen ist dagegen nur unter Laborbedingungen bekannt. Die wichtigste Sporenform sind die Uredosporen, die während der gesamten Vegetationsperiode gebildet werden und der Verbreitung dienen. Diese Sporen werden in großen Mengen gebildet und können mit dem Wind und Regen weite Strecken zurücklegen. P. pachyrhizi ist obligat biotroph. Gelangt eine Uredospore auf einen geeigneten Wirt, keimt diese mit einem einzelnen Keimschlauch aus. An dessen Ende entsteht ein Appressorium, welches schnell die Größe der Spore erreicht. Mit dem Appressorium ist der Pilz in der Lage großen Druck aufzubauen und kann mit einer Penetrationshyphe die Epidermiszellen direkt penetrieren. Die Penetrationshyphe von P. pachyrhizi durchwächst die Epidermiszelle und bildet, sobald sie den interzellulären Raum im Blatt erreicht, das erste Septum. Sie wächst nun als primäre Hyphe im Blatt weiter. Bereits 24 - 48 h nach der Infektion wird die erste Haustorienmutterzelle durch ein Septum abgeteilt und ein sackförmiges Haustorium in einer Mesohyllzelle des Blattes gebildet. Dabei wird die Zellwand der Mesophyllzelle penetriert, das Plasmalemma aber nur eingestülpt, so dass die Zelle am Leben bleibt und als Quelle für Nährstoffe dienen kann. Diese gelangen von der Membran der lebenden Wirtszelle über die extrahaustoriale Matrix zum Haustorium. Die zu Anfang penetrierte Epidermiszelle wird kurze Zeit nach der Penetration nekrotisch. Diese Art und Weise der Infektion eines biotrophen Pathogens, wie sie P. pachyrhizi vollführt, wird daher als "heminekrotroph" bezeichnet. Nur 11 - 12 Tage nach der Infektion werden die ersten Uredosporen gebildet und der Zyklus kann von neuem beginnen (Koch E. et al., Phytopath. Z. 106, 302 (1983)).

Die landwirtschaftlich genutzte Sojabohne Glycine max (L.) Merr. gehört zur Familie der Leguminosae, Unterfamilie Papilionoideae, Tribus Phaseoleae, Gattung Glycine Willd. und Untergattung Soja (Moench). Die Sojabohne wird in über 35 Ländern angebaut. Einige der wichtigsten Anbaugebiete befinden sich in den USA, China, Korea, Argentinien und Brasilien. Sie gilt als eine der ältesten Kulturpflanzen und wurde in China zwischen dem 11. und 17. Jahrhundert erstmals domestiziert (Hymowitz T., Econ. Bot. 24, 408 (1970)). Im Jahr 1765 wurde sie in die USA eingeführt, heute eines der größten Anbaugebiete von Soja. Wilde Sojabohnenarten sind in China, Korea, Japan, Taiwan und der früheren UDSSR zu finden. Morphologische, cytologische und molekulare Hinweise deuten auf G. soja als Vorfahre der Kulturform G. max hin. Die Sojabohne als subtropische Pflanze bevorzugt eine mittlere Jahrestemperatur von 5.9 - 27°C und ist nicht frostresistent (OECD, Consensus document on the biology of Gycine max (L.) Merr. (Soybean); Series on harmonization of regulatory oversight in biotechnology No. 15, ENV/JM/MONO(2000)9). Heute ist die Sojabohne eine wichtige Öl- und Proteinquelle. Diese umfangreiche Verwendung von Soja in der Nahrungsmittelproduktion unterstreicht die Bedeutung einer effektiven Bekämpfung des Sojarostes.

Die Infektion der Sojapflanzen durch P. pachyrhizi erfolgt durch Wind verbreitete Uredosporen. Als erste Symptome sind auf der Blattoberseite kleine gelbe bis rotbraune Läsionen zu erkennen, die sich später weiter ausbreiten bis das gesamte Blatt schließlich chlorotisch wird und abstirbt. Bei einer fortgeschrittenen Infektion treten die Läsionen auf der ganzen Pflanze auf. Die ersten Uredien mit 100-200 µm Durchmesser treten 10-14 Tage nach der Infektion auf der Blattunterseite auf und können drei bis sechs Wochen lang Sporen produzieren. Telia werden subepidermal gebildet und treten meist an der Peripherie der Läsionen auf. Die Sporen sind zunächst gelb bis braun und werden später schwarz. Die ersten Symptome treten häufig zuerst auf den älteren Blättern auf. Die schnelle Entwicklung der Krankheit korreliert mit dem Beginn der Blüte (R1+) und zerstört schließlich das gesamte Blattwerk. Die weitgehende Zerstörung der photosynthetisch aktiven Fläche und die Entnahme von Wasser und Nährstoffen durch den Pilz führen zu einer verringerten Produktivität der Pflanze (Sconyers E.L. et al., www.ers.usda.gov/Features/SoyBeanRust/ (2005)).

Für die Keimung benötigt P. pachyrhizi Feuchtigkeit in Form von Tau oder ähnlichem auf der Blattoberfläche. Besonders gefördert wird der Pilz durch häufigen Regen und Temperaturen zwischen 15 und 29 °C (Sconyers E.L. et al., www.ers.usda.gov/Features/SoyBeanRust/(2005)). Die Krankheit beginnt häufig an einzelnen Stellen und breitet sich anschließend schnell über das ganze Feld aus. Der Pilz ist autözisch d.h. er benötigt keinen Wirtswechsel für seine Entwicklung und kann auf seinen zahlreichen alternativen Wirtspflanzen gut überdauern. In den USA wird vor allem, das aus Japan stammende, Kudzu (Pueraria lobata) als potentielle Wirtspflanze angesehen, auf der P. pachyrhizi überwintern und im Frühjahr neues Inokulum liefern kann.

Zurzeit ist eine Bekämpfung von P. pachyrhizi im Feld nur mit Fungiziden möglich. Sojapflanzen mit einer Resistenz gegen das ganze Spektrum der Isolate sind nicht verfügbar. Bei der Suche nach resistenten Pflanzen wurden vier dominante Gene Rpp1-4 entdeckt, die eine Resistenz von Soja gegen P. pachyrhizi vermitteln, diese ist aber nur Isolat-spezifisch (Hartwig E.E. et al., Crop Science, 23, 237 (1983); Hartwig E.E., Crop Science 26, 1135 (1986)). Da die Resistenz nur auf einzelnen Genen beruhte, ging sie schnell verloren. Einzig die von Rpp4 vermittelte Resistenz wurde bisher nur unter Gewächshausbedingungen gebrochen (Posada-Buitrago M.L. et al., Fungal Genetics and Biology 42, 949 (2005). Die Nutzung potentieller Resistenzquellen aus Vertretern der mehrjährigen ausdauernden Untergattung Soja ist begrenzt (Hartman G..I. et al., Plant Disease 76, 396 (1992)). Bisher waren bei allen Kreuzungen die Nachkommen steril (Singh R. et al., Wendl. Theor. Appl. Genet. 74, 391 (1987).

Für eine effektive Bekämpfung des Sojarostes mit Fungiziden ist eine tiefe Applikation in das Blattwerk der Pflanzen wichtig, da die Infektion als erstes an den unteren Blättern auftritt. Bewährt hat sich eine zweimalige Behandlung. Ein Nachteil der dabei verwendeten Fungizide ist, dass aufgrund ihres spezifischen Wirkungsmechanismus leicht Resistenzen entstehen können. Eine potentielle Alternative zum Fungizideinsatz ist die Verwendung von Glyphosatresistenten Sojapflanzen. In einem Gewächshausversuch wurde nach der Behandlung der Sojapflanzen mit dem Herbizid drei Tage vor der Inokulation eine Reduktion der vom Rost verursachten Läsionen um 46 - 70 %, beobachtet (Feng C.C.P. et al., PNAS 102, 17290 (2005)). Es ist jedoch abzuwarten, ob diese Wirkung auch in Feldversuchen erhalten bleibt.

P. pachyrhizi gewinnt in den letzten Jahren eine immer größere Bedeutung als Schädling im Sojaanbau. Es besteht im Stand der Technik daher eine Nachfrage, Methoden zur Bekämpfung des Pilzes zu entwickeln. Von der Züchtung ist dabei vorerst kein Beitrag zu erwarten, da die vorhandenen Resistenzquellen nicht zugänglich sind. Die Behandlung mit Fungiziden hat eine begrenzte Wirkung und ist nur effektiv, wenn die Krankheit noch nicht ausgebrochen ist. Aus diesen Gründen bietet sich insbesondere das Pathosystem Soja mit P. pachyrhizi für einen transgenen Ansatz an. Für einen aussichtsreichen Ansatz ist es zunächst wichtig den Infektionsverlauf und die Reaktion der Pflanze besonders in den ersten Stadien der Infektion genau zu kennen. Potentielle Resistenz vermittelnde Kandidatengene müssen identifiziert und ihre Wirkung in der Interaktion zwischen der Pflanze und dem Pathogen charakterisiert werden. Die stabile Transformation von Pflanzen ist sehr zeit- und kostenintensiv, daher wird eine transiente Transformation, die eine Charakterisierung der Pflanze-Pathogen Interaktion auf dem Level der einzelnen Zelle ermöglicht, bevorzugt. Als Methode bietet sich dabei die transiente ballistische Transformation von Blättern an. Die Nicht-Wirtsresistenz ist bei Pflanzen besonders effektiv und dauerhaft und beruht auf einer im Vergleich zur kompatiblen Reaktion erhöhten Quantität und Geschwindigkeit der Resistenzreaktion. Eine Charakterisierung entscheidender Gene in der Nicht-Wirtsresistenz kann so der Identifizierung potentieller Kandidatengene für die Herstellung resistenter Pflanzen dienen. Um daher auch die Reaktion einer Nicht-Wirtspflanze auf den Sojarost zu untersuchen, wurde in den Beispielen das System Gerste (Hordeum vulgare) mit P. pachyrhizi gewählt, da Gerste eine gut beschriebene Modellpflanze ist. Zusätzlich ist das Pathosystem von Gerste mit Blumeria graminis f.sp. hordei (Bgh) und dem inkompatiblen Blumeria graminis f.sp. tritici (Bgt) sehr gut untersucht. Obwohl Bgh und P. pachyrhizi in mancher Hinsicht Unterschiede aufweisen, haben sie zumindest phänotypisch einige Schritte zu Beginn des Infektionsvorganges gemeinsam. So kann die Analyse in Gerste Aufschluss über die Mechanismen der Nicht-Wirtsresistenz gegenüber P. pachyrhizi im Vergleich zur Wirts-Resistenz der Gerste geben. In transient transformierten Gerstenblättern wurden daher Kandidatengene auf ihre Effekte in der Resistenz gegenüber P. pachyrhizi geprüft. Mit der transienten ballistischen Transformation steht eine Methode zur Verfügung eine Reihe von potentiell in der Resistenzantwort beteiligter Genen in dem Pathosystem Gerste mit P. pachyrhizi zu untersuchen.

Der programmierte Zelltod (,programmed cell death' PCD) ist ein wichtiger Prozess in der Entwicklung und der Stressantwort von Pflanzen und Tieren. Einige morphologische und biochemische Veränderungen der Zelle, wie Chromatinkondensation, Schrumpfen des Cytoplasmas und DNA-Fragmentierung scheinen Pflanzen und Tieren dabei gemeinsam zu sein (Lam E. et al., Nature 411, 848 (2001)). Eine klare Trennung des PCD und der durch biotischen oder abiotischen Stress hervorgerufenen HR ist nicht möglich (Heath M., Plant Mol. Biol. 44, 321 (2000)). Ein Aktivator des PCD in Tieren ist BAX. BAX bildet in der äußeren Mitochondrienmembran Kanäle und bewirkt die Abgabe von Cytochrom c. Dieses löst eine Caspase Kaskade und damit die Proteolyse von, für die Zelle lebensnotwendigen Proteinen aus (Green D.R. et al., Science 281, 1309 (1998). Die Überexpression von BAX in Tabak (N. tabacum, Lacomme C. et al., Proc. Nat. Acad. Sci. USA 96, 7956 (1999)) und Arabidopsis thaliana (Kawai-Yamada M. et al., Plant Cell 16, 21 (2004)) verursacht einen PCD und weist damit auf ähnliche Mechanismen bei Pflanzen und Tieren hin. Bisher konnten aber keine Homologe von BAX in Pflanzen identifiziert werden. Dagegen ist ein zu BAX antagonistischer Regulator des PCD, der BAX Inhibitor-1 (BI-1), in Pflanzen, Tieren und anderen Organismen, wie Hefe, konserviert. Ähnliche Proteine wurden inzwischen in A. thaliana, H. vulgare, Brassica napus, Brassica oleracea, Oryza sativa und N. tabacum identifiziert (Hückelhoven R., Apoptosis 9, 299 (2004)). In Experimenten mit BI-1-GFP Fusionsproteinen wurde eine Lokalisation von BI-1 in der Membran des Endoplasmatischen Refikulums (ER) und der Kernmembran beobachtet (Eichmann R. et al., Mol. Plant Microbe Interact. 17, 484 (2004)). Das Protein hat eine Größe von 25 - 27 kDa und besitzt 6 - 7 transmembranale Domänen. Der wahrscheinlich im Cytoplasma lokalisierte C-Terminus (Bolduc N. et al., Planta 216, 377 (2003)) ist essentiell für die Funktion von BI-1 (Kawai-Yamada et al., Plant Cell 16, 21 (2004)). Die transmembranalen Domänen bilden möglicherweise einen lonenkanal (Bolduc N. et al., Planta 216, 377 (2003)). So könnte, aufgrund der Speicherfunktion des ER für Ca²⁺, BI-1 eine Funktion in der Regulation des cytosolischen Ca²⁺-Spiegels und/oder des Redoxstatus der Zelle haben (Xu Q. et al., Mol. Cell 18, 1084 (1998); Balduc N. et al., FEBS Lett. 532, 111 (2003); Hückelhoven R. et al., Proc. Natl. Acad Sci. USA 29, 5555 (2003); Matsumura H. et al., Plant J. 33, 425 (2003)). In tierischen Zellen gibt es keine direkte physikalische Interaktion von BI-1 mit Bax. Aber BI-1 interagiert mit anderen Regulatoren des PCD (Xu Q. et al., Mol. Cell 18, 1084 (1998)). Wahrscheinlich interagiert BI-1 in Pflanzen ebenfalls mit anderen Regulatoren des PCD und beeinflusst so die Resistenzreaktionen. In Arabidopsis kann BI-1 den BAX und den H₂O₂ induzierten PCD unterdrücken (Baek D. et al., Plant Mol. Biol. 56, 15 (2004); Kawai-Yamada M.et al., Plant Cell 16, 21 (2004)). Es reguliert daher wahrscheinlich die Prozesse in einer tieferen Ebene als der oxidativen Stressantwort (Kawai-Yamada M. et al., Plant Cell 16, 21 (2004)).
Die Expression von BI-1 wird durch biotischen und abiotischen Stress, wie Pathogenangriff oder Verwundung, aber auch in alternden Geweben induziert (Balduc N. et al., FEBS Lett. 532, 111 (2003); Hückelhoven R., Apoptosis 9, 299 (2004)). In Arabidopsis sind die mRNA-Level von BI-1 nach Hitzeschock erhöht (Watanabe N. et al., Plant J. 45, 884 (2006)). In Tomate (Lycopesicum esculentum) wirkt H₂O₂ und in Arabidopsis H₂O₂ und Salicylsäure induzierend auf die BI-1 Expression (Hückelhoven R., Apoptosis 9, 299 (2004); Kawai-Yamada M.et al., Plant Cell 16, 21 (2004)). Dies weist auf eine Funktion von BI-1 in der Pahogenabwehr hin, denn beide Substanzen spielen bei dieser eine wichtige Rolle (Prell H.H., Interaktionen von Pflanzen und phytopathogenen Pilzen, Gustav Fischer Verlag, Jena, 1996).

Dementsprechend löst die Infektion von Gerste mit Bgh oder Bgt eine Steigerung der Expression von BI-1 aus (Hückelhoven R. et al., Plant Mol. Biol. 47, 739 (2001); Eichmann R. et al., Mol. Plant Microbe Interact. 17, 484 (2004)). In Reis folgt die Expression nach der Infektion mit M. grisea einem biphasischen Verlauf. Sie ist zunächst leicht erhöht, wird aber 12 h nach der Infektion wieder reduziert, um anschließend wieder zu steigen (Matsumura H. et al., Plant J. 33, 425 (2003)). Die BI-1 Expressionssteigerung in Arabidopsis Zellen nach der Behandlung mit Fumonisin B1 (Watanabe N. et al., Plant J. 45, 884 (2006)) und die Reduktion der BI-1 Expression nach der Behandlung von Reiszellen mit M. grisea Elicitorextrakt zeigt, dass die Expressionsmuster abhängig von dem induzierenden Faktor und der Pflanze sehr unterschiedlich sein können. Die Expressionsmuster deuten auf eine Rolle von Bl-1 in der Regulation des stressinduzierten PCD bzw. HR und der Resistenzreaktion gegen Pathogene hin. Die Beeinflussung der HR kann die Resistenz einer Pflanze erhöhen, vor allem, wenn es sich bei den Pathogenen um perto- oder hemibiotrophe Pilze handelt. Eine Überexpression von Bl-1 in Karotten (Daucus carota ssp. sativa) führt zu einer Resistenz der Pflanzen gegenüber Botrytis cinerea (Imani J. et al, Mol. Plant Physiol. in press). In Tomaten schützt die Expression des PCD Inhibitors p35 vor Alternaria alternata, Colletotrichum coccodes und Pseudomonas syringae (Lincoln J.E. et al., Proc. Nat. Acad. Sci. USA 99, 15217 (2002)).

Vor diesem Hintergrund bestand im Stand der Technik ein fortdauerndes Bedürfnis nach landwirtschaftlich genutzten Pflanzen, die eine erhöhte Resistenz gegen Pathogene aufweisen. Es gibt nur wenige Ansätze, die Pflanzen eine Resistenz gegen ein breiteres Spektrum von Pathogenen, vor allem Pilzpathogene, verleihen. Die systemische erworbene Resistenz ("systemic acquired resistance"; SAR) - ein Abwehrmechanismus bei verschiedenen Pflanze/Pathogen-Interaktionen - kann durch Applikation von endogene Botenstoffe wie Jasmonat (JA) oder Salizylsäure (SA) vermittelt werden (Ward J.M. et al., Plant Cell 3, 1085 (1991); Uknes et al., Plant Cell 4(6), 645 (1992)). Ähnliche Effekte können auch durch synthetische Verbindungen wie 2,6-Dichlorisonikotinsäure (DCINA) oder Benzo(1,2,3)thiadiazol-7-thiocarbonsäure-S-methylester (BTH; Bion®) (Friedrich et al., Plant J. 10(1), 61 (1996); Lawton et al., Plant J. 10, 71 (1996)) bewirkt werden. Auch die Expression der im Rahmen eines SAR hochregulierten "pathogenesis related" (PR) Proteine vermag zum Teil eine Pathogenresistenz zu bewirken.

In Gerste ist der Mlo-Locus als negativer Regulator der Pathogenabwehr beschrieben. Der Verlust oder Funktionsverlust ("loss-of-function") des Mlo-Gens bedingt eine erhöhte, rassenunspezifische Resistenz gegen zahlreiche Mehltauisolate (Büschges R. et al., Cell 88, 695 1997); Jorgensen J.H., Euphytica 26, 55 (1977); Lyngkjaer M.F. et al. Plant Pathol 44, 786 (1995)).

Das Mlo-Gen ist beschrieben (Büschges R. et al., Cell 88, 695 (1997); WO 98/04586; Schulze-Lefert P. et al.,Trends Plant Sci. 5, 343 (2000)). Verschiedene Mlo-Homologe aus anderen Getreidearten wurden isoliert. Verfahren unter Verwendung dieser Gene zum Erzielen einer Pathogenresistenz sind beschrieben (WO 98/04586; WO 00/01722; WO 99/47552). Nachteilig ist, dass Mlo-defiziente Pflanzen auch in Abwesenheit eines Pathogens den o.g. Abwehrmechanismus initiieren, was sich in einem spontanen Absterben von Blattzellen äußert (Wolter M. et al., Mol. Gen. Genet. 239, 122 (1993)). Dadurch erleiden mlo-resistente Pflanzen eine Ertragseinbuße von ca. 5% (Jörgensen J.H., Euphytica 63, 141 (1992)). Das spontane Absterben der Blattzellen bedingt ferner eine nachteilige Hypersuszeptibilität gegen nekrotrophe und hemibiotrophe Pathogene wie Magnaporthe grisea (M. grisea) oder Cochliobolus sativus (Bipolaris sorokiniana) (Jarosch B. et al., Mol Plant Microbe Interact. 12, 508 (1999); Kumar J. et al., Phytopathology 91, 127 (2001)).

Apoptose, auch als programmierter Zelltod bezeichnet, ist ein essentieller Mechanismus zur Aufrechterhaltung der Gewebehomöostase und steht damit der Zellteilung als negativ regulierender Mechanismus gegenüber. Im vielzelligen Organismus ist die Apoptose ein natürlicher Bestandteil der Ontogenese und u.a. an der Entwicklung der Organe und der Beseitigung von gealterten, infizierten oder mutierten Zellen beteiligt. Durch die Apoptose wird eine effiziente Elimination von unerwünschten Zellen erreicht. Eine Störung oder Inhibition der Apoptose trägt zur Pathogenese verschiedener Erkrankungen bei, unter anderem zur Karzinogenese. Die Haupteffektoren der Apoptose sind Aspartat-spezifische Cystein-Proteasen, die sogenannten Caspasen. Ihre Aktivierung kann im Allgemeinen durch mindestens zwei Apoptose-Signalwege stattfinden: Zum einen durch die Aktivierung der TNF-(Tumor Necrosis Factor) Rezeptorfamilie, zum anderen spielen Mitochondrien eine zentrale Rolle. Die Aktivierung des mitochondrialen Apoptose-Signalweges wird durch Proteine der Bcl-2-Familie reguliert. Diese Proteinfamilie besteht aus anti-apoptotischen sowie pro-apoptotischen Proteinen wie z.B. Bax. Im Falle eines apoptotischen Stimulus findet eine allosterische Konformationsänderung des Bax-Proteins statt, welche zur Verankerung des Proteins in der mitochondrialen Außenmembran und seiner Oligomerisierung führt. Durch diese Oligomere werden pro-apoptotischen Moleküle aus den Mitochondrien ins Zytosol freigesetzt, die eine apoptotische Signalkaskade und letztlich die Degradierung spezifischer zellulärer Substrate bedingen, was den Zelltod zur Folge hat. Der Bax Inhibitor-1 (BI1) wurde über seine Eigenschaft isoliert, die pro-apoptotische Wirkung von BAX zu inhibieren (Xu Q. et al., Mol Cell 1(3), 337 (1998)). BI1 stellt ein hochkonserviertes Protein dar. Es findet sich überwiegend als integraler Bestandteil intrazellulärer Membranen. BI1 interagiert mit bcl-2 und bcl-xl. Überexpression von BI1 in Säugetierzellen unterdrückt die pro-apoptotische Wirkung von BAX, Etoposid und Staurosporin, aber nicht von Fas-Antigen (Roth W. et al., Nat. Med. 8, 216 (2002)). Die Inhibition von BI1 durch antisense-RNA hingegen induziert Apoptose (Xu Q. et al. ,Mol Cell 1(3), 337 1998)). Die ersten pflanzlichen Homologen von BI1 wurden aus Reis und Arabidopsis isoliert (Kawai et al., FEBS Lett 464, 143 (1999); Sanchez et al., Plant J. 21, 393 (2000)). Diese pflanzlichen Proteine supprimieren BAX-induzierten Zelltod in Hefe. Die Aminosäure-Sequenzhomologie zu menschlichem BI1 beträgt ca. 45%. Das Arabidopsis-Homolog AtBl1 vermag in rekombinanten Pflanzen die pro-apoptotische Wirkung von BAX aus Maus zu supprimieren (Kawai-Yamada M. et al., Proc. Natl. Acad. Sci. USA, 98(21), 12295 (2001)). Das Reis (Oryza sativa) Bl1-Homolog OsBl1 wird in allen pflanzlichen Geweben exprimiert (Kawai et al., FEBS Lett 464, 143(1999)). Beschrieben sind ferner Bl1-Gene aus Gerste (Hordeum vulgare; GenBank Acc.-No.: AJ290421), Reis (GenBank Acc.-No.: AB025926), Arabidopsis (GenBank Acc.-No.: AB025927), Tabak (GenBank Acc.-No.: AF390556) und Raps (GenBank Acc.-No.: AF390555, Bolduc N. et al., Planta 216, 377-386(2003)). Die Expression von BI1 in Gerste wird infolge einer Infektion mit Mehltau hochreguliert (Hückelhoven R. et al., Plant Mol. Biol. 47(6), 739 (2001)).

WO 00/26391 beschreibt die Überexpression der anti-apoptotischen Gene Ced-9 aus C. elegans, sflAP aus Spodoptera frugiperda, bcl-2 aus Mensch sowie bcl-xl aus Huhn in Pflanzen zur Erhöhung der Resistenz gegen nekrotrophe bzw. hemibiotrophe Pilze. Pflanzliche BI1 Homologe werden nicht offenbart. Die Expression erfolgt unter Kontrolle konstitutiver Promotoren. Beschrieben ist ferner die Expression eines BI1 Proteins aus Arabidopsis unter dem starken konstitutiven 35S CaMV Promotor in Reiszellen und eine dadurch induzierte Resistenz gegen Zelltod induzierende Substanzen aus Magnaporthe grisea (Matsumura H. et al., Plant J. 33, 425 (2003)).

Im Stand der Technik wurde ursprünglich beschrieben, dass eine konstitutive Expression eines Inhibitors der programmierten Zelltods in Pflanzen eine Resistenz gegen nekrotrophe Pilze bedingen kann.

Dem Fachmann stellte sich hier jedoch insbesondere die Aufgabe, Verfahren für die Pathogenabwehr in Pflanzen bereitzustellen, und zwar insbesondere gegen biotrophe Pathogene.

Die Aufgabe wird überraschenderweise durch das in den Hauptansprüchen definierte erfindungsgemäße Verfahren gelöst. Die Unteransprüche definieren spezielle besonders bevorzugte Ausführungsformen der vorliegenden Erfindung.

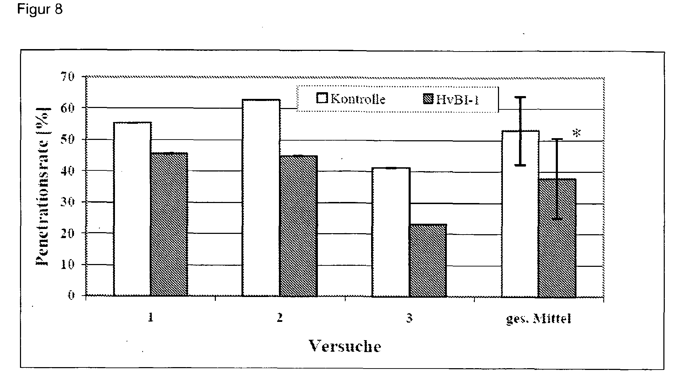

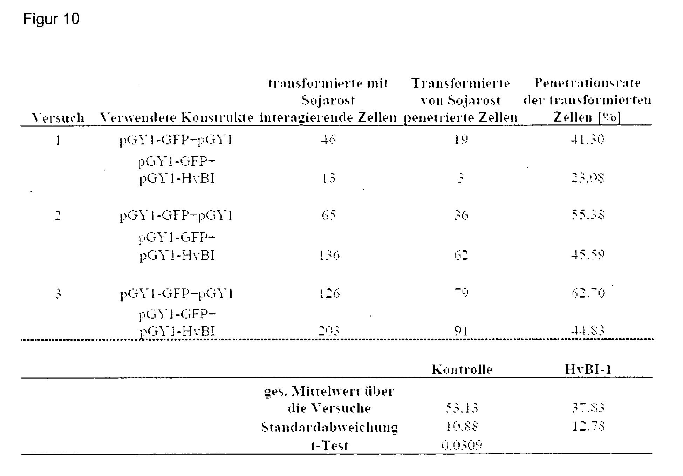

Die Rolle von BI-1 wurde im transienten Transformationssystem in drei voneinander unabhängigen Experimenten getestet. In der Kontrolle wurden über die Versuche gemittelt 53 % der transformierten mit P. pachyrhizi interagierenden Zellen penetriert, während von den mit BI-1 transformierten Zellen nur 37 % penetriert wurden (FIG. 8; Tabelle FIG. 10). Drei voneinander unabhängige Experimente liegen den Werten zugrunde. Als Kontrolle wurden Gersteblätter mit dem Reportergenkonstrukt pGY1-GFP und dem leeren Vektor transformiert. Die Penetrationsrate ist in den mit BI-1 transformierten Zellen signifikant verschieden vom WT (P<0.05). Nach der Auswertung zeigen die transient mit BI-1 transformierten Zellen daher überraschenderweise eine signifikant erhöhte Penetrationsresistenz gegen P. pachyrhizi (P< 0.05; Abb. 8; Tabelle FIG. 10). Auffällig war bei der mikroskopischen Beobachtung, dass die BI-1 bildenden Zellen deutlich vitaler wirkten als Zellen die GFP oder ADF3 exprimierten.

Bereits die transiente Überexpression des Zelltodinhibitors BI-1 in Gerste ließ somit überraschenderweise die Tendenz zu einer erhöhten Resistenz der Pflanzenzellen gegenüber der Penetration durch Sojarost erkennen. Um dies zu bestätigen, wurde die Interaktion zwischen transgenen Gerstenpflanzen der Sorte ,Golden Promise' (GP), die ein GFP-BI-1 Überexpressionskonstrukt enthalten im Vergleich zum Wildtyp (WT) der Sorte mikroskopisch untersucht. Für die Herstellung der transgenen Pflanzen wurde eine GFP-BI-1 Fusion unter der Kontrolle des konstitutiven CAMV 35S Promoters verwendet und so eine ausreichende Expression des Proteins gewährleistet.

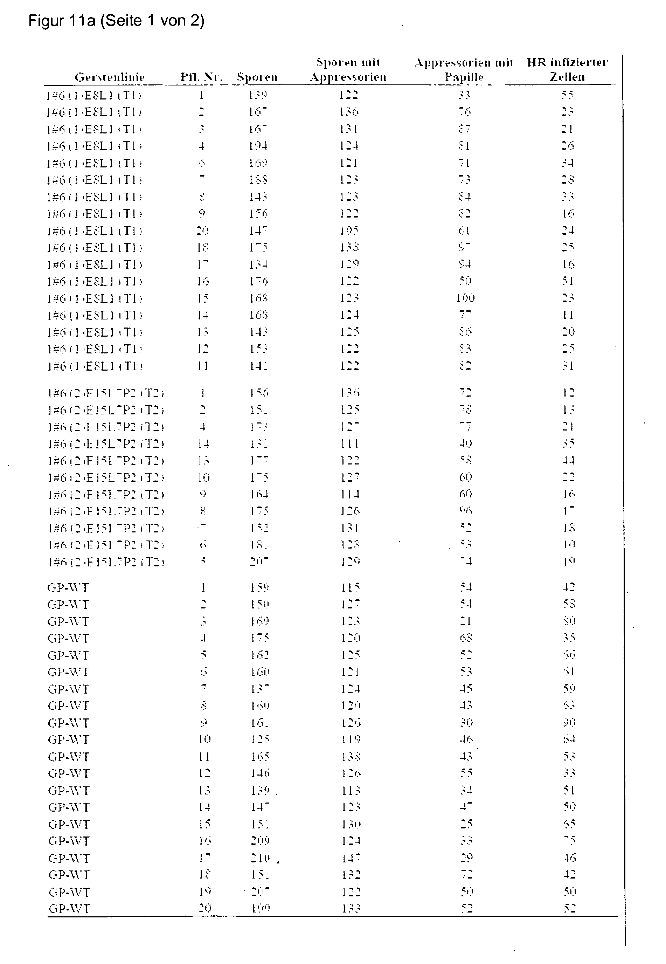

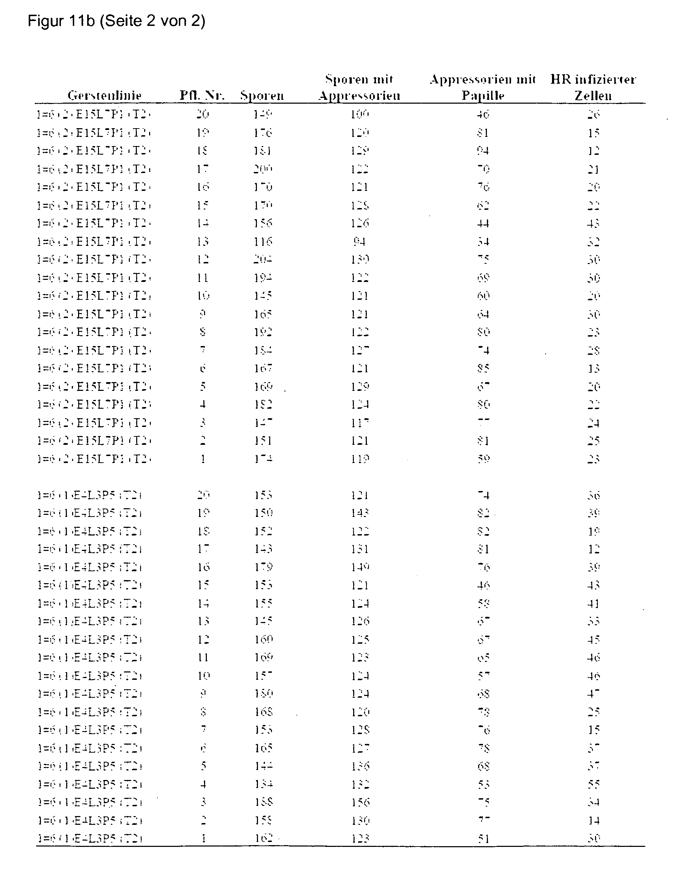

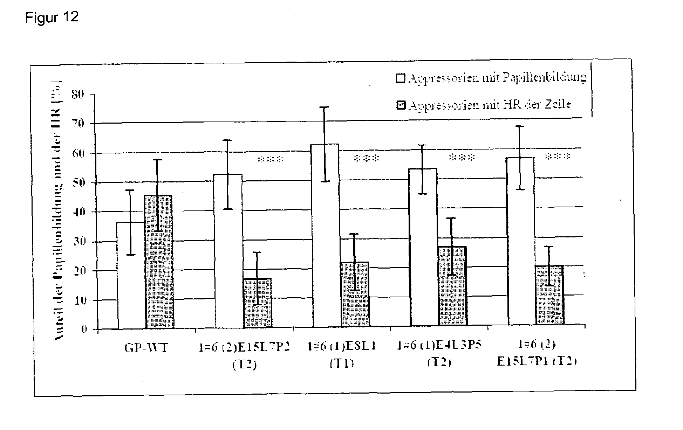

In einem Versuch wurden Blätter des WT der Sorte ,Golden Promise' und der transgenen Gerste-Linie (Sorte ,Golden Promise') #6(1)E8L1(T1)' mit P. pachyrhizi inokuliert und 24 h nach der Inokulation in Entfärbelösung fixiert. Nach der vollständigen Entfärbung wurden die Blätter mit Anillinblau gefärbt. Anilinblau lagert sich in die Struktur von Kallose ein und färbt so bevorzugt Papillen an, bei denen es zur Akkumulation und Vernetzung von Kallose mit anderen polymeren Substanzen kommt. Zellen, die durch eine hypersensitive Reaktion (H R), einen der Apoptose in Säugerzellen ähnlichen Prozess durchlaufen haben, zeigen nach Anillinblaufärbung ebenfalls eine helle Fluoreszenz. Auf den inokulierten Gersteblättern wurde die Anzahl der Sporen, die gekeimten Sporen mit Keimschläuchen die bereits ein Appressorium gebildet hatten und, als Zellreaktion, die Appressorien mit darunter liegenden Papillen sowie die HR gezählt. Größere Sporenzusammenballungen, bei denen eine Zuordnung der Appressorien zu den Sporen nicht mehr möglich war, wurden nicht mitgezählt. Es wurden, soweit möglich, pro Blatt mindestens 100 Sporen mit Appressorien ausgezählt. Schon in diesem Versuch konnte in der transgenen Linie eine signifikant gesteigerte Papillenbildung und eine signifikant verminderte H R der Zellen beobachtet werden (P<0.01; Tabelle FIG. 11 A/B, FIG. 12).

Sowohl Bgh (Hückelhoven R., FEMS Microbiol. Letters 245, 9 (2005)) als auch P. pachyrhizi (Koch E. et al., Phytopath. Z. 106, 302 (1983)) sind biotrophe Pathogene. Eine H R der befallenen Zellen kann daher die Entwicklung der Pilze stoppen, da sie ihnen die Nahrungsgrundlage entzieht. Wird die HR unterbunden, können die Zellen gegenüber einer Infektion durch biotrophe Pathogene empfindlicher werden. Trotzdem zeigen transient, mit einem BI-1 Überexpressionskonstrukt transformierte Gerstezellen überraschenderweise eine erhöhte Resistenz gegen die Penetration durch P. pachyrhizi. Dies überrascht, da eine erhöhte Sensitivität gegenüber dem biotrophen Pilz zu erwarten wäre. Allerdings ist die HR bei der Resistenz von Gerste und Weizen gegen das inkompatible Pathogen P. pachyrhizi nicht die einzige und möglicherweise nicht die entscheidende Resistenzreaktion. So wird P. pachyrhizi in Weizen durch die Bildung von Papillen abgewehrt (Hoppe H.H. et al., Pro. Intern. Congress of SABRAO (Bangkok 1985) 1986). Gerste reagiert, abhängig von der Sorte, mit Papillenbildung und HR der infizierten Zellen. Gerste mit dem mlo5 Allel reagiert zudem mit einer gesteigerten Papillenbildung. Bgt wird von Gerste auch durch Papillenbildung, aber vor allem durch eine HR infizierter Zellen abgewehrt (Hückelhoven R. et al., Mol. Plant Pathol. 2, 199 (2001). Im Gegensatz zu P. pachyrhizi führt aber die transiente Überexpression von BI-1 in Gerste zu einer erhöhten Pentrationsrate der Zellen durch Bgt (Eichmann R. et al., Mol. Plant Microbe Interact. 17, 484 (2004)). Ebenso zeigen Gerstenpflanzen mit dem mlo5 Allel, das eine breite Resistenz gegen Bgh vermittelt, bei transienter Überexpression von BI-1 eine größere Empfindlichkeit der Zellen gegenüber einer Penetration durch Bgh (Hückelhoven R. et al., Proc. Natl. Acad. Sci. USA, 29, 5555 (2003). Obwohl die Resistenz von Gerste mit dem mlo5 Allel nicht auf einer HR der infizierten Zellen sondern auf einer effizienteren Akkumulation von antimikrobiellen Verbindungen, H₂O₂ und einer gesteigerten Papillenbildung beruht, scheint die Inhibition der HR auch die anderen Resistenzreaktionen zu beeinflussen (Hückelhoven R. et al., Plant Physiol. 119, 1251 (1999)). Ohne durch Theorie eine Einschränkung herbeizuführen, diese Beobachtungen einer verringerten Penetrationsresistenz durch die Inhibition der HR deutet auf eine vernetzte Regulation der Resistenzreaktionen hin. Die Vermutung der vernetzten Regulation von Resistenzreaktionen wird durch die mikroskopische Analyse transgener mit P. pachyrhizi inokulierter Gerstepflanzen unterstützt. Diese tragen ein BI-1 Überexpressionskonstrukt und reagieren auf die Infektion mit einer gesteigerten Papillenbildung. Auf den Blättern der transgenen Pflanzen zeigten nur 16 - 26 % der infizierten Zellen eine HR, dagegen war bei ca. 50 % der befallenen Zellen auf den WT-Blättern eine H R zu sehen. In der Gerstensorte ,Golden Promise' scheint daher die H R der Zellen auch ein wichtiger Resistenzmechanismus gegen den biotrophen P. pachyrhizi zu sein, obwohl P. pachyrhizi zumindest in Soja die Epidermiszellen nicht zu seiner Ernährung heranzieht sondern erst im Mesophyll Haustorien bildet (Koch E. et al., Phytopath. Z. 106, 302 (1983). Dieses Stadium erreicht der Pilz aber auf der Nicht-Wirtspflanze Gerste im Allgemeinen nicht. Die Inhibition der HR ermöglicht Bgt die Penetration der Zellen und es gelingt dem Pilz sich erfolgreich zu etablieren (Eichmann R. et al., Mol. Plant Microbe Interact. 17, 484 (2004)). Für diesen Prozess scheinen weitere pilzspezifische Faktoren notwendig zu sein. Anscheinend können diese speziellen Faktoren des Bgt entweder die alternative Resistenzantwort der Pflanzenzelle oder die Erkennung durch diese supprimieren. Diese Faktoren fehlen P. pachyrhizi wahrscheinlich. So kann die Pflanzenzelle P. pachyrhizi möglicherweise erkennen und, da BI-1 die HR unterdrückt, eine alternative Resistenzantwort, die Papillenbildung, induzieren. Eine Erkennung von P. pachyrhizi durch die Pflanze wird durch die Beobachtung der gesteigerten Papillenbildung in Gerste mit dem mlo5 Allel unterstützt. Warum aber die Pflanze das Pathogen P. pachyrhizi, das auf Wirtspflanzen einer völlig anderen Ordnung spezialisiert ist, erkennen kann bleibt offen (Sinclair J. B. et al. (eds.), Proceedings of the soybean rust workshop (1995), National Soybean Research Laboratory, Publication No. 1 (1996)). Das ,Erkennungsmerkmal' von P. pachyrhizi könnte ein unspezifischer Elicitor (,pathogenesis-associated molecular patterns' PAMP) wie das INF1 aus P. infestans sein, das in Nicotiana sp. eine HR auslöst (Kamoun S. et al., Plant Cell 10, 1413 (1998)). PAMPs werden von der Pflanze als Fremdmoleküle erkannt und lösen eine Resistenzreaktion aus. Nicht alle Blätter der transgenen Pflanzen zeigten die gesteigerte Papillenbildung, obwohl das BI-1 Genkonstrukt in ihnen mittels PCR nachgewiesen werden konnte. Dies könnte die Folge eines ungünstigen Insertionsortes des Konstruktes sein, der eine effektive Expression des BI-1 verhindert und/oder zu antagonistischen Effekten durch andere Gene führt. Da der Nachweis auf DNA-Ebene erfolgte, kann keine Aussage über die Expression des BI-1 gemacht werden. Zu beachten ist auch, dass schon kleine Beschädigungen der Samen oder der Blätter die Entwicklung der Pflanze entscheidend beeinflussen oder verhindern können. So sind einige Samen der Linie #6(2)E15L7P2 (T2) nicht gekeimt.

Ein Gegenstand der Erfindung betrifft daher ein Verfahren zum Erzeugen oder Erhöhen einer Resistenz in einer Sojapflanze, oder einem Teil einer Sojapflanze, gegen mindestens einen biotrophen Pilz aus dem Genus Phakopsora. Das Verfahren umfasst einen Schritt, in dem die Menge eines Bax Inhibitor-1 (BI1) Proteins oder dessen Funktion in der Sojapflanze oder zumindest einem Teil einer Sojapflanze erhöht wird. Unter einem Teil einer Pflanze ist die ganze Pflanze, ein oder mehrere Organe von ihr, ein Gewebe oder mindestens eine Zelle gemeint. Das Verfahren umfasst ferner den Schritt, dass eine Pflanze oder ein Teil einer Pflanze, in welchem/ welcher das BI1 Protein oder dessen Funktion erhöht wurde, dahingehend ausgewählt wird, dass er/sie eine erhöhte Resistenz gegen mindestens einen biotrophen Pilz aus dem Genus Phakopsora im Vergleich zu einer Ausgangspflanze aufweist, bei dem/der die Erhöhung der Menge oder Funktion des BI1 Proteins gegenüber einer Ausgangspflanze oder deren Teil erhöht ist.

Gemäß der vorliegenden Erfindung ist der biotrophe Pilz ausgewählt aus der Gattung Phakopsora. In besonders bevorzugten Ausführungsformen ist das Pathogen Phakopsora pachyrhizi und/oder P. meibomiae (gemeinsam auch als "Sojabohnenrost" oder "Sojarost" bezeichnet), wobei der erstere am meisten bevorzugt ist.

Der Schritt der Erhöhung der Menge oder Funktion des BI1 Proteins wird durch biotechnologische Verfahren erzielt. "Biotechnologische" Verfahren umfasst unter anderem die rekombinante Expression des Proteins in einer Zelle, vorzugsweise funktionell verknüpft mit und angetrieben durch einen heterologen Promotor. Die genannte Erhöhung kann aber auch beispielsweise durch Austausch nur des endogenen Promotors oder bspw. Silencing von Faktoren, die die Expression des endogenen BI1 Proteins im allgemeinen hemmen, erreicht werden. Dementsprechend umfasst der Begriff der biotechnologischen Verfahren alle der modernen Molekularbiologie offenstehenden Verfahen, insbesondere die der rekombinanten Gentechnologie, die dem Fachmann bekannt sind, und auf die im Laufe der Beschreibung noch eingehender eingegangen werden wird.

DiePflanze ist aus den Leguminosen ausgewählt, umfassend Sojabohne (Glycine max (L.) Merill)).

In jeder der hierin offenbarten Ausführungsformen der vorliegenden Erfindung ist es besonders bevorzugt, dass die Menge oder Funktion des BI1 Proteins zumindest in der Epidermis erhöht wird, vorzugsweise im wesentlichen gewebespezifisch in der Epidermis; insbesondere bevorzugt ist es, dass die Menge oder Funktion des BI1 Proteins spezifisch in der Epidermis erhöht wird und/oder im Mesophyll im wesentlichen nicht erhöht wird.

Unter der Epidermis versteht der Fachmann das vorherrschende Abschlussgewebe primärer oberirdischer Pflanzenteile, so des Sprosses, der Blätter, Blüten, Früchte und Samen. Nach außen scheiden die Epidermiszellen eine wasserabstoßende Schicht, die Kutikula ab. Die Wurzeln sind von der Rhizodermis umgeben, welche der Epidermis in vieler Hinsicht ähnelt, jedoch auch markante Unterschiede zu ihr aufweist. Die Epidermis entsteht aus der äußersten Schicht des Apikalmeristems. Die Ableitung der Rhizodermis hingegen ist weniger klar. Je nach Art kann sie entwicklungsgeschichtlich entweder der Wurzelhaube oder der primären Rinde zugerechnet werden. Der Epidermis können zahlreiche Funktionen zugeschrieben werden: Sie bietet der Pflanze Schutz vor Austrocknung und regelt die Transpirationsrate. Sie schützt die Pflanze vor den verschiedensten chemischen und physikalischen Fremdeinflüssen sowie vor Tierfraß und Befall durch Parasiten. Sie ist am Gasaustausch, an der Sekretion bestimmter Stoffwechselprodukte und an der Absorption von Wasser beteiligt. In ihr sind Rezeptoren für Licht und mechanische Reize enthalten. Sie wirkt damit als ein Signalwandler zwischen Umwelt und Pflanze. Entsprechend den verschiedenen Funktionen enthält die Epidermis eine Anzahl unterschiedlich differenzierter Zellen. Hinzu kommen artspezifische Varianten und unterschiedliche Organisation der Epidermen in den einzelnen Teilen einer Pflanze. Im wesentlichen besteht sie aus drei Kategorien von Zellen: den "eigentlichen" Epidermiszellen, den Zellen der Stomata (Spaltöffnungen) und den Trichomen (griech.: Trichoma, Haar), epidermalen Anhangsgebilden verschiedener Form, Struktur und Funktion.

Die "eigentlichen", d.h., die am wenigsten spezialisierten Epidermiszellen machen die Hauptmasse der Zellen des Abschlussgewebes aus. Sie sind in der Aufsicht entweder polygonal (von platten- oder tafelförmiger Gestalt) oder gestreckt. Die zwischen ihnen ausgebildeten Wände sind vielfach gewellt oder gebuchtet. Wodurch diese Form während der Entwicklung induziert wird, ist unbekannt, die vorliegenden Hypothesen erklären den Sachverhalt nur unbefriedigend. Gestreckte Epidermiszellen findet man an Organen oder Organteilen, die selbst gestreckt sind, so z.B. an Stengeln, Blattstielen und Blattrippen sowie an den Blättern der meisten Monokotyledonen. Ober- und Unterseite von Blattspreiten können von unterschiedlich strukturierten Epidermen bedeckt sein wobei sowohl die Form der Zellen, die Dicke der Wände als auch die Verteilung und Zahl spezialisierter Zellen (Stomata und/oder Trichome) pro Flächeneinheit variieren kann. Große Variationen findet man auch innerhalb einzelner Familien, z. B. bei den Crassulaceen. Meist ist die Epidermis einschichtig, jedoch sind bei Arten aus mehreren Familien (Moraceae: hier die meisten Ficus-Arten, Piperaceae: Peperonia [Peperonie], Begoniaceae, Malvaceae u.a.) mehrschichtige wasserspeichernde Epidermen nachgewiesen worden. Epidermiszellen sondern nach außen eine Cutinschicht (Kutikula) ab, die als ein im wesentlichen ununterbrochener Film im wesentlichen alle epidermalen Oberflächen überzieht. Sie kann entweder glatt oder durch Vorwölbungen, Leisten, Falten und Furchen strukturiert sein. Doch nicht immer beruht eine durch Betrachtung der Oberfläche sichtbare Faltung der Kutikula auf der Ausbildung von Kutikularleisten. Es gibt durchaus Fälle, wo eine Kutikulafaltung nur der Ausdruck der darunterliegenden Ausstülpungen der Zellwand ist. Epidermale Anhangsgebilde verschiedener Form, Struktur und Funktion werden als Trichome bezeichnet und hierin ebenfalls unter dem Begriff "Epidermis" verstanden.. Sie treten als Schutz-, Stütz- und Drüsenhaare in Form von Schuppen, verschiedenen Papillen und bei Wurzeln als absorbierende Haare auf. An ihrer Bildung sind allein Epidermiszellen beteiligt. Oft entsteht ein Trichom aus nur einer solchen Zelle, manchmal sind an der Entstehung mehrere beteiligt.

Ebenfalls umfasst unter dem Begriff "Epidermis" sind Papillen. Papillen sind Ausstülpungen der Epidermisoberfläche. Das Lehrbuchbeispiel hierfür sind die Papillen auf Blütenoberflächen des Stiefmütterchens (Viola tricolor) sowie die Blattoberflächen vieler Arten im tropischen Regenwald. Sie verleihen der Oberfläche eine samtartige Konsistenz. Einige Zellen von Epidermen können als Wasserspeicher ausgebildet sein. Ein typisches Beispiel stellen die Blasenzellen an Oberflächen vieler Mittagsblumenarten und anderer Sukkulenten dar. Bei manchen Pflanzen, z.B. bei der Glockenblume (Campanula persicifolia) sind die Außenwände der Epidermis linsenförmig verdickt.

Die Hauptmasse aller Gewebe bildet das Grundgewebe oder Parenchym. Zu den parenchymatischen Geweben gehört das Mesophyll , das in Blättern in Palisadenparenchym und Schwammparenchym differenziert sein kann.

Folglich versteht der Fachmann unter Mesophyll ein parenchymatisches Gewebe. Parenchymatische Zellen sind durchweg lebend, meist isodiametrisch, seltener gestreckt. Das Mark der Sprosse, die Speichergewebe der Früchte, Samen, der Wurzel und anderer unterirdischer Organe sind ebenso als Parenchyme zu betrachten wie das Mesophyll.

Das Mesophyll ist in den Blättern der meisten Farne und Phanerogamen, besonders ausgeprägt bei den Dikotyledonen und vielen Monokotyledonen, in Palisaden- und Schwammparenchym untergliedert. Ein "typisches" Blatt ist dorsiventral gebaut. Das Palisadenparenchym liegt dabei meist an der Blattoberseite unmittelbar unter der Epidermis. Das Schwammparenchym füllt den darunterliegenden Raum aus. Es ist von einem voluminösen Interzellularsystem durchsetzt, dessen Gasraum über die Spaltöffnungen in direktem Kontakt zur Außenwelt steht.

Das Palisadenparenchym besteht aus langgestreckten, zylindrischen Zellen. Bei einigen Arten sind die Zellen irregulär, gelegentlich sind sie gegabelt (Y-förmig: Armpalisadenparenchym). Solche Varianten kommen bei Farnen, Coniferen und einigen wenigen Angiospermen (z.B. bei einigen Ranunculaceen- und Caprifoliaceenarten [Beispiel: Holunder]) vor. Neben der eben beschriebenen, am weitesten verbreiteten Organisationsform sind die folgenden Varianten nachgewiesen worden:
- Palisadenparenchym an der Blattunterseite. Besonders auffällig bei Schuppenblättern. Beispiel: Lebensbaum (Thuja), sowie bei den Blättern des Bärlauchs (Allium ursinum).
- Palisadenparenchym an beiden Blattseiten (Ober- und Unterseite). Häufig bei Pflanzen trockener Standorte (Xerophyten). Beispiel: Kompasspflanze (Lactuca serriola);
- Ringförmig geschlossenes Palisadenparenchym: In zylindrisch organisierten Blättern und in Nadeln der Koniferen.

Die Variabilität der Schwammparenchymzellen und die Ausbildung des Schwammparenchyms selbst sind noch vielgestaltiger als die des Palisadenparenchyms. Es wird meist als Durchlüftungsgewebe bezeichnet, denn es enthält eine Vielzahl untereinander verbundener Interzellularen.

Das Mesophyll kann das so genannte Assimilationsgewebe umfassen, jedoch sind die Begriffe Mesophyll und Assimilationsgewebe nicht als Synonyme zu verwenden. Es gibt chloroplastenfreie Blätter, die sich in ihrem Aufbau nur unwesentlich von vergleichbaren, grünen Blättern unterscheiden. Folglich enthalten sie Mesophyll, doch eine Assimilation unterbleibt; umgekehrt findet eine Assimilation z.B. auch in Sproßabschnitten statt.

In der vorliegenden Beschreibung wird die Epidermis vorzugsweise biochemisch charakterisiert. Die Epidermis kann in einer bevorzugten Ausführungsform durch die Aktivität eines oder mehrer der folgenden Promotoren gekennzeichnet werden:
- WIR5 (=GstA1), acc. X56012; Dudler & Schweizer,
- GLP4, acc. AJ310534; Wei Y., Zhang Z., Andersen C.H., Schmelzer E., Gregersen P.L., Collinge D.B., Smedegaard-Petersen V. und Thordal-Christensen H., Plant Molecular Biology 36, 101 (1998),
- GLP2a, acc. AJ237942, Schweizer P., Christoffel A. und Dudler R., Plant J. 20, 541 (1999).;
- Prx7, acc. AJ003141, Kristensen B.K., Ammitzböll H., Rasmussen S.K. and Nielsen K.A., Molecular Plant Pathology, 2(6), 311 (2001);
- GerA, acc. AF250933 ; Wu S., Druka A., Horvath H., Kleinhofs A., Kannangara G. and von Wettstein D., Plant Phys Biochem 38, 685 (2000);
- OsROC1, acc. AP004656
- RTBV, acc. AAV62708, AAV62707 ; Klöti A., Henrich C., Bieri S., He X., Chen G., Burkhardt P.K., Wünn J., Lucca P., Hohn T., Potrykus I. und Fütterer J,. PMB 40, 249 (1999);
- Chitinase ChtC2-Promotor aus Kartoffel (Ancillo et al., Planta. 217(4), 566 (2003));
- AtProT3 Promotor (Grallath et al., Plant Physiology. 137(1), 117 (2005))
- SHN-Promotoren aus Arabidopsis (AP2/EREBP transcription factors involved in cutin and wax production) (Aarón et al., Plant Cell. 16(9), 2463 (2004));
- GSTA1 aus Weizen (Dudler et al., WP2005306368 bzw. Altpeter et al., Plant Molecular Biology. 57(2),271 (2005)).

In der vorliegenden Beschreibung wird die Epidermis dadurch gekennzeichnet, dass alle genannten Promoter in dem Gewebe oder der Zelle aktiv sind. In der vorliegenden Beschreibung wird die Epidermis dadurch gekennzeichnet, dass nur ein Teil der Promotoren aktiv ist, z.B. bevorzugt 2, 3, 5 oder am meisten bevorzugt 7 oder mehr, mindestens jedoch einer der oben aufgezählten.

In der vorliegenden Beschreibung wird das Mesophyll biochemisch charakterisiert. Das Mesophyll kann durch die Aktivität eines oder mehrer der folgenden Promotoren gekennzeichnet werden:
- PPCZm1 (=PEPC); Kausch A.P., Owen T.P., Zachwieja S.J., Flynn A.R. und Sheen J., Plant Mol. Biol. 45, 1 (2001);
- OsrbcS, Kyozuka et al., PIaNT Phys 102, 991 (1993); Kyozuka J., McElroy D., Hayakawa T., Xie Y., Wu R. und Shimamoto K., Plant Phys. 102, 991 (1993);
- OsPPDK, acc. AC099041;
- TaGF-2.8, acc. M63223; Schweizer P., Christoffel A. and Dudler R. , Plant J. 20, 541 (1999);
- TaFBPase, acc. X53957;
- TaWIS1, acc. AF467542; US 200220115849;
- HvBIS1, acc. AF467539; US 200220115849;
- ZmMIS1, acc. AF467514; US 200220115849;
- HvPR1a, acc. X74939 ; Bryngelsson et al., Mol. Plant Microbe Interacti. 7(2), 267 (1994);
- HvPR1b, acc. X74940; Bryngelsson et al., Mol. Plant Microbe Interact, 7(2), 267 (1994);
- HvB1,3gluc; acc. AF479647;
- HvPrx8, acc. AJ276227; Kristensen et al., Molecular Plant Pathology, 2(6), 311 (2001);
- HvPAL, acc. X97313 ; Wei Y., Zhang Z., Andersen C.H., Schmelzer E., Gregersen P.L., Collinge D.B., Smedegaard-Petersen V. und Thordal-Christensen H. Plant Molecular Biology 36, 101 (1998).

In der vorliegenden Beschreibung wird das Mesophyll dadurch gekennzeichnet, dass alle genannten Promotoren in dem Gewebe oder der Zelle aktiv sind. In einer anderen Ausführungsform wird das Mesophyll dadurch gekennzeichnet, dass nur ein Teil der Promotoren aktiv ist, z.B. bevorzugt 2, 3, 5 oder besonders bevorzugt 7 oder mehr, mindestens jedoch einer der oben aufgezählten.

In der vorliegenden Beschreibung sind in einer verwendeten oder hergestellten Pflanze oder in einer erfindungsgemäßen Pflanze in der Epidermis und im Mesophyll alle genannten Promotoren aktiv. In der vorliegenden Beschreibung sind nur ein Teil der genannten Promotoren aktiv, z.B. bevorzugt 2, 5, oder insbesondere bevorzugt 7 oder mehr, mindestens ist jedoch einer der oben aufgezählten Promotoren jeweils aktiv.

In bevorzugten Ausführungsformen erfolgt die Erhöhung der Proteinmenge oder -funktion des BI1-Proteins konstitutiv oder gewebespezifisch. In besonders bevorzugten Ausführungsformen erfolgt eine im wesentlichen gewebespezifische Erhöhung der Proteinmenge oder -funktion im wesentlichen epidermisspezifisch , beispielsweise durch rekombinante Expression einer für das BI1-Protein kodierenden Nukleinsäuresequenz unter Kontrolle eines Epidermis-spezifischen Promotors. Insbesondere bevorzugt erfolgt die Erhöhung der Expression oder Funktion des BI1 Proteins in der Epidermis, wobei aber die Expression des BI-1 Proteins im Mesophyll im wesentlichen unverändert bleibt, oder sie wird vermindert, und wobei andere Gewebe außer Betracht bleiben.

In einer Ausführungsform wird wie hierin beschrieben, die Expression oder Funktion des hierin charakterisierten BI-1 zumindest in der Epidermis einer Pflanze erhöht. Eine Erhöhung der Expression kann wie unten beschrieben erreicht werden. Unter Erhöhung der Expression oder Funktion wird hierein sowohl die Aktivierung oder Steigerung der Expression oder Funktion des endogenen Proteins einschließlich einer de novo Expression als auch eine Erhöhung oder Steigerung durch die Expression eines transgenen Proteins oder Faktors verstanden.

In einer besonders bevorzugten Ausführungsform kann die Erhöhung der Proteinmenge oder Funktion mindestens eines pflanzlichen BI1-Proteins kombiniert werden mit einem mloresistenten Phänotyp oder mit der Inhibierung oder Reduzierung im Vergleich zu einer Kontrollpflanze der Expression von MLO, RacB und/oder NaOx in der Pflanze oder einem Teil davon, z.B. in einem Gewebe, besonders vorteilhaft jedoch mindestens in der Epidermis oder einem wesentlichen Teil der Epidermiszellen und/oder mit der Erhöhung der Expression oder Funktion von PEN2 und/oder PEN1 in der Pflanze, z.B. konstitutiv, oder einem Teil davon, z.B. in einem Gewebe, besonders vorteilhaft jedoch mindestens in der Epidermis oder einem wesentlichen Teil der Epidermiszellen wird, mit der Maßgabe, dass die Expression eines pflanzlichen BI1-Proteins in der Blattepidermis im wesentlichen unverändert bleibt oder reduziert wird.

In Gerste ist der Mlo-Locus als negativer Regulator der Pathogenabwehr beschrieben. Der Verlust oder Funktionsverlust ("loss-of-function") des Mlo-Gens bedingt eine erhöhte, rassenunspezifische Resistenz gegen zahlreiche Mehltauisolate (Büschges R. et al., Cell 88, 695 (1997); Jorgensen J.H., Euphytica 26, 55 (1977); Lyngkjaer M.F. et al., Plant Pathol. 44, 786 (1995)). Ein mlo-resistenter Phänotyp kann wie im Stand der Technik beschrieben erreicht werden. Verfahren unter Verwendung dieser Gene zum Erzielen einer Pathogenresistenz sind beschrieben u.a. in WO 98/04586; WO 00/01722; WO 99/47552.

Vorteilhaft kann in einer Ausführungsform der vorliegenden Erfindung die Aktivität, Expression oder Funktion von MLO, RacB und/oder NaOx in der Pflanze oder einem Teil davon, z.B. in einem Gewebe, besonders vorteilhaft jedoch mindestens in der Epidermis oder einem wesentlichen Teil der Epidermiszellen inhibiert oder im Vergleich zu einer Kontrollpflanze oder einem Teil davon reduziert werden. Durch die Reduzierung der Aktivität oder Funktion von MLO, RacB und/oder NaOx in der Pflanze oder einem Teil davon, z.B. in einem Gewebe, besonders vorteilhaft jedoch mindestens in der Epidermis oder einem wesentlichen Teil der Epidermiszellen wird vorzugsweise die Resistenz oder Widerstandskraft gegen biotrophe Pathogene bei erfindungsgemäß hergestellten Pflanzen erhöht. Die Aktivität oder Funktion von MLO, RacB und/oder NaOx kann analog wie für MLO in WO 98/04586; WO 00/01722; WO 99/47552 und den weiteren unten genannten Schriften beschrieben reduziert oder inhibiert werden, deren Inhalt hiermit ausdrücklich als mit aufgenommen gilt, insbesondere um die Aktivität und Inhibierung von MLO zu beschreiben. Die Beschreibung der genannten Schriften beschreibt Verfahren Methoden und besonders bevorzugte Ausführungsformen zur Erniedrigung oder Inhibierung der Aktivität oder Funktion von MLO, die Beispiele geben konkret an, wie dies ausgeführt werden kann.

Die Reduzierung der Aktivität oder Funktion, ggf. der Expression von RacB ist in der WO 2003/20939 ausführlich beschrieben, die hiermit ausdrücklich mit als in die vorliegenden Beschreibung mit aufgenommen gilt.

Die Beschreibung der genannten Schrift beschreibt Verfahren und Methoden zur Erniedrigung oder Inhibierung der Aktivität oder Funktion von Proteinen, die Beispiele geben konkret an, wie dies ausgeführt werden kann. Besonders bevorzugt wird die Reduktion oder Inhibierung der Aktivität oder Funktion von RacB gemäß den in der WO 2003/20939 besonders bevorzugten Ausführungsformen und den Beispielen und in den dort als besonders bevorzugt dargestellten Organismen durchgeführt, insbesondere in einer Pflanze oder einem Teil davon, z.B. in einem Gewebe, besonders vorteilhaft jedoch mindestens in der Epidermis oder einem wesentlichen Teil der Epidermiszellen. Die Reduzierung der Aktivität oder Funktion, ggf. der Expression von RacB ist in der WO 2003/20939 ausführlich beschrieben. Der Fachmann findet in der WO 2003/20939 die Sequenzen, die für RacB Proteine codieren und kann mit dem in der WO 2003/20939 zur Verfügung gestellten Verfahren auch RacB identifizieren.

Die Reduzierung der Aktivität oder Funktion, ggf. der Expression von NaOx ist in WO 2004/09820 (= PCT/EP/03/07589) ausführlich beschrieben, die hiermit ausdrücklich mit als in die vorliegenden Beschreibung mit aufgenommen gilt. Die Beschreibung der genannten Schrift beschreibt Verfahren und Methoden zur Erniedrigung oder Inhibierung der Aktivität oder Funktion von NaOx, die Beispiele geben konkret an, wie dies ausgeführt werden kann. Besonders bevorzugt wird die Reduktion oder Inhibierung der Aktivität oder Funktion von NaOx gemäß den in WO 2004/09820 (= PCT/EP/03/07589) besonders bevorzugten Ausführungsformen und den Beispielen und in den dort als besonders bevorzugt dargestellten Organismen durchgeführt insbesondere in einer Pflanze oder einem Teil davon, z.B. in einem Gewebe, besonders vorteilhaft jedoch mindestens in der Epidermis oder einem wesentlichen Teil der Epidermiszellen. Der Fachmann findet in WO 2004/09820 (= PCT/EP/03/07589) die Sequenzen, die für NaOx Proteine codieren und kann mit dem in WO 2004/09820 (= PCT/EP/03/07589) zur Verfügung gestellten Verfahren auch NaOx identifizieren.

Vorteilhaft kann in einer Ausführungsform der vorliegenden Erfindung die Aktivität, Expression oder Funktion von PEN1, PEN2 und/oder SNAP34 in der Pflanze, z.B. konstitutiv, oder einem Teil davon, z.B. in einem Gewebe, besonders vorteilhaft jedoch mindestens in der Epidermis oder einem wesentlichen Teil der Epidermiszellen erhöht werden. Die Erhöhung der Aktivität, die auch eine de novo Expression umschließt, von PEN1, PEN2 und/oder SNAP 34 in der Pflanze, z.B. konstitutiv, oder einem Teil davon, z.B. in einem Gewebe, besonders vorteilhaft jedoch mindestens in der Epidermis oder einem wesentlichen Teil der Epidermiszellen wird vorzugsweise die Resistenz oder Widerstandskraft gegen biotrophe Pathogene bei erfindungsgemäß hergestellten Pflanzen erhöhen. Die Erhöhung der Aktivität oder Funktion, ggf. der Expression von PEN2 ist in der WO03/074688 ausführlich beschrieben und die hiermit ausdrücklich als in der vorliegenden Beschreibung mit aufgenommen gilt. Die Beschreibung der genannten Schrift beschreibt Verfahren und Methoden zur Erniedrigung oder Inhibierung der Aktivität oder Funktion von PEN2, die Beispiele geben konkret an, wie dies ausgeführt werden kann. Besonders bevorzugt wird die Reduktion oder Inhibierung der Aktivität oder Funktion von PEN2 gemäß den in der WO03/074688 besonders bevorzugten Ausführungsformen und den Beispielen und in den dort als besonders bevorzugt dargestellten Organismen durchgeführt, insbesondere in Pflanzen, z.B. konstitutiv, oder einem Teil davon, z.B. in einem Gewebe, besonders vorteilhaft jedoch mindestens in der Epidermis oder einem wesentlichen Teil der Epidermiszellen. Der Fachmann findet in der WO03074688 die Sequenzen, die für PEN2 Proteine codieren und kann mit dem in der WO03/074688 zur Verfügung gestellten Verfahren auch PEN2 identifizieren.

Die Expression von PEN1 und SNAP34 kann analog zu den in der WO03/074688 beschriebenen Verfahren erhöht werden. Der Fachmann kann aufgrund des allgemeinen Fachwissens und des ihm bekannten Stands der Technik PEN1 und SNAP34-Nukleinsäuresequenzen und - Proteinsequenzen isolieren und überexprimieren. SEQ ID NO. 39 beschreibt die Nukleinsäuresequenz, die für PEN1 aus Gerste kodiert, die Proteinsequenz ist in SEQ ID No.: 40 beschrieben. SEQ ID NO.: 41 beschreibt die Nukleinsäuresequenz, die für PEN1 1 aus Arabidopsis thaliana kodiert, die Proteinsequenz ist in SEQ ID No. 42 beschrieben. PEN1 aus Arabidopsis thaliana ist veröffentlicht unter den accession numbers N M 202559 und N M 112015. Das Homolog aus Gerste wird als ROR2 offenbart in accession numbers AY246907 und AY246906. Es handelt sich um Mitglieder der recht großen Syntaxin-Proteinfamilie. Der Fachmann kann somit durch einfache Homologievergleiche weitere Syntaxin-Proteine identifzieren, die als potentielle Resistenzgene in dem erfindungsgemäßen Verfahren exprimiert werden.

SEQ ID NO. 43 beschreibt die Nukleinsäuresequenz, die für SNAP34 aus Gerste kodiert, die Proteinsequenz ist in SEQ ID No.: 44 beschrieben. Das SNAP-34 Homolog aus Gerste ist auch veröffentlicht unter AY 247208 (SNAP-34). Homologe unbekannter Funktion, die eine Rolle in der Resistenz spielen könnten, sind veröffentlicht unter AY 247209 (SNAP-28) und AY 247210 (SNAP-25). Folgende Arabidopsis-Gene zeigen eine höhere Homologie zu Gerste SNAP34 als Gersten SNAP-28 bzw. SNAP-25 zu SNAP-34 und können somit als potentielle Resistenzvermittelnde Gene vorteilhaft coüberexprimiert werden:
AAM 62553 - Arabidopsis SNAP25a
NP 200929 - Arabidopsis SNAP33b
NP 172842 - Arabidopsis SNAP30
NP 196405 - Arabidopsis SNAP29

Folglich betrifft die Beschreibung auch eine Pflanze, in der mindestens weiterhin in der Epidermis ein Polypeptid überexprimiert wird, das codiert wird von einem Nukleinsäuremolekül, das die in Seq. ID No. 39, 41 oder 43 oder eine der in den genannten Datenbankveröffentlichungen gezeigten Sequenzen umfasst oder das die in Seq. ID No. 40, 42 oder 44 gezeigte oder eine der in den genannten Datenbankveröffentlichungen gezeigten Aminosäuresequenzen umfasst, oder das ein funktionelles Äquivalent davon ist oder das eine Homologie von mindestens 50 %, bevorzugt 70 %, mehr bevorzugt 80 %, noch mehr bevorzugt 90 %, 95 % oder mehr zu den genannten Sequenzen auf Ebene des codierenden Nukleinsäuremoleküls oder bevorzugt auf A-minosäureebene hat, oder eine Pflanze, in der konstitutiv oder in einem Teil, z.B. in einem Gewebe, besonders vorteilhaft jedoch mindestens in der Epidermis oder einem wesentlichen Teil der Epidermiszellen das vorhergehend charakterisierte Polypeptide aktiviert wird oder dessen Aktivität oder Funktion erhöht wird.

Eine Reduktion oder Expression oder Aktivität eines Proteins kann durch einen Fachmann geläufige Verfahren bewirkt werden, z.B. Mutagenese, z.B. EMS, ggf. Tilling, iRNA; Ribozyme, Silencing, Knockout, etc. Verfahren zur Reduzierung sind insbesondere beschrieben in WO 2003/20939, dessen Methoden an die hierin beschriebenen Sequenzen leicht angepasst werden können. Daher wird der Inhalt der WO 2003/20939 explizit hierin mit aufgenommen.

Die Reduzierung oder Verminderung der Expression eines Proteins, der Aktivität oder der Funktion kann auf vielfältige Art und Weise realisiert werden.

"Reduzierung", "reduzieren", "Verminderung" oder "vermindern" ist weit auszulegen und umfasst die teilweise oder im wesentlichen vollständige, auf unterschiedliche zellbiologische Mechanismen beruhende Unterbindung oder Blockierung der Funktionalität eines Proteins.

Eine Verminderung umfasst auch eine mengenmäßige Verringerung eines Proteins bis hin zu einem im wesentlichen vollständigen Fehlen des Proteins (d.h. fehlende Nachweisbarkeit von Aktivität bzw. Funktion oder fehlende immunologische Nachweisbarkeit des Proteins). Dabei wird die Expression eines bestimmten Proteins oder die Aktivität bzw. Funktion in einer Zelle oder einem Organismus bevorzugt um mehr als 50 %, besonders bevorzugt um mehr als 80 %, ganz besonders bevorzugt um mehr als 90 % vermindert.

Die Methoden der dsRNAi, der Kosuppression mittels sense-RNA und der "VIGS" ("virus induced gene silencing") werden auch als "post-transcriptional gene silencing" (PTGS) bezeichnet. PTGS-Verfahren wie auch die Verminderung der Funktion oder Aktivität mit dominant-negativen Varianten sind besonders vorteilhaft, weil die Anforderungen an die Homologie zwischen dem zu supprimierenden endogenem Gen und der transgen exprimierten sense- oder dsRNA-Nukleinsäuresequenz (bzw. zwischen dem endogenen Gen und seiner dominant-negativen Variante) geringer sind als beispielsweise bei einem klassischen antisense-Ansatz. Entsprechende Homologie-Kriterien sind bei der Beschreibung des dsRNAI-Verfahrens genannt und allgemein für PTGS-Verfahren oder dominant-negative Ansätze übertragbar.

"Einbringung" umfasst alle Verfahren, die dazu geeignet eine Verbindung, direkt oder indirekt, in der Epidermis oder einem wesentlichen Teil der Epidermiszellen, Kompartiment oder Gewebe derselben einzuführen oder dort zu generieren. Direkte und indirekte Verfahren sind umfasst. Die Einbringung kann zu einer vorübergehenden (transienten) Präsenz einer Verbindung (beispielsweise einer dsRNA) führen oder aber auch zu einer dauerhaften (stabilen). Einführen umfasst beispielsweise Verfahren wie Transfektion, Transduktion oder Transformation.

In Expressionskonstrukten steht ein hierin offenbartes Nukleinsäuremolekül, dessen Expression (Transkription und ggf. Translation) ein entsprechendes Aminosäuremolekül generiert, bevorzugt in funktioneller Verknüpfung mit mindestens einem genetischen Kontrollelement (beispielsweise einem Promotor), das eine Expression in einem Organismus, bevorzugt in Pflanzen, gewährleistet, vorzugsweise Epidermis-spezifisch. Soll das Expressionskonstrukt direkt in die Pflanze eingeführt werden, so sind pflanzenspezifische genetische Kontrollelemente (beispielsweise Promotoren) bevorzugt, wobei aus dem oben gesagten die Epidermis-spezifische Aktivität des Promoters in den meisten Ausführungsformen wie oben beschrieben zwingend ist.

Unter einer funktionellen Verknüpfung versteht man zum Beispiel die sequentielle Anordnung eines Promotors mit der zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz, je nach Anordnung der Nukleinsäuresequenzen zu sense oder anti-sense RNA, erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben (cis- bzw. trans-Lokalisation). Bevorzugt sind Anordnungen, in denen die transgen zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung einer Expressionskassette kann mittels gängiger Rekombinations- und Klonierungstechniken realisiert werden, wie sie beispielsweise in Maniatis T., Fritsch E.F. und Sambrook J., Molecular Cloning: A Laboratory Manual, Cold Spring Harbor Laboratory, Cold Spring Harbor (NY) (1989), in Silhavy T.J., Berman M.L. und Enquist L.W., Experiments with Gene Fusions, Cold Spring Harbor Laboratory, Cold Spring Harbor (NY) (1984), in Ausubel F.M. et al.. Current Protocols in Molecular Biology, Greene Publishing Assoc. and Wiley Interscience (1987) und bei Gelvin et al. in Plant Molecular Biology Manual (1990) beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann die Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden. Die Kontrollelemente vermitteln bevorzugt eine Epidermis-spezifische Expression.

"Ungefähr" meint im Zusammenhang mit Zahlen- oder Größenangaben einen Zahlen- oder Größenbereich um den angegebenen Zahlen- oder Größenwert herum. Im allgemeinen meint der Begriff ungefähr einen Bereich von jeweils 10% des angegebenen Wertes nach oben und nach unten.

Eingeschlossen unter dem Begriff "Pflanze" sind die reifen Pflanzen, Saatgut, Sprossen und Keimlinge, sowie davon abgeleitete Teile, Vermehrungsgut, Pflanzenorgane, Gewebe, Protoplasten, Kallus und andere Kulturen, zum Beispiel Zellkulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium.

"Pflanze" umfaßt alle einjährigen und mehrjährige Pflanzen.

"Pathogenresistenz" meint das Vermindern oder Abschwächen von Krankheitssymptomen einer Pflanze infolge eines Befalls durch mindestens ein Pathogen. Die Symptome können vielfältiger Art sein, umfassen aber bevorzugt solche die direkt oder indirekt zu einer Beeinträchtigung Qualität der Pflanze, der Quantität des Ertrages, der Eignung zur Verwendung als Futter- oder Nahrungsmittel führen oder aber auch Aussaat, Anbau, Ernte oder Prozessierung des Erntegutes erschweren. Insbesondere ist für der Zwecke der vorliegenden Erfindung die Pathogentoleranz als von der Pathogenresistenz umfasst anzusehen.

"Verleihen", "bestehen", "erzeugen" oder "erhöhen" einer Resistenz meint, dass die Abwehrmechanismen einer bestimmten Pflanzenart oder -sorte durch Anwendung des erfindungsgemäßen Verfahrens im Vergleich zu dem Wildtyp der Pflanze ("Ausgangspflanze"), auf den das erfindungsgemäße Verfahren nicht angewendet wurde, unter ansonsten im wesentlichen gleichen Bedingungen (wie beispielsweise Klima- oder Anbaubedingungen, Stress- oder Pathogenart etc.) eine erhöhte Resistenz gegen ein und mehrere Pathogene aufweist. Dabei äußert sich die erhöhte Resistenz bevorzugt in einer verminderten Ausprägung der Krankheitssymptome, wobei Krankheitssymptome - neben den oben erwähnten Beeinträchtigungen - auch beispielsweise die Penetrationseffizienz eines Pathogens in die Pflanze oder pflanzliche Zellen oder die Proliferationseffizienz in oder auf denselben umfaßt. Dabei sind die Krankheitssymptome bevorzugt um mindestens 5 %, 10 % oder mindestens 20 %, besonders bevorzugt um mindestens 40 % oder 60 %, ganz besonders bevorzugt um mindestens 70 % oder 80 %, am meisten bevorzugt um mindestens 90 % oder 95 % vermindert.

"Auswahl" meint in Bezug auf Pflanzen, bei denen - im Unterschied oder Vergleich zur Ausgangspflanze - die Resistenz gegen mindestens ein Pathogen besteht oder erhöht ist, all die Verfahren, die eine zur Erkennung einer vorliegenden oder erhöhten Pathogenresistenz geeignet sind. Dies können beispielsweise Symptome der Pathogeninfektion sein (z.B. Nekrosen-Ausbildung bei Pilzinfektion) aber auch die oben beschriebenen Symptome umfassen, die die Qualität der Pflanze, die Quantität des Ertrages, die Eignung zur Verwendung als Futter- oder Nahrungsmittel usw. betreffen.

"Pathogen" meint mindestens einen biotrophen Pilz aus dem Genus Phakopsora. Es ist jedoch anzunehmen, dass die mesophyll-spezifische Expression eines BI1-Proteins auch eine Resistenz gegen weitere Pathogene bewirkt, da insgesamt eine Resistenz gegen Streßfaktoren erzeugt wird.

Beispielsweise jedoch nicht einschränkend seien nachfolgende Pathogene zu nennen:

### 1. Pilzpathogene oder pilz-ähnliche Pathogene:

Pilzpathogene oder pilz-ähnliche Pathogene (wie z.B. Chromista) stammen vorzugsweise aus der Gruppe umfassend Plasmodiophoramycota, Oomycota, Ascomycota, Chytridiomyceten, Zygomyceten, Basidiomycota und Deuteromyceten (Fungi imperfecti). Beispielhaft jedoch nicht einschränkend seien die in Tabelle 1 bis 4 genannten Pathogene und die mit ihnen in Zusammenhang gebrachten Erkrankungen zu nennen. Folgende englische und deutsche Termini können alternativ verwendet werden:

| | |
|---|---|
| Ährenfäule | ear rot / head blight |
| Stengelfäule | stalk rot |
| Wurzelfäule | root rot |
| Rost | rust |
| Falscher Mehltau | downy mildew |

**Tabelle 1: Erkrankungen hervorgerufen durch biotrophe, phytopathogene Pilze**

| Erkrankung | Pathogen |
|---|---|
| Braunrost | Puccinia recondita |
| Gelbrost | P. striiformis |
| Echter Mehltau | Erysiphe graminis / Blumeria graminis |
| Rost (gemeiner Mais) | Puccinia sorghi |
| Rost (südlicher Mais) | Puccinia polysora |
| Tabak Blattflecken | Cercospora nicotianae |
| Rost (Soja) | Phakopsora pachyrhizi, P. meibomiae |
| Rost (tropischer Mais) | Physopella pallescens, P. zeae = Angiopsora zeae |

**Tabelle 2: Erkrankungen hervorgerufen durch nekrotrophe und/oder hemibiotrophe Pilze und Oomyceten**

| Erkrankung | Pathogen |
|---|---|
| Spelzenbräune | Septoria (Stagonospora) nodorum |
| Blattdürre | Septoria tritici |
| Ährenfusariosen | Fusarium spp. |
| Halmbruchkrankheit | Pseudocercosporella herpotrichoides |
| Flugbrand | Ustilago spp. |
| Kraut- und Knollenfäule | Phytohpthora infestans |
| Weizensteinbrand | Tilletia caries |
| Schwarzbeinigkeit | Gaeumannomyces graminis |
| Anthrocnose leaf blight Anthracnose stalk rot | Colletotrichum graminicola (teleomorph: Glomerella graminicola Politis); Glomerella tucumanensis (anamorph: Glomerella falcatum Went) |
| Aspergillus ear and kernel rot | Aspergillus flavus |
| Banded leaf and sheath spot ("Wurzeltöter") | Rhizoctonia solani Kuhn = Rhizoctonia microsclerotia J. Matz (telomorph: Thanatephorus cucumeris) |
| Black bundle disease | Acremonium strictum W. Gams = alosporium acremonium Auct. non Corda |
| Black kernel rot | Lasiodiplodia theobromae = Botryodiplodia theobromae |
| Borde blanco | Marasmiellus sp. |
| Brown spot (black spot, stalk rot) | Physoderma maydis |
| Cephalosporium kernel rot | Acremonium strictum = Cephalosporium acremonium |
| Charcoal rot | Macrophomina phaseolina |
| Corticium ear rot | Thanatephorus cucumeris = Corticium sasakii |
| Curvularia leaf spot | Curvularia clavata, C. eragrostidis, = C. maculans (teleomorph: Cochliobolus eragrostidis), Curvularia inaequalis, C. intermedia (teleomorph: Cochliobolus intermedius), Curvularia lunata (teleomorph: Cochliobolus lunatus), Curvularia pallescens (teleomorph: Cochliobolus pallescens), Curvularia senegalensis, C. tuberculata (teleomorph: Cochliobolus tuberculatus) |
| Didymella leaf spot | Didymella exitalis |
| Diplodia Ähren- und Stengelfäule | Diplodia frumenti (teleomorph: Botryosphaeria festucae) |
| Diplodia Ähren- und Stengelfäule, seed rot and seedling blight | Diplodia maydis = Stenocarpella maydis |
| Diplodia leaf spot or streak | Stenocarpella macrospora = Diplodialeaf macrospora |
| Brown stripe downy mildew | Sclerophthora rayssiae var. zeae |
| Crazy top downy mildew | Sclerophthora macrospora = Sclerospora macrospora |
| Green ear downy mildew (graminicola downy mildew) | Sclerospora graminicola |
| Dry ear rot (cob, kernel and stalk rot) | Nigrospora oryzae (teleomorph: Khuskia oryzae) |
| Ährenfäulen (minor) | Alternaria altemata = A. tenuis, Aspergillus glaucus, A. niger, Aspergillus spp., Botrytis cinerea (teleomorph: Botryotinia fuckeliana), Cunninghamella sp., Curvularia pallescens, Doratomyces stemonitis = Cephalotrichum stemonitis, Fusarium culmorum, Gonatobotrys simplex, Pithomyces maydicus, Rhizopus microsporus Tiegh., R. stolonifer = R. nigricans, Scopulariopsis brumptii |
| Ergot (horse's tooth) | Claviceps gigantea (anamorph: Sphacelia sp.) |
| Eyespot | Aureobasidium zeae = Kabatiella zeae |
| Fusarium Ähren- und Stengelfäule | Fusarium subglutinans = F. moniliforme var.subglutinans |
| Fusarium kernel, root and stalk rot, seed rot and seedling blight | Fusarium moniliforme (teleomorph: Gibberella fujikuroi) |
| Fusarium Stengelfäule, seedling root rot | Fusarium avenaceum (teleomorph: Gibberella avenacea) |
| Gibberella Ähren- u. Stengelfäule | Gibberella zeae (anamorph: Fusarium graminearum) |
| Graue Ährenfäule | Botryosphaeria zeae = Physalospora zeae (anamorph: Macrophoma zeae) |
| Gray leaf spot (Cercospora leaf spot) | Cercospora sorghi = C. sorghi var. maydis, C. zeae-maydis |
| Helminthosporium root rot | Exserohilum pedicellatum = Helminthosporium pedicellatum (teleomorph: Setosphaeria pedicellata) |
| Hormodendrum Ährenfäule (Cladosporium Fäule) | Cladosporium cladosporioides = Hormodendrum cladosporioides, C. herbarum (teleomorph: Mycosphaerella tassiana) |
| Leaf spots, minor | Alternaria alternata, Ascochyta maydis, A. tritici, |
| | A. zeicola, Bipolaris victoriae = Helminthosporium victoriae (teleomorph: Cochliobolus victoriae), C. sativus (anamorph: Bipolaris sorokiniana = H. sorokinianum = H. sativum), Epicoccum nigrum, Exserohilum prolatum = Drechslera prolata (teleomorph: Setosphaeria prolata) Graphium penicillioides, Leptosphaeria maydis, Leptothyrium zeae, Ophiosphaerella herpotricha, (anamorph: Scolecosporiella sp.), Paraphaeosphaeria michotii, Phoma sp., Septoria zeae, S. zeicola, S. zeina |
| Northern corn leaf blight (white blast, crown stalk rot, stripe) | Setosphaeria turcica (anarnorph: Exserohilum turcicum = Helminthosporium turcicum) |
| Northern corn leaf spot Helminthosporium ear rot (race 1) | Cochliobolus carbonum (anamorph: Bipolaris zeicola = Helminthosporium carbonum) |
| Penicillium Ährenfäule (blue eye, blue mold) | Penicillium spp., P. chrysogenum, P. expansum, P. oxalicum |
| Phaeocytostroma Stengel- und Wurzelfäule | Phaeocytostroma ambiguum, = Phaeocytosporella zeae |
| Phaeosphaeria leaf spot | Phaeosphaeria maydis = Sphaerulina maydis |
| Physalospora Ährenfäule (Botryosphaeria Ährenfäule) | Botryosphaeria festucae = Physalospora zeicola (anamorph: Diplodia frumenti) |
| Purple leaf sheath | Hemiparasitic bacteria and fungi |
| Pyrenochaeta Stengel- und Wurzelfäule | Phoma terrestris = Pyrenochaeta terrestris |
| Pythium Wurzelfäule | Pythium spp., P. arrhenomanes, P. graminicola |
| Pythium Stengelfäule | Pythium aphanidermatum = P. butleri L. |
| Red kernel disease (ear mold, leaf and seed rot) | Epicoccum nigrum |
| Rhizoctonia Ährenfäule (sclerotial rot) | Rhizoctonia zeae (teleomorph: Waitea circinata) |
| Rhizoctonia Wurzel- und Stengelfäule | Rhizoctonia solani, Rhizoctonia zeae |
| Wurzelfäulen (minor) | Alternaria alternata, Cercospora sorghi, Dictochaeta fertilis, Fusarium acuminatum (teleomorph: Gibberella acuminata), F. equiseti (tele- |
| | omorph: G. intricans), F. oxysporum, F. pallidoroseum, F. poae, F. roseum, G. cyanogena, (anamorph: F. sulphureum), Microdochium bolleyi, Mucor sp., Periconia circinata, Phytophthora cactorum, P. drechsleri, P. nicotianae var. parasitica, Rhizopus arrhizus |
| Rostratum leaf spot (Helminthosporium leaf disease, ear and stalk rot) | Setosphaeria rostrata, (anamorph: xserohilum rostratum = He/minthosporium rostratum) |
| Falscher Java Mehltau | Peronosclerospora maydis = Sclerospora maydis |
| Falscher Philippinen Mehltau | Peronosclerospora philippinensis = Sclerospora philippinensis |
| Falscher Sorghum Mehltau | Peronosclerospora sorghi = Sclerospora sorghi |
| Spontaneum downy mildew | Peronosclerospora spontanea = Sclerospora spontanea |
| Falscher Zuckerrohr-Mehltau | Peronosclerospora sacchari = Sclerospora sacchari |
| Sclerotium Ährenfäule (southern blight) | Sclerotium rolfsii Sacc. (teleomorph: Athelia rolfsii) |
| Seed rot-seedling blight | Bipolaris sorokiniana, B. zeicola = Helminthosporium carbonum, Diplodia maydis, Exserohilum pedicillatum, Exserohilum turcicum = Helminthosporium turcicum, Fusarium avenaceum, F. culmorum, F. moniliforme, Gibberella zeae (anamorph: F. graminearum), Macrophomina phaseolina, Penicillium spp., Phomopsis sp., Pythium spp., Rhizoctonia solani, R. zeae, Sclerotium rolfsii, Spicaria sp. |
| Selenophoma leaf spot | Selenophoma sp. |
| Sheath rot | Gaeumannomyces graminis |
| Shuck rot | Myrothecium gramineum |
| Silage mold | Monascus purpureus, M ruber |
| Flugbrand (Smut, common) | Ustilago zeae = U. maydis |
| Smut, false | Ustilaginoidea virens |
| Kolbenbrand (Smut, head) | Sphacelotheca reiliana = Sporisorium holcisorghi |
| Southern corn leaf blight and stalk rot | Cochliobolus heterostrophus (anamorph: Bipolaris maydis = Helminthosporium maydis) |
| Southern leaf spot | Stenocarpella macrospora = Diplodia macrospora |
| Stengelfäulen (minor) | Cercospora sorghi, Fusarium episphaeria, F. merismoides, F. oxysporum Schlechtend, F. poae, F. roseum, F. solani (teleomorph: Nectria haematococca), F. tricinctum, Mariannaea elegans, Mucor sp., Rhopographus zeae, Spicaria sp. |
| Lagerfäulen (Storage rots) | Aspergillus spp., Penicillium spp. und weitere Pilze |
| Tar spot | Phyllachora maydis |
| Trichoderma ear rot and root rot | Trichoderma viride = T. lignorum teleomorph: Hypocrea sp. |
| White ear rot, root and stalk rot | Stenocarpella maydis = Diplodia zeae |
| Yellow leaf blight | Ascochyta ischaemi, Phyllosticta maydis (teleomorph: Mycosphaerella zeae-maydis) |
| Zonate leaf spot | Gloeocercospora sorghi |

**Tabelle 4: Erkrankungen hervorgerufen durch Pilze und Oomyceten mit unklarer Einstufung hinsichtlich biotrophen, hemibiotrophen bzw. nekrotrophen Verhaltens**

| Erkrankung | Pathogen |
|---|---|
| Hyalothyridium leaf spot | Hyalothyridium maydis |
| Late wilt | Cephalosporium maydis |

### Besonders bevorzugt sind

- Plasmodiophoromycota wie Plasmodiophora brassicae (Kohlhernie), Spongospora subterranea, Polymyxa graminis,
- Oomycota wie Bremia lactucae (Falscher Mehltau an Salat), Peronospora (Falscher Mehltau) bei Löwenmaul (P. antirrhini), Zwiebel (P. destructor), Spinat (P. effusa), Sojabohne (P. manchurica), Tabak (Blauschimmel; P. tabacina) Alfalfa und Klee (P. trifolium), Pseudoperonospora humuli (Falscher Mehltau an Hopfen), Plasmopara (Falscher Mehltau bei Trauben) (P. viticola) und Sonnenblume (P. halstedii), Sclerophtohra macrospora (Falscher Mehltau bei Cerealien und Gäsern), Pythium (z.B. Wurzelbrand an Beta-Rübe durch P. debaryanum), Phytophthora infestans (Kraut- und Knollenfäule bei Kartoffel, Braunfäule bei Tomate etc.), Albugo spec.
- Ascomycota wie Microdochium nivale (Schneeschimmel an Roggen und Weizen), Fusarium graminearum, Fusarium culmorum (Ährenfäule v.a. bei Weizen), Fusarium oxysporum (Fusarium-Welke an Tomate), Blumeria graminis (Echter Mehltau an Gerste (f.sp. hordei) und Weizen (f.sp. tritici)), Erysiphe pisi (Erbsenmehltau), Nectria galligena (Obstbaumkrebs), Unicnula necator (Echter Mehltau der Weinrebe), Pseudopeziza tracheiphila (Roter Brenner der Weinrebe), Claviceps purpurea (Mutterkorn an z.B. Roggen und Gräsern), Gaeumannomyces graminis (Schwarzbeinigkeit an Weizen, Roggen u.a. Gräsern), Magnaporthe grisea, Pyrenophora graminea (Streifenkrankheit an Gerste), Pyrenophora teres (Netzfleckenkrankheit an Gerste), Pyrenophora tritici-repentis (Blattfleckenkrankheit (Blattdürre) an Weizen), Venturia inaequalis (Apfelschorf), Sclerotinia sclerotium (Weißstengeligkeit, Rapskrebs), Pseudopeziza medicaginis (Klappenschorf an Luzerne, Weiß- und Rotklee).
- Basidiomyceten wie Typhula incamata (Typhula-Fäule an Gerste, Roggen, Weizen), Ustilago maydis (Beulenbrand an Mais), Ustilago nuda (Flugbrand an Gerste), Ustilago tritici (Flugbrand an Weizen, Dinkel), Ustilago avenae (Flugbrand an Hafer), Rhizoctonia solani (Wurzeltöter an Kartoffeln), Sphacelotheca spp. (Kolbenbrand bei Sorghum), Melampsora lini (Rost bei Flachs), Puccinia graminis (Schwarzrost an Weizen, Gerste, Roggen, Hafer), Puccinia recondita (Braunrost an Weizen), Puccinia dispersa (Braunrost an Roggen), Puccinia hordei (Braunrost an Gerste), Puccinia coronata (Kronenrost an Hafer), Puccinia striiformis (Gelbrost an Weizen, Gerste, Roggen sowie zahlreichen Gräsern), Uromyces appendiculatus (Bohnenrost), Sclerotium rolfsii (Wurzel- und Stengelfäule bei zahlreichen Pflanzen).
- Deuteromyceten (Fungi imperfecti) wie Septoria (Stagonospora) nodorum (Spelzenbräune) an Weizen (Septoria tritici), Pseudocercosporella herpotrichoides (Halmbruchkrankheit an Weizen, Gerste, Roggen), Rynchosporium secalis (Blattfleckenkrankheit an Roggen und Gerste), Alternaria solani (Dürrfleckenkrankheit an Kartoffel, Tomate), Phoma betae (Wurzelbrand an Beta-Rübe), Cercospora beticola (Cercospora-Blattfleckenkrankheit an Beta-Rübe), (Alternaria brassicae (Rapsschwärze an Raps, Kohl u.a. Kreuzblütlern), Verticillium dahliae (Rapswelke und -stengelfäule), Colletotrichum lindemuthianum (Brennfleckenkrankheit an Bohne), Phoma lingam - Umfallkrankheit (Schwarzbeinigkeit an Kohl; Wurzelhals- oder Stengelfäule an Raps), Botrytis cinerea (Grauschimmel an Weinrebe, Erdbeere, Tomate, Hopfen etc.).

Besonders bevorzugt sind biotrophe Pathogene, und unter diesen insbesondere heminekrotrophe Pathogene, d.h. Phakopsora pachyrhizi und/oder jene, die im wesentlichen einen ähnlichen Infektionsmechanismus wie Phakopsora pachyrhizi, wie er hierin beschrieben ist, aufweisen. Insbesondere bevorzugt sind Pathogene aus der Gruppe der Uredinales (Rostpilze), und unter diesen besonders die Melompsoraceae. Besonders bevorzug sind Phakopsora pachyrhizi und/oder Phakopsora meibomiae.

### 2. Bakterielle Pathogene:

Beispielhaft jedoch nicht einschränkend seien die in Tabelle 5 genannten Pathogene und die mit ihnen in Zusammenhang gebrachten Erkrankungen zu nennen.

**Tabelle 5: Bakterielle Erkrankungen**

| Erkrankung | Pathogen |
|---|---|
| Bacterial leaf blight and stalk rot | Pseudomonas avenae subsp. avenae |
| Bacterial leaf spot | Xanthomonas campestris pv. holcicola |
| Bakterielle Stengelfäule | Enterobacter dissolvens = Erwinia dissolvens |
| Schwarzbeinigkeit ("Bacterial stalk and top rot") | Erwinia carotovora subsp. carotovora, Erwinia chrysanthemi pv. zeae |
| Bacterial stripe | Pseudomonas andropogonis |
| Chocolate spot | Pseudomonas syringae pv. coronafaciens |
| Goss's bacterial wilt and blight (leaf freckles and wilt) | Clavibacter michiganensis subsp. nebraskensis = Corynebacterium michiganense pv.andnebraskense |
| Holcus spot | Pseudomonas syringae pv. syringae |
| Purple leaf sheath | Hemiparasitic bacteria |
| Seed rot-seedling blight | Bacillus subtilis |
| Stewart's disease (bacterial wilt) | Pantoea stewartii = Erwinia stewartii |
| Corn stunt (achapparramiento,maize stunt, Mesa Central or Rio Grande maize stunt) | Spiroplasma kunkelii |

### 3. Virale Pathogene:

"Virale Pathogene" schließt sämtliche Pflanzenviren ein wie beispielsweise Tabak- oder oder Cucumber-Mosaiv Virus, Ringspot-Virus, Nekrose-Virus, Mais Dwarf-Mosaic Virus etc.

Beispielhaft jedoch nicht einschränkend sind die in Tabelle 6 genannten Pathogene und die mit ihnen in Zusammenhang gebrachten Erkrankungen zu nennen.

**Tabelle 6: Virale Erkrankungen**

| Krankheit | Pathogen |
|---|---|
| American wheat striate (wheat striate mosaic) | American wheat striate mosaic virus (AWSMV) |
| Barley stripe mosaic | Barley stripe mosaic virus (BSMV) |
| Barley yellow dwarf | Barley yellow dwarf virus (BYDV) |
| Brome mosaic | Brome mosaic virus (BMV) |
| Cereal chlorotic mottle | Cereal chlorotic mottle virus (CCMV) |
| Corn chlorotic vein banding (Braizilian maize mosaic) | Corn chlorotic vein banding virus (CCVBV) |
| Corn lethal necrosis | Viruskomplex aus Maize chlorotic mottle virus (MCMV) und Maize dwarf mosaic virus (MDMV) A oder B oder Wheat streak mosaic virus(WSMV) |
| Cucumber mosaic | Cucumber mosaic virus (CMV) |
| Cynodon chlorotic streak | Cynodon chlorotic streak virus (CCSV) |
| Johnsongrass mosaic | Johnsongrass mosaic virus (JGMV) |
| Maize bushy stunt | Mycoplasma-like organism (MLO) associated |
| Maize chlorotic dwarf | Maize chlorotic dwarf virus (MCDV) |
| Maize chlorotic mottle | Maize chlorotic mottle virus (MCMV) |
| Maize dwarf mosaic | Maize dwarf mosaic virus (MDMV) strains A, D, E and F |
| Maize leaf fleck | Maize leaf fleck virus (MLFV) |
| Maize line | Maize line virus (MLV) |
| Maize mosaic (corn leaf stripe, enanismo rayado) | Maize mosaic virus (MMV) |
| Maize mottle and chlorotic stunt | Maize mottle and chlorotic stunt virus |
| Maize pellucid ringspot | Maize pellucid ringspot virus (MPRV) |
| Maize raya gruesa | Maize raya gruesa virus (MRGV) |
| maize rayado fino (fine striping disease) | Maize rayado fino virus (MRFV) |
| Maize red leaf and red stripe | Mollicute |
| Maize red stripe | Maize red stripe virus (MRSV) |
| Maize ring mottle | Maize ring mottle virus (MRMV) |
| Maize rio IV | Maize rio cuarto virus (MRCV) |
| Maize rough dwarf (nanismo ruvido) | Maize rough dwarf virus (MRDV) (Cereal tillering disease virus) |
| Maize sterile stunt | Maize sterile stunt virus (strains of barley yellow striate virus) |
| Maize streak | Maize streak virus (MSV) |
| Maize stripe (maize chlorotic stripe, maize hoja blanca) | Maize stripe virus |
| Maize stunting | Maize stunting virus |
| Maize tassel abortion | Maize tassel abortion virus (MTAV) |
| Maize vein enation | Maize vein enation virus (MVEV) |
| Maize wallaby ear | Maize wallaby ear virus (MWEV) |
| Maize white leaf | Maize white leaf virus |
| Maize white line mosaic | Maize white line mosaic virus (MWLMV) |
| Millet red leaf | Millet red leaf virus (MRLV) |
| Northern cereal mosaic | Northern cereal mosaic virus (NCMV) |
| Oat pseudorosette (zakuklivanie) | Oat pseudorosette virus |
| Oat sterile dwarf | Oat sterile dwarf virus (OSDV) |
| Rice black-streaked dwarf | Rice black-streaked dwarf virus (RBSDV) |
| Rice stripe | Rice stripe virus (RSV) |
| Sorghum mosaic | Sorghum mosaic virus (SrMV) (auch: sugarcane mosaic virus (SCMV) Stämme H, I and M) |
| Sugarcane Fiji disease | Sugarcane Fiji disease virus (FDV) |
| Sugarcane mosaic | Sugarcane mosaic virus (SCMV) strains A, B, D, E, SC, BC, Sabi and MB (formerly MDMV-B) |
| Wheat spot mosaic | Wheat spot mosaic virus (WSMV) |

### 4. Tierische Schädlinge

### 4.1 Insekten Pathogene:

Beispielhaft jedoch nicht einschränkend seien Insekten wie beispielsweise Käfer, Raupen, Läuse oder Milben zu nennen.

Bevorzugt sind Insekten der Gattungen Coleoptera, Diptera, Hymenoptera, Lepidoptera, Mallophaga, Homoptera, Hemiptera, Orthoptera, Thysanoptera. Dermaptera, Isoptera, Anoplura, Siphonaptera, Trichoptera, etc.. Besonders bevorzugt sind Coleoptera and Lepidoptera Insekten, wie beispielsweise den Maiszünsler (European Corn Borer), Diabrotica barberi, Diabrotica undecimpunctata, Diabrotica virgifera, Agrotis ipsilon , Crymodes devastator, Feltia ducens, Agrotis gladiaria, Melanotus spp., Aeolus mellillus, Aeolus mancus, Horistonotus uhlerii, Sphenophorus maidis, Sphenophorus zeae, Sphenophorus parvulus, Sphenophorus callosus, Phyllogphaga spp., Anuraphis maidiradicis, Delia platura, Colaspis brunnea, Stenolophus lecontei und Clivinia impressifrons.

Ferner sind zu nennen: Das Getreidehähnchen (Oulema melanopus), die Fritfliege (Oscinella frit), Drahtwürmer (Agrotis lineatus) und Blattläuse (wie z.B. Haferblattlaus Rhopalosiphum padi, Grosse Getreideblattlaus Sitobion avenae).

### 4.2 Nematoden:

Beispielhaft jedoch nicht einschränkend seien die in Tabelle 7 genannten Pathogene und die mit ihnen in Zusammenhang gebrachten Erkrankungen zu nennen.

**Tabelle 7: Parasitäre Nematoden**

| Schädigung | Pathogene Nematode |
|---|---|
| Awl | Dolichodorus spp., D. heterocephalus |
| Stengel- oder Stockälchen, Rübenkopfälchen | Ditylenchus dipsaci |
| Burrowing | Radopholus similis |
| Haferzystenälchen | Heterodera avenae, H. zeae, Punctodera chalcoensis |
| Dagger | Xiphinema spp., X. americanum, X. mediterraneum |
| False root-knot | Nacobbus dorsalis |
| Lance, Columbia | Hoplolaimus columbus |
| Lance | Hoplolaimus spp., H. galeatus |
| Lesion | Pratylenchus spp., P. brachyurus, P. crenatus, P. hexincisus, P. neglectus, P. penetrans, P. scribneri, P. thornei, P. zeae |
| Needle | Longidorus spp., L. breviannulatus |
| Ring | Criconemella spp., C. ornata |
| Wurzelgallenälchen | Meloidogyne spp., M. chitwoodi, M. incognita, M. javanica |
| Spiral | Helicotylenchus spp. |
| Sting | Belonolaimus spp., B. longicaudatus |
| Stubby-root | Paratrichodorus spp., P. christiei, P. minor, Quinisulcius acutus, Trichodorus spp. |
| Stunt | Tylenchorhynchus dubius |

Ganz besonders bevorzugt sind Globodera rostochiensis und G. pallida (Zystenälchen an Kartoffel, Tomate u.a. Nachtschattengewächsen), Heterodera schachtii (Rübenzystenälchen an Zucker- und Futterrübe, Raps, Kohl etc.), Heterodera avenae (Haferzystenälchen an Hafer u.a. Getreidearten), Ditylenchus dipsaci (Stengel- oder Stockälchen, Rübenkopfälchen an Roggen, Hafer, Mais, Klee, Tabak, Rübe), Anguina tritici (Weizenälchen, Radekrankheit an Weizen (Dinkel, Roggen), Meloidogyne hapla (Wurzelgallenälchen an Möhre, Gurke, Salat, Tomate, Kartoffel, Zuckerrübe, Luzerne).

Als für die einzelnen Sorten bevorzugte Pilz- oder Virus-Pathogene sind beispielsweise zu nennen:

### 1. Gerste:

Pilz-, bakterielle und virale Pathogene: Puccinia graminis f.sp. hordei, barley yellow dwarf virus (BYDV).

Pathogene Insekten / Nematoden: Ostrinia nubilalis (European corn borer); Agrotis ipsilon; Schizaphis graminum; Blissus leucopterus leucopterus; Acrosternum hilare; Euschistus servus; Deliaplatura; Mayetiola destructor; Petrobia latens.

### 2. Sojabohne:

Pilz-, bakterielle oder virale Pathogene: Phytophthora megasperma fsp.glycinea, Macrophomina phaseolina, Rhizoctonia solani, Sclerotinia sclerotiorum, Fusarium oxysporum, Diaporthe phaseolorum var. sojae (Phomopsis sojae), Diaporthe phaseolorum var. caulivora, Sclerotium rolfsii, Cercospora kikuchii, Cercospora sojina, Peronospora manshurica, Colletotrichum dematium (Colletotrichum truncatum), Corynespora cassiicola, Septoria glycines, Phyllosticta sojicola, Alternaria alternata, Pseudomonas syringae p.v. glycinea, Xanthomonas campestris p.v. phaseoli, Microsphaera diffussa, Fusarium semitectum, Phialophora gregata, Sojabohnen Mosaikvirus, Sojabohnenrost, Glomerella glycines, Tobacco Ring spot virus, Tobacco Streak virus, Phakopsorapachyrhizi, Pythium aphanidermatum, Pythium ultimum, Pythium debaryanum, Tomato spotted wilt virus, Heterodera glycines Fusarium solani; insbesondere Sojabohnenrost.

Pathogene Insekten / Nematoden: Pseudoplusia includens; Anticarsia gemmatalis; Plathypena scabra; Ostrinia nubilalis; Agrotis ipsilon; Spodoptera exigua; Heliothis virescens; Helicoverpa zea; Epilachna varivestis; Myzus persicae; Empoasca fabae; Acrosternum hilare; Melanoplus femurrubrum; Melanoplus differentialis; Hylemya platura; Sericothrips variabilis; Thrips tabaci; Tetranychus turkestani; Tetranychus urticae.

### 3. Raps:

Pilz-, bakterielle oder virale Pathogene: Albugo candida, Alternaria brassicae, Leptosphaeria maculans, Rhizoctonia solani, Sclerotinia sclerotiorum, Mycosphaerella brassiccola, Pythium ultimum, Peronospora parasitica, Fusarium roseum, Alternaria alternata.

### 4. Alfalfa:

Pilz,-, bakterielle oder virale Pathogene: Clavibater michiganese subsp. insidiosum, Pythium ultimum, Pythium irregulare, Pythium splendens, Pythium debaryanum, Pythium aphanidermatum, Phytophthora megasperma, Peronospora trifoliorum, Phoma medicaginis var. medicaginis, Cercospora medicaginis, Pseudopeziza medicaginis, Leptotrochila medicaginis, Fusarium, Xanthomonas campestris p.v. alfalfae, Aphanomyces euteiches, Stemphylium herbarum, Stemphylium alfalfae.

### 5. Weizen:

Pilz-,bakterielle oder virale Pathogene: Pseudomonas syringae p.v. atrofaciens, Urocystis agropyri, Xanthomonas campestris p.v. translucens, Pseudomonas syringae p.v. syringae, Alternaria alternata, Cladosporium herbarum, Fusarium graminearum, Fusarium avenaceum, Fusarium culmorum, Ustilago tritici, Ascochyta tritici, Cephalosporium gramineum, Collotetrichum graminicola, Erysiphe graminis f.sp. tritici, Puccinia graminis f.sp. tritici, Puccinia recondita f.sp. tritici, Puccinia striiformis, Pyrenophora tritici-repentis, Septoria (Stagonospora) nodorum, Septoria tritici, Septoria avenae, Pseudocercosporella herpotrichoides, Rhizoctonia solani, Rhizoctonia cerealis, Gaeumannomyces graminis var. tritici, Pythium aphanidermatum, Pythium arrhenomanes, Pythium ultimum, Bipolaris sorokiniana, Barley Yellow Dwarf Virus, Brome Mosaic Virus, Soil Borne Wheat Mosaic Virus, Wheat Streak Mosaic Virus, Wheat Spindle Streak Virus, American Wheat Striate Virus, Claviceps purpurea, Tilletia tritici, Tilletia laevis, Ustilago tritici, Tilletia indica, Rhizoctonia solani, Pythium arrhenomannes, Pythium gramicola, Pythium aphanidermatum, High Plains Virus, European Wheat Striate Virus, Puccinia graminis f.sp. tritici (Wheat stem rust), Blumeria (Erysiphe) graminis f.sp. tritici (Wheat Powdery Mildew).

Pathogene Insekten / Nematoden: Pseudaletia unipunctata; Spodoptera,frugiperda; Elasmopalpus lignosellus; Agrotis orthogonia; Elasmopalpus Zignosellus; Oulema melanopus; Hypera punctata; Diabrotica undecimpunctata howardi; Russian wheat aphid; Schizaphis graminum; Macrosiphum avenae; Melanoplus femurrubrum; Melanoplus differentialis; Melanoplus sanguinipes; Mayetiola destructor; Sitodiplosis mosellana; Meromyza americana; Hylemya coarctata; Frankliniella fusca; Cephus cinctus; Aceria tulipae.

### 6. Sonnenblume:

Pilz-, bakterielle oder virale Pathogene: Plasmophora halstedii, Sclerotinia sclerotiorum, Aster Yellows, Septoria helianthi, Phomopsis helianthi, Alternaria helianthi, Alternaria zinniae, Botrytis cinerea, Phoma macdonaldii, Macrophomina phaseolina, Erysiphe cichoracearum, Rhizopus oryzae, Rhizopus arrhizus, Rhizopus stolonifer, Puccinia helianthi, Verticillium dahliae, Erwinia carotovorum p.v. Carotovora, Cephalosporium acremonium, Phytophthora cryptogea, Albugo tragopogonis.

Pathogene Insekten / Nematoden: Suleima helianthana; Homoeosoma electellum; zygogramma exclamationis; Bothyrus gibbosus; Neolasioptera murtfeldtiana.

### 7. Mais:

Pilz-, bakterielle oder virale Pathogene: Fusarium moniliforme var. subglutinans, Erwinia stewartii, Fusarium moniliforme, Gibberella zeae (Fusarium graminearum), Stenocarpella maydi (Diplodia maydis), Pythium irregulare, Pythium debaryanum, Pythium graminicola, Pythium splendens, Pythium ultimum, Pythium aphanidermatum, Aspergillus flavus, Bipolaris maydis 0, T (Cochliobolus heterostrophus), Helminthosporium carbonum I, II & III (Cochliobolus carbonum), Exserohilum turcicum I, II & III, Helminthosporium pedicellatum, Physoderma maydis, Phyllosticta maydis, Kabatiella maydis, Cercospora sorghi, Ustilago maydis, Puccinia sorghi, Puccinia polysora, Macrophomina phaseolina, Penicillium oxalicum, Nigrospora oryzae, Cladosporium herbarum, Curvularia lunata, Curvularia inaequalis, Curvularia pallescens, Clavibacter michiganese subsp. nebraskense, Trichoderma viride, Maize Dwarf Mosaic Virus A & B, Wheat Streak Mosaic Virus, Maize Chlorotic Dwarf Virus, Claviceps sorghi, Pseudonomas avenae, Erwinia chrysanthemi p.v. Zea, Erwinia corotovora, Cornstunt spiroplasma, Diplodia macrospora, Sclerophthora macrospora, Peronosclerospora sorghi, Peronosclerospora philippinesis, Peronosclerospora maydis, Peronosclerospora sacchari, Spacelotheca reiliana, Physopella zeae, Cephalosporium maydis, Caphalosporium acremonium, Maize Chlorotic Mottle Virus, High Plains Virus, Maize Mosaic Virus, Maize Rayado Fino Virus, Maize Streak Virus (MSV, Maisstrichel-Virus), Maize Stripe Virus, Maize Rough Dwarf Virus.

Pathogene Insekten / Nematoden: Ostrinia nubilalis; Agrotis ipsilon; Helicoverpa zea; Spodoptera frugiperda; Diatraea grandiosella; Elasmopalpus lignosellus; Diatraea saccharalis; Diabrotica virgifera; Diabrotica longicornis barberi; Diabrotica undecimpunctata howardi; Melanotus spp.; Cyclocephala borealis; Cyclocephala immaculata; Popillia japonica; Chaetocnema pulicaria; Sphenophorus maidis; Rhopalosiphum maidis; Anuraphis maidiradicis; Blissus leucopterus leucopterus; Melanoplus femurrubrum; Melanoplus sanguinipes; Hylemva platura; Agromyza parvicornis; Anaphothrips obscrurus; Solenopsis milesta; Tetranychus urticae.

### 8. Sorghum:

Pilz-, bakterielle oder virale Pathogene: Exserohilum turcicum, Colletotrichum graminicola (Glomerella graminicola), Cercospora sorghi, Gloeocercospora sorghi, Ascochyta sorghina, Pseudomonas syringae p.v. syringae, Xanthomonas campestris p.v. holcicola, Pseudomonas andropogonis, Puccinia purpurea, Macrophomina phaseolina, Perconia circinata, Fusarium monilifonne, Alternaria alternate, Bipolaris sorghicola, Helminthosporium sorghicola, Curvularia lunata, Phoma insidiosa, Pseudomonas avenae (Pseudomonas alboprecipitans), Ramulispora sorghi, Ramulispora sorghicola, Phyllachara sacchari, Sporisorium reilianum (Sphacelotheca reiliana), Sphacelotheca cruenta, Sporisorium sorghi, Sugarcane mosaic H, Maize Dwarf Mosaic Virus A & B, Claviceps sorghi, Rhizoctonia solani, Acremonium strictum, Sclerophthona macrospora, Peronosclerospora sorghi, Peronosclerospora philippinensis, Sclerospora graminicola, Fusarium graminearum, Fusarium oxysporum, Pythium arrhenomanes, Pythium graminicola.

Pathogene Insekten / Nematoden: Chilo partellus; Spodoptera frugiperda; Helicoverpa zea; Elasmopalpus lignosellus; Feltia subterranea; Phvllophaga crinita ; Eleodes, Conoderus und Aeolus spp.; Oulema melanopus; Chaetocnema pulicaria; Sphenophorus maidis; Rhopalosiphum maidis; Siphaflava; Blissus leucopterus leucopterus; Contarinia sorghicola; Tetranychus cinnabarinus; Tetranychus urticae.

### 9. Baumwolle:

Pathogene Insekten / Nematoden: Heliothis virescens; Helicoverpa zea; Spodoptera exigua; Pectinophora gossypiella; Anthonomus grandis grandis; Aphis gossypii; Pseudatomoscelis seriatus; Trialeurodes abutilonea; Lygus lineolaris; Melanoplus femurrubrum; Melanoplus differentialis; Thrips tabaci (onion thrips); Franklinkiella fusca; Tetranychus cinnabarinus; Tetranychus urticae.

### 10. Reis:

Pathogene Insekten / Nematoden: Diatraea saccharalis; Spodoptera frugiperda; Helicoverpa zea; Colaspis brunnea; Lissorhoptrus oryzophilus; Sitophilus oryzae; Nephotettix nigropictus; Blissus leucopterus leucopterus; Acrosternum hilare.

### 11. Raps:

Pathogene Insekten / Nematoden: Brevicoryne brassicae; Phyilotreta cruciferae; Mamestra conjgurata; Plutella xylostella; Delia ssp..

"BI1-Protein" meint im Rahmen der Erfindung Polypeptide die mindestens eine Sequenz die eine Homologie von bevorzugt mindestens 80%, besonders bevorzugt mindestens 90%, ganz besonders bevorzugt mindestens 95 %, und insbesondere 100% aufweisen zu einem BI1-Konsensusmotiv ausgewählt aus der Gruppe bestehend aus
a) H(L/I)KXVY
b) AXGA(Y/F)XH
c) NIGG
d) P(V/P)(Y/F)E(E/Q)(R/Q)KR
e) (E/Q)G(A/S)S(V/I)GPL
f) DP(S/G)(L/I)(I/L)
g) V(G/A)T(A/S)(L/I)AF(A/G)CF(S/T)
h) YL(Y/F)LGG, bevorzugt EYLYLGG
i) L(L/V)SS(G/W)L(S/T)(I/M)L(L/M)W
j) DTGX(I/V)(I/V)E

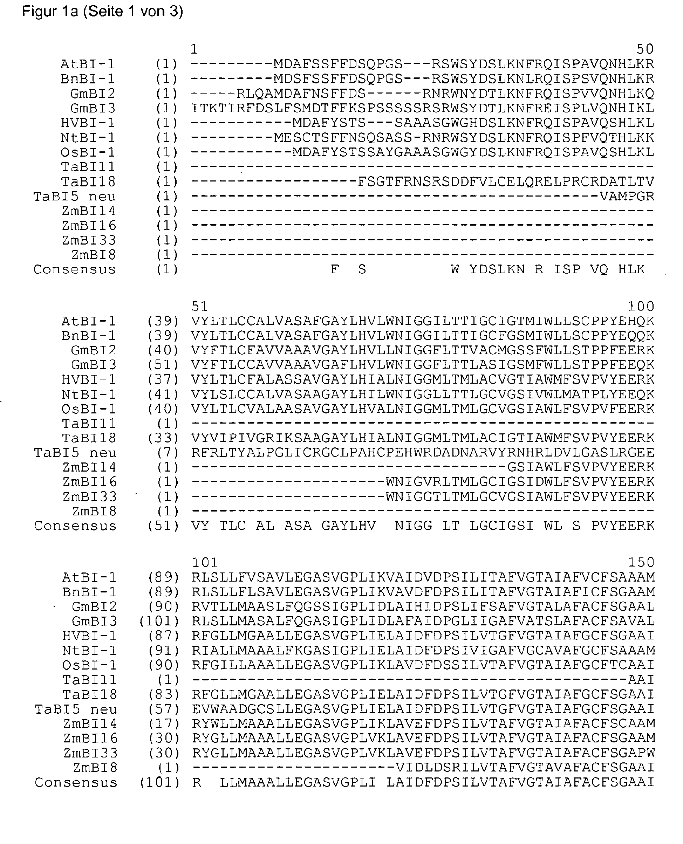

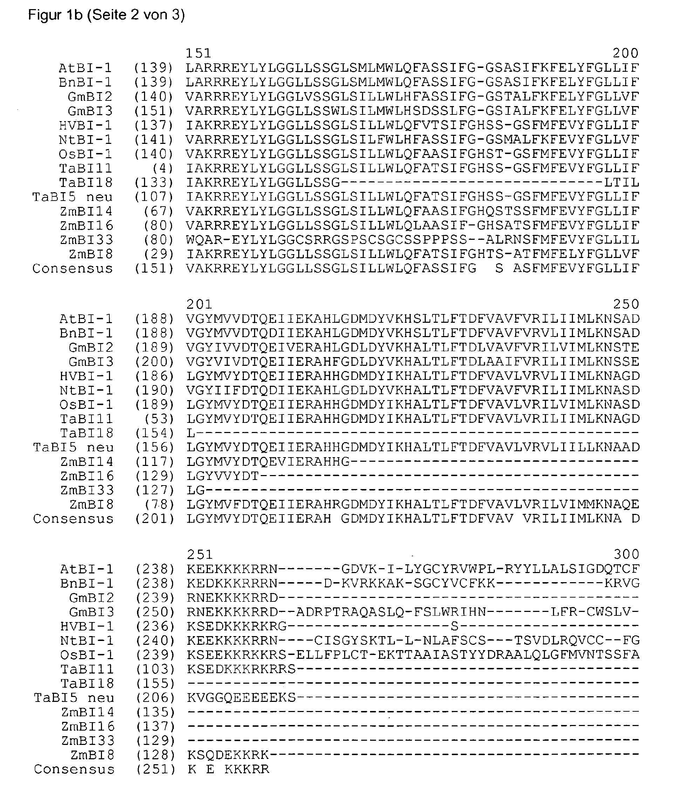

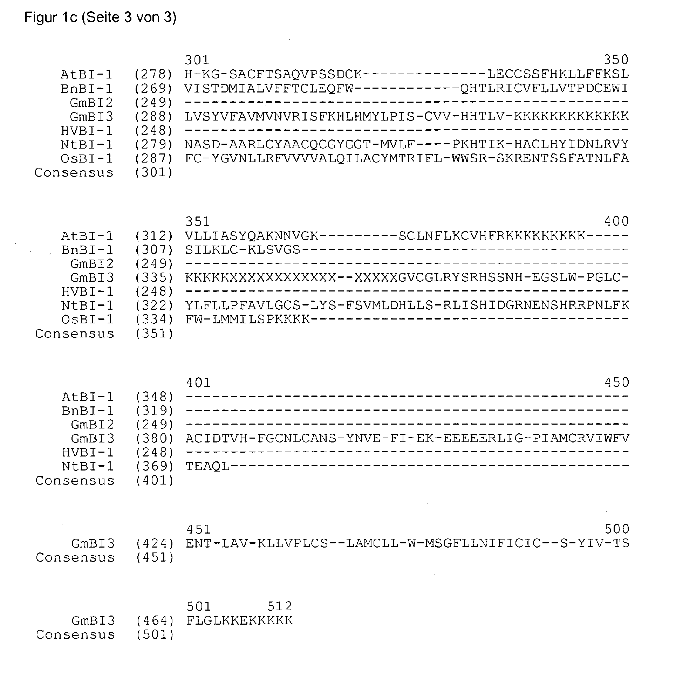

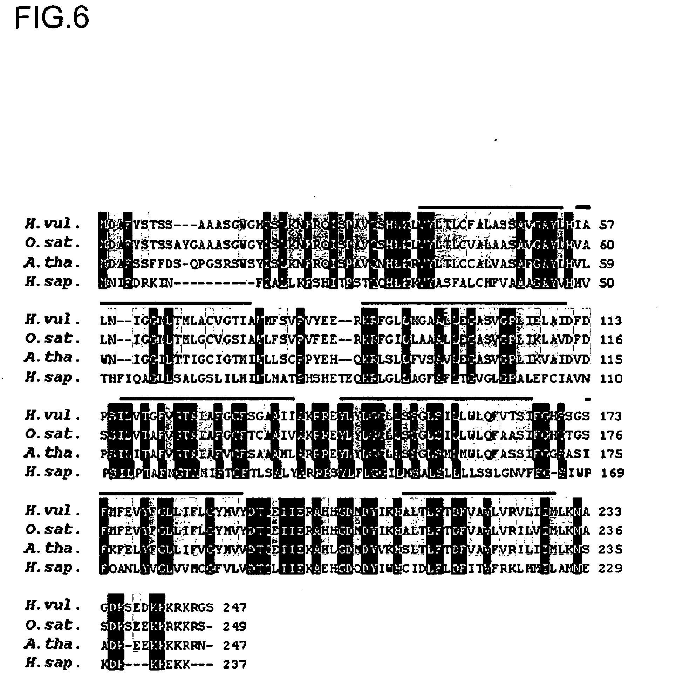

Besonders bevorzugt ist dabei das BI- Konsensusmotiv h) YL(Y/F)LGG, ganz besonders bevorzugt (EYLYLGG). Dieses Motiv ist charakteristisch für pflanzliche BI1-Proteine. Besonders bevorzugt kommen Sequenzen mit Homologie zu mindestens 2 oder 3 dieser Motive (a bis j) in einem BI1-Protein vor, ganz besonders bevorzugt mindestens 4 oder 5, am meisten bevorzugt alle Motive a bis j. Weitere BI1 typischen Sequenzmotive kann der Fachmann unschwer aus dem Sequenzvergleich von BI1-Proteine - wie in FIG. 1 oder 6 dargestellt - ableiten.

Insbesondere bevorzugt für die Verwendung in den hierin offenbarten Verfahren sind BI1-Proteine, die kodiert werden durch ein Polypeptid das mindestens eine Sequenz umfaßt ausgewählt aus der Gruppe bestehend aus:
(a) den Sequenzen gemäß SEQ ID NO 2, 4, 6, 8, 10, 12, 14, 16, 18, 20, 22, 24, 26, 28, oder 38 ; und
(b) Sequenzen, die eine Identität von mindestens 70%, bevorzugter 80%, 85% oder 90%, insbesondere bevorzugt 95, 97 oder 99% oder mehr zu einer der Sequenzen gemäß SEQ ID NO 2, 4, 6, 8, 10, 12, 14, 16, 18, 20, 22, 24, 26, 28, oder 38 aufweisen.

Von dem Begriff BI-Protein umfasst sind insbesondere natürliche oder künstliche Mutationen der BI1-Polypeptide gemäß SEQ ID NO: 2, 4, 6, 8, 10, und insbesondere 38 sowie homologe bzw. ähnliche Polypeptide aus anderen Organismen, bevorzugt Pflanzen, welche weiterhin im wesentlichen gleiche Eigenschaften aufweisen. Mutationen umfassen Substitutionen, Additionen, Deletionen, Inversion oder Insertionen eines oder mehrerer Aminosäurereste.

Umfaßt sind insofern auch Ausführungsformen unter Verwendung von BI1-Proteinen aus nicht-pflanzlichen Organismen wie beispielsweise Mensch (GenBank Acc.-No.: P55061), Ratte (GenBank Acc.-No.: P55062) oder Drosophila (GenBank Acc.-No.: Q9VSH3). Zwischen pflanzlichen und nicht-pflanzlichen BI1-Proteinen konservierte Motive können durch Sequenzvergleiche leicht identifiziert werden (vgl. Alignment in Bolduc N. et al., Planta 216, 377 (2003); Fig. 1 und 6).

Somit werden beispielsweise auch solche Polypeptide durch die vorliegende Erfindung mit umfaßt, welche man durch Modifikation eines Polypeptides gemäß SEQ ID NO 2, 4, 6, 8, 10 und 38 erhält.

Die offenbarten BI1-Sequenzen homologen Sequenzen aus anderen Pflanzen können z.B. durch
(a) Datenbanksuche in Banken von Organismen, deren genomische oder cDNA Sequenz ganz oder teilweise bekannt ist, unter Verwendung der bereitgestellten BI1-Sequenzen als Suchsequenz oder
(b) Durchmustern von Gen- oder cDNA-Bibliotheken unter Verwendung der bereitgestellten BI1-Sequenzen als Sonde -
aufgefunden werden. Die Durchmusterung von cDNA- oder genomischen Bibliotheken (beispielsweise unter Verwendung einer der unter SEQ ID NO 1, 3, 5, 7, 9, 11, 13 , 15, 17, 19, 21, 23, 25, 27, und 37 beschriebene Nukleinsäuresequenzen oder Teilen derselben als Sonde), ist ein dem Fachmann geläufiges Verfahren, um homologe bzw. ähnliche oder identische Sequenzen zu identifizieren. Dabei haben die von den Nukleinsäuresequenzen gemäß SEQ ID NO 1, 3, 5, 7, 9, 11, 13, 15, 17, 19, 21, 23, 25, 27, , und 37 abgeleiteten Sonden eine Länge von mindestens 20 bp, bevorzugt mindestens 50 bp, besonders bevorzugt mindestens 100 bp, ganz besonders bevorzugt mindestens 200 bp, am meisten bevorzugt mindestens 400 bp. Für die Durchmusterung der Bibliotheken kann auch ein zu den unter SEQ ID NO 1, 3, 5, 7, 9, 11, 13, 15, 17, 19, 21, 23, 25, 27, und 37 beschriebenen Sequenzen komplementärer DNA-Strang eingesetzt werden.

Unter Homologie zwischen zwei Nukleinsäuresequenzen wird hierin die Identität der Nukleinsäuresequenz über die jeweils gesamte Sequenzlänge verstanden, welche wiederum durch Vergleich mit Hilfe des Programmalgorithmus GAP (Wisconsin Package Version 10.0, University of Wisconsin, Genetics Computer Group (GCG), Madison, USA; Altschul et al., Nucleic Acids Res. 25, 3389 (1997)) unter Einstellung folgender Parameter berechnet wird:

| | |
|---|---|
| Gap Weight: | 50 Length Weight: 3 |
| Average Match: | 10 Average Mismatch: 0 |

Beispielhaft wird unter einer Sequenz, die eine Homologie von mindestens 80 % auf Nukleinsäurebasis mit der Sequenz SEQ ID NO 1 aufweist, eine Sequenz verstanden, die bei einem Vergleich mit der Sequenz SEQ ID NO 1 nach obigem Programmalgorithmus mit obigem Parametersatz eine Homologie von mindestens 80 % aufweist.

Unter Homologie zwischen zwei Polypeptiden wird die Identität der Aminosäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: | 8 Length Weight: 2 |
| Average Match: | 2,912 Average Mismatch:-2,003 |

Beispielhaft wird unter einer Sequenz, die eine Homologie von mindestens 80 % auf Proteinbasis mit der Sequenz SEQ ID NO 2 aufweist, eine Sequenz verstanden, die bei einem Vergleich mit der Sequenz SEQ ID NO 2 nach obigem Programmalgorithmus mit obigem Parametersatz eine Homologie von mindestens 80 % aufweist.

BI1-Proteine umfassen auch solche Polypeptide die durch Nukleinsäuresequenzen kodiert werden, die unter Standardbedingungen mit einer der durch SEQ ID NO 1, 3, 5, 7, 9, 11, 13 , 15, 17, 19, 21, 23, 25, 27, und 37 beschriebenen BI1 Nukleinsäuresequenz, der zu ihr komplementären Nukleinsäuresequenz oder Teilen der vorgenannten hybridisieren und die gleichen wesentlichen Eigenschaften wie die unter SEQ ID NO 2, 4, 6, 8, 10, und 38 beschriebenen Proteine haben.

Der Begriff "Standardhybridisierungsbedingungen" ist breit zu verstehen und meint stringente als auch weniger stringente Hybridisierungsbedingungen. Solche Hybridisierungsbedingungen sind unter anderem bei Sambrook J., Fritsch E.F., Maniatis T. et al., in Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6. beschrieben. Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3M Natriumcitrat, 3M NaCl, pH 7.0). Darüber hinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50% Formamid wird die Hybridisierung bevorzugt bei 42°C ausgeführt.

"Wesentliche Eigenschaften" meint in Bezug auf ein BI-Protein eine oder mehrere nachfolgender Eigenschaften:
(a) Verleihung oder Steigerung der Pathogenresistenz gegen zumindest ein Pathogen bei Erhöhung der Proteinmenge oder Funktion des besagten BI-Proteins in mindestens einem Gewebe der Pflanze, vorzugsweise zumindest in der Epidermis der Pflanze.
(b) Ausbleiben eines spontan-induzierten Zelltodes bei Erhöhung der Proteinmenge oder Funktion des besagten BI-Proteins.
(c) Die Eigenschaft bei transienter co-Transfektion von Bax mit besagtem BI1-Protein beispielsweise in HEK293 Zellen die BAX-induzierte Apoptose signifikant zu inhibieren. Entsprechende Verfahren sind beschrieben (Bolduc N. et al., Planta 216, 377 (2003)).
(d) Das Vorliegen von fünf bis sieben putativen Transmembrandomänen innerhalb des besagten BI1-Proteins.
(e) Eine präferentielle Lokalisation in Zellmembranen, insbesondere der Kernhüll-, ER- und/oder Thylakoidmembran.

Dabei kann die quantitative Ausprägung besagter Eigenschaften eines BI1-Proteins nach unten als auch nach oben im Vergleich zu einem Wert erhalten für das BI1-Protein gemäß SEQ ID NO 2, 4, 6, 8, 10, oder 38 abweichen.

Der Begriff "Erhöhung der BI1 Proteinmenge oder Funktion" ist breit zu verstehen und kann auf unterschiedlichen zellbiologischen Mechanismen beruhen.

"Proteinmenge" meint die Menge eines BI1-Proteins in dem jeweils angegebenen Organismus, Gewebe, Zelle oder Zellkompartiment.

"Erhöhung der Proteinmenge" meint die mengenmäßige Erhöhung der Menge eines BI1-Proteins in dem jeweils angegebenen Organismus, Gewebe, Zelle oder Zellkompartiment - beispielsweise durch eines der unten beschriebenen Verfahren - im Vergleich zu dem Wildtyp derselben Gattung und Art auf den dieses Verfahren nicht angewendet wurde, aber unter ansonsten im wesentlichen gleichen Rahmenbedingungen (wie beispielsweise Kulturbedingungen, Alter der Pflanzen etc.). Die Erhöhung beträgt dabei mindestens 10 %, bevorzugt mindestens 30 % oder mindestens 50 %, besonders bevorzugt mindestens 70 % oder 100 %, ganz besonders bevorzugt um mindestens 200 % oder 500 %, am meisten bevorzugt um mindestens 1000 %. Die Bestimmung der Proteinmenge kann durch verschiedene dem Fachmann geläufige Verfahren erfolgen. Beispielhaft, jedoch nicht einschränkend, sind zu nennen: Das Mikro-Biuret Verfahren (Goa J., Scand J. Clin. Lab. Invest. 5, 218 (1953)), die Folin-Ciocalteu-Methode (Lowry O.H. et al., J Biol Chem 193, 265 (1951)) oder die Messung der Adsorption von CBB G-250 (Bradford M.M., Analyt.Biochem. 72, 248 (1976)). Ferner kann eine Quantifizierung über immunologische Methoden wie beispielsweise Western-Blot erfolgen. Die Herstellung entsprechender BI1-Antikörper sowie die Durchführung von BI1-Western-Blots ist beschrieben (Bolduc N.et al., FEBS Lett 532, 111 (2002)). Eine indirekte Quantifizierung kann über Northern-Blots realisiert werden, wobei die mRNA Menge in der Regel gut mit der resultierenden Proteinmenge korreliert. Entsprechende Verfahren sind beschrieben (u.a. Bolduc N. et al., Planta 216, 377 (2003); Matsumura H. et al., Plant J. 33,425 (2003)).

"Funktion" meint bevorzugt die Eigenschaft eines BI1-Proteins den spontan-induzierten Zelltodes zu vermindern und/oder die Eigenschaft, die apoptose-indizierende Wirkung von Bax zu inhibieren. Entsprechende Funktionen zählen zu den wesentlichen Eigenschaft eines BI1-Proteins.

"Erhöhung" der Funktion meint beispielsweise die mengenmäßige Steigerung der inhibitorischen Wirkung auf den Bax-induzierten apoptotischen Zelltod, welche durch dem Fachmann geläufige Verfahren quantifiziert werden kann (s.o.) Der Erhöhung beträgt dabei mindestens 10 %, bevorzugt mindestens 30 % oder mindestens 50 %, besonders bevorzugt mindestens 70 % oder 100 %, ganz besonders bevorzugt um mindestens 200 % oder 500 %, am meisten bevorzugt um mindestens 1000 %. Verfahren zur Erhöhung der Funktion umfassen neben den oben beschriebenen Verfahren zur Erhöhung der Proteinmenge (die in der Regel auch die Funktion erhöht) ferner beispielhaft - jedoch nicht einschränkend - insbesondere das Einführen von Mutationen in ein BI1-Protein oder Inhibierung eines putativen Inhibitors von BI1 etc.

Die BI1-Proteinmenge kann beispielsweise, jedoch nicht einschränkend, durch nachfolgenden Verfahren erhöht werden:

Rekombinante Expression oder Überexpression eines BI1-Proteins durch Einbringen einer rekombinanten Expressionskassette umfassend eine Nukleinsäuresequenz kodierend für ein BI1-Protein unter Kontrolle eines gewebespezifischen Promotors, wobei besagter Promotor bevorzugt im wesentlichen spezifisch in der Blattepidermis Aktivität aufweist, und/oder keine Aktivität im Mesophyll aufweist.

Der Begriff "Einbringen" umfaßt allgemein alle Verfahren, die dazu geeignet sind, die einzubringende Verbindung, direkt oder indirekt, in eine Pflanze oder eine Zelle, Kompartiment, Gewebe, Organ oder Samen derselben einzuführen oder dort zu generieren. Direkte und indirekte Verfahren sind umfaßt. Das Einbringen kann zu einer vorübergehenden (transienten) Präsenz besagter Verbindung führen oder aber auch zu einer dauerhaften (stabilen) oder induzierbaren. Einführen umfaßt beispielsweise Verfahren wie Transfektion, Transduktion oder Transformation.

In den zum Einsatz kommenden rekombinanten Expressionskassetten steht ein Nukleinsäuremolekül (beispielsweise kodierend für ein BI1-Protein) in funktioneller Verknüpfung mit mindestens einem gewebespezifischen Promotor, wobei besagter Promotor bevorzugt im wesentlichen spezifisch in der Blattepidermis Aktivität aufweist, und/oder keine Aktivität im Mesophyll aufweist und wobei der Promotor in Bezug auf die zu exprimierende Nukleinsäuresequenz heterolog ist d.h. natürlicherweise nicht mit derselben kombiniert vorkommt. Die rekombinanten Expressionskassetten können optional weitere genetische Kontrollelemente umfassen.

Unter einer funktionellen Verknüpfung versteht man zum Beispiel die sequentielle Anordnung des besagten Promotors mit der zu exprimierenden Nukleinsäuresequenz und ggf. weiterer regulativer Elemente wie zum Beispiel einem Terminator derart, dass jedes der regulativen Elemente seine Funktion bei der rekombinanten Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die rekombinant zu exprimierende Nukleinsäuresequenz hinter der als Promoter fungierenden Sequenz positioniert wird, so dass beide Sequenzen kovalent miteinander verbunden sind. Bevorzugt ist dabei der Abstand zwischen der Promotorsequenz und der rekombinant zu exprimierende Nukleinsäuresequenz geringer als 200 Basenpaare, besonders bevorzugt kleiner als 100 Basenpaare, ganz besonders bevorzugt kleiner als 50 Basenpaare. Die Herstellung einer funktionellen Verknüpfung als auch die Herstellung einer rekombinanten Expressionskassette kann mittels gängiger Rekombinations- und Klonierungstechniken realisiert werden, wie sie oben beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen.

Bevorzugt kann die rekombinante Expressionskassette, bestehend aus einer Verknüpfung von Promoter und zu exprimierender Nukleinsäuresequenz, integriert in einem Vektor vorliegen und durch zum Beispiel Transformation in ein pflanzliches Genom insertiert werden.

Unter einer rekombinanten Expressionskassette sind aber auch solche Konstruktionen zu verstehen, bei denen der Promoter - zum Beispiel durch eine homologe Rekombination - vor ein endogenes BI1-Gen plaziert wird, und so die Expression des BI1-Proteins steuert. Analog kann auch die zu exprimierende Nukleinsäuresequenz (z.B. kodierend für ein BI1-Protein) derart hinter einen endogenen Promotor plaziert werden, dass der gleiche Effekt auftritt. Beide Ansätze führen zu rekombinanten Expressionskassetten im Sinne der Erfindung.

Unter einem "gewebespezifischen Promotor, der im wesentlichen spezifisch in der Blattepidermis Aktivität aufweist" sind allgemein solche Promotoren zu verstehen, die geeignet sind, eine rekombinante Expression einer Nukleinsäuresequenz mindestens in einem pflanzlichen Gewebe zu gewährleisten oder zu erhöhen, mit der Maßgabe, dass
(a) die Expression mindestens in der Epidermis stattfindet, und bevorzugt nicht im Mesophyll bzw. im Mesophyll im Wesentlichen unverändert bleibt, wobei andere als diese beiden Gewebe außer Betracht bleiben, und
(b) die rekombinante Expression unter Kontrolle des besagten Promotors in besagtem pflanzlichen Gewebe mindestens das fünffache, bevorzugt mindestens das zehnfache, besonders bevorzugt mindestens das einhundertfache der Expression gegenüber einer Vergleichspflanze beträgt.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)). Es ist gezeigt worden, dass diese eine signifikante Funktionen bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al., Nucl. Acids Res. 15, 8693 (1987)) und dergleichen. Sie können ferner die Gewebsspezifität fördern (Rouster J. et al., Plant J 15, 435 (1998)).

Die rekombinante Expressionskassette kann vorteilhafterweise eine oder mehrere sogenannte "enhancer Sequenzen" funktionell verknüpft mit dem Promoter enthalten, die eine erhöhte rekombinante Expression der Nukleinsäuresequenz ermöglichen. Auch am 3'-Ende der rekombinant zu exprimierenden Nukleinsäuresequenzen können zusätzliche vorteilhafte Sequenzen inseriert werden, wie weitere regulatorische Elemente oder Terminatoren. Die rekombinant zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignale aus Agrobacterium tumefaciens, insbesondere der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel der natürliche Promoter eines BI1-Gens gegen einen der bevorzugten gewebespezifischen Promoter ausgetauscht werden. Methoden wie die cre/lox-Technologie erlauben eine gewebespezifische, unter Umständen induzierbare Entfernung der rekombinanten Expressionskassette aus dem Genom des Wirtsorganismus (Sauer B., Methods. 14(4), 381(1998)). Hier werden bestimmte flankierende Sequenzen dem Zielgen angefügt (lox-Sequenzen), die später eine Entfernung mittels der cre-Rekombinase ermöglichen.

Eine rekombinante Expressionskassetten und die von ihr abgeleiteten Vektoren können weitere Funktionselemente enthalten. Der Begriff Funktionselement ist breit zu verstehen und meint all solche Elemente, die einen Einfluß auf Herstellung, Vermehrung oder Funktion der erfindungsgemäßen rekombinanten Expressionskassetten, Vektoren oder rekombinanten Organismen haben. Beispielhaft aber nicht einschränkend seien zu nennen:
(a) Selektionsmarker, die eine Resistenz gegen einen Metabolismusinhibitor wie 2-Desoxy-glucose-6-phosphat (WO 98/45456), Antibiotika oder Biozide, bevorzugt Herbizide, wie zum Beispiel Kanamycin, G 418, Bleomycin, Hygromycin, oder Phosphinotricin etc. verleihen. Besonders bevorzugte Selektionsmarker sind solche die eine Resistenz gegen Herbizide verleihen. Beispielhaft seien genannt: DNA Sequenzen, die für Phosphinothricinacetyltransferasen (PAT) kodieren und Glutaminsynthaseinhibitoren inaktivieren (bar und pat Gen), 5-Enolpyruvylshikimat-3-phosphatsynthasegene (EPSP Synthasegene), die eine Resistenz gegen Glyphosat® (N-(Phosphonomethyl)glycin) verleihen, das für das Glyphosat® degradierende Enzyme kodierende gox Gen (Glyphosatoxidoreduktase), das deh Gen (kodierend für eine Dehalogenase, die Dalapon® inaktiviert)sowie bxn Gene, die für Bromoxynil degradierende Nitrilaseenzyme kodieren, das aasa-Gen, das eine Resistenz gegen das Antibiotikum Apectinomycin verleih, das Streptomycinphosphotransferase (SPT) Gen, das eine Resistenz gegen Streptomycin gewährt, das Neomycinphosphotransferas (NPTII) Gen, das eine Resistenz gegen Kanamycin oder Geneticidin verleiht, das Hygromycinphosphotransferase (HPT) Gen, das eine Resistenz gegen Hygromycin vermittelt, das Acetolactatsynthas Gen (ALS), das eine Resistenz gegen SulfonylharnstoffHerbizide verleiht (z.B. mutierte ALS-Varianten mit z.B. der S4 und/oder Hra Mutation), sowie das Acetolactatsynthase-Gen (ALS), das eine Resistenz gegen Imidazolinon-Herbizide verleiht.
(b) Reportergene, die für leicht quantifizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E., Groskreutz D., Mol, Biotechnol. 13(1), 29(1999)) wie das "green fluorescent protein" (GFP) (Sheen et al., Plant Journal 8(5), 777 (1995); Haseloff et al., Proc. Natl. Acad.Sci.USA 94(6), 2122 (1997); Reichel et al., Proc. Natl. Acad. Sci. USA 93(12), 5888 (1996); Tian et al., Plant Cell Rep. 16, 267 (1997); WO 97/41228; Chui W.L. et al., Curr. Biol. 6, 325 (1996); Leffel S.M. et al., Biotechniques 23(5), 912 (1997)), die Chloramphenicoltransferase, eine Luziferase (Ow et al., Science 234, 856 (1986); Millar et al., Plant Mol. Biol. Rep. 10, 324 (1992)), das Aequoringen (Prasher et al., Biochem. Biophys. Res. Commun. 126(3), 1259 (1985)), die β-Galactosidase, R-Locus Gen (kodieren ein Protein, das die Produktion von Anthocyaninpigmenten (rote Färbung) in pflanzlichen Gewebe reguliert und so eine direkte Analyse der Promotoraktivität ohne Zugabe zusätzlicher Hilfstoffe oder chromogener Substrate ermöglicht; Dellaporta et al. in Chromosome Structure and Function: Impact of New Concepts, 18th Stadler Genetics Symposium, 11 (1988)), ganz besonders bevorzugt ist die β-Glucuronidase (Jefferson et al., EMBO J. 6, 3901 (1987)).
(c) Replikationsursprünge, die eine Vermehrung der erfindungsgemäßen rekombinanten Expressionskassetten oder Vektoren in zum Beispiel E.coli gewährleisten. Beispielhaft seien genannt ORI (origin of DNA replication), der pBR322 ori oder der P15A ori (Sambrook et al., Molecular Cloning. A Laboratory Manual, 2nd ed. Cold Spring Harbor Laboratory Press, Cold Spring Harbor, NY, 1989).
(d) Elemente, die für eine Agrobakterium vermittelte Pflanzentransformation erforderlich sind, wie zum Beispiel die rechte oder linke Begrenzung der T-DNA oder die vir-Region.

Zur Selektion erfolgreich homolog rekombinierter oder auch transformierter Zellen ist es in der Regel erforderlich, einen selektionierbaren Marker zusätzlich einzuführen, der den erfolgreich rekombinierten Zellen eine Resistenz gegen ein Biozid (zum Beispiel ein Herbizid), einen Metabolismusinhibitor wie 2-Desoxyglucose-6-phosphat (WO 98/45456) oder ein Antibiotikum verleiht. Der Selektionsmarker erlaubt die Selektion der transformierten Zellen von untransformierten (McCormick et al., Plant Cell Reports 5, 81 (1986)).

Die Einführung einer rekombinanten Expressionskassette in einen Organismus oder Zellen, Geweben, Organe, Teile bzw. Samen desselben (bevorzugt in Pflanzen bzw. pflanzliche Zellen, Gewebe, Organe, Teile oder Samen), kann vorteilhaft unter Verwendung von Vektoren realisiert werden, in denen die rekombinanten Expressionskassetten enthalten sind. Die rekombinante Expressionskassette kann in den Vektor (zum Beispiel ein Plasmid) über eine geeignete Restriktionsschnittstelle eingeführt werden. Das entstandene Plasmid wird zunächst in E.coli eingeführt. Korrekt transformierte E.coli werden selektioniert, gezüchtet und das rekombinante Plasmid mit dem Fachmann geläufigen Methoden gewonnen. Restriktionsanalyse und Sequenzierung können dazu dienen, den Klonierungsschritt zu überprüfen.

Vektoren können beispielhaft Plasmide, Cosmide, Phagen, Viren oder auch Agrobacterien sein. Es wird wird die Einführung der rekombinante Expressionskassette mittels Plasmidvektoren beschrieben. Bevorzugt sind solche Vektoren, die eine stabile Integration der rekombinanten Expressionskassette in das Wirtsgenom ermöglichen.

Die Herstellung eines transformierten Organismus (bzw. einer transformierten Zelle oder Gewebes) erfordert, dass die entsprechende DNA, RNA oder Protein in die entsprechende Wirtszelle eingebracht wird.

Für diesen Vorgang, der als Transformation (oder Transduktion bzw. Transfektion) bezeichnet wird, steht eine Vielzahl von Methoden zur Verfügung (Keown et al., Methods Enzymol. 185, 527 (1990); Jenes B. et al., Techniques for Gene Transfer, in: Transgenic Plants, Vol. 1, Engineering and Utilization, herausgegeben von Kung S.D. und Wu R., Academic Press, S. 128-143 (1993), sowie in Potrykus, Annu. Rev. Plant Physiol. Plant Molec. Biol. 42, 205 (1991)).

So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglykol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zur Einführung von DNA, bei der die Zellen reversibel durch einen elektrischen Impuls permeabilisiert werden. Entsprechende Verfahren sind beschrieben (beispielsweise bei Bilang et al., Gene 100, 247 (1991); Scheid et al., Mol. Gen. Genet. 228, 104 (1991); Guerche et al., Plant Science 52, 111 (1987); Neuhause et al., Theor. Appl. Genet. 75, 30 (1987); Klein et al., Nature 327, 70 (1987); Howell et al., Science 208, 1265 (1980); Horsch et al., Science 227, 1229 (1985); DeBlock et al., Plant Physiol 91, 694 (1989)).

Bei Pflanzen werden dabei die beschriebenen Methoden zur Transformation und Regeneration von Pflanzen aus Pflanzengeweben oder Pflanzenzellen zur stabilen Transformation genutzt. Geeignete Methoden sind vor allem die Protoplastentransformation durch Polyethylenglykolinduzierte DNA-Aufnahme, das biolistische Verfahren mit der Genkanone, die sogenannte "particle bombardment" Methode, die Elektroporation, die Inkubation trockener Embryonen in DNAhaltiger Lösung und die Mikroinjektion.

Neben diesen "direkten" Transformationstechniken kann eine Transformation auch durch bakterielle Infektion mittels Agrobacterium tumefaciens oder Agrobacterium rhizogenes durchgeführt werden. Die Agrobacterium-vermittelte Transformation ist am besten für dicotyledone Pflanzenzellen geeignet. Die Verfahren sind beispielsweise beschrieben bei Horsch R.B. et al., Science 225, 1229 (1985)).

Werden Agrobacterien verwendet, so ist die rekombinante Expressionskassette in spezielle Plasmide zu integrieren, entweder in einen Zwischenvektor (englisch: shuttle or intermediate vector) oder einen binären Vektor. Wird ein Ti oder Ri Plasmid zur Transformation verwendet werden soll, ist zumindest die rechte Begrenzung, meistens jedoch die rechte und die linke Begrenzung der Ti oder Ri Plasmid T-DNA als flankierende Region mit der einzuführenden rekombinanten Expressionskassette verbunden.

Bevorzugt werden binäre Vektoren verwendet. Binäre Vektoren können sowohl in E.coli als auch in Agrobacterium replizieren. Sie enthalten in der Regel ein Selektionsmarkergen und einen Linker oder Polylinker flankiert von der rechten und linken T-DNA Begrenzungssequenz. Sie können direkt in Agrobacterium transformiert werden (Holsters et al., Mol. Gen. Genet. 163, 181 (1978)). Das Selektionsmarkergen erlaubt eine Selektion transformierter Agrobakteria und ist zum Beispiel das nptll Gen, das eine Resistenz gegen Kanamycin verleiht. Das in diesem Fall als Wirtsorganismus fungierende Agrobacterium sollte bereits ein Plasmid mit der vir-Region enthalten. Diese ist für die Übertragung der T-DNA auf die pflanzliche Zelle erforderlich. Ein so transformiertes Agrobacterium kann zur Transformation pflanzlicher Zellen verwendet werden. Die Verwendung von T-DNA zur Transformation pflanzlicher Zellen ist intensiv untersucht und beschrieben (EP 120 516; Hoekemain: The Binary Plant Vector System, Offsetdrukkerij Kanters B.V., Alblasserdam, Chapter V; An et al., EMBO J. 4, 277 (1985)). Verschiedene binäre Vektoren sind bekannt und teilweise kommerziell erhältlich wie zum Beispiel pBI101.2 oder pBIN19 (Bevan et al., Nucl. Acids Res. 12, 8711(1984) ; Clontech Laboratories, Inc. USA). Weitere zur Expression in Pflanzen geeignet Promotoren sind beschrieben (Rogers et al., Methods Enzymol. 153, 253 (1987); Schardl et al., Gene 61, 1 (1987); Berger et al., Proc. Natl. A-cad. Sci. USA 86, 8402 (1989)).

Direkte Transformationstechniken eignen sich im Prinzip für jeden Organismus und Zelltyp. Im Falle von Injektion oder Elektroporation von DNA bzw. RNA in pflanzliche Zellen sind keine besonderen Anforderungen an das verwendete Plasmid gestellt. Einfache Plasmide wie die der pUC-Reihe können verwendet werden. Sollen vollständige Pflanzen aus den transformierten Zellen regeneriert werden, so ist er erforderlich, dass sich auf dem Plasmid ein zusätzliches selektionierbares Markergen befindet.

Stabil transformierte Zellen d.h. solche, die die eingeführte DNA integriert in die DNA der Wirtszelle enthalten, können von untransformierten selektioniert werden, beispielsweise wenn ein selektionierbarer Marker Bestandteil der eingeführten DNA ist. Als Marker kann beispielhaft jedes Gen fungieren, dass eine Resistenz gegen Antibiotika oder Herbizide (wie Kanamycin, G 418, Bleomycin, Hygromycin oder Phosphinotricin etc.) zu verleihen vermag (s.o.). Transformierte Zellen, die ein solches Markergen exprimieren, sind in der Lage, in der Gegenwart von Konzentrationen eines entsprechenden Antibiotikums oder Herbizides zu überleben, die einen untransformierten Wildtyp abtöten. Beispiel für geeignete Selektionsmarker sind oben genannt. Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen aus. Aus diesen noch undifferenzierten Zellmassen kann die Bildung von Sproß und Wurzel in bekannter Weise induziert werden. Die erhaltenen Sprößlinge können ausgepflanzt und gezüchtet werden. Dem Fachmann sind Verfahren bekannt, um aus Pflanzenzellen, Pflanzenteile und ganze Pflanzen zu regenerieren. Beispielsweise werden hierzu Verfahren beschrieben von Fennell et al., Plant Cell Rep. 11, 567 (1992); Stoeger et al., Plant Cell Rep. 14, 273 (1995); Jahne et al., Theor. Appl. Genet. 89, 525 (1994) verwendet. Die erhaltenen Pflanzen können in der üblichen Weise gezüchtet und/oder gekreuzt werden. Zwei oder mehr Generationen sollten kultiviert werden, um sicherzustellen, dass die genomische Integration stabil und vererblich ist.

Das beschriebene Verfahren kann vorteilhaft mit weiteren Verfahren die eine Pathogenresistenz (beispielsweise gegen Insekten, Pilze, Bakterien, Nematoden etc.), Streßresistenz oder eine andere Verbesserung der pflanzlichen Eigenschaften bewirken kombiniert werden. Beispiele sind u.a. genannt bei Dunwell J.M., J. Exp. Bot. 51 (Spec No), 487 (2000).

"Rekombinant" meint bezüglich zum Beispiel einer Nukleinsäuresequenz, einer Expressionskassette oder einem Vektor enthaltend besagte Nukleinsäuresequenz oder einem Organismus transformiert mit besagter Nukleinsäuresequenz, Expressionskassette oder Vektor alle solche durch gentechnische Methoden zustandegekommene Konstruktionen, in denen sich entweder
(a) die B11 Nukleinsäuresequenz, oder
(b) eine mit der BI1 Nukleinsäuresequenz funktionell verknüpfte genetische Kontrollsequenz, zum Beispiel ein Promotor, oder
(c) (a) und (b)
sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des BI1-Promotors mit dem entsprechenden BI1-Gen - wird zu einer rekombinanten Expressionskassette, wenn diese durch nicht-natürliche, synthetische ("künstliche") Verfahren wie beispielsweise einer Mutagenisierung geändert wird. Entsprechende Verfahren sind beschrieben (US 5,565,350; WO 00/15815; siehe auch oben).

Ein anderer Gegenstand betrifft rekombinante Organismen, transformiert mit wenigstens einer Nukleinsäuresequenzen, Expressionskassette oder einem Vektor, sowie Zellen, Zellkulturen, Gewebe, Teile - wie zum Beispiel bei pflanzlichen Organismen Blätter, Wurzeln usw.- oder Vermehrungsgut abgeleitet von solchen Organismen. Organismus ist breit zu verstehen und meint prokaryotische und eukaryotische Organismen, bevorzugt Bakterien, Hefen, Pilze, tierische und pflanzliche Organismen. Als rekombinante Organismen bevorzugte Wirts- oder Ausgangsorganismen sind vor allem Pflanzen gemäß der oben genannten Definition.

Ein weiterer Gegenstand betrifft die Verwendung der erfindungsgemäßen, rekombinanten Organismen und der von ihnen abgeleitete Zellen, Zellkulturen, Teile - wie zum Beispiel bei rekombinanten pflanzlichen Organismen Wurzeln, Blätter etc.-, und rekombinantes Vermehrungsgut wie Saaten oder Früchte, zur Herstellung von Nahrungs- oder Futtermitteln, Pharmazeutika oder Feinchemikalien.

Weiterhin wird ein Nukleinsäuremolekül, das antisense zu der Nukleinsäure ist, ein monoclonaler, spezifisch an das Polypeptide bindender Antikörper und ein Fungizid, das die Nukleinsäure, den Vektor, insbesondere einen infektiösen, z.B. viralen Vektor, das erfindungsgemäße Polypeptide in einer zur Auftragung auf Pflanzen geeigneten Form enthält, z.B. verkapselt oder in einem infektiösen, vorzugsweise zur Übertragung von Nukleinsäuren oder Expression von Genen in einer Zelle geeigneten Organismus, wie ein Agrobacterium oder ein Virus.

Weiterhin beschrieben ist die Verwendung eines BI-1 codierenden Nukleinsäuremoleküls oder eines BI-1 Proteins zur Herstellung einer Pathogen-resistenten Pflanze, vorzugsweise zur Herstellung einer gegen Pilze resistenten Pflanze oder zur Herstellung eines dies bewirkenden Fungizids oder zur Bekämpfung oder Behandlung von mit Pathogenen befallenen oder durch Pathogene bedrohten Pflanzen.

### Sequenzen

- 1. SEQ ID NO: 1 :: Nukleinsäuresequenz kodierend für ein BI1 Protein aus Gerste (Hordeum vulgare).
- 2. SEQ ID NO: 2 :: Aminosäuresequenz kodierend für ein BI1 Protein aus Gerste (Hordeum vulgare).
- 3. SEQ ID NO: 3 :: Nukleinsäuresequenz kodierend für ein BI1 Protein aus Arabidopsis thaliana.
- 4. SEQ ID NO: 4 :: Aminosäuresequenz kodierend für ein BI1 Protein aus Arabidopsis thaliana.
- 5. SEQ ID NO: 5 :: Nukleinsäuresequenz kodierend für ein BI1 Protein aus Tabak.
- 6. SEQ ID NO: 6 :: Aminosäuresequenz kodierend für ein BI1 Protein aus Tabak.
- 7. SEQ ID NO: 7 :: Nukleinsäuresequenz kodierend für ein BI1 Protein aus Reis.
- 8. SEQ ID NO: 8:: Aminosäuresequenz kodierend für ein BI1 Protein aus Reis.
- 9. SEQ ID NO: 9 :: Nukleinsäuresequenz kodierend für ein BI1 Protein aus Raps.
- 10. SEQ ID NO: 10 :: Aminosäuresequenz kodierend für ein BI1 Protein aus Raps.
- 11. SEQ ID NO: 11 :: Nukleinsäuresequenz kodierend für Teileines BI1 Protein aus Soja.
- 12. SEQ ID NO: 12:: Aminosäuresequenz kodierend für Teileines BI1 Protein aus Soja.
- 13. SEQ ID NO: 13 :: Nukleinsäuresequenz kodierend für Teileines BI1 Protein aus Soja.
- 14. SEQ ID NO: 14:: Aminosäuresequenz kodierend für Teileines BI1 Protein aus Soja.
- 15. SEQ ID NO: 15 :: Nukleinsäuresequenz kodierend für Teil eines BI1 Protein aus Weizen.
- 16. SEQ ID NO: 16 :: Aminosäuresequenz kodierend für Teileines BI1 Protein aus Weizen.
- 17. SEQ ID NO: 17 :: Nukleinsäuresequenz kodierend für Teileines BI1 Protein aus Mais.
- 18. SEQ ID NO: 18 :: Aminosäuresequenz kodierend für Teileines BI1 Protein aus Mais.
- 19. SEQ ID NO: 19 :: Nukleinsäuresequenz kodierend für Teileines BI1 Protein aus Weizen.
- 20. SEQ ID NO: 20 :: Aminosäuresequenz kodierend für Teileines BI1 Protein aus Weizen.
- 21. SEQ ID NO: 21 :: Nukleinsäuresequenz kodierend für Teil eines BI1 Protein aus Mais.
- 22. SEQ ID NO: 22 :: Aminosäuresequenz kodierend für Teil eines BI1 Protein aus Mais.
- 23. SEQ ID NO: 23 :: Nukleinsäuresequenz kodierend für Teil eines BI1 Protein aus Mais.
- 24. SEQ ID NO: 24 :: Aminosäuresequenz kodierend für Teil eines BI1 Protein aus Mais.
- 25. SEQ ID NO: 25 :: Nukleinsäuresequenz kodierend für Teil eines BI1 Protein aus Weizen.
- 26. SEQ ID NO: 26 :: Aminosäuresequenz kodierend für Teil eines BI1 Protein aus Weizen.
- 27. SEQ ID NO: 27 :: Nukleinsäuresequenz kodierend für Teil eines BI1 Protein aus Mais.
- 28. SEQ ID NO: 28 :: Aminosäuresequenz kodierend für Teil eines BI1 Protein aus Mais.
- 29. SEQ ID NO: 29 :: Nukleinsäuresequenz kodierend für den Patatin Promotor aus Kartoffel.
- 30. SEQ ID NO: 30 :: Nukleinsäuresequenz kodierend für den Germin 9f-3.8 Promotor aus Weizen.
- 31. SEQ ID NO: 31 :: Nukleinsäuresequenz kodierend für den Arabidopsis CAB-2 Promotor.
- 32. SEQ ID NO: 32 :: Nukleinsäuresequenz kodierend für den PPCZm1 Promotor aus Mais.
- 33. SEQ ID NO: 33 :: Nukleinsäuresequenz kodierend für rekombinanten Expressionsvektor pUbiBI-1.
- 34. SEQ ID NO: 34 :: Nukleinsäuresequenz kodierend für rekombinanten Expressionsvektor pLo114UbiBI-1.
- 35. SEQ ID NO: 35 :: Nukleinsäuresequenz kodierend für rekombinanten Expressionsvektor pOXoBI-1.
- 36. SEQ ID NO: 36 :: Nukleinsäuresequenz kodierend für rekombinanten Expressionsvektor pLo114OXoBI-1.
- 37. SEQ ID NO: 37:: Nukleinsäuresequenz kodierend für BI-1 Protein aus Weizen.
- 38. SEQ ID NO: 38:: Aminosäuresequenz kodierend für BI-1 Protein aus Weizen.
- 39. SEQ ID NO: 39:: Nukleinsäuresequenz für PEN1 (= ROR2) aus Gerste.
- 40. SEQ ID NO: 40:: Aminosäuresequenz kodierend für PEN1 (= ROR2) aus Gerste.
- 41. SEQ ID NO: 41:: Nukleinsäuresequenz für PEN1 (= ROR2) aus Arabidopsis thaliana.
- 42. SEQ ID NO: 42:: Aminosäuresequenz kodierend für PEN1 (= ROR2) aus Arabidopsis thaliana.
- 43. SEQ ID NO: 43:: Nukleinsäuresequenz kodierend für SNAP34 aus Gerste.
- 44. SEQ ID NO: 44:: Aminosäuresequenz kodierend für SNAP34 aus Gerste.
- 45. SEQ ID NO: 45:: Nukleinsäuresequenz kodierend für BI-1 aus Soja.
- 46. SEQ ID NO: 46:: Aminosäuresequenz kodierend für BI-1 aus Soja.
- 47. SEQ ID NO: 47:: GFP- Primer 1 (siehe unten).
- 48. SEQ ID NO: 48:: GFP- Primer 2 (siehe unten).

### Abbildungen:

1. Fig. 1 a-d: Vergleich von Proteinsequenzen verschiedener BI-1 Proteine aus Pflanzen. AtBI-1: Arabidopsis; BnBI-1: Brassica napus (Raps); GmBI2: Glycine max (Soja; Variante 1); GmBI3: Glycine max (Soja; Variante 2); HVBI-1: Hordeum vulgare (Gerste); NtBI-1: Nicotiana tabacum (Tabak); OsBI-1: Oryza sativa (Reis); TaBI11: Triticum aestivum (Weizen, Variante 1); TaBI18: Triticum aestivum (Weizen, Variante 2); TaBI5 neu: Triticum aestivum (Weizen, Variante 3); ZmBI14: Zea mays (Mais; Variante 1); ZmBI16: Zea mays (Mais; Variante 2); ZmBI33: Zea mays (Mais; Variante 3); ZmBI8: Zea mays (Mais; Variante 4); Consensus: Aus dem Alignment abgeleitete Konsensussequenz.

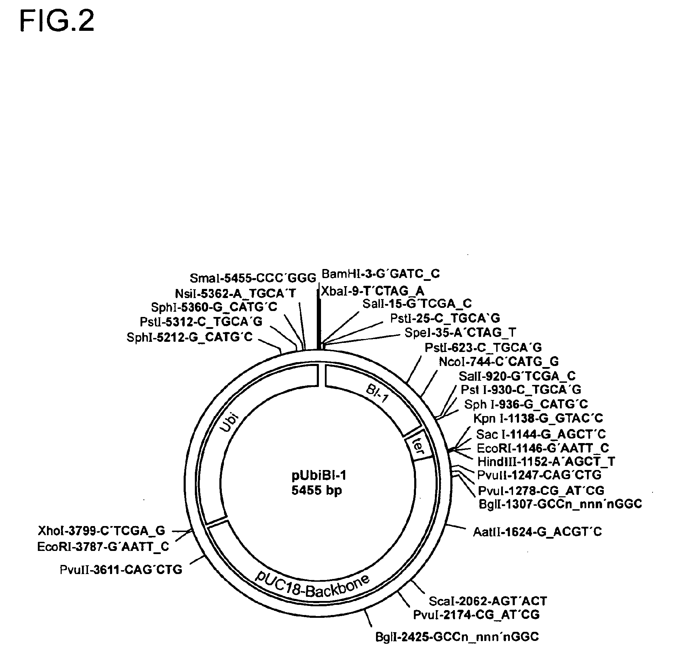

2. Fig. 2: Vektorkarte für Vektor pUbiBI-1 (Ubi: Ubiquitin-Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.

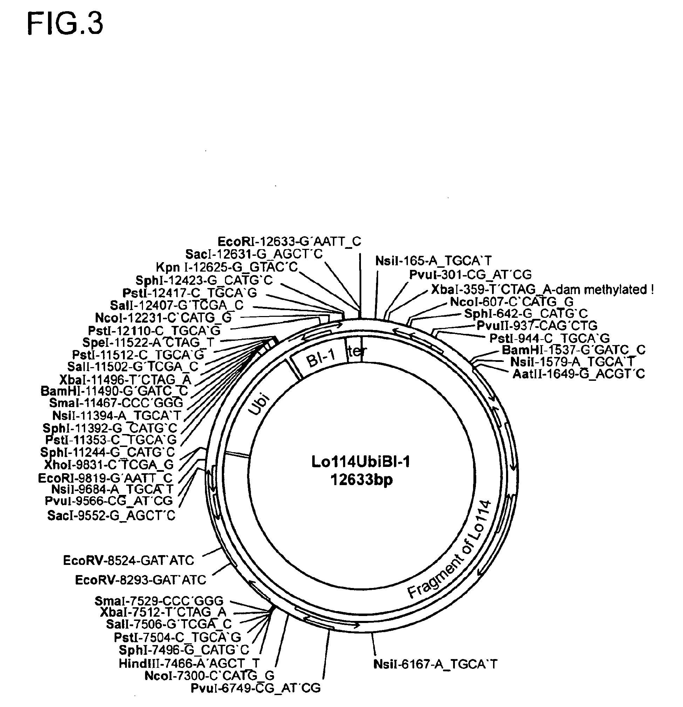

3. Fig. 3: Vektorkarte für Vektor pLO114UbiBI-1 (Ubi: Ubiquitin-Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.

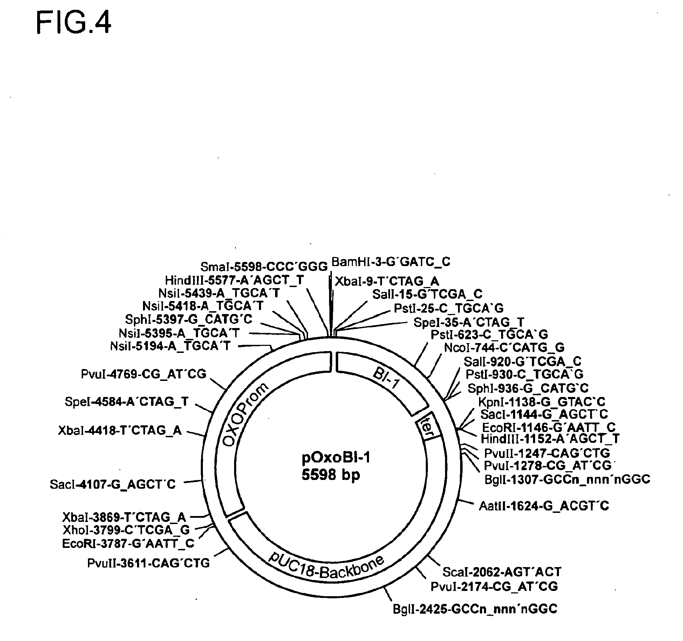

4. Fig. 4: Vektorkarte für Vektor pOxoBI-1 (Oxo: TaGermin 9f-2.8 Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.

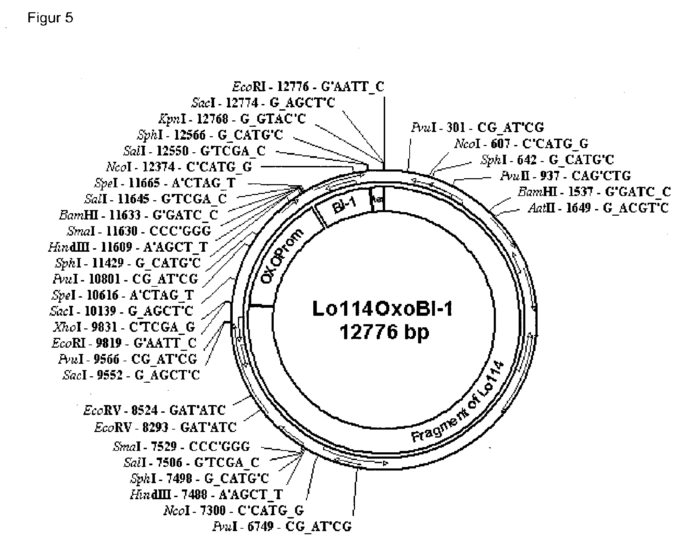

5. Fig. 5: Vektorkarte für Vektor pLO114OxoBI-1 (Oxo: TaGermin 9f-2.8 Promotor; BI-1 Nukleinsäuresequenz kodierend für Gerste BI1-Protein; ter: Transkriptionsterminator). Angegeben sind ferner die Lokalisation der Schnittstellen verschiedener Restriktionsenzyme.
6. Fig. 6: Vergleich der Proteinsequenzen von BI-1 Proteinen aus Gerste (Hordeum vulgare, GenBank Acc.-No.: CAC37797), Reis (Oryza sativa, GenBank Acc.-No.: Q9MBD8), Arabidopsis thaliana (GenBank Acc.-No.: Q9LD45) und Mensch (Homo sapiens, GenBank Acc.-No.: AAB87479). Schwarzhinterlegte Aminosäuren sind identisch in allen Arten. Grau hinterlegte Aminosäuren sind nur in Pflanzen identisch. Balken zeigen die vorausgesagten sieben Transmembrandomänen in HvBI-1 an.

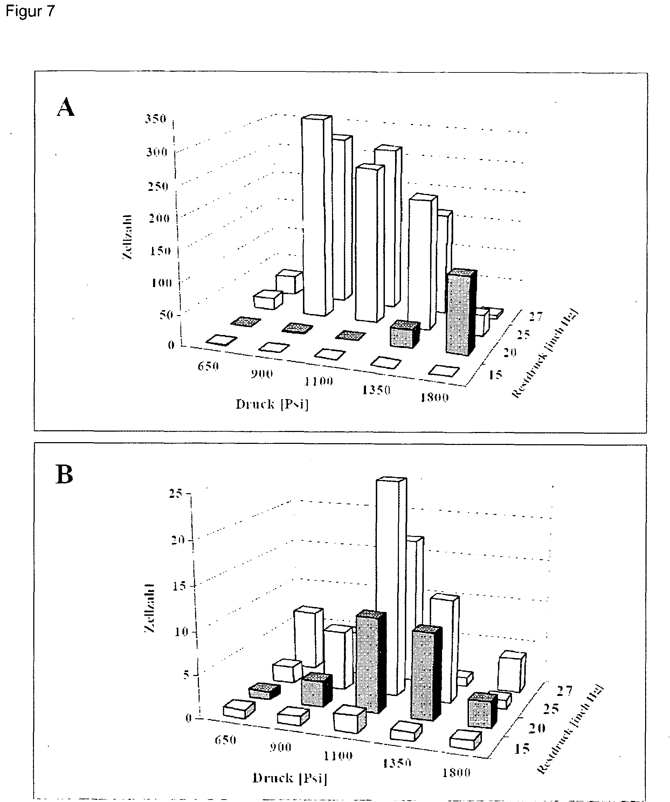

7. Fig. 7: Graphische Darstellung der Transformationsrate in Abhängigkeit von den Bedingungen bei der ballistischen Transformation von Soja- (A) und Gersteblättem (B). Für die Transformation wurden jeweils 1.6 µg DNA pro Schuss und für Gersteblätter Partikel mit 0.6 µm sowie für Soja mit 1 µm Durchmesser verwendet. Die Zahl der transformierten Zellen wurde 24 h nach der Transformation bestimmt.
8. Fig. 8: Graphische Darstellung der Penetrationsrate von P. pachyrhizi in transient mit einem BI-1 Überexpressionskonstrukt transformierten Gerstenzellen im Vergleich zur Kontrolle. Drei voneinander unabhängige Experimente liegen den Werten zugrunde. Als Kontrolle wurden Gersteblätter mit dem Reportergenkonstrukt pGY1-GFP und dem leeren Vektor transformiert. Die Penetrationsrate ist in den mit BI-1 transformierten Zellen signifikant verschieden vom WT (P<0.05).

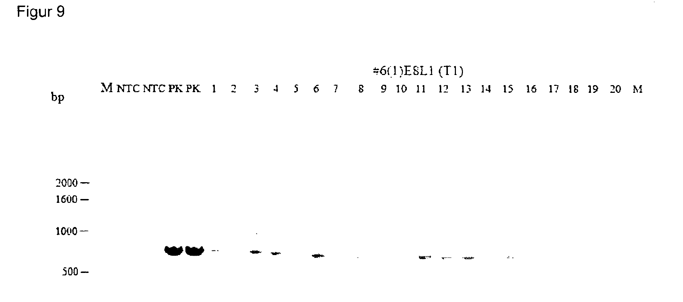

9. Fig. 9: PCR zum Nachweis des GFP-BI-1 Konstruktes in den transgenen Gerstelinien #6(1)E4L3P5 (T2), 1#6(2)E15L7P1 (T2), #6(2)E15L7P2 (T2) und #6(1)E8L1(T1) (Sorte, ,Golden Promise'). Die PCR wurde mit genomischer DNA der Pflanzen mit den GFP-spezifischen Primern durchgeführt. In positiven transgenen Pflanzen wird das vollständige GFP mit 740 bp amplifiziert. Als Negativkontrolle diente genomische DNA aus dem WT ,Golden Promise' (WT) oder Wasser (NTC). Als Positiv-Kontrolle (PK) wurde das GFP vom Plasmid pGY1-GFP amplifiziert.
10. Fig. 10: Ermittelte Daten zur Penetrationsrate von P. pachyrhizi in transient mit einem BI-1 Überexpressionskonstrukt transformierten Gerstenzellen. Gezeigt sind die Fig. 8 zugrundeliegenden Daten (siehe auch unten Beispiele).
11. Fig. 11 A/B: Ausgezählte Sporen und Zellreaktionen bei der Interaktion zwischen Sojarost und transgenen Gerstelinien mit dem GFP-BI-1 Überexpressionskonstrukt #6(1)E4L3P5(T2), 1#6(2)E15L7P1(T2), #6(2)E15L7P2(T2) und #6(1)E8L1(T1) der Sorte ,Golden Promise'. Als Kontrolle diente der WT. Blätter von 7 d alten Pflanzen wurden mit Sojarost inokuliert nach 24 h fixiert und für das Auszählen mit Anillinblau gefärbt.
12. Fig. 12: Relativer Anteil der Papillenbildung und der HR nach der Inokulation mit P. pachyrhizi in Gerstenblättem von den transgenen Linien (,Sorte ,Golden Promise') #6(1)E4L3P5 (T2), 1#6(2)E15L7P1 (T2), #6(2)E15L7P2 (T2) und #6(1)E8L1(T1) im Vergleich zum WT. Die Primärblätter wurden sieben Tage nach der Aussaat der Gerstesamen abgenommen, mit P. pachyrhizi inokuliert und 24 h nach der Inokulation ausgewertet. Dargestellt sind die Mittelwerte des WT und der einzelnen Linien. Die Fehlerbalken stellen die Standardabweichung dar. Die relative Papillenbildung und die HR sind in den transgenen Linien nach einem studentschen Test gegenüber dem WT signifikant verschieden P < 0.001.

### Beispiele

### Allgemeine Methoden:

Die chemische Synthese von Oligonukleotiden kann beispielsweise, in bekannter Weise, nach der Phosphoamiditmethode (Voet, Voet, 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. Cold Spring Harbor Laboratory Press (1989), ISBN 0-87969-309-6; beschrieben durchgeführt. Die Sequenzierung rekombinanter DNA-Moleküle erfolgt mit einem Laserfluoreszenz-DNA-Sequenzierer der Firma MWG-Licor nach der Methode von Sanger (Sanger et al.,Proc. Natl. Acad. Sci. USA 74, 5463 (1977)).

### Beispiel 1 : Inokulation mit P. pachyrhizi

Geeignetes Sporenmaterial (Uredosporen) des Erregers Phakopsora pachyrhizi stammt von BASF Aktiengesellschaft. Dafür wurden Sporen von 2-3 Wochen zuvor inokulierten Sojapflanzen direkt verwendet oder auf Alufolie abgeklopft und auf einem Trockenmittel (TROPAGel, Tropack, Lahnau) im Dunkeln bei Raumtemperatur gelagert.

Für die Herstellung einer Sporensuspension wurden die Sporen von den Blättern in Tween-H₂O (0,1 %) abgewaschen. Das Auszählen der Sporen erfolgte mittels einer Thoma-Zählkammer unter dem Lichtmikroskop. Für die Inokulation wurden die Blätter auf 1 % H₂O-Agar mit einem Metallring fixiert. Zur Inokulation wurden schließlich verschiedene Methoden verwendet: Für die Sprühinokulation wurde die Sporensuspension in eine mit Druckluft betriebene Sprühflasche gegeben und gleichmäßig auf den Pflanzen verteilt bis die Blattoberfläche gut befeuchtet war. Um eine höhere Sporendichte zu erreichen, wurden außerdem Blätter, nachdem sie zuvor mit Tween-H₂O eingesprüht worden waren, in einem 'Fallturm' trocken inokuliert. Hierzu wird der Turm über die Platten mit den angefeuchteten Blättern gestellt. Die jungen Sporen von infizierten Sojapflanzen werden durch eine Öffnungsklappe in den Turm geblasen. Durch den Luftzug werden die Sporen verwirbelt, fein verteilt und fallen auf die Blätter.

Um höhere Inokulationsdichten ohne Aggregation der Sporen zu erreichen, wurden die Blätter alternativ mit einer Sporensuspension überschichtet und nach 15 min aus der Suspension entnommen (Überschichtungsmethode). Nach dieser Zeit war bereits eine ausreichende Anheftung der präzipitierten Sporen gegeben. Die Inkubation der inokulierten Blätter erfolgte in einer Kammer mit durchschnittlich 25 °C und 71,2 % Luftfeuchtigkeit.

Um den Infektionsverlauf mikroskopisch zu beurteilen, wurden 0 hours post inokulation (hpi), 6 hpi, 24 hpi und 48 hpi Blattproben genommen und mit verschiedenen Methoden eingefärbt.

### a) Coomassie-Färbung

Zur Coomassie-Färbung wurde das infizierte Blattmaterial geerntet und über Nacht bei Raumtemperatur entfärbt. Zur Mikroskopie wurde das Blattmaterial mit Coomassie-Lösung bedeckt und nach 5 min die Lösung mit etwas Wasser abgespült. Direkt danach wurde die Probe mikroskopiert.

### b) Calcofluor-Färbung

Zur Herstellung der Calcofluor-Färbelösung wurden 0,03% Calcofluor White (Sigma-Aldrich) in 50mM Tris/HCI pH 8,0 und 0,01 % Tween 20 gelöst. Zur Anfärbung der extrazellulären Pilzstrukturen wurde das infizierte Blatt 30 sec in die Färbelösung getaucht und anschließend 10 sec mit Wasser gewaschen. Die angefärbten pilzlichen Strukturen fluoreszieren unter UV-Anregung hellblau.

### c) Anillinblau-Färbung

Das Blattmaterial wurde in Falcons oder in Schalen mit Entfärbelösung überführt und über Nacht bei RT inkubiert. Danach wurde die Entfärbelösung entfernt und die Blätter 2 x mit Wasser gewaschen. Für die Färbung wurden die Blätter 1.5 - 2 h mit Anillinblau-Färbelösung bedeckt und anschließend direkt mikroskopiert.

### d) Weizenkeim Agglutinin-Alexa Fluor 488-Färbung (WGA-Färbung)

Für die WGA-Färbung von P. pachyrhizi wurden inokulierte Gerstenblätter 30 - 45 min bei RT in 10 % (w/v) KOH eingelegt. Inokulierte Sojablätter wurden in 1 cm2 Stückchen geschnitten und 5 min in 10 % (w/v) KOH gekocht. Anschließend wurden die Gerste- und die Sojablätter 5 x 3 - 5 min mit 1 x PBS-Puffer gewaschen. Zur Färbung wurde das Blattmaterial in WGA-Färbelösung eingelegt und 10 min bei 100 mbar Restdruck infiltriert. Daraufhin wurde das Material direkt mikroskopiert oder in der Färbelösung bei 4 °C im Dunkeln aufbewahrt.

### Beispiel 2 Transiente ballistische Transformation von Pflanzenzellen

Für die transiente Überexpression des Bax-Inhibitors (für Konstrukte und sonstige Methoden siehe Patent WO2004/081217) durch ballistische Transformation wurden 30 - 100 mg Goldpartikel abgewogen in 1 ml 70 % EtOH resuspendiert und 3-5 min geschüttelt. Nach einer Inkubation von 1 h bei Raumtemperatur erfolgte eine Pelletierung durch Zentrifugation (1 min 10,000 rpm, Mikrozentrifuge Eppendorf). Danach wurden die Partikel 3 x mit sterilem Wasser gewaschen und anschließend in sterilen 50 % (v/v) Glycerin aufgenommen und bei 4 °C gelagert. Die Lösung enthielt bei Einwaage von 30 mg Goldpartikeln ca. 25 mg/ml Gold. Vor der Verwendung wurden die Partikel noch einmal zur gründlichen Verteilung geschüttelt und für 15 sec im Ultraschallbad sonifiziert.

Im Allgemeinen wurde eine Goldpartikelmenge für mindestens drei Schuss vorbereitet. Bis zur Fällung wurde nach jedem Schritt der Ansatz mit dem Vortexer für einige Sekunden kräftig geschüttelt. Für die DNA-Fällung auf Goldpartikel für 3 Schuss wurden aus der gut aufgeschüttelten Goldpartikel-Glycerinlösung 12.5 µl entnommen und mit der gewünschten DNA (pGY1-BI-1, pGY1-GFP, pGY1, siehe WO2004/081217) versetzt. Dabei wurden pro Schuss und Plasmid 1.6 µg/µl DNA eingesetzt. Anschließend erfolgte die Zugabe von 12.5 µl 2.5 M CaCl₂-Lösung und 5 µl 0.1 M Spermidine-Lösung. Das Gemisch wurde 3 min geschüttelt, mit 70 µl 70% (v/v) Ethanol versetzt und vorsichtig invertiert. Nach Zugabe von 70 µl 100% Ethanol wurde der Ansatz nochmals durch invertieren gründlich gemischt und bis zu 1 h bei -20 °C inkubiert. Die Partikel wurden durch Zentrifugation pelletiert (1 min, 11000 rpm, Mikrozentrifuge Eppendorf, Wesseling-Berzdort) in 18 µl 100 % Ethanol resuspendiert und möglichst gleichmäßig in einem Radius von ca. 1 cm auf den Macrocarrieren (Bio-Rad, München) verteilt, die zuvor mit 100 % Ethanol gewaschen und getrocknet worden waren. Die Makrocarrier wurden dann bei Raumtemperatur inkubiert bis der Ethanol vollständig verdampft war.

Die transiente ballistische Transformation von Gerste und Sojablättern erfolgte mit dem Biolistic Particle Delivery System PDS-1000/He System (Bio-Rad, München). Für die Transformation wurden bevorzugt Gersteblätter von 7 Tage alten Gerstepflanzen (Sorte,Hanna') oder die ersten Laubblätter (Zweiblattstadium) von Sojapflanzen (Sorte ,Oxford') verwendet. Die zu transformierenden Blätter wurden auf 1 % (w/v) Wasser-Agar gelegt und mit einem Metallring fixiert.

Um das optimale Verhältnis zwischen angelegtem Druck und Restdruck in der Vakuumkammer für die Transformation zu bestimmen, wurden verschiedene Druck/Restdruck-Kombinationen getestet. Dabei wurden Restdrücke von 15-27 inch Hg und ,Rupture Disks' von 650 - 1800 Psi Sollbruchstärke verwendet. Nach dem Beschuss wurde die Kammer wieder belüftet, die Platte mit den Blättern entnommen und diese vor der Mikroskopie oder Färbung mindesten 24 h bei Raumtemperatur inkubiert.

Für die ballistische Transformation der Blätter wurden Goldpartikel verwendet. Dabei stellte sich heraus, dass für Gerste der Beschuss mit Partikeln von 0,6 µm Durchmesser am besten geeignet war, da sie weniger Verletzungen des Gewebes hervorriefen. Bei der transienten Transformation von Soja wurde mit den Partikeln von 1 µm die höchste Transformationsrate erreicht.

Für die erfolgreiche transiente Transformation müssen die Verhältnisse zwischen dem Druck zur Beschleunigung der Partikel, dem Restdruck in der Vakuumkammer und dem Abstand der Probe zu den Partikeln aufeinander abgestimmt werden. Um dies zu erreichen wurden für Gerste- und Sojablätter verschiedene Bedingungen getestet. Bei Soja bewährte sich ein Druck von 900 Psi mit 25 inch Hg Restdruck in der Vakuumkammer und einem Abstand der Probe von 9 cm (FIG. 7A). Bei Gersteblättern wurden die höchsten Zellzahlen mit einem Druck von 1100 psi, 25 inch Hg Restdruck in der Vakuumkammer und einem Abstand der Blätter zu den Partikeln von 9 cm erreicht (FIG. 7B).

Zur Untersuchung der Effekte von BI-1 auf die Interaktion zwischen P. pachyrhizi und Gerste wurde die transiente ballistische Transformation angewandt. Die Primärblätter von sieben Tage alten Gerstepflanzen (Sorte ,Hanna') wurden mit Überexpressionskonstrukten von BI-1 (pGY1-BI-1, Hückelhoven R.et al., 2001) beschossen. Diese Plasmide tragen einen CAMV 35S Promoter, der eine konstitutive Expression der Gene gewährleistet. Als Reportergen wurde ein CAMV 35S-GFP Konstrukt zusammen mit den Expressionsplasmiden in die Zellen geschossen (Schweizer P. et al., MPMI 12, 647 (1999)). Für die Kontrolle wurde der leere Vektor pGY-1 zusammen mit dem GFP-Reportergenkonstrukt verwendet. Die Inokulation mit P. pachyrhizi erfolgte 24 h nach der Transformation durch Präzipitation aus einer Sporensuspension (Überschichtungsmethode, 2-3 x 104 Sporen/ml). Nach 18 h Inkubation wurden nach Calcofluor-Färbung der extrazellulären Pilzstrukturen die Interaktionen mikroskopisch ausgewertet. Gezählt wurden GFP-bildende Zellen, die von dem Pilz penetriert wurden und GFP-bildende Zellen die von dem Pilz attackiert wurden, diesen aber entweder erfolgreich abwehren konnten (oder wo der Pilz vor einer Penetration abgestorben ist). Zusätzlich wurden die GFP-bildenden Zellen insgesamt gezählt. Für die Auswertung wurden die relativen Penetrationsraten in Bezug auf die gesamten transformierten mit Sojarost interagierenden Zellen berechnet.

In der Kontrolle wurden über die Versuche gemittelt 53 % der transformierten mit P. pachyrhizi interagierenden Zellen penetriert, während von den mit BI-1 transformierten Zellen nur 37 % penetriert wurden (siehe Tabelle FIG. 10 und FIG. 8). Nach der Auswertung zeigen die transient mit BI-1 transformierten Zellen eine signifikant erhöhte Penetrationsresistenz gegen P. pachyrhizi. Auffällig war bei der mikroskopischen Beobachtung, dass die BI-1 bildenden Zellen deutlich vitaler wirkten als Zellen die nur GFP exprimierten.

### Beispiel 3 Stabile Transformation von Gerste und Nachweis des GFP:BI-1-Transgens

Zur genaueren Untersuchung des Einflusses von BI-1 auf die Interaktion von Gerste mit Phakopsora pachyrhizi wurden stabil transformierte Gerstenpflanzen hergestellt, die ein GFP:BI-1 Fusionsprotein überexprimieren. Die dazu verwendete Agrobakterium-vermittelte stabile Transformation ist eine seit Jahren etablierte Technik, deren Methoden bereits in vielen Review-Artikeln veröffentlicht sind (Cheng Z. et al., Plant Mol. Biol. 60, 583 (2004);Taylor et al., DNA & Cell Biology. 21(12), 963 (2002); Rakoczy-Trojanowska, Cellular & Molecular Biology Letters. 7(3), 849 (2002); Grabowska A., Acta Physiologiae Plantarum. 26(4), 451 (2004); Chesnokov V., Sel'skokhozyaistvennaya Biologiya. 1, 26 (2004). Auch die Transformation von monokotylen Spezies wie z.B. Gerste, die in den 1990er Jahren noch schwierig war, ist inzwischen zu einer Standardtechnik geworden (Travella S. et al., Plant Cell Reports 23(12), 780 (2005); Murray F. et al., Plant Cell Reports 22(6), 397 (2004)).

Da es bei den erhaltenen transgenen Pflanzen nicht bekannt war, ob es sich um homozygote Linien handelte, musste zunächst das Vorhandensein des Überexpressionskonstrukts nachgewiesen werden. Dazu wurde von allen Pflanzen aus einem Blatt die genomische DNA isoliert. Verwendet wurde das DNeasy 96 Plant Kit (Quiagen, Hilden; nach Angaben des Herstellers).

Der Nachweis des Überexpressionskonstrukts GFP-BI-1 erfolgte mit GFP-spezifischen Primern mittels PCR auf das Vorhandensein des Konstruktes überprüft. Bei der In vitro-Amplifikation von DNA durch PCR (Mullis & Faloona, 1987) wurde die Phusion Hot Start (Finnzymes, Espoo) verwendet.
- Primer 1:: 5'-ATGGTGAGCAAGGGCGAGGA-3' (SEQ ID NO: 47)
- Primer 2:: 5'-TTGAACAACGATGTGCAAGACTCCTTGTACAGCTCGTCCATGC-3') (SEQ ID NO: 48)

### PCR- Ansatz zum Nachweis des GFP:BI-1 Transgens:

| | |
|---|---|
| DMSO | 0,6 µl |
| Puffer (5x) (F-519 GC) | 4 µl |
| DNA | 3 µl |
| dNTP-Mix (10mM each) | 0.4 µl |
| Primer 1 (20 pmol) | 1 µl |
| Primer 2 (20 pmol) | 1 µl |
| Hot Start Phusion F 540L | 0,2 µl |
| Wasser | 9.8 µl |

Die PCR wurde in einem Thermocycler mit beheizbarem Deckel (Tgradient; Biometra, Göttingen) durchgeführt.

### PCR- Programm:

| | | | | |
|---|---|---|---|---|
| 1 | Initiale Denaturation | 98°C | 30sec | |
| 2. | Denaturation | 98°C | 10ecs | 35 Zyklen der Schritte 2-4 |
| 3. | Annealing | 58°C | 30sec | |
| 4. | Elongation | 72°C | 30sec | |
| 5. | Elongation | 72°C | 5 min | |
| 6. | Storage | 4°C | | |

Unter Verwendung von Standardmethoden wurde die DNA durch Gelelektrophorese in einem 1 % Agarosegel ([w/v], INVITROGEN, Karlsruhe; in 1 x TAE Puffer) aufgetrennt und analysiert (Sambrook et al., Molecular Cloning: A Laboratory Manual, Cold Spring Harbor Laboratory, Cold Spring Harbor (NY) 1989).

Bei insgesamt sechs Pflanzen von den transgenen Linien #6(2)E15L7P2 (T2) und #6(1)E8L1(T1) konnte das GFP-Fragment von 740 bp und damit das Konstrukt nicht nachgewiesen werden. Die Werte dieser Pflanzen [Pflanze 5, 10 und 19 der Linie #6(1)E8L1(T1) sowie Pflanze 3, 11 und 12 der Linie #6(2)E15L7P2 (T2)] wurden daher in den folgenden Berechnungen nicht berücksichtigt. (siehe FIG. 9).

### Beispiel 4 : Interaktion von P. pachyrhizi mit transgener Bax Inhibitor-1 überexprimierender Gerste

Um den resistenzerhöhenden Effekt durch transiente Überexpression von BI-1 zu bestätigen, wurde die Interaktion zwischen P. pachyrhizi und transgenen Gerstenpflanzen der Sorte ,Golden Promise' (GP), die ein GFP-BI-1 Überexpressionskonstrukt enthalten im Vergleich zum Wildtyp (WT) der Sorte mikroskopisch untersucht. Für die Herstellung der transgenen Pflanzen wurde eine GFP-BI-1 Fusion unter der Kontrolle des konstitutiven CAMV 35S Promoters verwendet und so eine ausreichende Expression des Proteins gewährleistet.

Es wurden jeweils 20 Pflanzen des Wildtyps und von vier transgenen Linien #6(1)E4L3P5 (T2), 1#6(2)E15L7P1 (T2), #6(2)E15L7P2 (T2) und #6(1)E8L1(T1) angezogen. Von der Linie #6(2)E15L7P2 (T2) sind nur 14 Pflanzen aus den Samen aufgelaufen. Da nur begrenzt Saatgut zur Verfügung stand, wurden die Versuche mit einer geringeren Anzahl von Pflanzen dieser Linie durchgeführt. Sieben Tage nach der Aussaat wurde das Primärblatt entnommen, mit Hilfe des Fallturms mit P. pachyrhizi inokuliert und 24 h nach der Inokulation in Entfärbelösung fixiert. Nach der vollständigen Entfärbung wurden die Blätter mit Anillinblau gefärbt. Anilinblau lagert sich in die Struktur von Kallose ein und färbt so bevorzugt Papillen an, bei denen es zur Akkumulation und Vernetzung von Kallose mit anderen polymeren Substanzen kommt. Zellen, die durch eine hypersensitive Reaktion (HR), einen der Apoptose in Säugerzellen ähnlichen Prozess durchlaufen haben, zeigen nach Anillinblaufärbung ebenfalls eine helle Fluoreszenz. Auf den inokulierten Gersteblättern wurde die Anzahl der Sporen, die gekeimten Sporen mit Keimschläuchen die bereits ein Appressorium gebildet hatten und, als Zellreaktion, die Appressorien mit darunter liegenden Papillen sowie die HR gezählt. Größere Sporenzusammenballungen, bei denen eine Zuordnung der Appressorien zu den Sporen nicht mehr möglich war, wurden nicht mitgezählt. Es wurden, soweit möglich, pro Blatt mindestens 100 Sporen mit Appressorien ausgezählt (siehe Tabelle in FIG. 11)

Bei der mikroskopischen Analyse der Blätter war eine Papillenbildung zu erkennen, welche in den transgenen Linien häufig deutlich verstärkt war. Dagegen war in den transgenen Pflanzen das Auftreten der HR bei den infizierten Zellen seltener zu beobachten. Im Wildtyp wurden durchschnittlich in 36 % der befallenen Zellen Papillen als Abwehrreaktion gebildet, 45 % der Zellen reagierten mit einer HR. Dagegen betrug der Anteil der Papillenbildung in den transgenen Linien zwischen 50-60 %. Deutlich niedriger war im Vergleich zum Wildtyp auch der Anteil der HR mit 16-26 % (FIG. 12). Nach der Überprüfung mit dem sogenannten "Student Test" (t-Test) ist die relative Papillenbildung in den transgenen Linien gegenüber dem WT signifikant erhöht und die HR signifikant erniedrigt (P< 0.001). Der BI-1verhindert den Beobachtungen in diesen Versuchen gemäß den programmierten Zelltod und fördert als alternative Abwehr die Papillenbildung der Zellen.

### Beispiel 5 : Interaktion von P. pachyrhizi mit transgener Bax Inhibitor-1 überexprimierender Soja

Nach an sich bekannten Methoden wurden Sojapflanzen, welche NtBI-1 überexpremieren hergestellt und wie voranstehend beschrieben mit P. pachyrhizi inokuliert. Die mit NtBI-1 transformierten Sojapflanzen zeigen im Vergleich zu den Wildtyp-Sojapflanzen eine deutliche Reduktion an Befall mit Sojarost - die Reduktion liegt im Durchschnitt im Bereich von über 30%.

Ebenso wurden Sojapflanzen, welche mit NTBI-1 und mit SELDA transformiert wurden, bereitgestellt und wie voranstehend beschrieben mit P. pachyrhizi inokuliert. Die mit NtBI-1 + SELDA transformierten Sojapflanzen zeigen im Vergleich zu den Wildtyp-Sojapflanzen ebenfall eine signifikante Reduktion an Befall mit Sojarost - die Reduktion liegt hier im Durchschnitt im Bereich von über 15 %.

## Patentansprüche

1. Verfahren zur Erzeugung oder Erhöhung einer Resistenz gegen mindestens einen biotrophen Pilz aus dem Genus Phakopsora in einer Pflanze oder einem Teil einer Pflanze, wobei der Teil einer Pflanze ein Gewebe oder eine Zelle umfasst, umfassend die Schritte:
(a) Erhöhung der Proteinmenge oder -funktion mindestens eines Bax Inhibitor-1 (BI1) Proteins in der Pflanze oder mindestens einem Teil einer Pflanze, gegenüber der Proteinmenge oder -funktion in der Ausgangspflanze oder in deren Teil, wobei
(i) pflanzliche Zellen stabil mit einer rekombinanten Expressionskassette transformiert werden, welche eine für ein BI1-Protein kodierende Nukleinsäuresequenz in funktioneller Verknüpfung mit einem Promotor enthält, wobei der Promotor in Bezug auf die das BI1-Protein kodierende Nukleinsäuresequenz heterolog ist;
(ii) die Pflanze aus der pflanzlichen Zellen regeneriert wird; und
(iii) die für das BI1-Protein kodierenden Nukleinsäuresequenz in einer Menge, in mindestens einem Gewebe und für eine Zeit, hinreichend um eine Pilzresistenz gegen mindestens einen biotrophen Pilz aus dem Genus Phakopsora in der Pflanze zu erzeugen oder zu erhöhen, expremiert wird
und
(b) Auswahl der Pflanze oder eines Teils einer Pflanze, bei der/dem im Vergleich zur Ausgangspflanze bzw. deren Teil eine Resistenz gegen mindestens einen biotrophen Pilz aus dem Genus Phakopsora erzeugt oder erhöht wurde, wobei
das BI1-Protein kodiert wird durch ein Polypeptid, das mindestens eine Sequenz umfaßt ausgewählt aus der Gruppe bestehend aus:
(a) den Sequenzen gemäß SEQ ID NO: 2, 4, 6, 8, 10, 12, 14, 16, 18, 20, 22, 24, 26, 28, oder, 38; und
(b) Sequenzen, die eine Identität von mindestens 70% zu einer der Sequenzen gemäß SEQ ID NO: 2, 4, 6, 8, 10, 12, 14, 16, 18, 20, 22, 24, 26, 28, oder 38 aufweisen,
wobei die Pflanze Soja ist

2. Verfahren nach Anspruch 1, wobei das BI1 Protein mindestens eine Sequenz umfaßt, die eine Identität von mindestens 80% aufweist zu mindestens einem BI1-Konsensusmotiv ausgewählt aus der Gruppe bestehend aus
(a) H(L/I)KXVY,
(b) AXGA(Y/F)XH,
(c) NIGG,
(d) P(V/P)(Y/F)E(E/Q)(R/Q)KR,
(e) (E/Q)G(A/S)S(V/I)GPL,
(f) DP(S/G)(L/I)(I/L),
(g) V(G/A)T(A/S)(L/I)AF(A/G)CF(S/T),
(h) YL(Y/F)LGG,
(i) L(L/V)SS(G/W)L(S/T)(I/M)L(L/M)W, und
(j) DTGX(I/V)(I/V)E.

3. Verfahren nach einem der vorherigen Ansprüche, wobei der heterologe Promotor ein gewebespezifischer Promotor ist.

4. Verfahren nach einem der vorstehenden Ansprüche, wobei die Expression des BI1 Proteins in einer Pflanze zumindest in der Epidermis, bevorzugt im wesentlichen gewebespezifisch in der Epidermis erhöht wird und/oder im Mesophyll im wesentlichen nicht erhöht wird.

5. Verfahren nach einem der vorstehenden Ansprüche, wobei die Pflanze zudem einen mloresistenten Phänotyp aufweist, oder die Expression oder Funktion von MLO, RacB und/oder NaOx mindestens in der Epidermis inhibiert oder im Vergleich zu einer Kontrollpflanze reduziert ist und/oder die Expression oder Funktion von PEN2, SNAP34 und/oder PEN1 mindestens in der Epidermis erhöht ist, im Vergleich zu einer Kontrollpflanze.

## Claims

1. A method of generating or increasing a resistance to at least one biotrophic fungus from the genus Phakopsora in a plant or a part of a plant, where the part of a plant comprises a tissue or a cell, comprising the following steps:
a) increasing the protein quantity or function of at least one Bax inhibitor-1 (BI1) protein in the plant or at least a part of a plant over the protein quantity or function in the starting plant or part thereof, where
(i) plant cells are stably transformed with a recombinant expression cassette comprising a nucleic acid sequence, which codes for a BI1 protein, in operable linkage with a promoter, the promoter being heterologous with regard to the nucleic acid sequence which codes for the BI1 protein;
(ii) the plant is regenerated from the plant cell; and
(iii) the nucleic acid sequence which codes for the BI1 protein is expressed in an amount, in at least one tissue and over a period sufficient for generating or increasing a fungal resistance to at least one biotrophic fungus from the genus Phakopsora in the plant and,
b) selecting the plant or a part of a plant where a resistance to at least one biotrophic fungus from the genus Phakopsora has been generated or increased in comparison with the starting plant or part thereof where
the BI1 protein is encoded by a polypeptide which comprises at least one sequence selected from the group consisting of:
a) the sequences as shown in SEQ ID NO: 2, 4, 6, 8, 10, 12, 14, 16, 18, 20, 22, 24, 26, 28 or 38; and
b) sequences with at least 70% identity with one of the sequences as shown in SEQ ID NO: 2, 4, 6, 8, 10, 12, 14, 16, 18, 20, 22, 24, 26, 28 or 38,
where the plant is soya

2. The method according to claim 1, where the BI1 protein comprises at least one sequence which has at least 80% identity with at least one BI1 consensus motif selected from the group consisting of
a) H(L/I)KXVY,
b) AXGA(Y/F)XH,
c) NIGG,
d) P(V/P)(Y/F)E(E/Q)(R/Q)KR,
e) (E/Q)G(A/S)S(V/I)GPL,
f) DP(S/G)(L/I)(I/L),
g) V(G/A)T(A/S)(L/I)AF(A/G)CF(S/T),
h) YL(Y/F)LGG,
i) L(L/V)SS(G/W)L(S/T)(I/M)L(L/M)W, and
j) DTGX(I/V)(I/V)E.

3. The method according to either of the preceding claims, where the heterologous promoter is a tissue-specific promoter.

4. The method according to any of the preceding claims, wherein the expression of the BI1 protein in a plant is increased at least in the epidermis, preferably essentially tissue-specifically in the epidermis, and/or essentially not increased in the mesophyll.

5. The method according to any of the preceding claims, where the plant additionally has an mlo-resistent phenotype, or the expression or function of MLO, RacB and/or NaOx is inhibited or reduced, in comparison with a control plant, at least in the epidermis and/or the expression or function of PEN2, SNAP34 and/or PEN1 is increased at least in the epidermis in comparison with a control plant.

## Revendications

1. Procédé de production ou d'augmentation d'une résistance à au moins un champignon biotrophen du genre Phakopsora dans une plante ou une partie d'une plante, la partie d'une plante comprenant un tissu ou une cellule, comprenant les étapes :
(a) augmentation de la quantité ou de la fonction d'au moins une protéine inhibiteur-1 de Bax (BI1) dans la plante ou au moins une partie de la plante, par comparison avec la quantité ou la fonction de la protéine dans la plante de départ ou dans sa partie,
(i) les cellules végétales subissant une Transformation stable avec une cassette d'expression recombinante, qui contient une séquence d'acide nucléique codant pour une protéine BI1 en liaison fonctionnelle avec un promoteur, le promoteur étant hétérologue par rapport à la séquence d'acide nucléique codant pour la protéine BI1 ;
(ii) la plante subissant une régénération à partir des cellules végétales ; et
(iii) la séquence d'acide nucléotidique codant pour la protéine BI1 étant exprimée en une quantité dans au moins un tissu et pendant un temps suffisant pour produire ou augmenter une résistance fongique à au moins un champignon biotrophe du genre Phakopsora dans la plante,
et
(b) sélection de la plante ou une partie de la plante dans laquelle, par comparaison avec la plante de départ ou sa partie, une résistance à au moins un champignon biotrophe du genre Phakopsora a été produite ou augmentée,
la protéine BI1 étant codée par un Polypeptide qui comprend au moins une séquence choisie dans le groupe consistant en :
(a) les séquences selon SEQ ID N° 2, 4, 6, 8, 10, 12, 14, 16, 18, 20, 22, 24, 26, 28 ou 38 ; et
(b) les séquences qui présentent une identité d'au moins 70 % avec l'une des séquences selon SEQ ID N° 2, 4, 6, 8, 10, 12, 14, 16, 18, 20, 22, 24, 26, 28 ou 38,
la plante étant le soja.

2. Procédé selon la revendication 1, dans lequel la protéine BI1 comprend au moins une séquence qui présente une identité d'au moins 80 % avec au moins un motif consensus de BI1 choisi dans le groupe consistant en
(a) H(L/I)KXVY,
(b) AXGA(Y/F)XH,
(c) NIGG,
(d) P(V/P)(Y/F)E(E/Q)(R/Q)KR,
(e) (E/Q)G(A/S)S(V/I)GPL,
(f) DP(S/G)(L/I)(I/L),
(g) V(G/A)T(A/S)(L/I)AF(A/G)CF(S/T),
(h) YL(Y/F)LGG,
(i) L(L/V)SS(G/W)L(S/T)(I/M)L(L/M)W,
et
(j) DTGX(I/V)(I/V)E.

3. Procédé selon l'une des revendications précédentes, dans lequel le promoteur hétérologue est un promoteur spécifique de plante.

4. Procédé selon l'une des revendications précédentes, dans lequel l'expression de la protéine BI1 dans une plante est augmentée au moins dans l'épiderme, de préférence pour l'essentiel d'une manière spécifique de tissu dans l'épiderme, et/ou pour l'essentiel n'est pas augmentée dans le mésophylle.

5. Procédé selon l'une des revendications précédentes, dans lequel la plante comprend en outre un phénotype de résistance MLO, ou l'expression ou la fonction du MLO, du RacB et/ou du NaOx est inhibée au moins dans l'épiderme ou est réduite par comparison avec une plante témoin, et/ou l'expression ou la fonction du PEN2, du SNAP34 et/ou du PEN1 est augmentée au moins dans l'épiderme, par comparison arec une plante témoin.
